(19) Europäisches Patentamt
European Patent Office
Office européen des brevets

(11) EP 3 831 819 B1

(12) **EUROPEAN PATENT SPECIFICATION**

(45) Date of publication and mention of the grant of the patent:
**14.01.2026 Bulletin 2026/03**

(21) Application number: **20210262.0**

(22) Date of filing: **27.11.2020**

(51) International Patent Classification (IPC):
***C07D 307/91*** *(2006.01)* ***H10K 50/11*** *(2023.01)*

(52) Cooperative Patent Classification (CPC):
**C07D 307/91; C07D 345/00**

(54) **HETEROCYCLIC COMPOUND AND ORGANIC LIGHT-EMITTING DEVICE INCLUDING THE SAME**

ORGANISCHE HETEROZYKLISCHE LICHTEMITTIERENDE VERBINDUNG UND VORRICHTUNG DAMIT

COMPOSÉ HÉTÉROCYCLIQUE ET ÉLÉMENT ÉLÉCTROLUMINESCENT ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2019 KR 20190158454**

(43) Date of publication of application:
**09.06.2021 Bulletin 2021/23**

(73) Proprietor: **Samsung Electronics Co., Ltd.**
**Suwon-si 16677 (KR)**

(72) Inventors:
- **CHUNG, Yeonsook**
  **16678 Gyeonggi-do (KR)**
- **JUNG, Yongsik**
  **16678 Gyeonggi-do (KR)**
- **KANG, Hosuk**
  **16678 Gyeonggi-do (KR)**
- **KIM, Sangmo**
  **16678 Gyeonggi-do (KR)**
- **KIM, Joonghyuk**
  **16678 Gyeonggi-do (KR)**
- **NAM, Sungho**
  **16678 Gyeonggi-do (KR)**
- **CHWAE, Jun**
  **16678 Gyeonggi-do (KR)**

(74) Representative: **Elkington and Fife LLP**
**Prospect House**
**8 Pembroke Road**
**Sevenoaks, Kent TN13 1XR (GB)**

(56) References cited:
**CN-A- 109 867 646** **KR-A- 20170 096 860**
**KR-A- 20180 131 963**

- **KIM YOUNG SEOK ET AL: "Blue fluorescent materials based on bis(10-phenylanthracen-9-yl) derivatives containing heterocyclic moiety", OPTICAL MATERIALS, ELSEVIER SCIENCE PUBLISHERS B.V. AMSTERDAM, NL, vol. 46, 28 April 2015 (2015-04-28), pages 247 - 253, XP029184966, ISSN: 0925-3467, DOI: 10.1016/J.OPTMAT.2015.04.027**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

One or more embodiments relate to a heterocyclic compound and an organic light-emitting device including the same.

### BACKGROUND OF THE INVENTION

Organic light-emitting devices are self-emission devices, which have improved characteristics in terms of a viewing angle, a response time, brightness, a driving voltage, and a response speed, and produce full-color images.
In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.
CN 109 867 646 relates to the field of organic electroluminescent materials and discloses a heterocyclic compound and use thereof.
KR 2018 0131963 relates to a heterocyclic compound an organic light emitting device having the same.
KR 2017 0096860 relates to an organic compound, to an organic optoelectronic device comprising the organic compound, and to a display device.
Y.S. Kim et al., in "Blue fluorescent materials based on bis(10-phenylanthracen-9-yl) derivatives containing heterocyclic moiety", synthesized a series of bis(10-phenylanthracen-9-yl) derivatives containing various heterocyclic moieties such as 9-ethylcarbazole, dibenzofuran and dibenzothiophene (1-3) by Suzuki cross-coupling reactions, and explored the electroluminescent properties of these materials.

### SUMMARY OF THE INVENTION

One or more embodiments provide a heterocyclic compound and an organic light-emitting device including the same.
Additional aspects will be set forth in part in the description which follows and, in part, will be apparent from the description, or may be learned by practice of the presented embodiments of the disclosure.
An aspect of the present invention provides a heterocyclic compound in accordance with claim 1.
An organic light-emitting device according to an embodiment includes a first electrode, a second electrode, and an organic layer between the first electrode and the second electrode and including at least one of the heterocyclic compounds.

### BRIEF DESCRIPTION OF THE DRAWING

The above and other aspects, features, and advantages of certain embodiments of the disclosure will be more apparent from the following description taken in conjunction with FIGURE which shows a schematic cross-sectional view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.
It will be understood that when an element is referred to as being "on" another element, it can be directly on the other element or intervening elements may be present therebetween In contrast, when an element is referred to as being "directly on" another element, there are no intervening elements present
It will be understood that, although the terms "first," "second," "third" etc. may be used herein to describe various elements, components, regions, layers and/or sections, these elements, components, regions, layers and/or sections should not be limited by these terms These terms are only used to distinguish one element, component, region, layer or

section from another element, component, region, layer or section Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise.

"Or" means "and/or." As used herein, the term "and/or" includes any and all combinations of one or more of the associated listed items It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures It will be understood that relative terms are intended to encompass different orientations of the device in addition to the orientation depicted in the Figures For example, if the device in one of the figures is turned over, elements described as being on the "lower" side of other elements would then be oriented on "upper" sides of the other elements The exemplary term "lower," can therefore, encompasses both an orientation of "lower" and "upper," depending on the particular orientation of the figure Similarly, if the device in one of the figures is turned over, elements described as "below" or "beneath" other elements would then be oriented "above" the other elements The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within $\pm$ 30%, 20%, 10% or 5% of the stated value.

Unless otherwise defined, all terms (including technical and scientific terms) used herein have the same meaning as commonly understood by one of ordinary skill in the art to which this disclosure belongs It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments As such, variations from the shapes of the illustrations as a result, for example, of manufacturing techniques and/or tolerances, are to be expected Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features Moreover, sharp angles that are illustrated may be rounded

A heterocyclic compound according to the invention is represented by one of Formula 1-1 to 1-6:

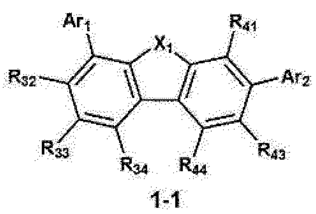

1-1

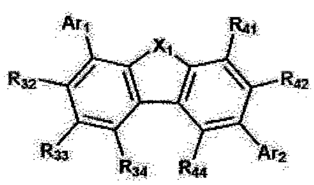

1-2

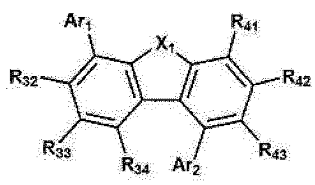

1-3

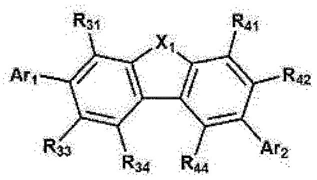

1-4

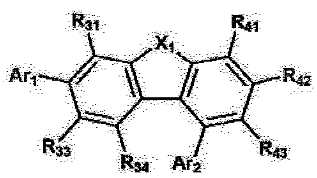

1-5

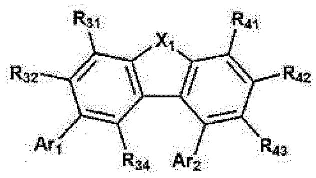

1-6

$X_1$ is O or Se.

In one or more embodiments, $X_1$ may be O. In one or more embodiments, $X_1$ may be Se.

$Ar_1$ is a group represented by Formula 1A, and $Ar_2$ is a group represented by Formula 1B.

In one or more embodiments, $Ar_1$ may be a group represented by one of Formulae 1A-1 to 1A-5:

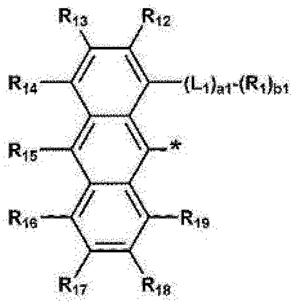
1A-1

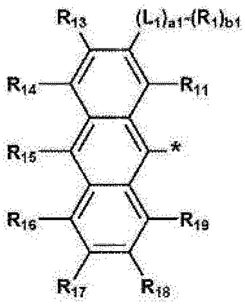
1A-2

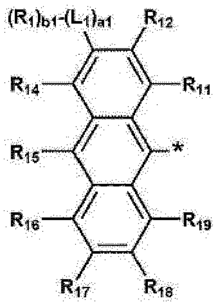
1A-3

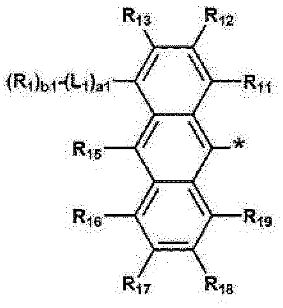
1A-4

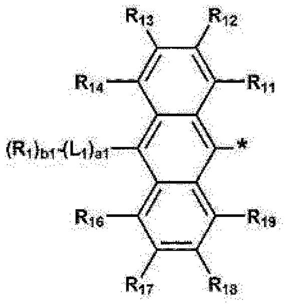
1A-5

In Formulae 1A-1 to 1A-5,

$L_1$, a1, $R_1$, and b1 are the same as described in the present specification,
$R_{11}$ to $R_{19}$ are the same as described in connection with $R_{10}$, and
* indicates a binding site to a neighboring atom.

In one or more embodiments, $Ar_2$ may be a group represented by one of Formulae 2A-1 to 2A-5:

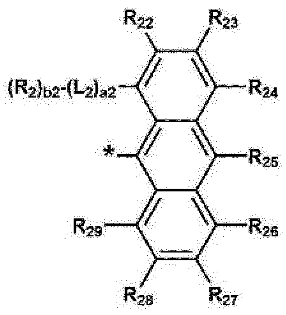
2A-1

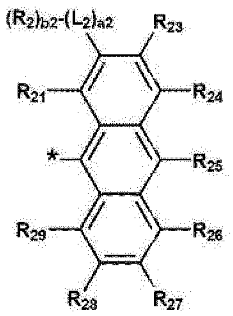
2A-2

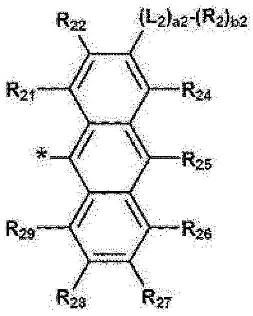
2A-3

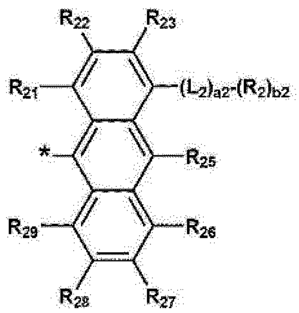
2A-4

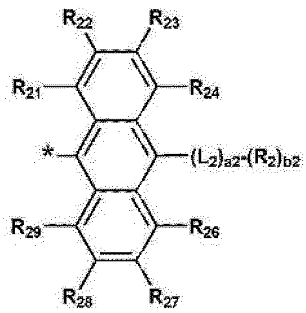
2A-5

In Formulae 2A-1 to 2A-5,

$L_2$, a2, $R_2$, and b2 may be the same as described in the present specification,
$R_{21}$ to $R_{29}$ may be the same as described in connection with $R_{20}$, and
* indicates a binding site to a neighboring atom.

In Formulae 1-1 to 1-6,
$X_1$, $Ar_1$ and $Ar_2$ are the same as described in the present specification,
$L_1$ and $L_2$ in Formulae 1A and 1B are each independently a substituted or unsubstituted $C_5$-$C_{30}$ carbocyclic group, a substituted or unsubstituted $C_1$-$C_{30}$ heterocyclic group, or any combination thereof.

In one or more embodiments, $L_1$ and $L_2$ may each independently be: a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof; or
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof.

a1 and a2 in Formulae 1A and 1B are each independently be an integer from 0 to 3. When a1 is 2 or more, two or more of $L_1$(s) may be identical to or different from each other. When a2 is 2 or more, two or more of $L_2$(s) may be identical to or different from each other.

$R_1$, $R_2$, $R_{10}$, $R_{20}$, $R_{31}$ to $R_{34}$, and $R_{41}$ to $R_{44}$ are each independently hydrogen, deuterium, -F, -Cl, -Br, -I, $-SF_5$, a hydroxyl group, a cyano group, a nitro group, an amidino group, a hydrazine group, a hydrazone group, a carboxylic acid or a salt thereof, a sulfonic acid or a salt thereof, a phosphoric acid or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-B(Q_6)(Q_7)$, $-P(=O)(Q_8)(Q_9)$, or any combination thereof.

In one or more embodiments, $R_1$, $R_2$, $R_{10}$, $R_{20}$, $R_{31}$ to $R_{34}$, and $R_{41}$ to $R_{44}$ may be each independently:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, a $C_1$-$C_{20}$ alkyl group, a $C_1$-$C_{20}$ alkoxy group, or any combination thereof;
a $C_1$-$C_{20}$ alkyl group, a $C_1$-$C_{20}$ alkoxy group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an

indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof;

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof; or

$-N(Q_1)(Q_2)$, $-Si(Q_3)(Q_4)(Q_5)$, $-B(Q_6)(Q_7)$, $-P(=O)(Q_8)(Q_9)$, or any combination thereof,

wherein $Q_1$ to $Q_9$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CH_3$, $-CD_2CD_3$, $-CD_2CD_2H$, $-CD_2CDH_2$, or any combination thereof;

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof; or

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof, each substituted with at least one deuterium, a $C_1$-$C_{10}$ alkyl group, a phenyl group, or any combination thereof.

b1 and b2 in Formulae 1, 1A, and 1B may each independently be an integer from 1 to 5. When b1 is 2 or more, two or more of $R_1$(s) may be identical to or different from each other. When b2 is 2 or more, two or more of $R_2$(s) may be identical to or different from each other.

b10 and b20 are each independently an integer from 1 to 8. When b10 is 2 or more, two or more of $R_{10}$(s) are identical to or different from each other. When b20 is 2 or more, two or more of $R_{20}$(s) are identical to or different from each other.

c1 and c2 are each independently an integer from 1 to 8. When c1 is 2 or more, two or more of $-(L_1)_{a1}-(R_1)_{b1}$ group(s) are identical to or different from each other. When c2 is 2 or more, two or more of $-(L_2)_{a2}-(R_2)_{b2}$ group(s) are identical to or different from each other.

The sum of b10 and c1 is 9 and the sum of b20 and c2 is 9.

In one or more embodiments, b10 may be 8 and c1 may be 1.

In one or more embodiments, b20 may be 8 and c2 may be 1.

In one or more embodiments, $R_1$ and $R_2$ may each independently be deuterium, -F, -Cl, -Br, -I, $-CF_3$, $-SF_5$, a hydroxyl group, a cyano group, a nitro group, $-SF_5$, an amidino group, a hydrazine group, a hydrazone group, a carboxylic acid or a salt thereof, a sulfonic acid or a salt thereof, a phosphoric acid or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-B(Q_6)(Q_7)$, $-P(=O)(Q_8)(Q_9)$, or any combination thereof.

In one or more embodiments, $R_1$ and $R_2$ may each independently be deuterium, -F, $-CF_3$, a cyano group, a nitro group, -SFs, a group represented by Formulae 9-1 to 9-19, a group represented by Formulae 10-1 to 10-208, or any combination thereof:

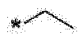
9-1
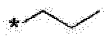
9-2
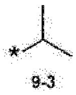
9-3
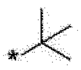
9-4
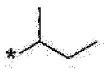
9-5
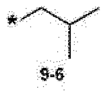
9-6
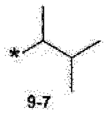
9-7

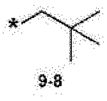
9-8
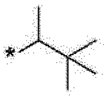
9-9
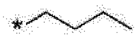
9-10
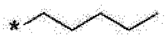
9-11
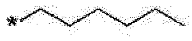
9-12

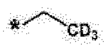
9-13
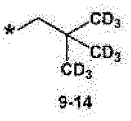
9-14
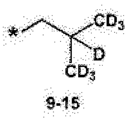
9-15
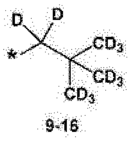
9-16
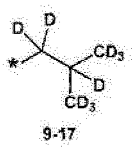
9-17
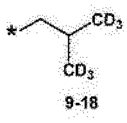
9-18

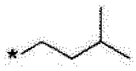
9-19

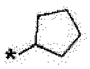
10-1
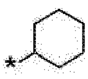
10-2
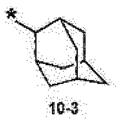
10-3
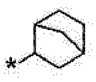
10-4
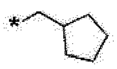
10-5
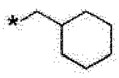
10-6
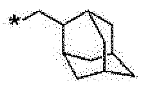
10-7

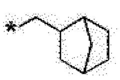
10-8
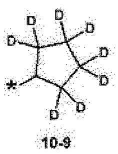
10-9
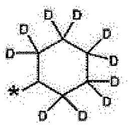
10-10
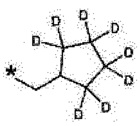
10-11
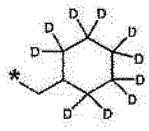
10-12
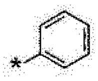
10-13

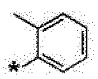
10-14
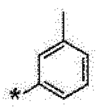
10-15
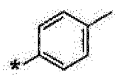
10-16
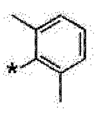
10-17
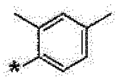
10-18
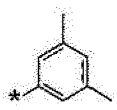
10-19
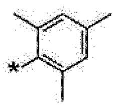
10-20

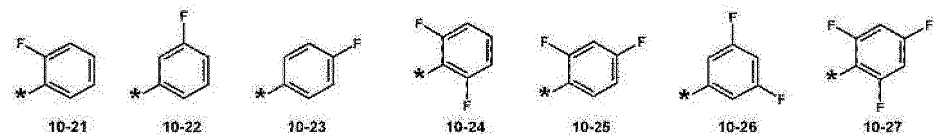

10-21 10-22 10-23 10-24 10-25 10-26 10-27

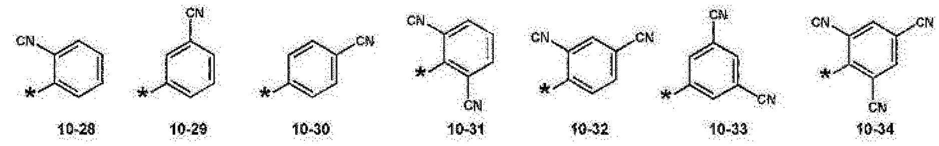

10-28 10-29 10-30 10-31 10-32 10-33 10-34

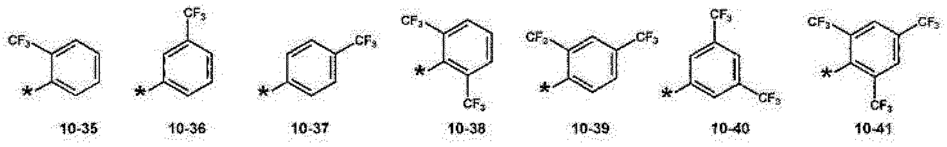

10-35 10-36 10-37 10-38 10-39 10-40 10-41

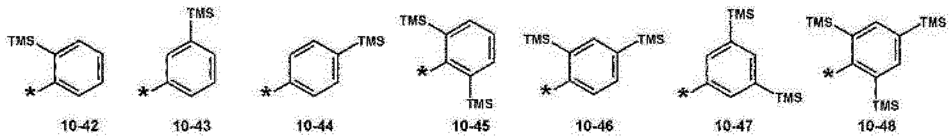

10-42 10-43 10-44 10-45 10-46 10-47 10-48

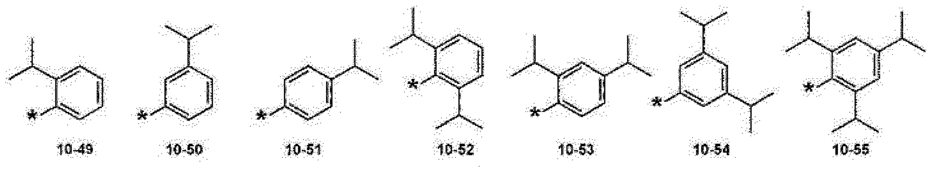

10-49 10-50 10-51 10-52 10-53 10-54 10-55

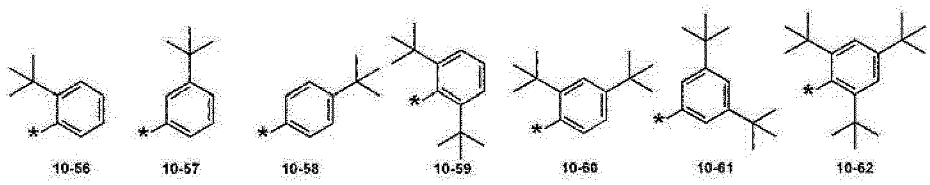

10-56 10-57 10-58 10-59 10-60 10-61 10-62

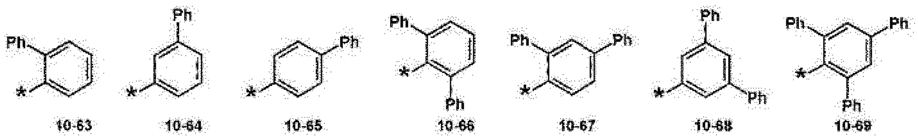

10-63 10-64 10-65 10-66 10-67 10-68 10-69

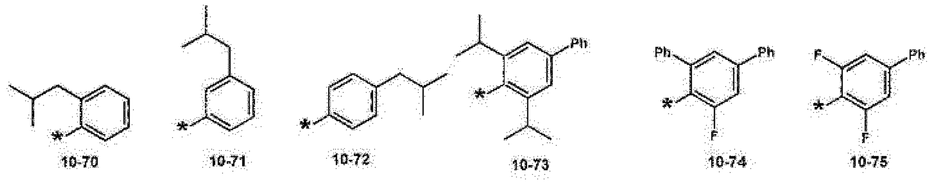

10-70 10-71 10-72 10-73 10-74 10-75

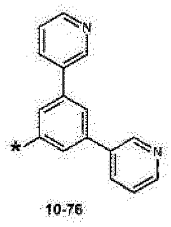
10-76
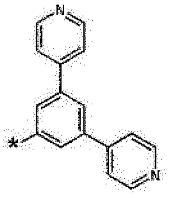
10-77
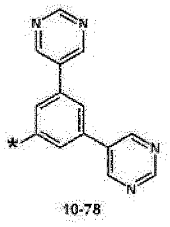
10-78
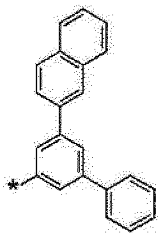
10-79
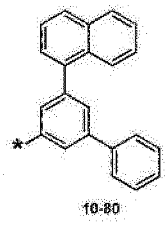
10-80

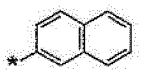
10-81
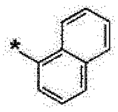
10-82
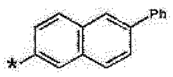
10-83
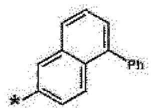
10-84
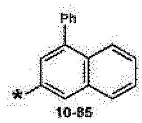
10-85

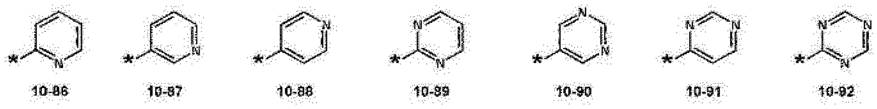
10-86 10-87 10-88 10-89 10-90 10-91 10-92

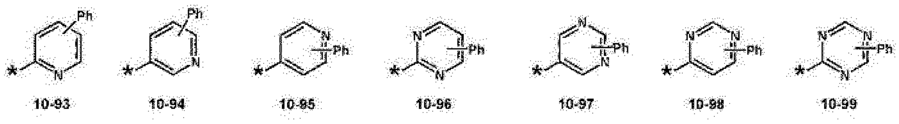
10-93 10-94 10-95 10-96 10-97 10-98 10-99

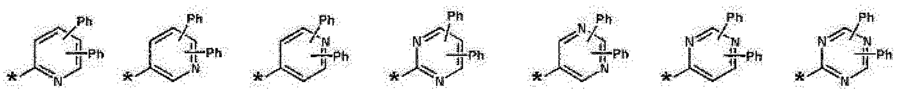
10-100 10-101 10-102 10-103 10-104 10-105 10-106

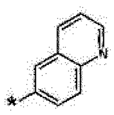
10-107
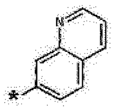
10-108
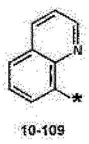
10-109
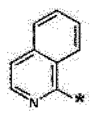
10-110
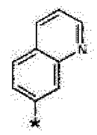
10-111
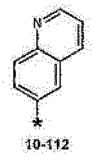
10-112

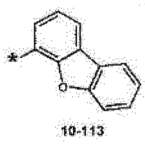
10-113
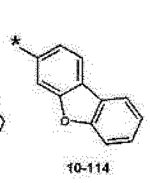
10-114
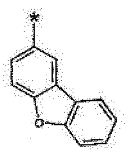
10-115
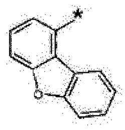
10-116
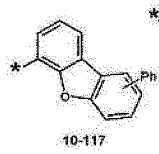
10-117
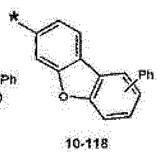
10-118

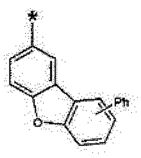
10-119
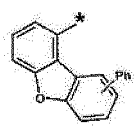
10-120
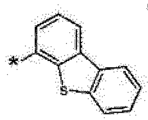
10-121
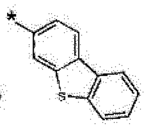
10-122
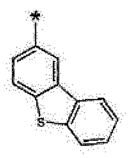
10-123
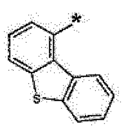
10-124

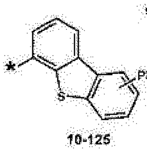
10-125
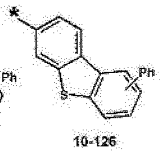
10-126
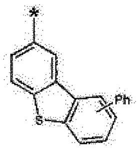
10-127
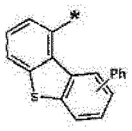
10-128
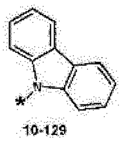
10-129
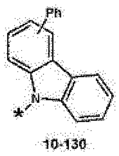
10-130

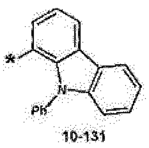
10-131
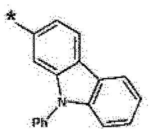
10-132
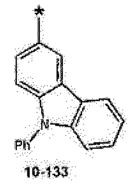
10-133
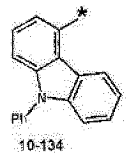
10-134
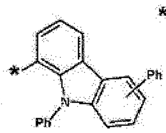
10-135
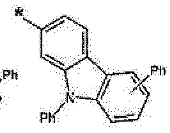
10-136

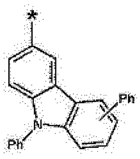
10-137
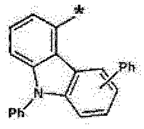
10-138
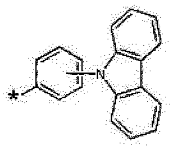
10-139
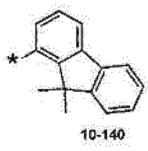
10-140
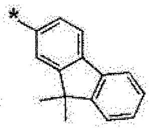
10-141
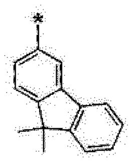
10-142

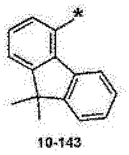
10-143
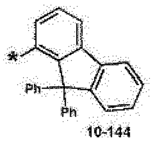
10-144
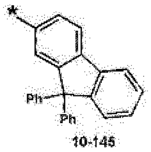
10-145
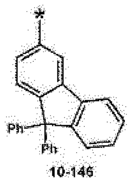
10-146
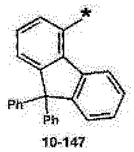
10-147

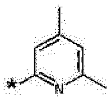
10-148
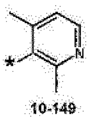
10-149
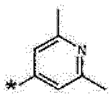
10-150
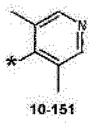
10-151
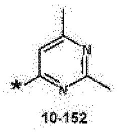
10-152
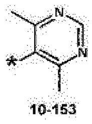
10-153
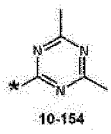
10-154

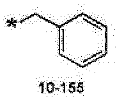
10-155
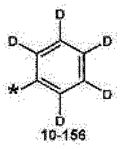
10-156
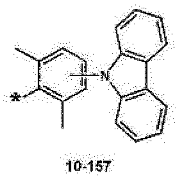
10-157
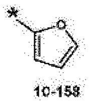
10-158
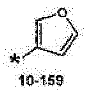
10-159
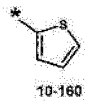
10-160
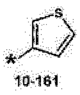
10-161

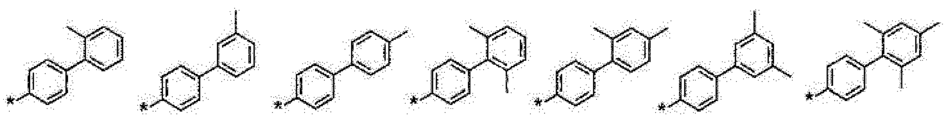
10-162 10-163 10-164 10-165 10-166 10-167 10-168

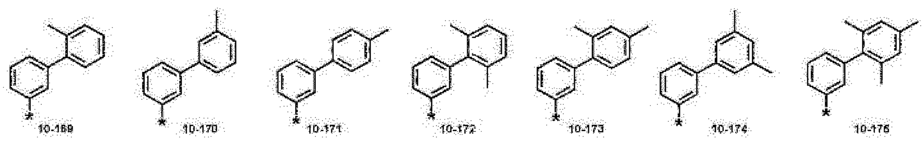
10-169 10-170 10-171 10-172 10-173 10-174 10-175

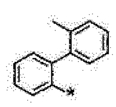 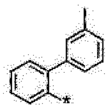 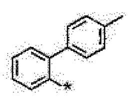 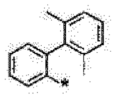 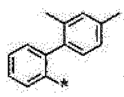 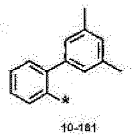 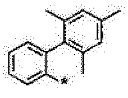

10-176 10-177 10-178 10-179 10-180 10-181 10-182

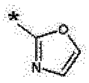 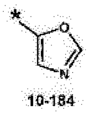 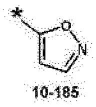 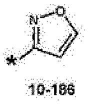 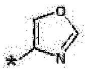 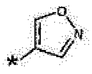 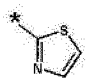 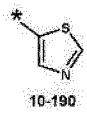 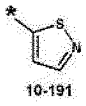

10-183 10-184 10-185 10-186 10-187 10-188 10-189 10-190 10-191

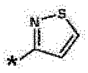 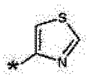 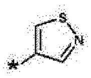

10-192 10-193 10-194

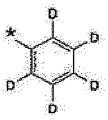 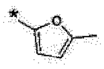 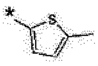 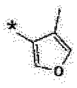 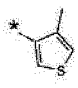 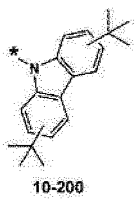 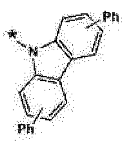 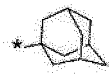

10-195 10-196 10-197 10-198 10-199 10-200 10-201 10-202

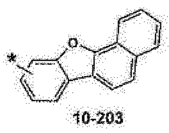 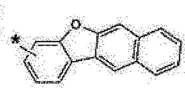 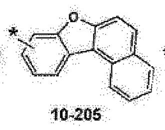 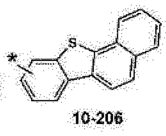 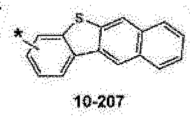 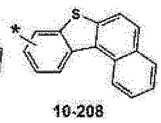

10-203 10-204 10-205 10-206 10-207 10-208 .

In Formulae 9-1 to 9-19 and 10-1 to 10-208, * indicates a binding site to a neighboring atom, Ph may be a phenyl group, and TMS may be a trimethylsilyl group.

In one or more embodiments, $R_1$ and $R_2$ may each independently be: hydrogen, deuterium, -F, -Cl, -Br, -I, $-CF_3$, a hydroxyl group, a cyano group, a nitro group, $-SF_5$, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, or any combination thereof;

a $C_1$-$C_{20}$ alkyl group, a $C_1$-$C_{20}$ alkoxy group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a phenyl group, a naphthyl group, a pyridinyl group, a pyrimidinyl group, a pyrazinyl group, a pyridazinyl group, a triazinyl group, or any combination thereof;

a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyrimidinyl group, an imidazopyridinyl group, or any combination thereof; or

a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group,

a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyrimidinyl group, an imidazopyridinyl group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_2$-$C_{20}$ alkenyl group, a $C_2$-$C_{20}$ alkynyl group, a $C_1$-$C_{20}$ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, or any combination thereof.

In one or more embodiments, $R_{10}$, $R_{20}$, $R_{31}$ to $R_{34}$, and $R_{41}$ to $R_{44}$ may each independently be hydrogen, deuterium, -F, $-CF_3$, a cyano group, a nitro group, $-SF_5$, $-CH_3$, $-CD_3$, $-CD_2H$, $-CDH_2$, $-CF_3$, $-CF_2H$, $-CFH_2$, a group represented by Formulae 9-1 to 9-19, a group represented by Formulae 10-1 to 10-208, or any combination thereof:

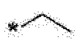
9-1
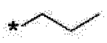
9-2
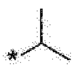
9-3
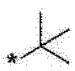
9-4
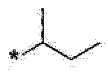
9-5
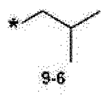
9-6
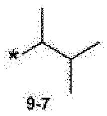
9-7

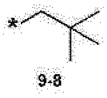
9-8
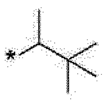
9-9
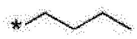
9-10
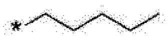
9-11
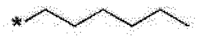
9-12

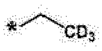
9-13
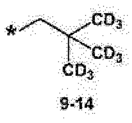
9-14
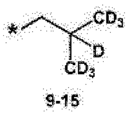
9-15
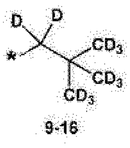
9-16
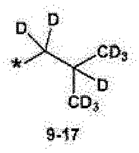
9-17
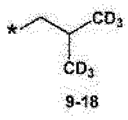
9-18

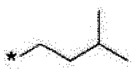
9-19

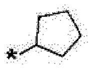
10-1
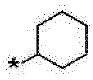
10-2
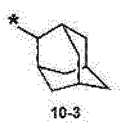
10-3
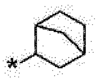
10-4
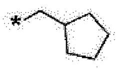
10-5
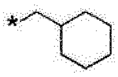
10-6
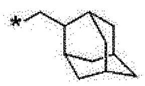
10-7

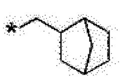
10-8
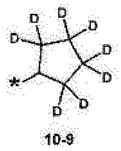
10-9
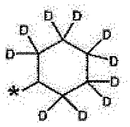
10-10
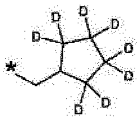
10-11
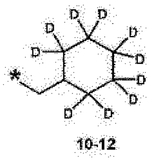
10-12
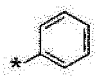
10-13

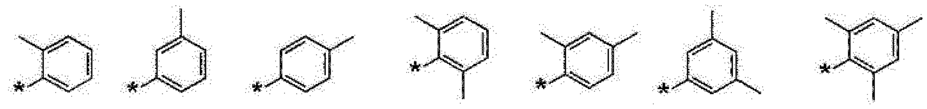

10-14 10-15 10-16 10-17 10-18 10-19 10-20

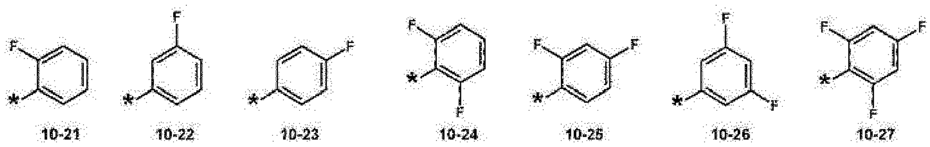

10-21 10-22 10-23 10-24 10-25 10-26 10-27

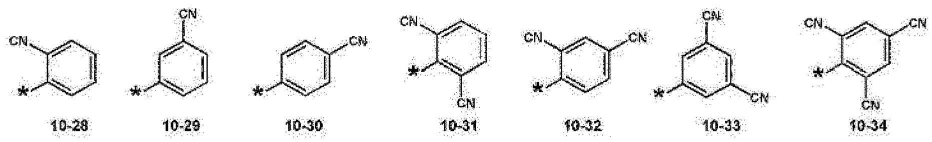

10-28 10-29 10-30 10-31 10-32 10-33 10-34

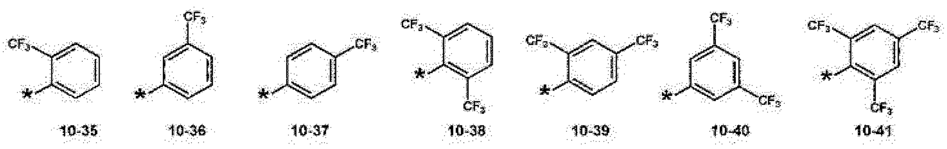

10-35 10-36 10-37 10-38 10-39 10-40 10-41

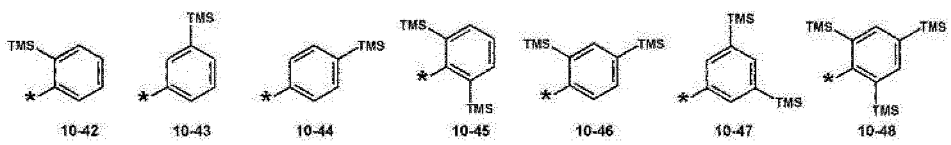

10-42 10-43 10-44 10-45 10-46 10-47 10-48

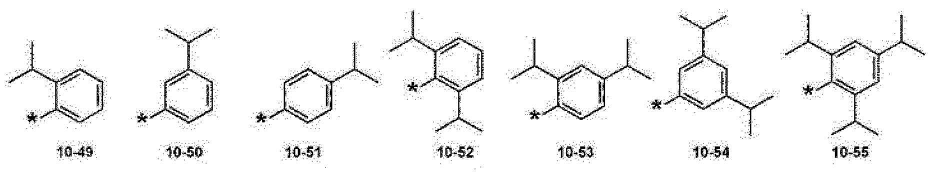

10-49 10-50 10-51 10-52 10-53 10-54 10-55

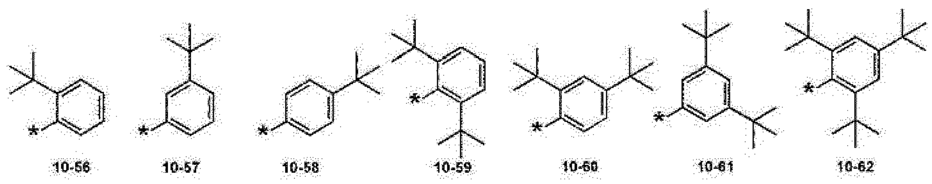

10-56 10-57 10-58 10-59 10-60 10-61 10-62

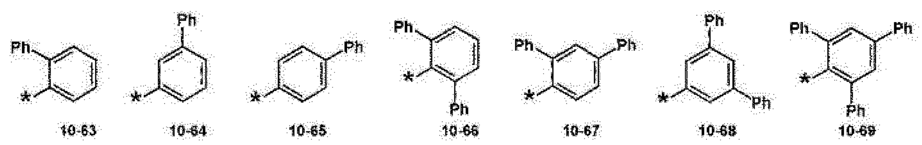

10-63 10-64 10-65 10-66 10-67 10-68 10-69

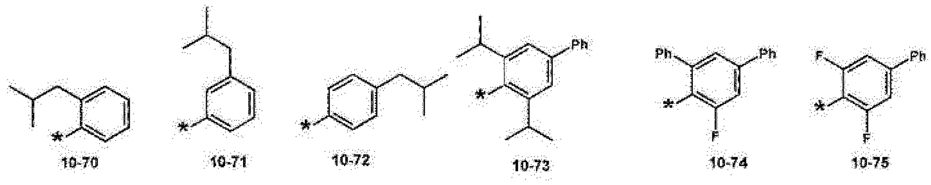

10-70 10-71 10-72 10-73 10-74 10-75

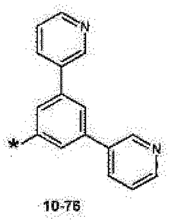
10-76

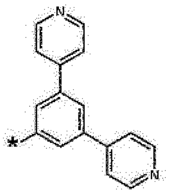
10-77

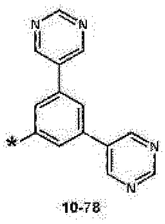
10-78

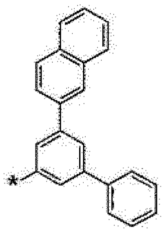
10-79

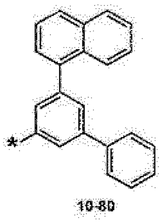
10-80

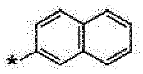
10-81

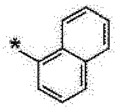
10-82

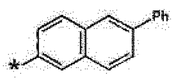
10-83

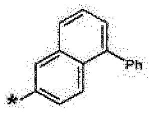
10-84

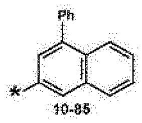
10-85

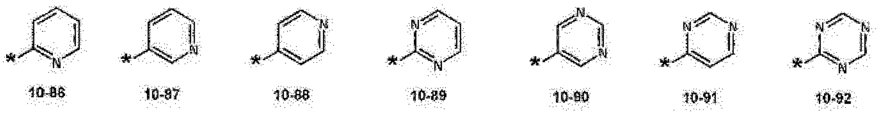
10-86 10-87 10-88 10-89 10-90 10-91 10-92

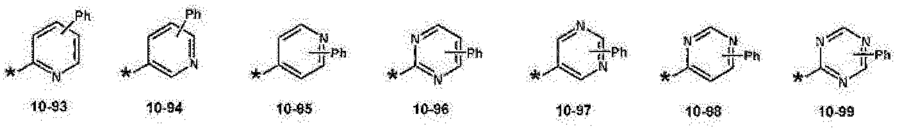
10-93 10-94 10-95 10-96 10-97 10-98 10-99

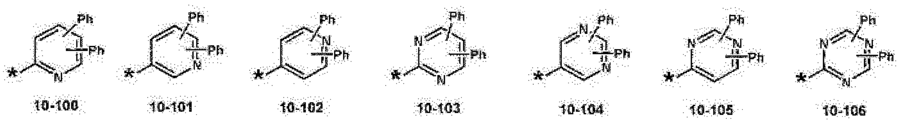
10-100 10-101 10-102 10-103 10-104 10-105 10-106

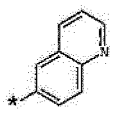
10-107

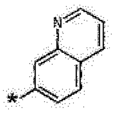
10-108

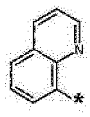
10-109

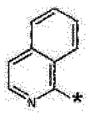
10-110

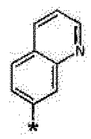
10-111

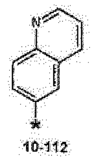
10-112

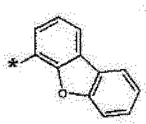
10-113

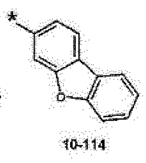
10-114

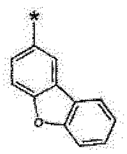
10-115

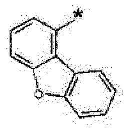
10-116

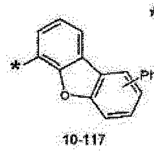
10-117

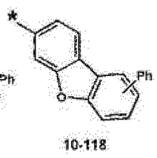
10-118

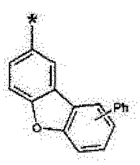
10-119

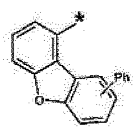
10-120

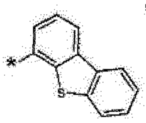
10-121

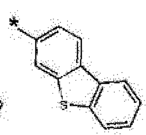
10-122

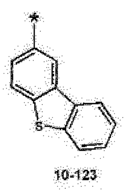
10-123

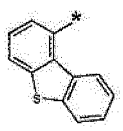
10-124

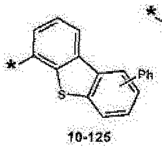
10-125
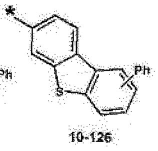
10-126
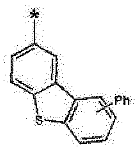
10-127
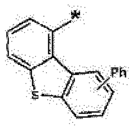
10-128
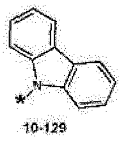
10-129
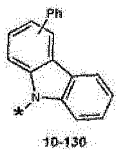
10-130

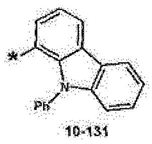
10-131
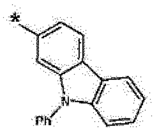
10-132
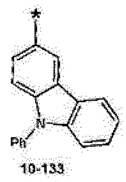
10-133
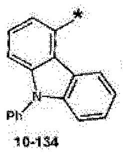
10-134
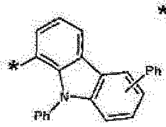
10-135
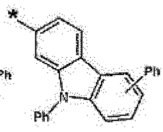
10-136

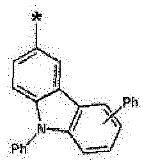
10-137
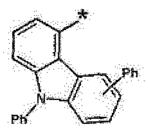
10-138
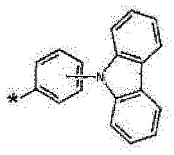
10-139
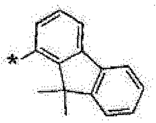
10-140
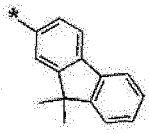
10-141
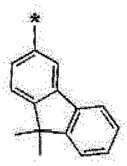
10-142

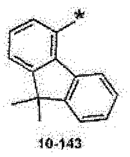
10-143
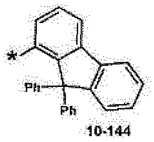
10-144
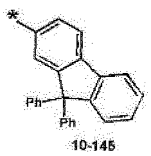
10-145
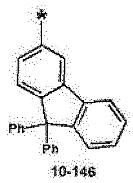
10-146
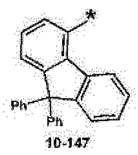
10-147

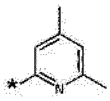
10-148
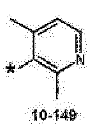
10-149
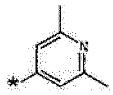
10-150
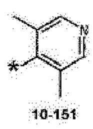
10-151
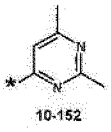
10-152
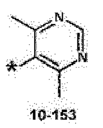
10-153
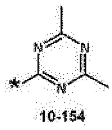
10-154

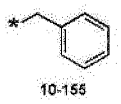
10-155
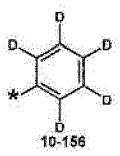
10-156
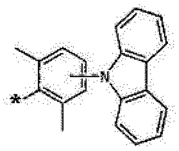
10-157
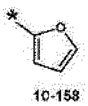
10-158
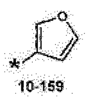
10-159
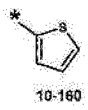
10-160
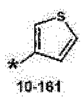
10-161

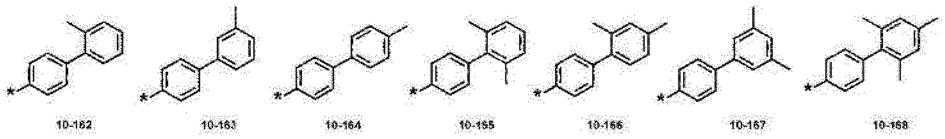
10-162 10-163 10-164 10-165 10-166 10-167 10-168

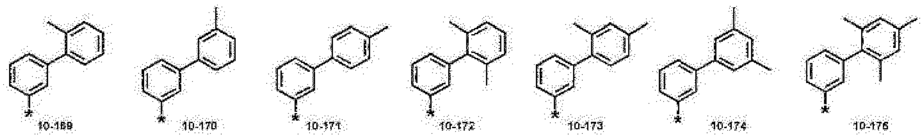
10-169 10-170 10-171 10-172 10-173 10-174 10-175

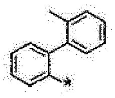
10-176
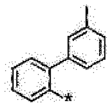
10-177
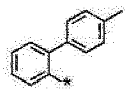
10-178
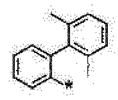
10-179
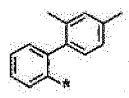
10-180
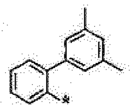
10-181
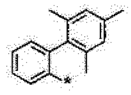
10-182

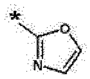
10-183
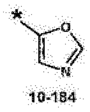
10-184
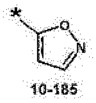
10-185
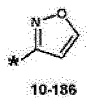
10-186
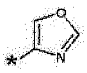
10-187
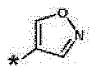
10-188
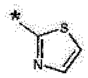
10-189
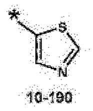
10-190
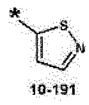
10-191

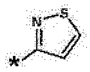
10-192
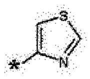
10-193
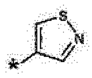
10-194

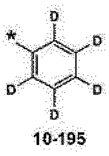
10-195
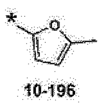
10-196
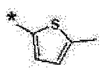
10-197
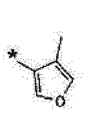
10-198
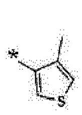
10-199
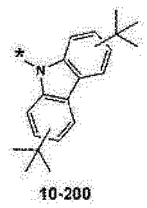
10-200
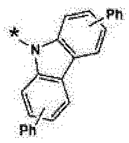
10-201
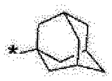
10-202

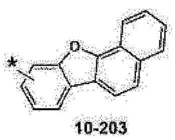
10-203
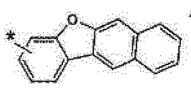
10-204
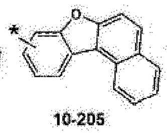
10-205
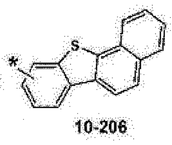
10-206
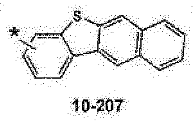
10-207
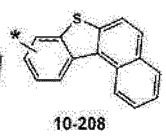
10-208

In Formulae 9-1 to 9-19 and 10-1 to 10-208, * indicates a binding site to a neighboring atom, Ph may be a phenyl group, and TMS may be a trimethylsilyl group.

In one or more embodiments, $R_{10}$, $R_{20}$, $R_{31}$ to $R_{34}$, and $R_{41}$ to $R_{44}$ may each be hydrogen. In one or more embodiments, the heterocyclic compound may be represented by one of Formulae 10-1 to 10-6:

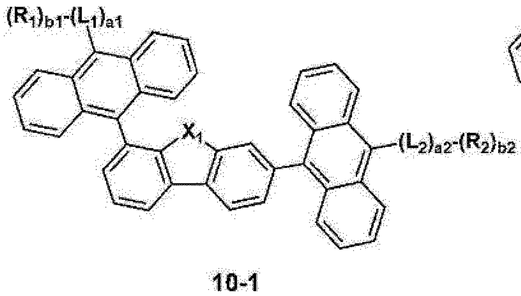
10-1

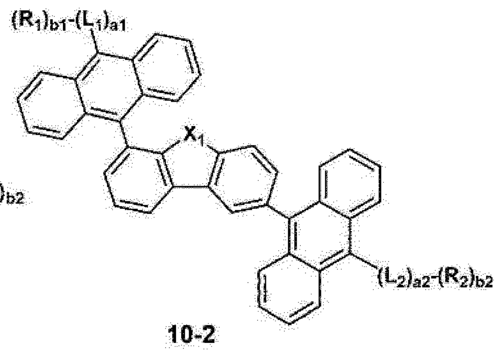
10-2

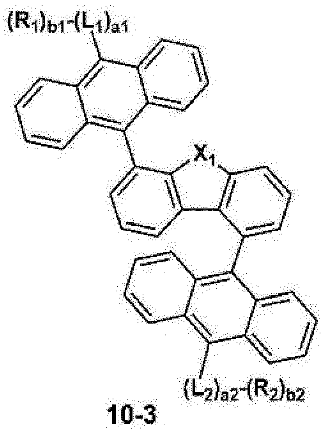

10-3

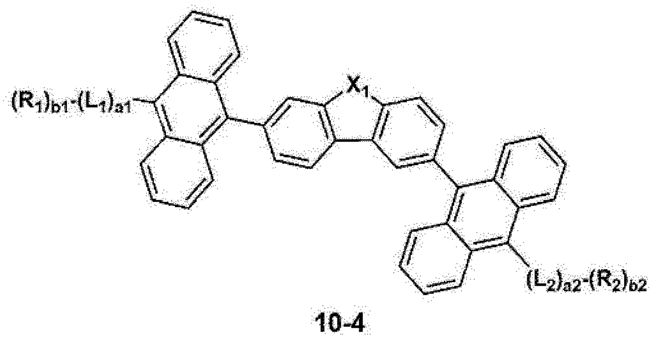

10-4

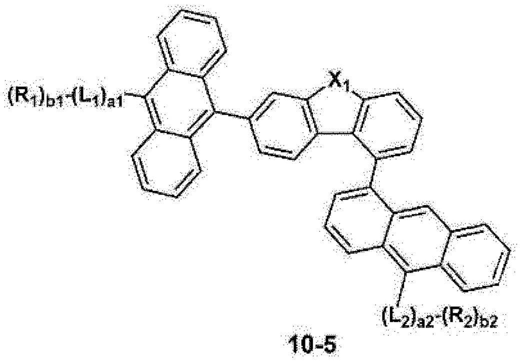

10-5

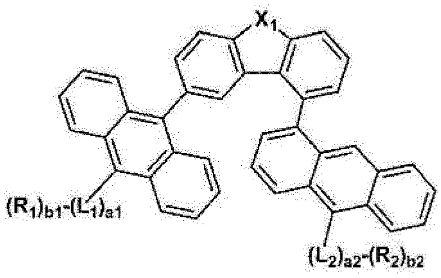

10-6 .

In Formulae 10-1 to 10-6,

$X_1$, $L_1$, $L_2$, a1, a2, $R_1$, $R_2$, b1, and b2 are the same as described above.

In one or more embodiments, the heterocyclic compound may be one of Compounds 1 to 2120:

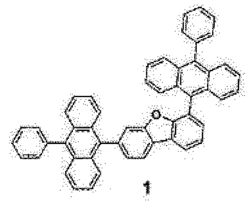

1

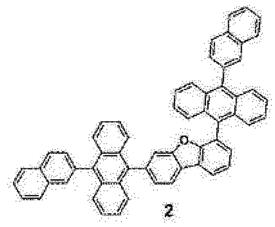

2

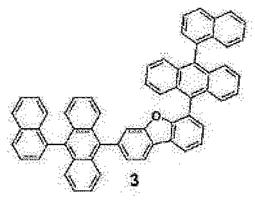

3

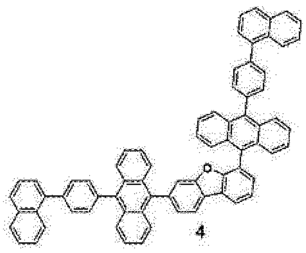

4

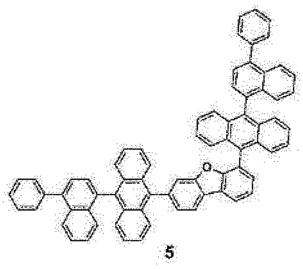

5

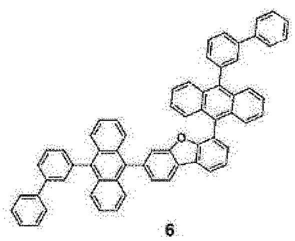

6

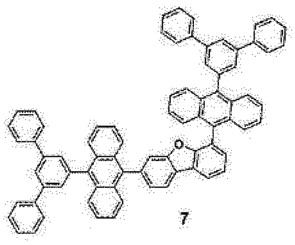

7

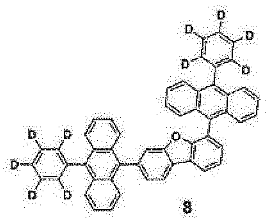

8

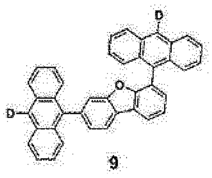

9

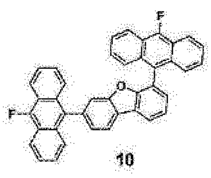

10

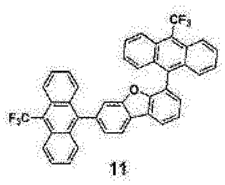

11

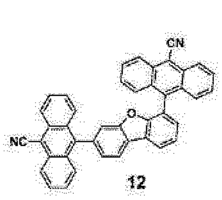

12

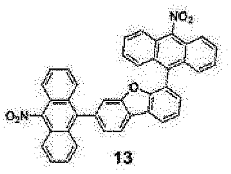
13

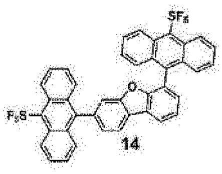
14

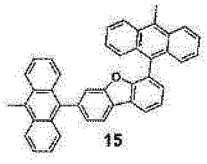
15

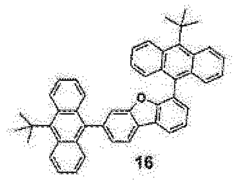
16

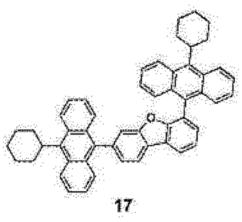
17

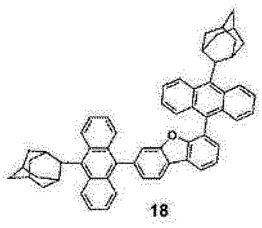
18

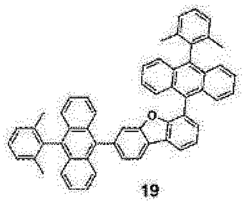
19

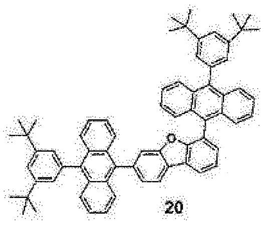
20

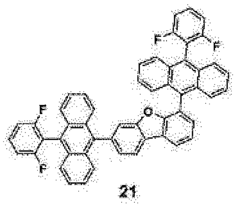
21

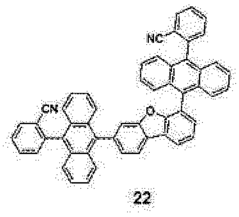
22

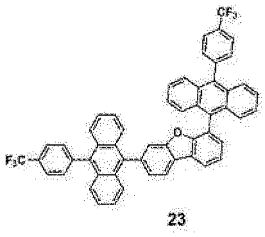
23

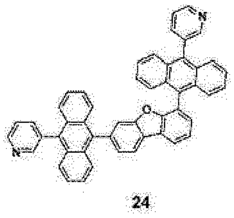
24

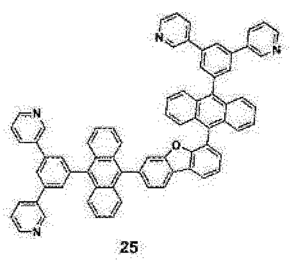
25

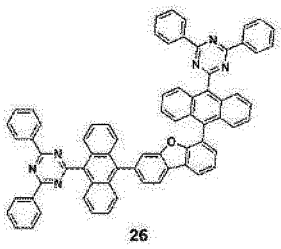
26

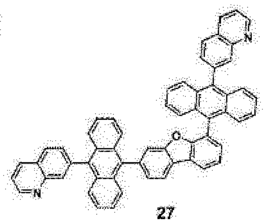
27

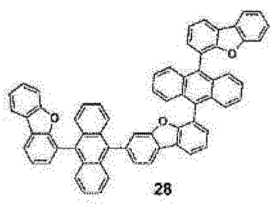
28

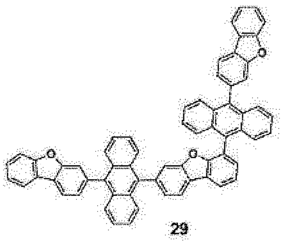
29

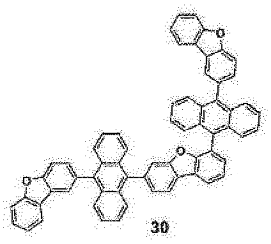
30

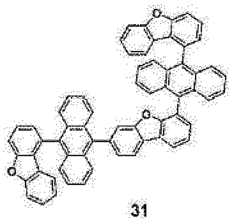
31

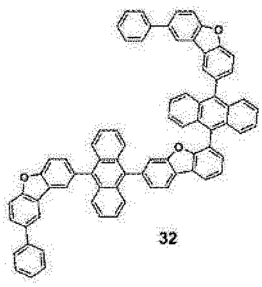
32

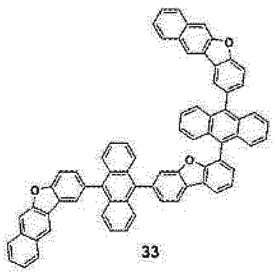
33

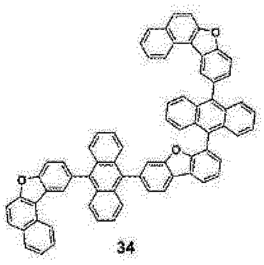
34

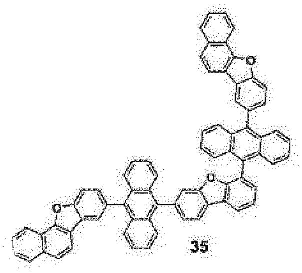
35

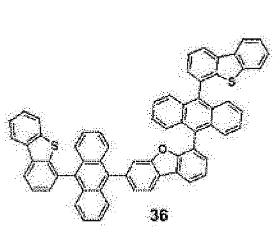
36

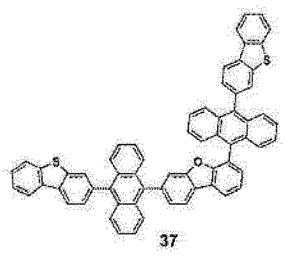
37

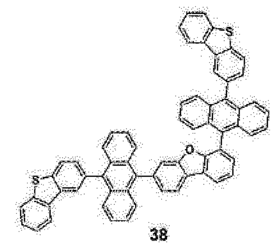
38

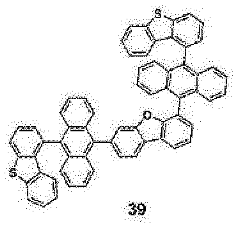
39

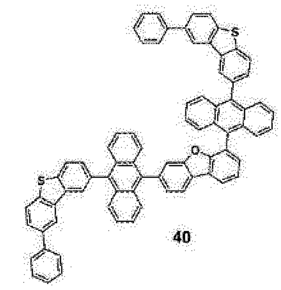
40

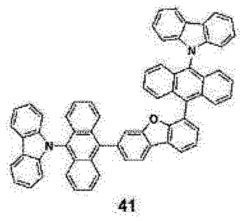
41

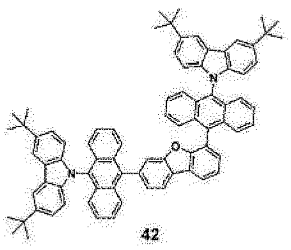
42

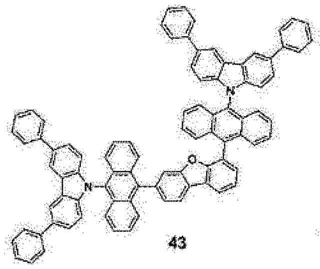
43

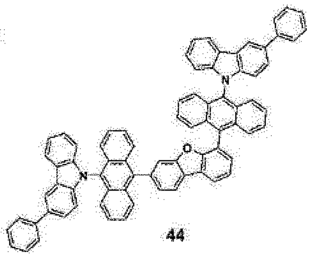
44

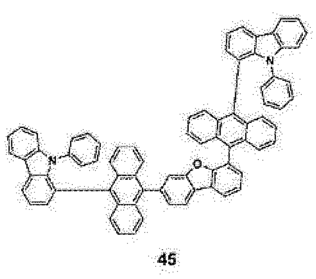
45

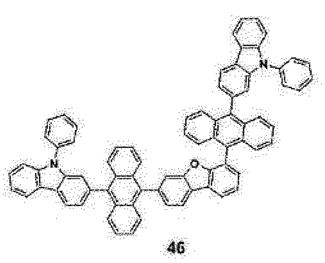
46

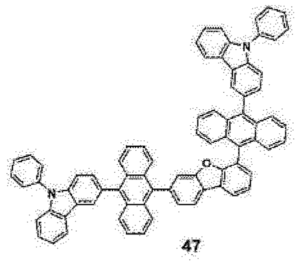
47

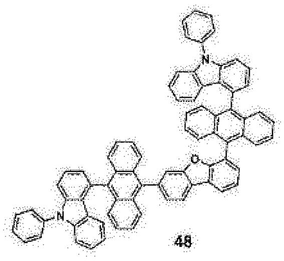
48

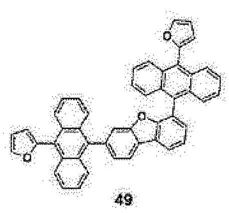
49

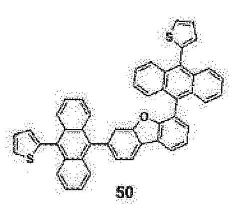
50

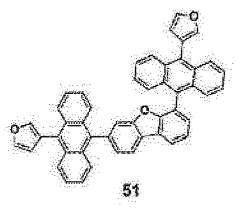
51

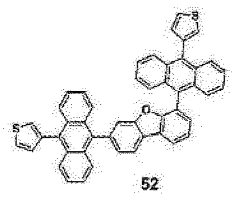
52

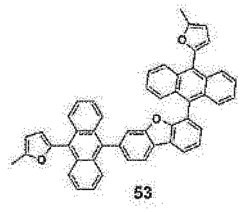
53

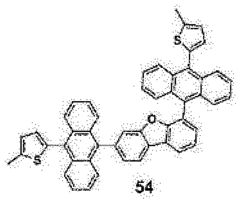
54

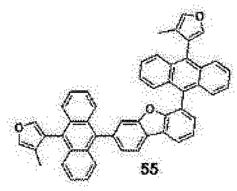
55

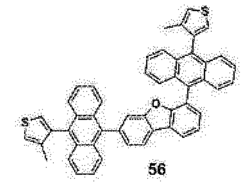
56

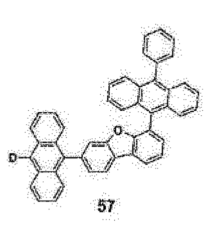
57

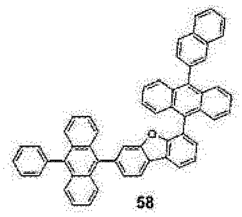
58

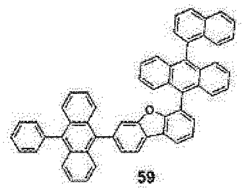
59

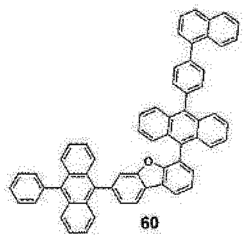
60

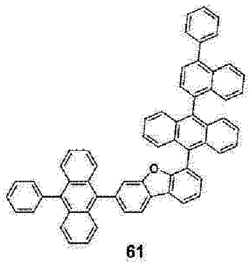
61

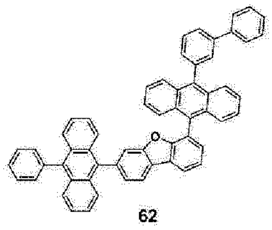
62

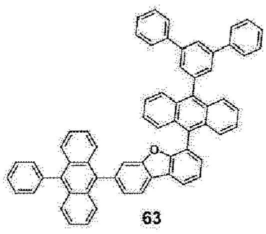
63

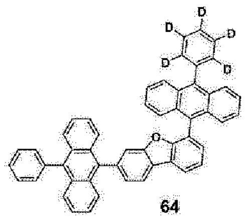
64

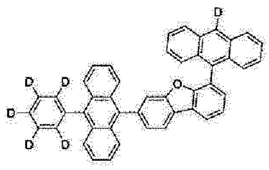
65

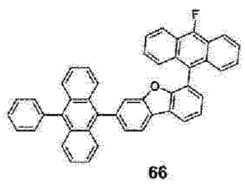
66

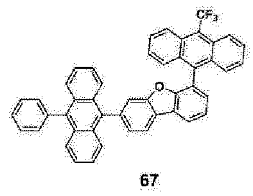
67

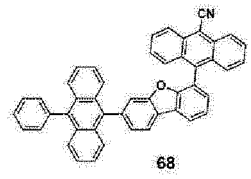
68

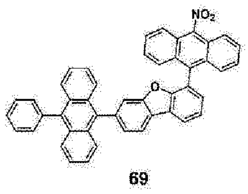
69

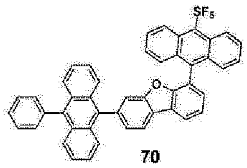
70

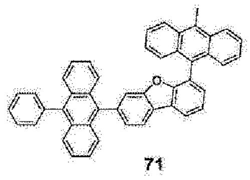
71

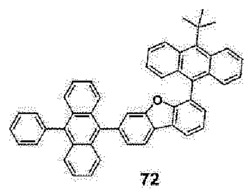
72

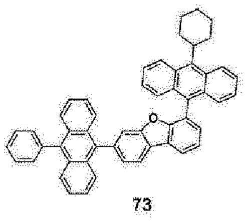
73

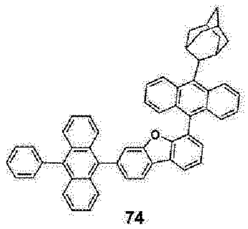
74

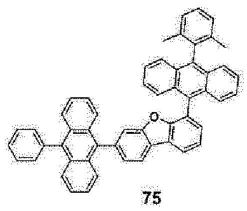
75

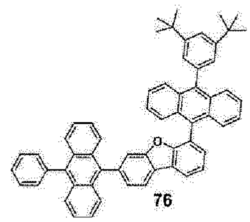
76

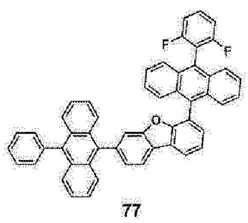
77

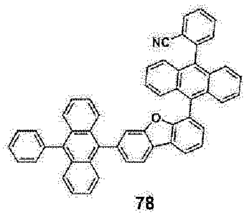
78

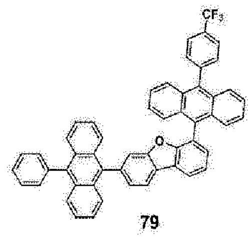
79

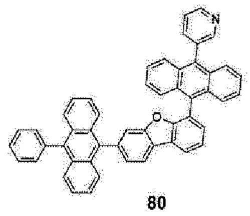
80

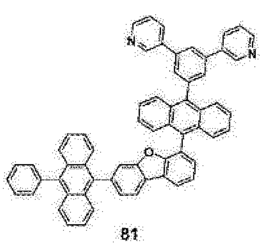
81

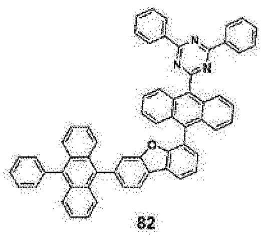
82

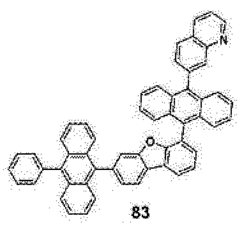
83

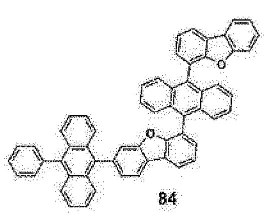
84

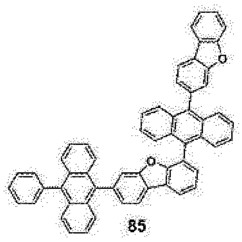
85

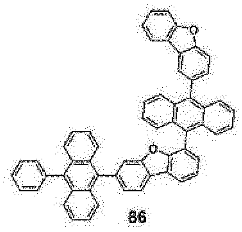
86

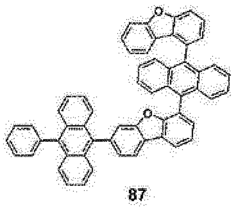
87

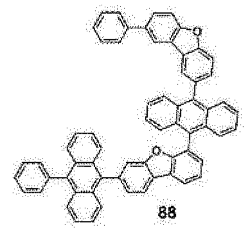
88

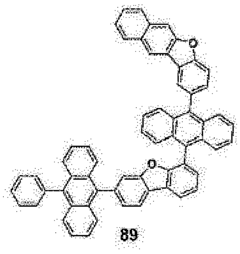
89

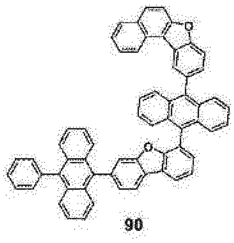
90

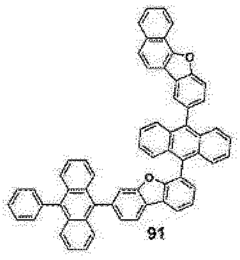
91

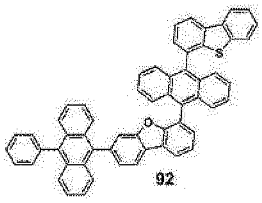
92

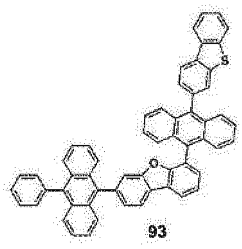
93

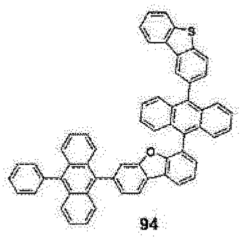
94

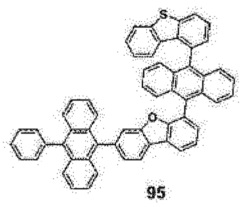
95

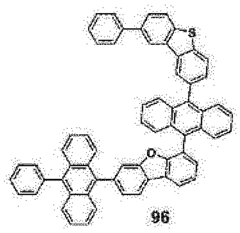
96

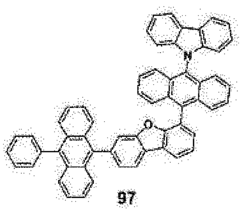
97

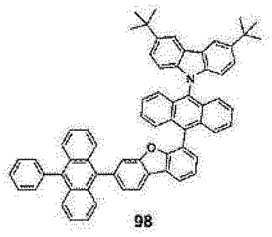
98

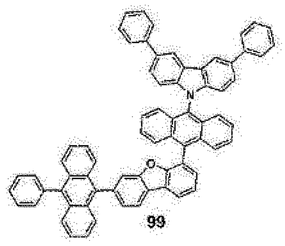
99

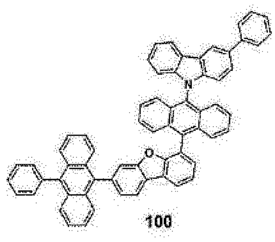
100

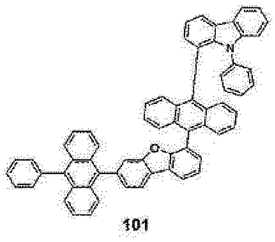
101

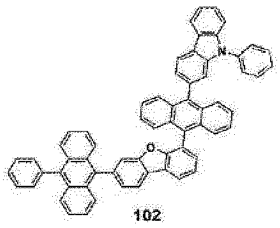
102

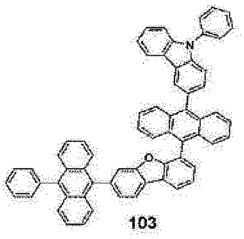
103

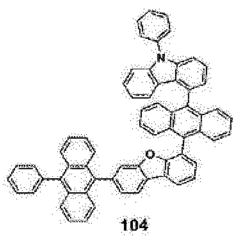
104

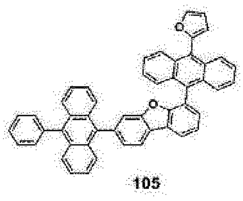
105

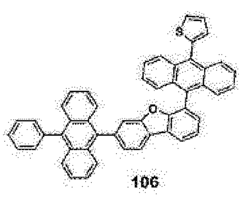
106

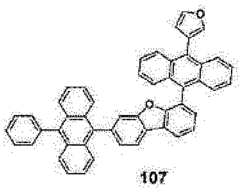
107

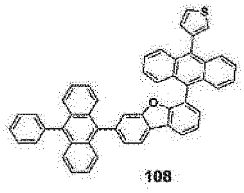
108

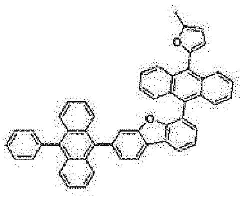
109

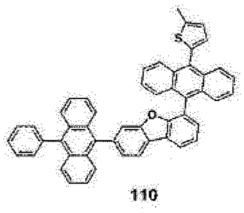
110

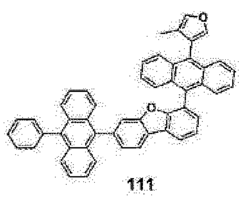
111

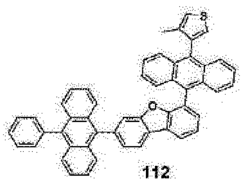
112

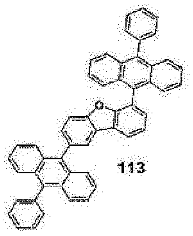
113

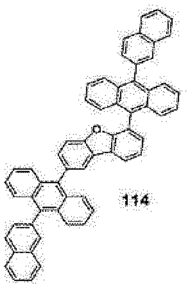
114

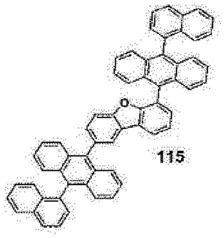
115

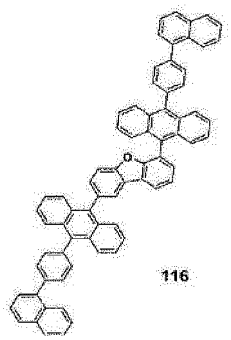
116

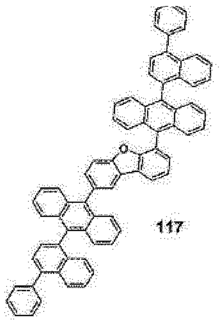
117

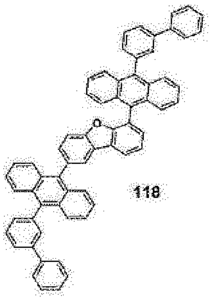
118

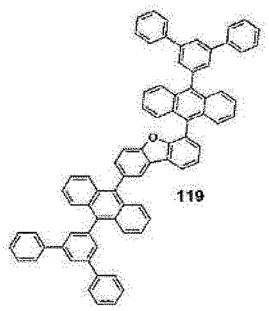
119

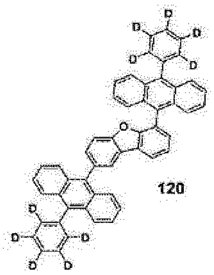
120

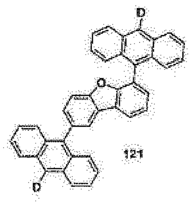
121

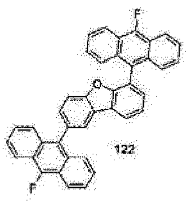
122

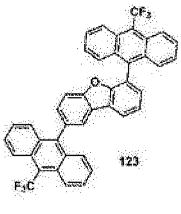
123

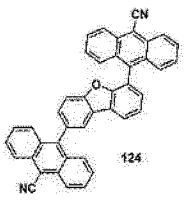
124

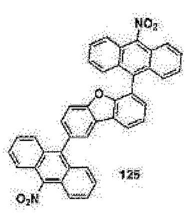
125

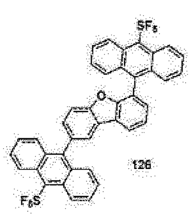
126

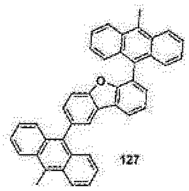
127

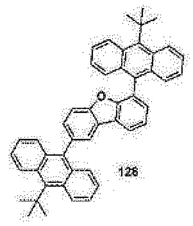
128

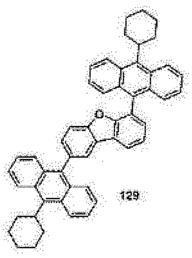
129

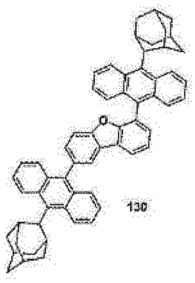
130

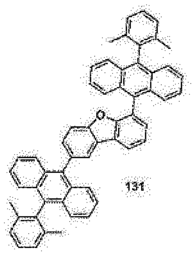
131

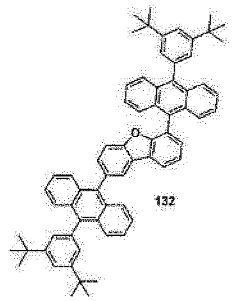
132

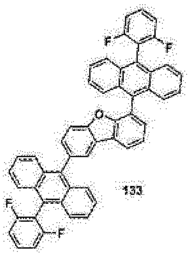
133

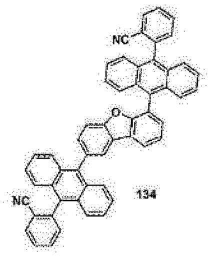
134

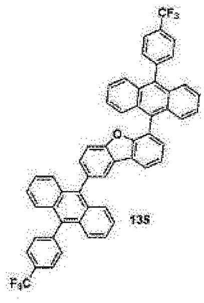
135

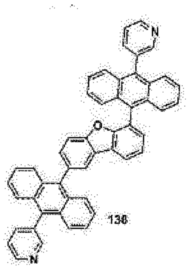
136

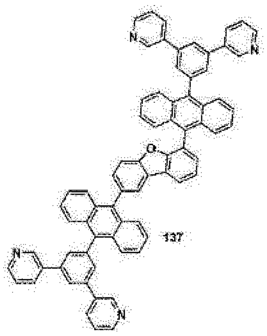
137

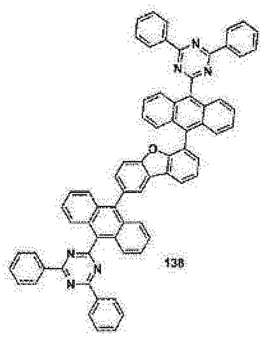
138

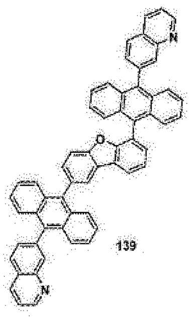
139

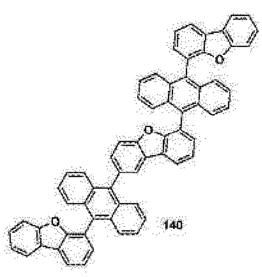
140

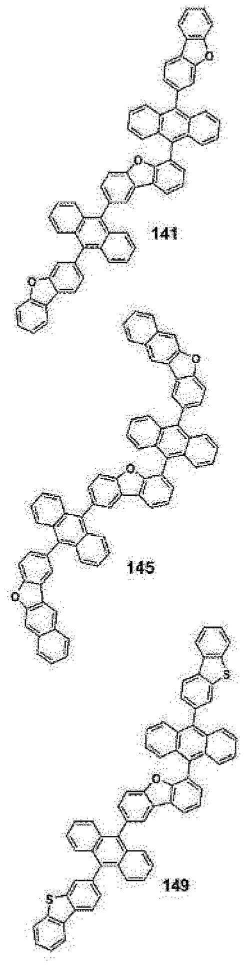

141

145

149

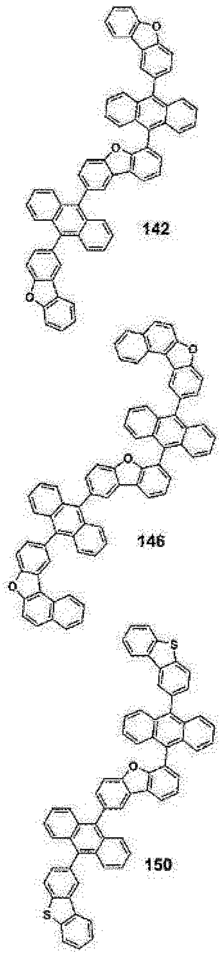

142

146

150

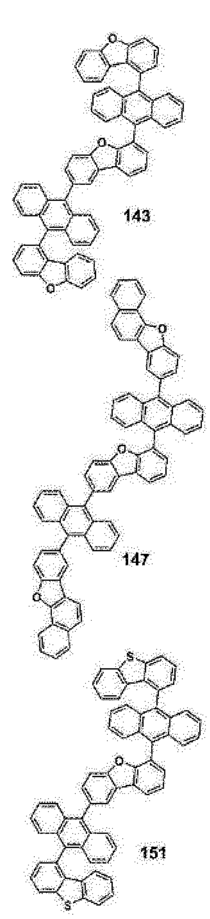

143

147

151

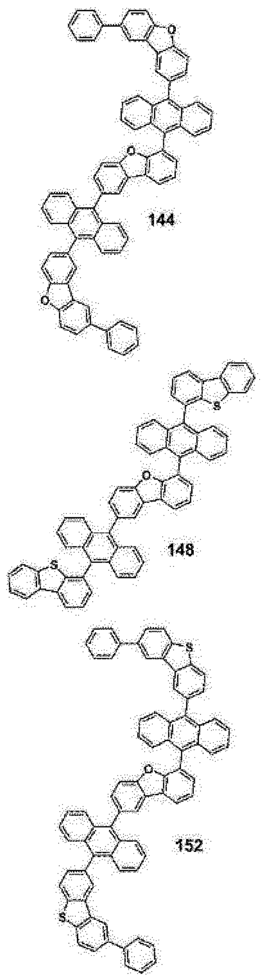

144

148

152

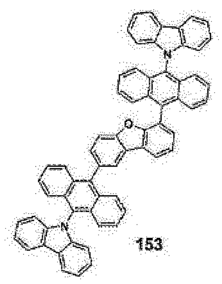

153

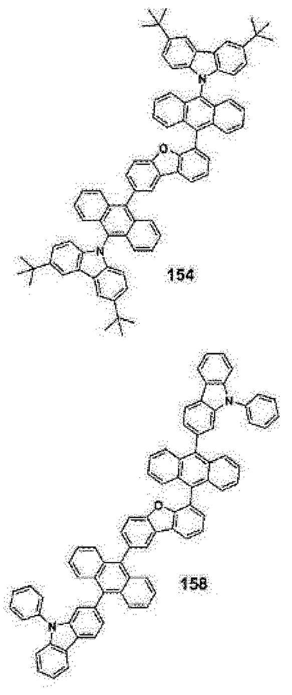

154

158

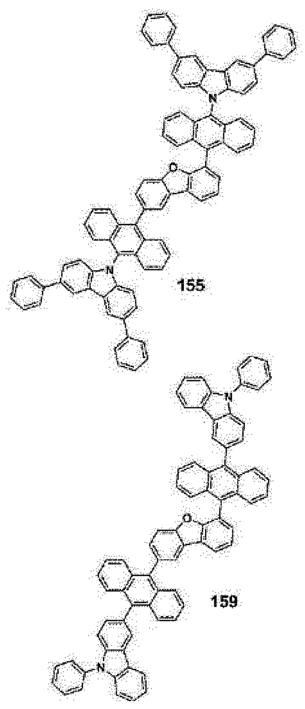

155

159

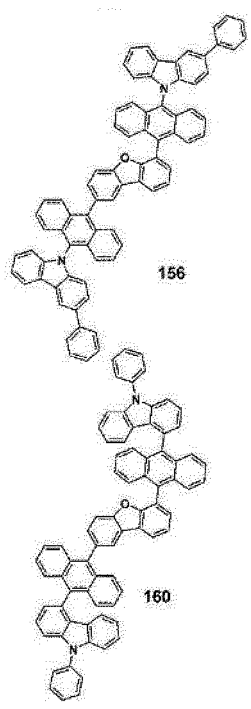

156

160

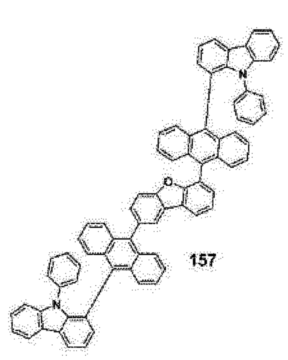

157

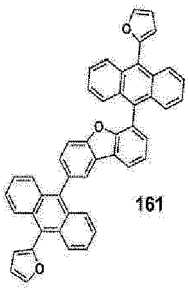
161

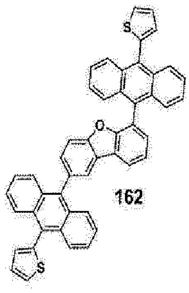
162

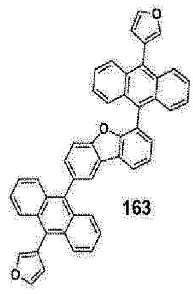
163

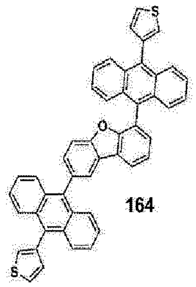
164

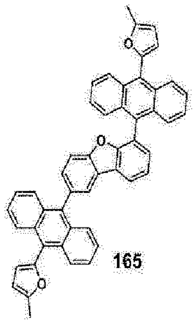
165

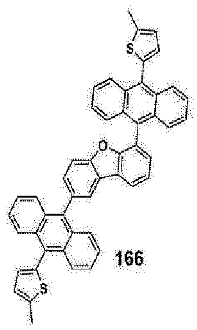
166

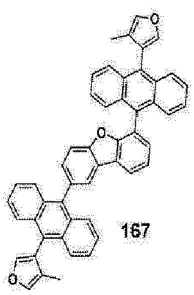
167

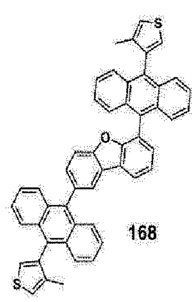
168

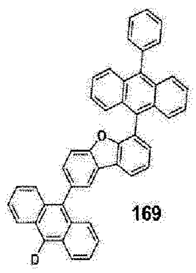
169

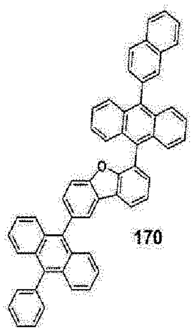
170

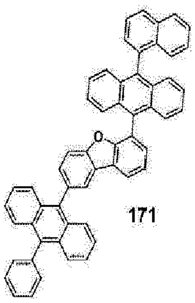
171

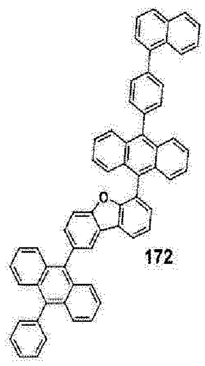
172

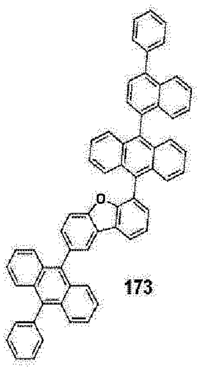
173

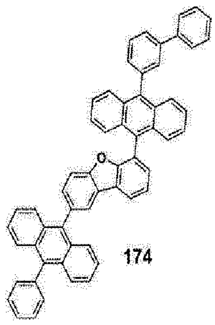
174

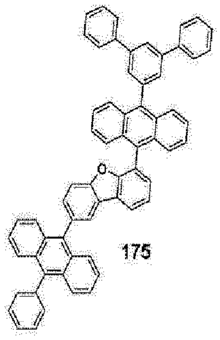
175

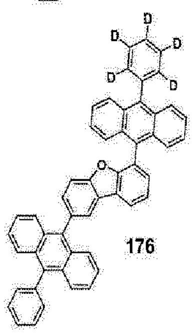
176

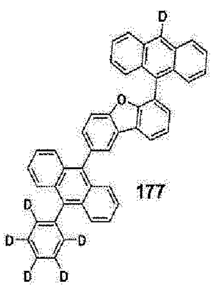
177

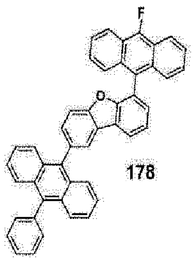
178

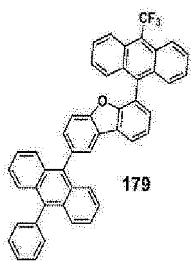
179

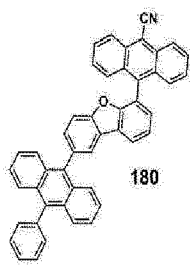
180

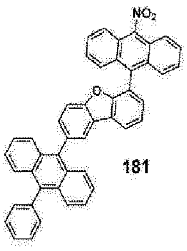
181

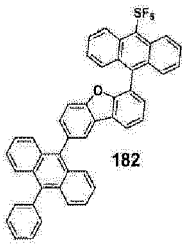
182

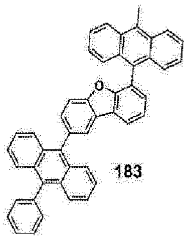
183

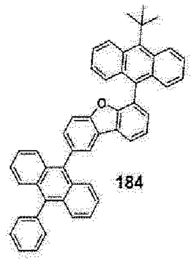
184

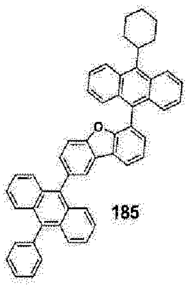
185

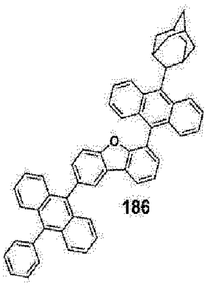
186

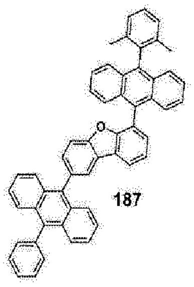
187

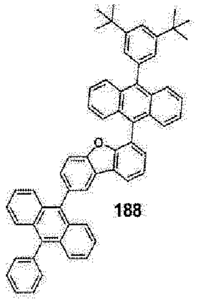
188

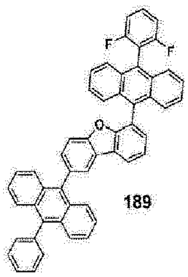
189

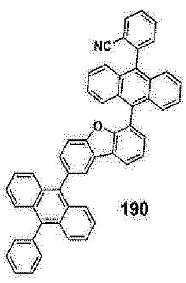
190

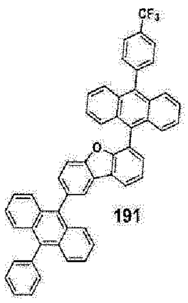
191

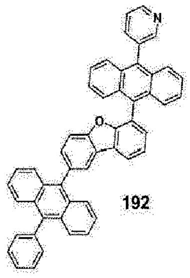
192

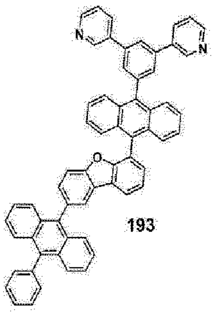
193

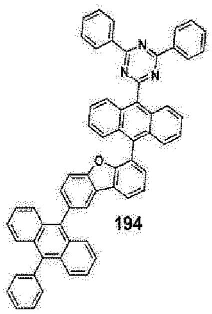
194

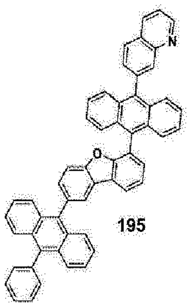
195

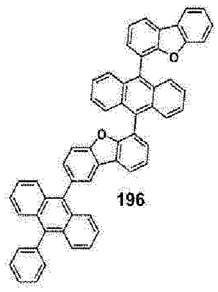
196

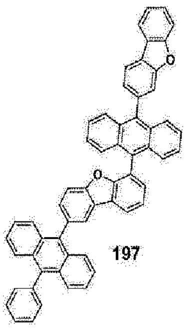
197

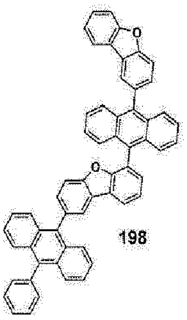
198

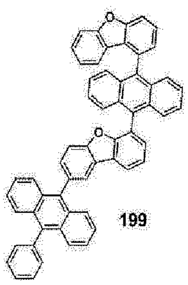
199

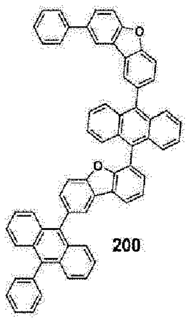
200

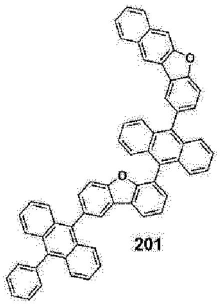
201

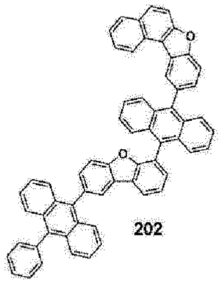
202

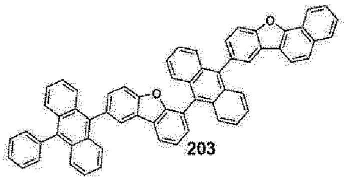
203

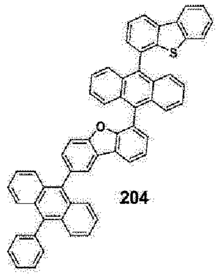
204

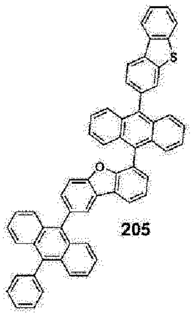
205

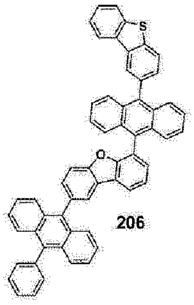
206

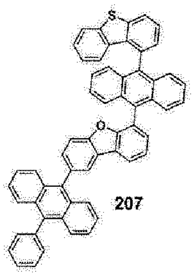
207

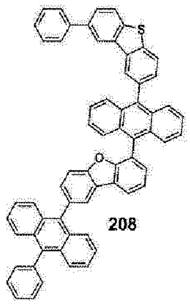
208

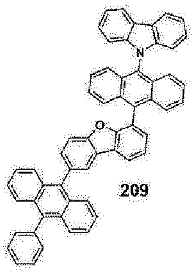
209

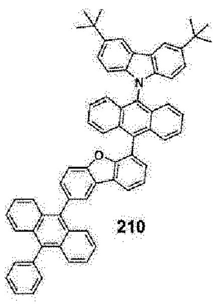
210

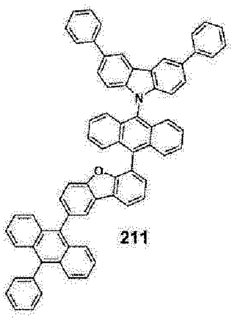
211

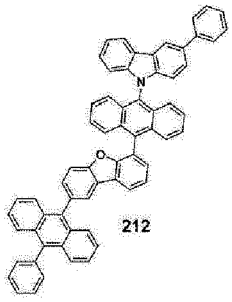
212

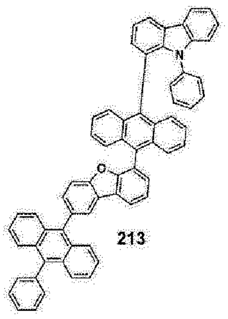
213

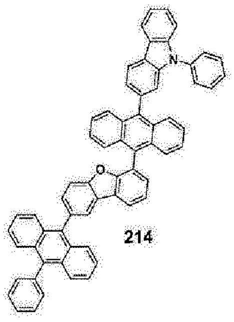
214

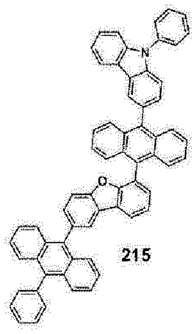
215

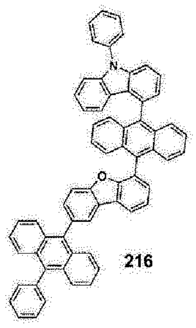
216

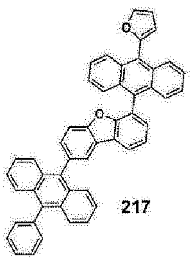
217

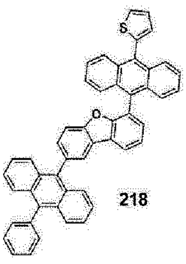
218

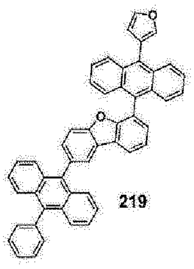
219

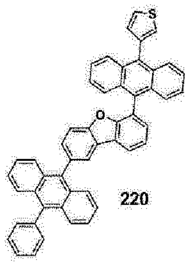
220

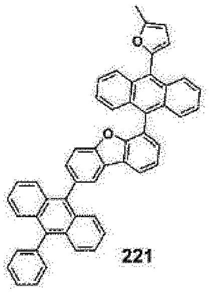
221

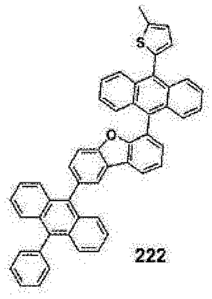
222

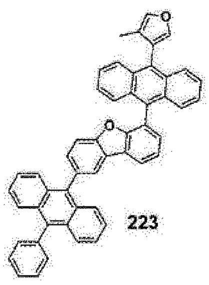
223

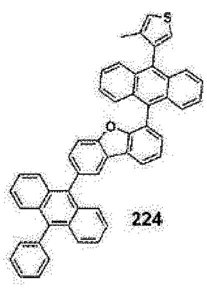
224

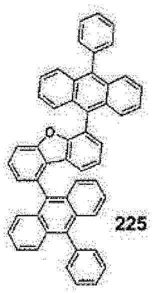
225

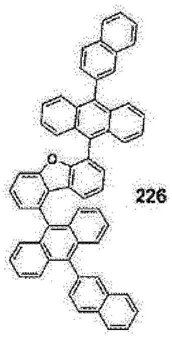
226

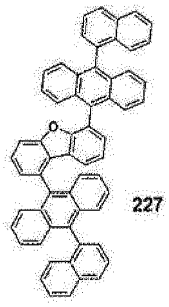
227

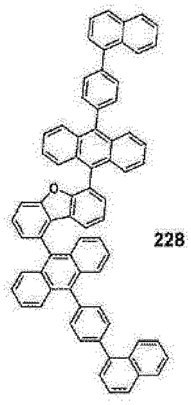
228

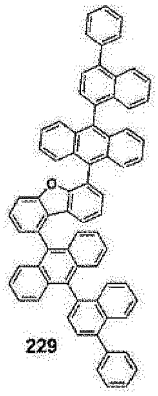
229

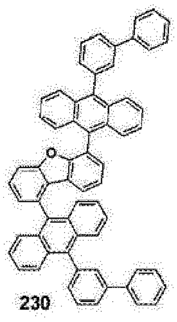
230

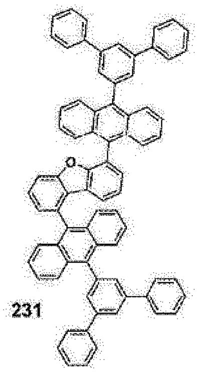
231

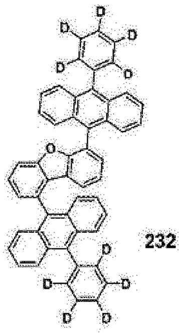
232

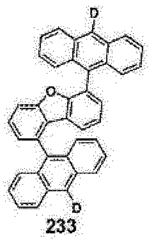
233

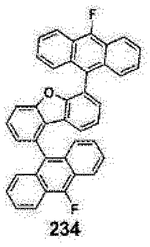
234

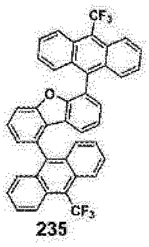
235

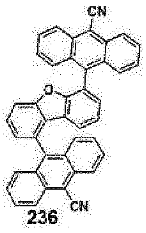
236

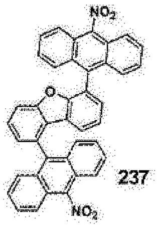
237

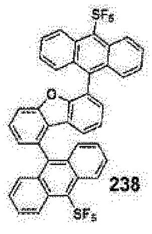
238

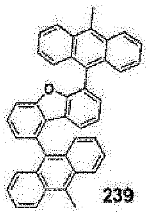
239

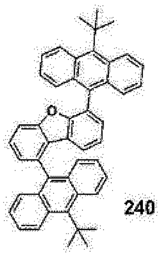
240

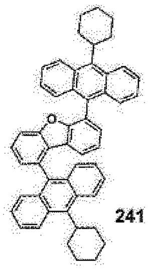
241

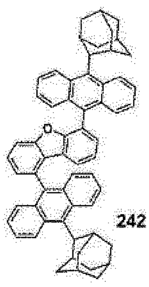
242

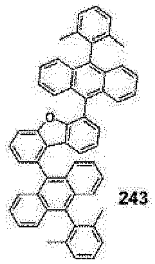
243

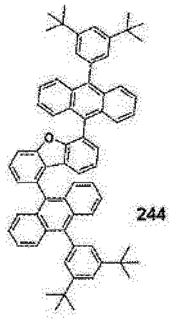
244

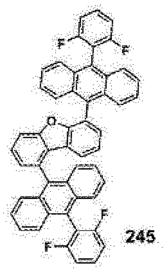
245

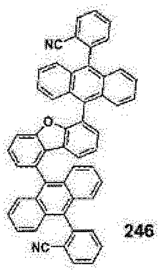
246

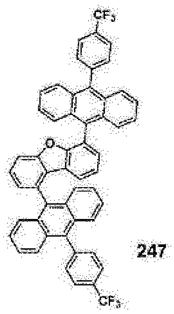
247

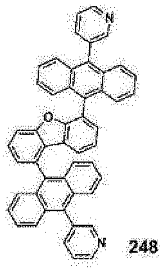
248

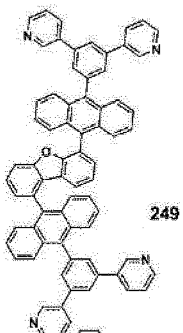
249

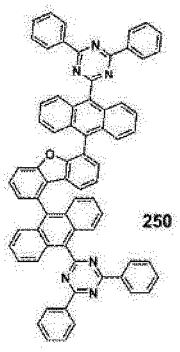
250

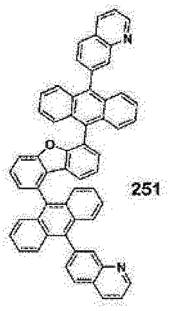
251

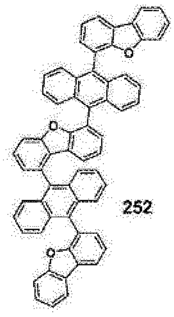
252

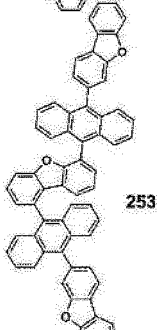
253

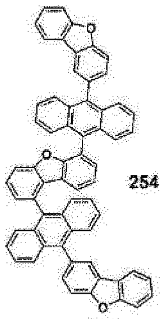
254

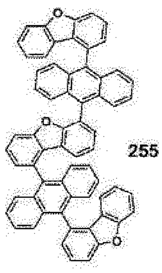
255

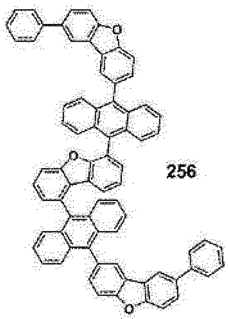
256

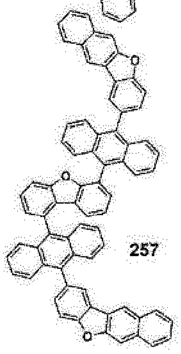
257

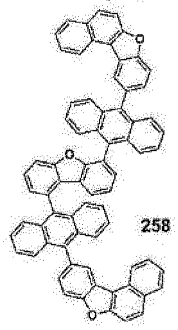
258

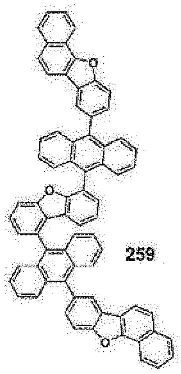
259

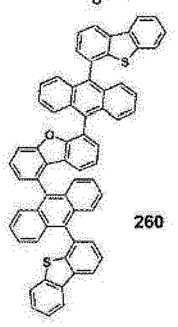
260

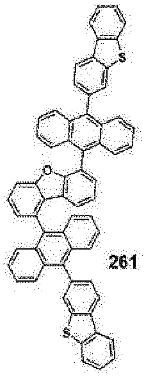
261

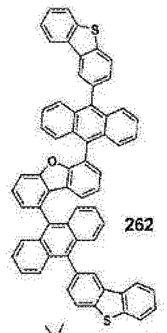
262

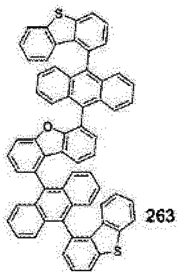
263

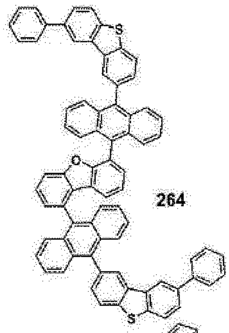
264

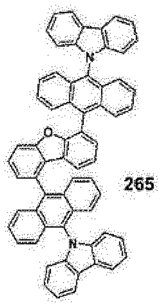
265

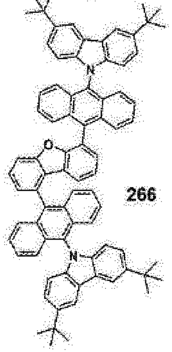
266

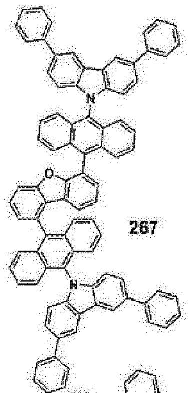
267

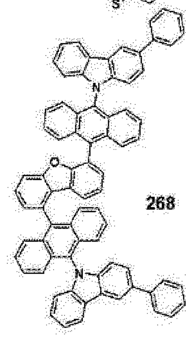
268

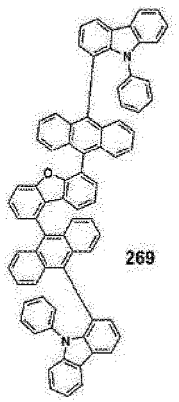
269

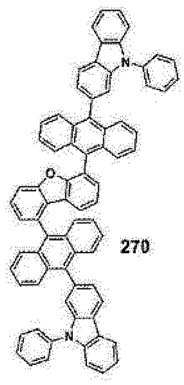
270

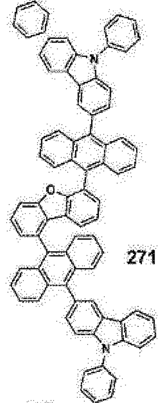
271

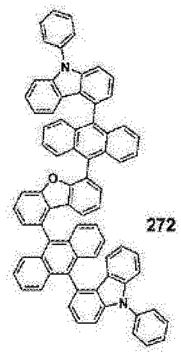
272

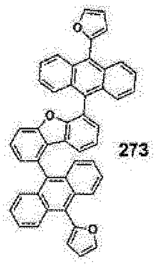
273

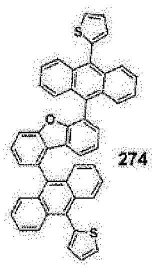
274

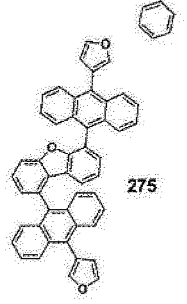
275

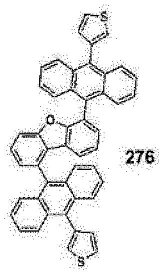
276

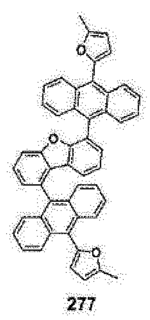
277

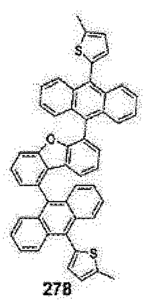
278

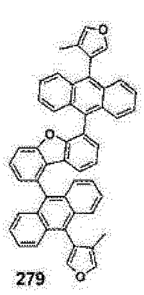
279

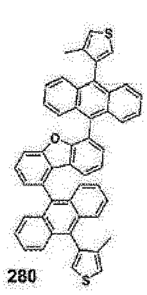
280

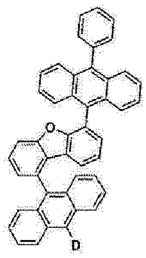
281

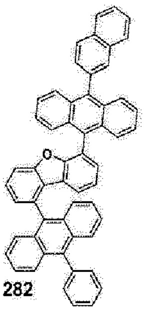
282

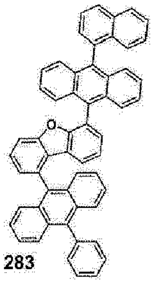
283

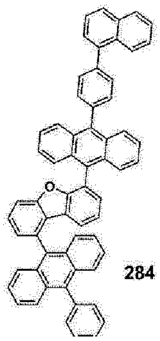
284

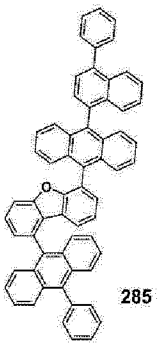
285

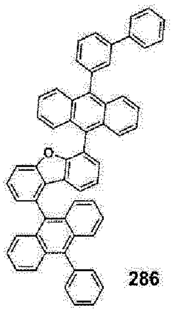
286

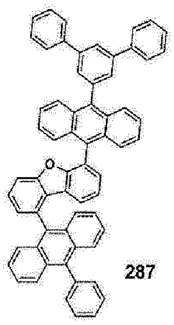
287

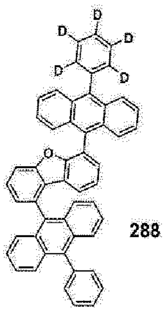
288

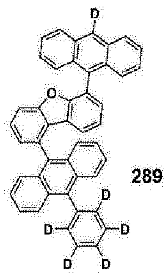
289

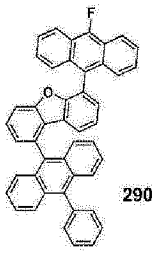
290

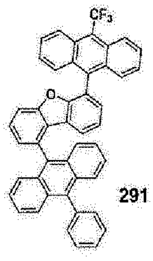
291

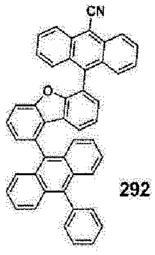
292

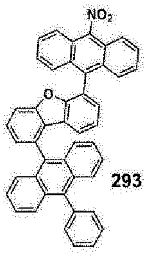
293

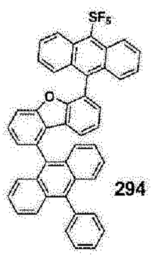
294

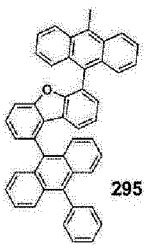
295

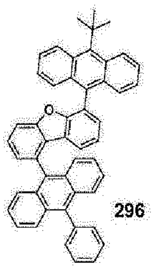
296

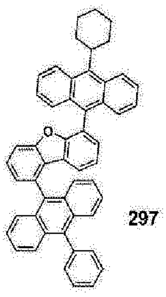
297

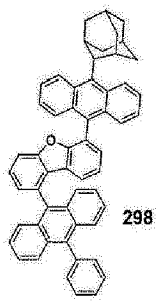
298

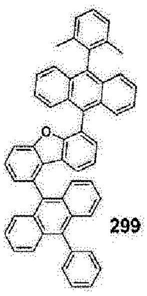
299

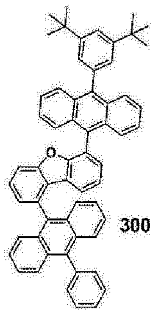
300

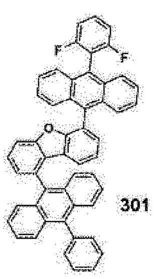
301

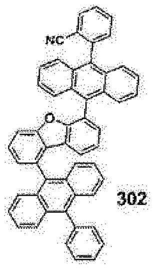
302

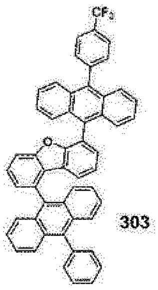
303

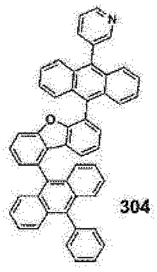
304

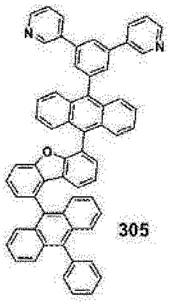
305

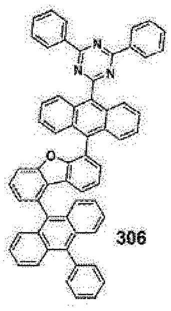
306

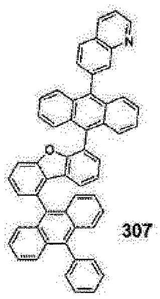
307

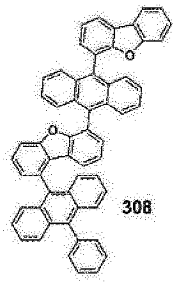
308

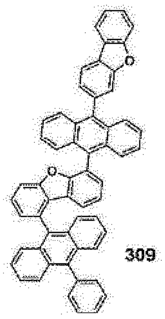
309

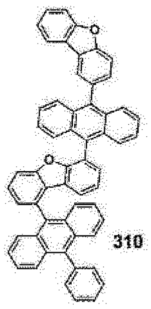
310

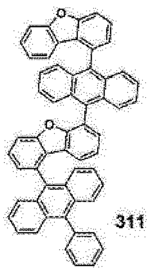
311

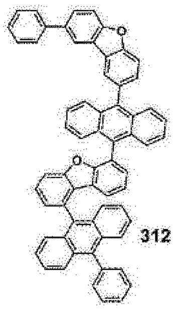
312

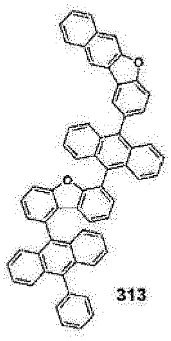
313

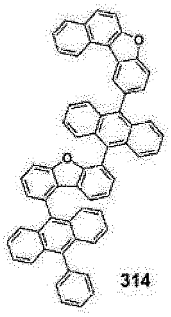
314

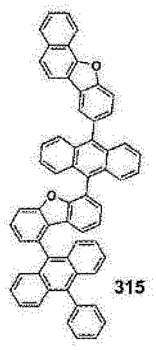
315

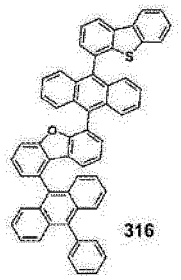
316

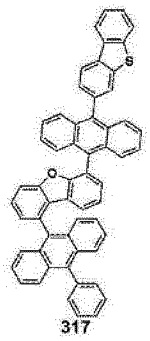
317

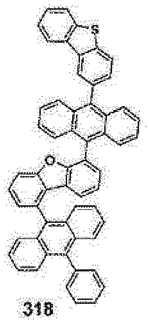
318

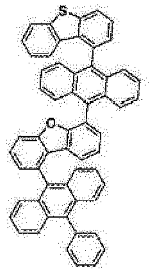
319

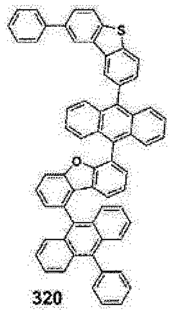
320

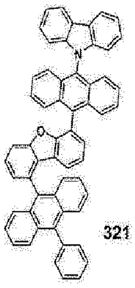
321

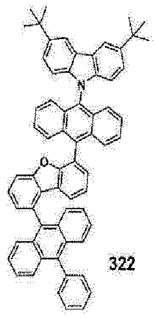
322

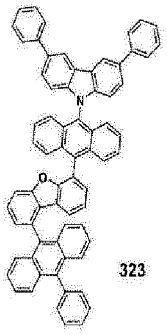
323

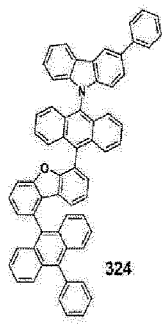
324

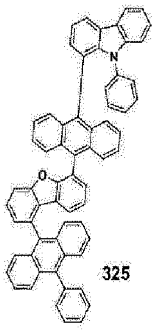
325

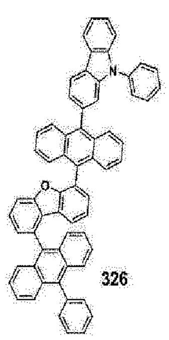
326

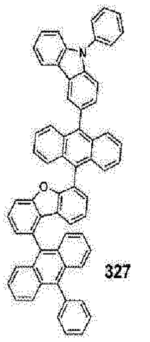
327

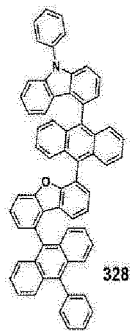
328

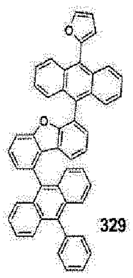
329

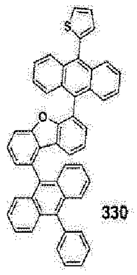
330

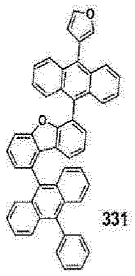
331

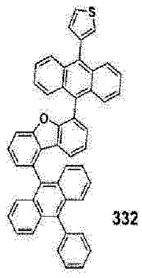
332

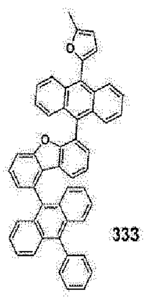
333

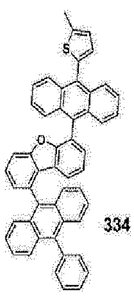
334

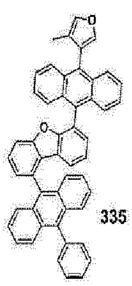
335

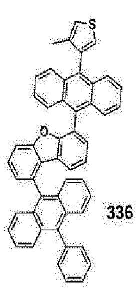
336

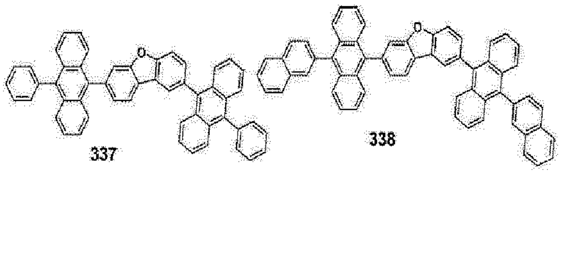
337

338

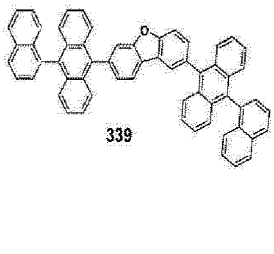
339

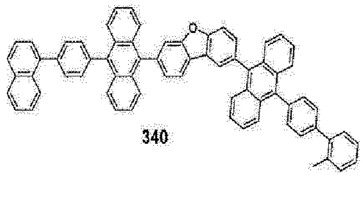
340

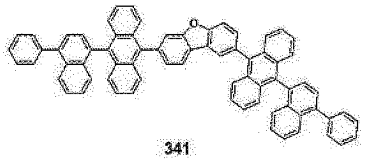

341

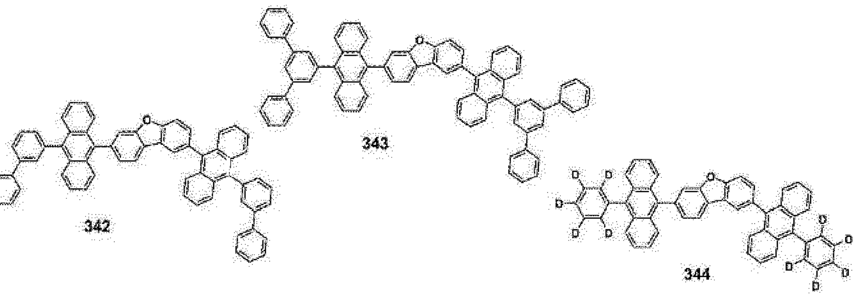

342

343

344

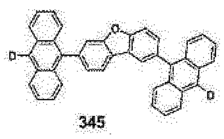

345

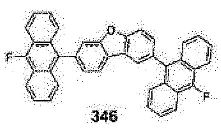

346

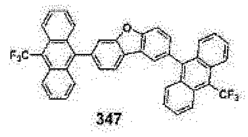

347

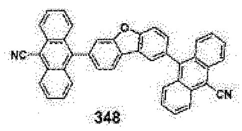

348

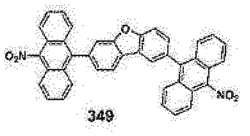

349

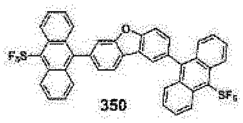

350

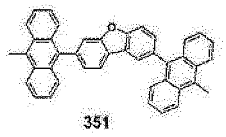

351

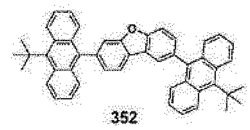

352

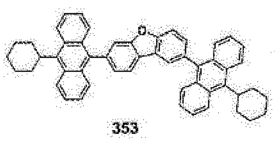

353

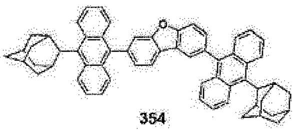

354

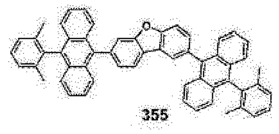

355

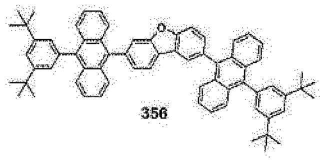

356

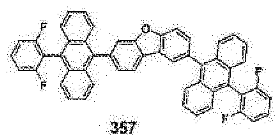

357

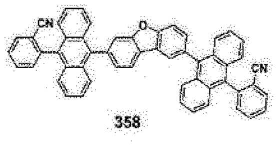

358

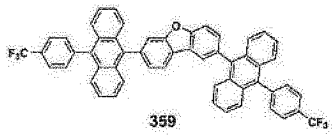

359

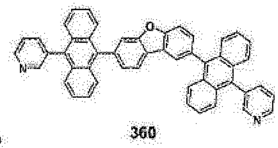

360

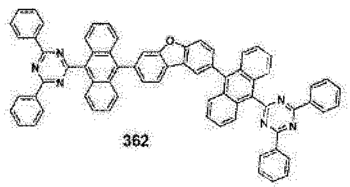

362

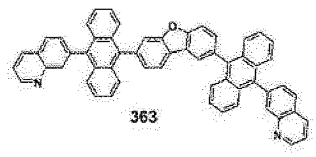

363

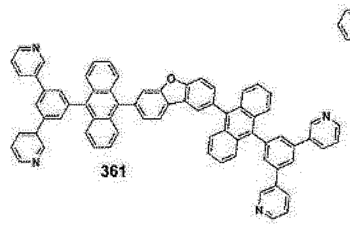

361

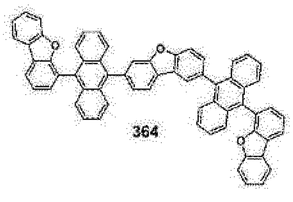

364

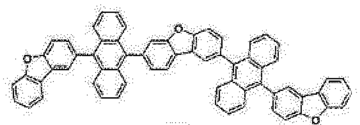

366

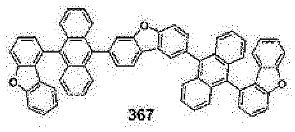

367

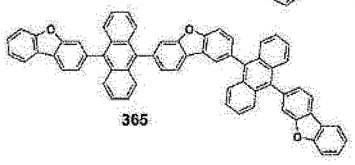

365

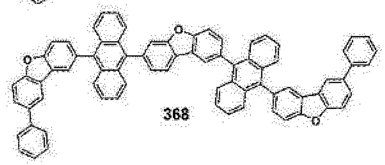

368

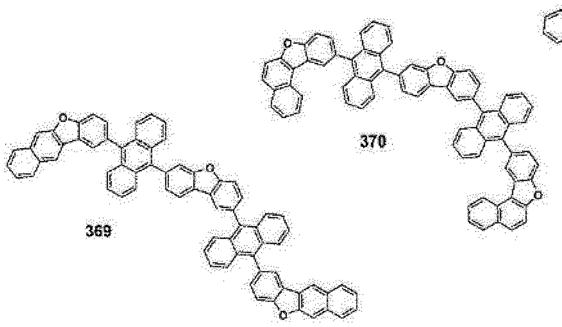

369

370

371

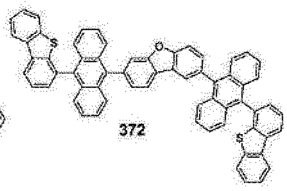

372

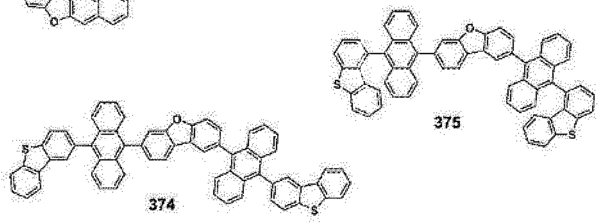

374

375

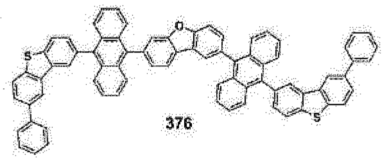

376

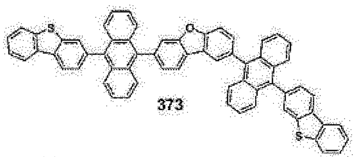

373

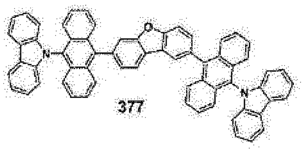

377

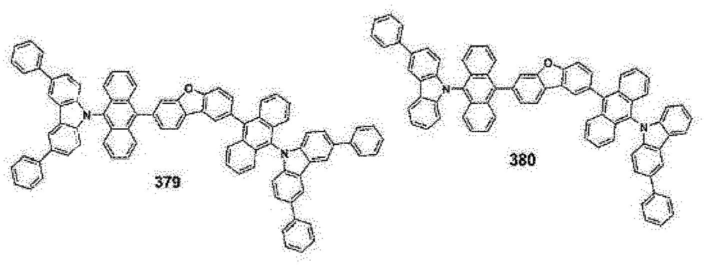

379

380

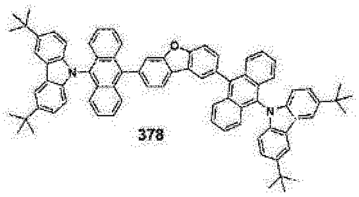

378

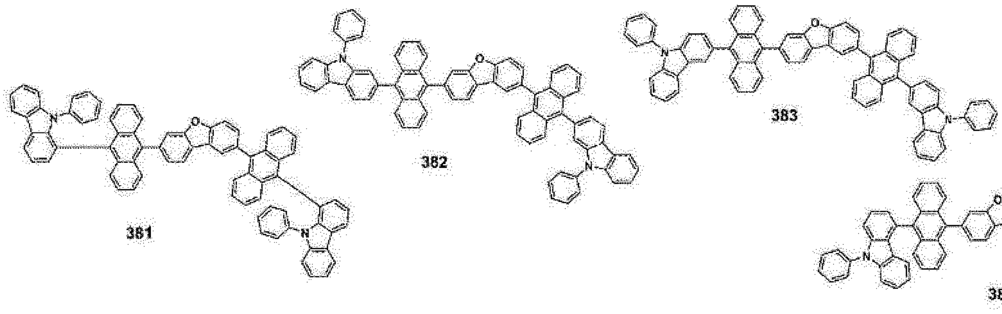

381

382

383

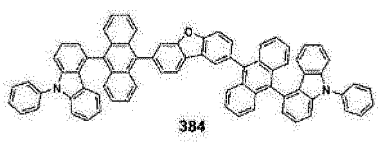

384

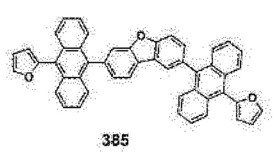

385

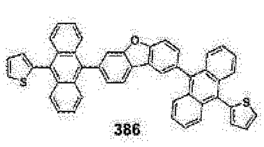

386

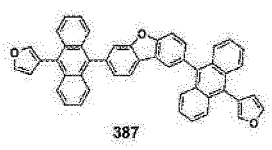

387

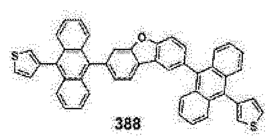

388

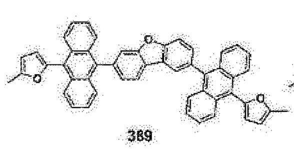

389

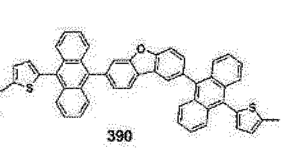

390

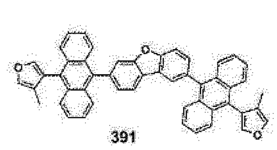

391

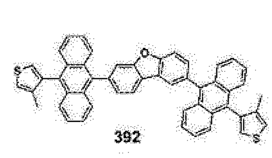

392

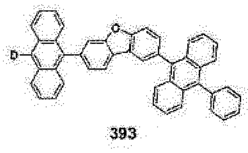
393

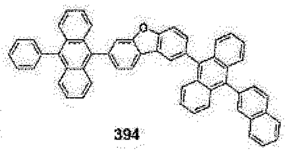
394

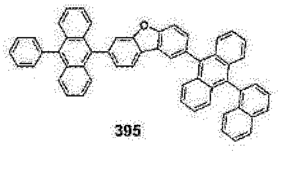
395

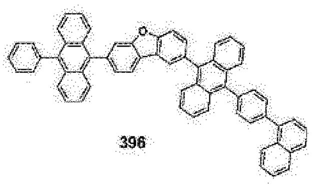
396

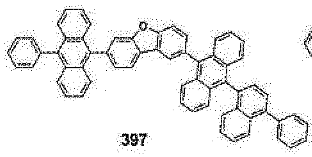
397

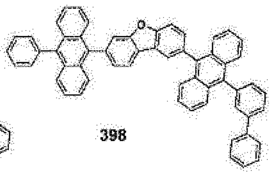
398

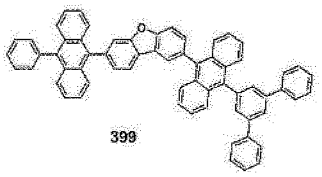
399

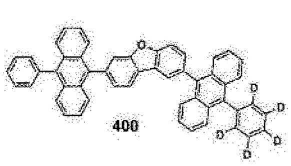
400

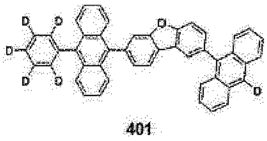
401

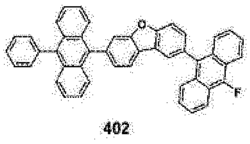
402

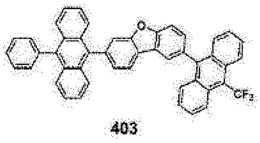
403

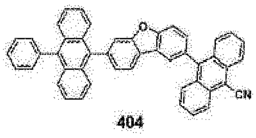
404

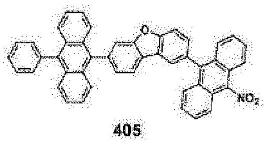
405

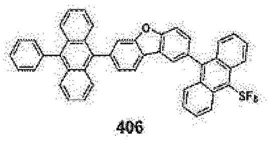
406

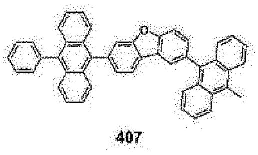
407

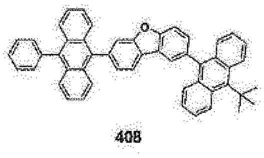
408

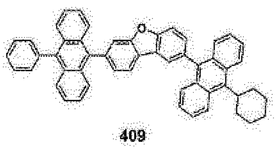
409

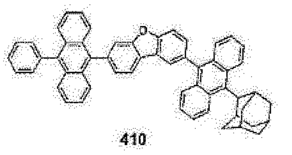
410

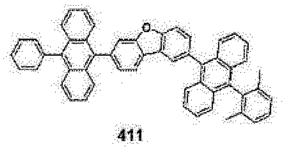
411

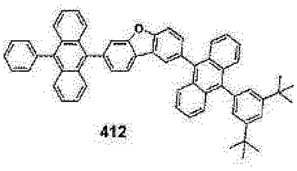
412

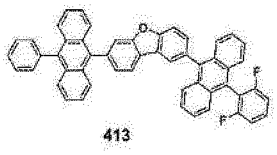
413

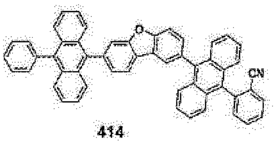
414

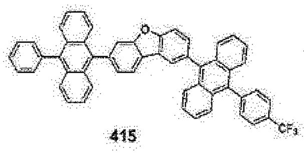
415

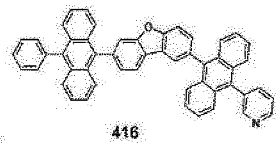
416

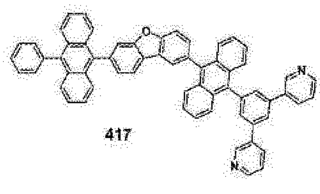
417

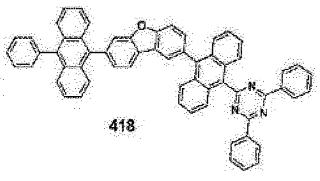
418

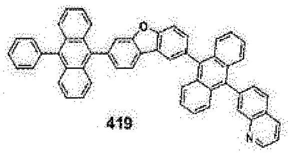
419

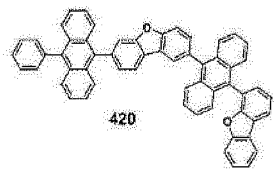
420

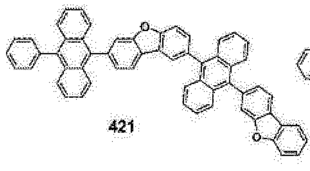

421

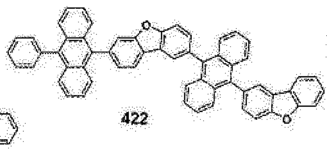

422

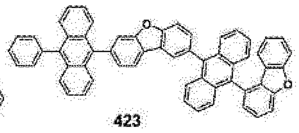

423

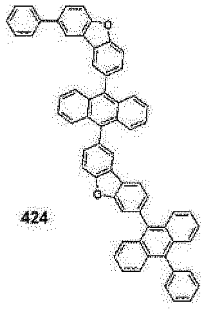

424

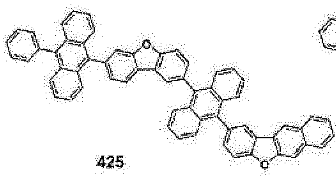

425

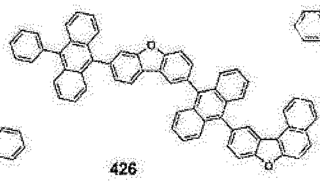

426

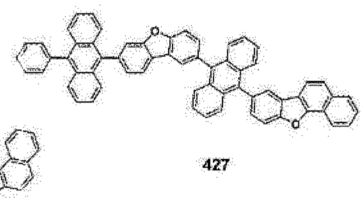

427

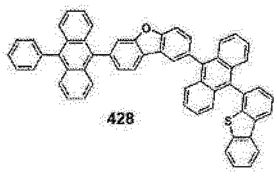

428

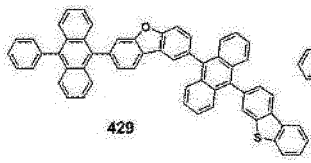

429

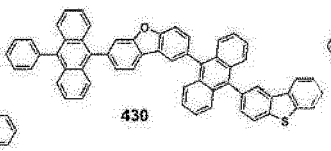

430

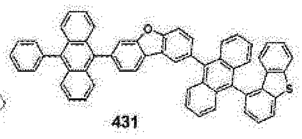

431

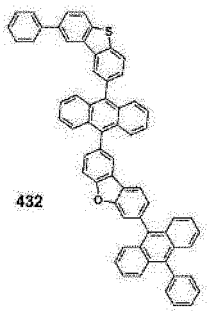

432

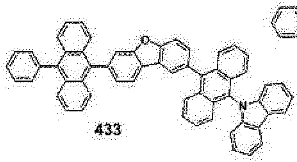

433

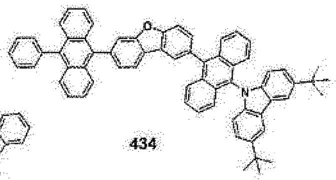

434

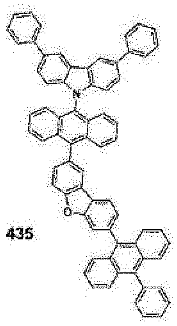

435

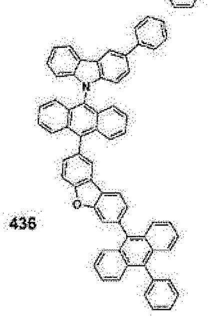

436

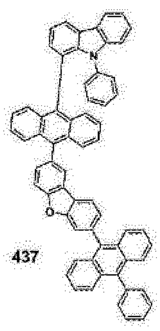

437

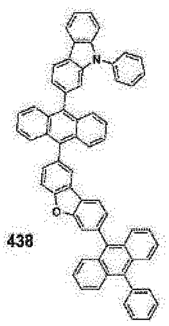

438

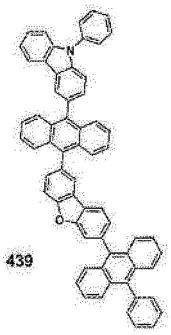

439

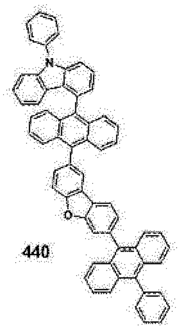

440

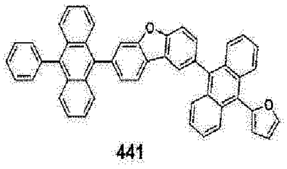
441
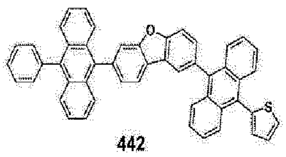
442
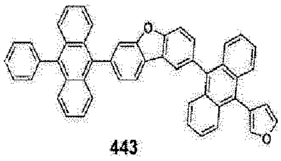
443
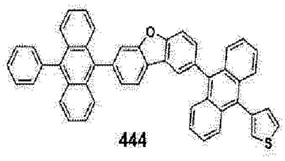
444

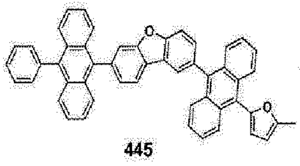
445
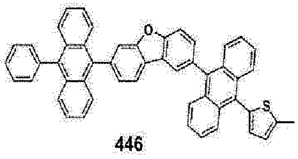
446
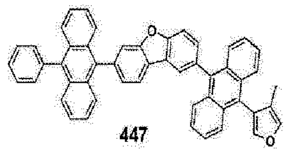
447
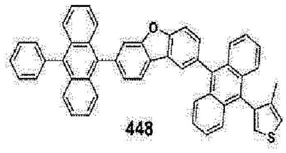
448

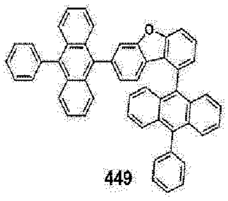
449
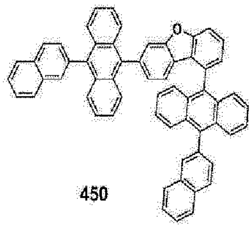
450
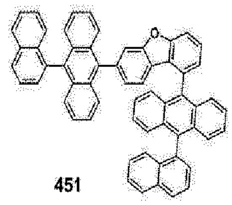
451
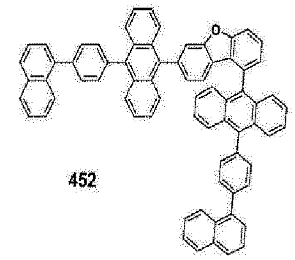
452

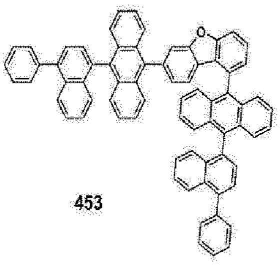
453
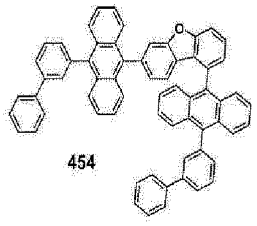
454
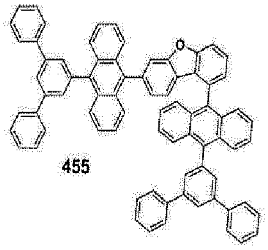
455
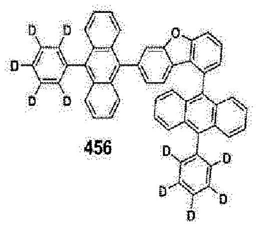
456

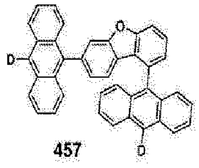
457
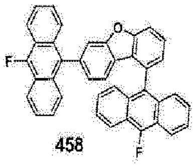
458
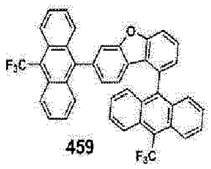
459
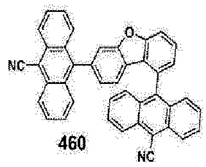
460

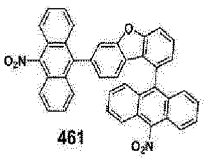
461
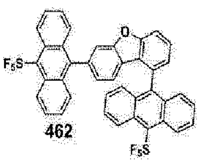
462
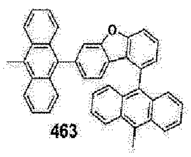
463
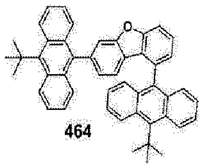
464

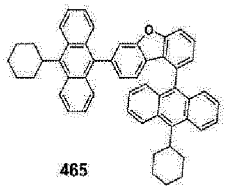
465
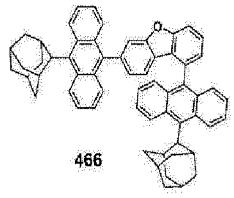
466
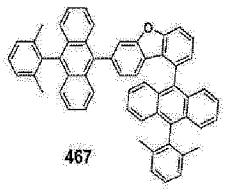
467
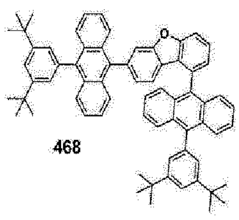
468

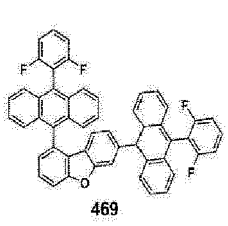
469

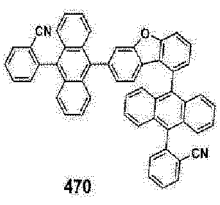
470

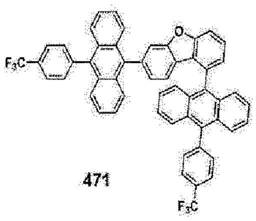
471

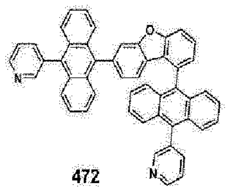
472

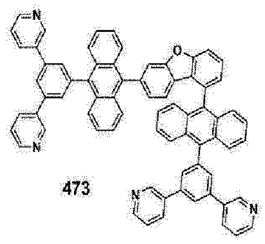
473

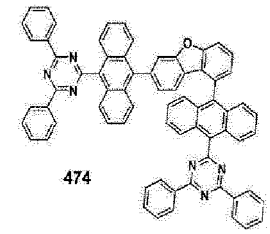
474

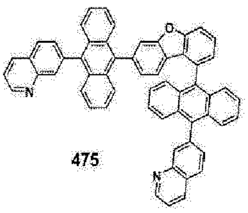
475

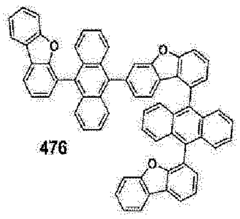
476

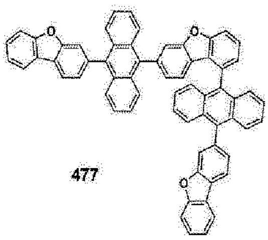
477

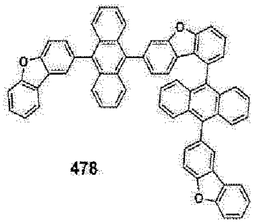
478

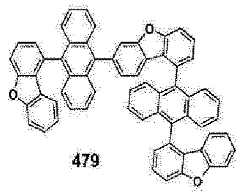
479

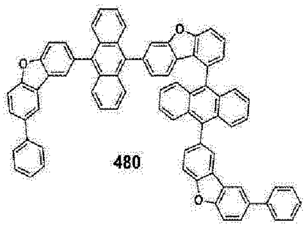
480

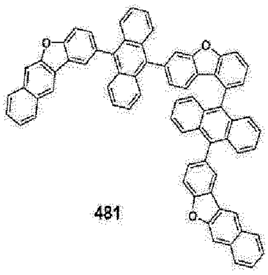
481

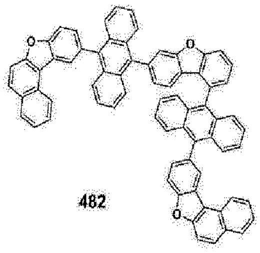
482

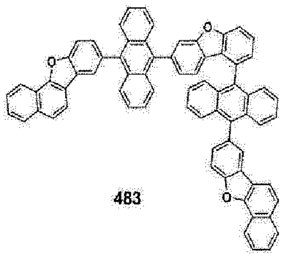
483

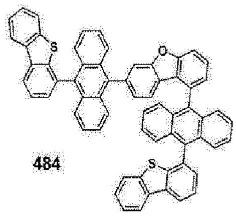
484

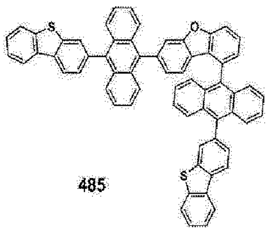
485

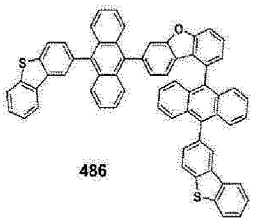
486

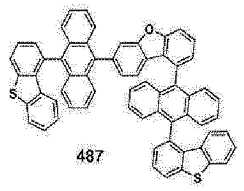
487

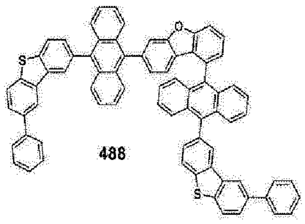
488

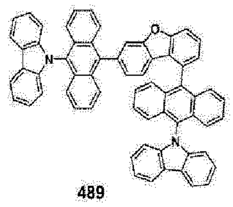
489

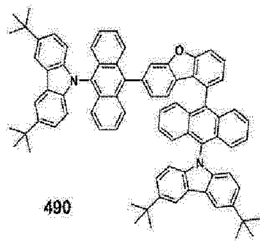
490

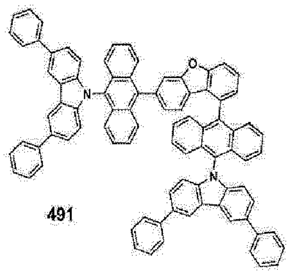
491

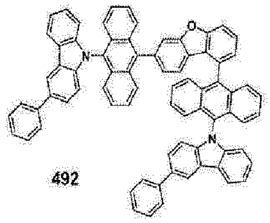
492

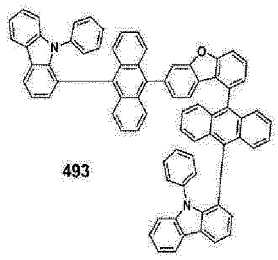
493

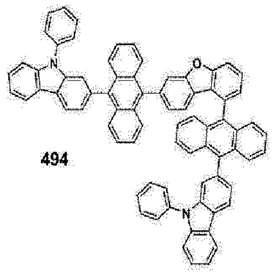
494

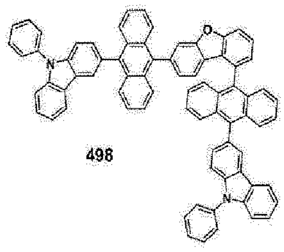
498

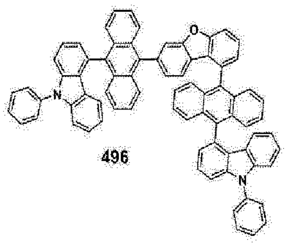
496

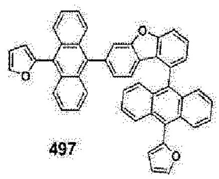
497

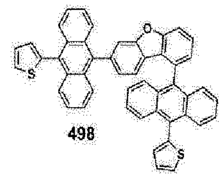
498

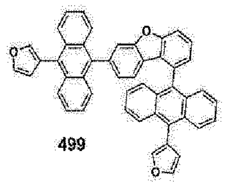
499

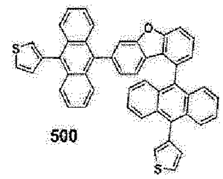
500

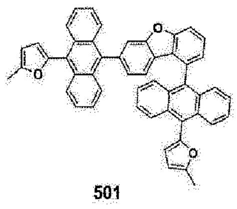
501

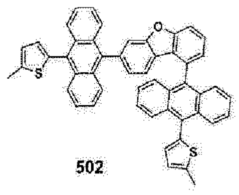
502

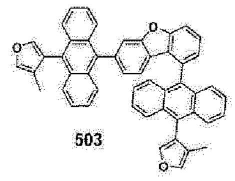
503

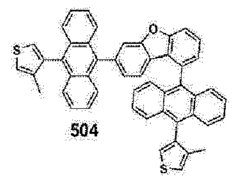
504

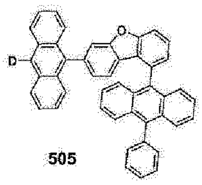
505

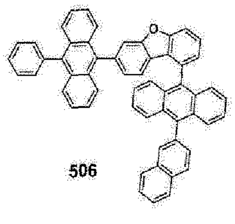
506

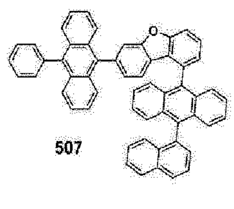
507

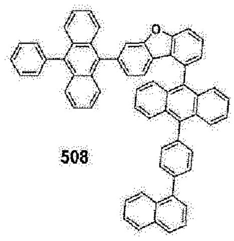
508

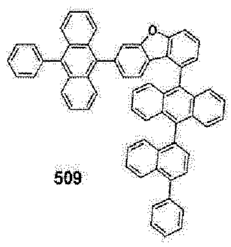
509

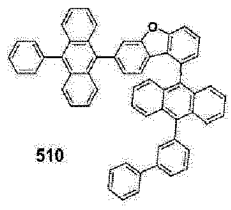
510

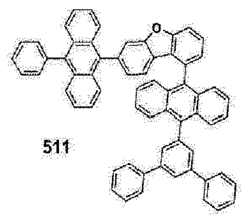
511

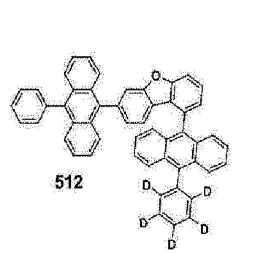
512

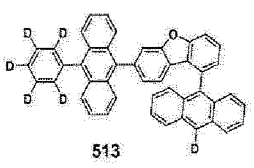
513

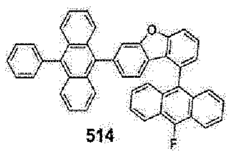
514

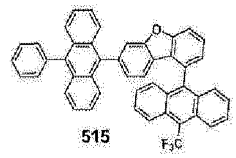
515

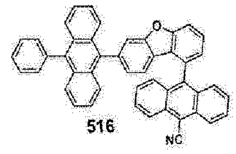
516

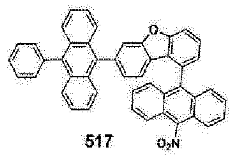
517

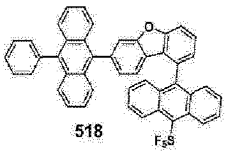
518

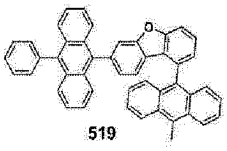
519

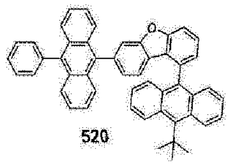
520

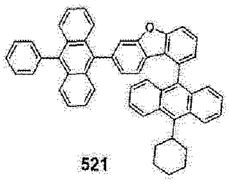
521

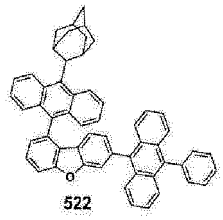
522

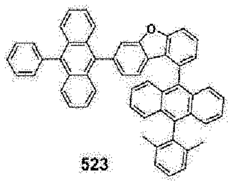
523

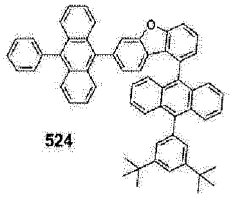
524

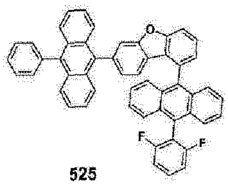
525

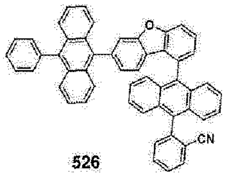
526

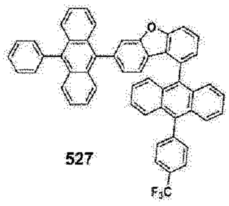
527

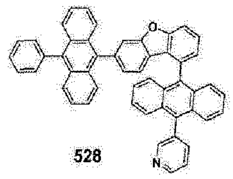
528

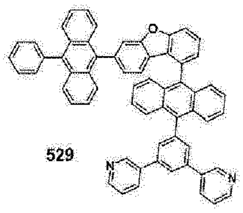
529

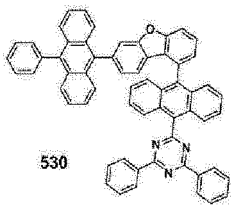
530

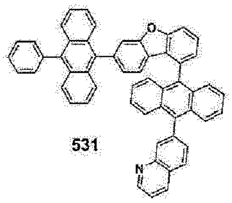
531

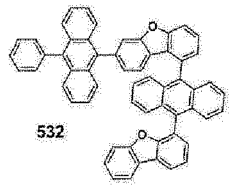
532

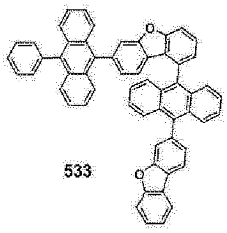
533

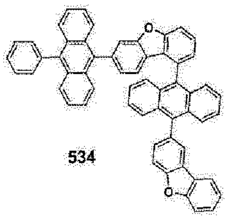
534

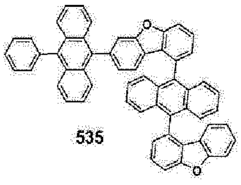
535

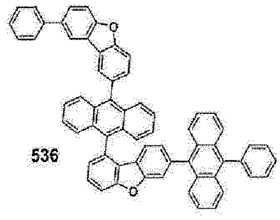
536

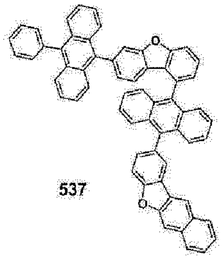
537

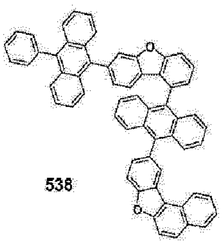
538

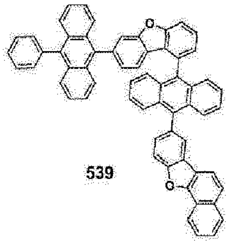
539

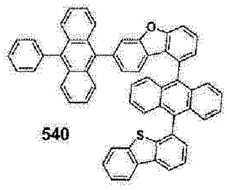
540

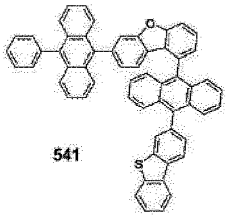
541

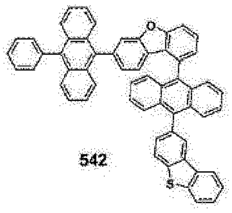
542

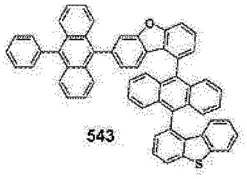
543

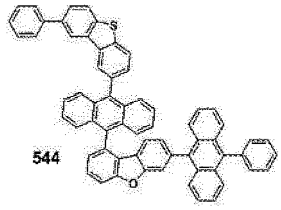
544

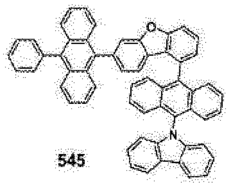
545

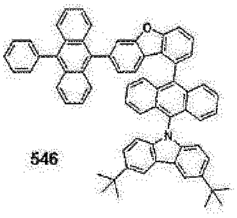
546

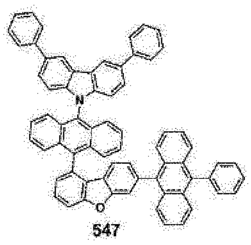
547

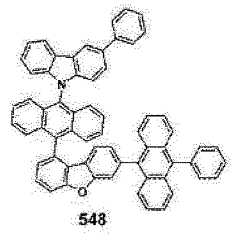
548

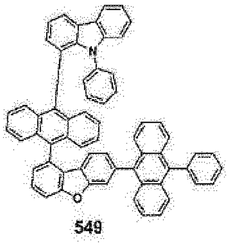
549

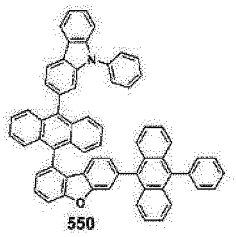
550

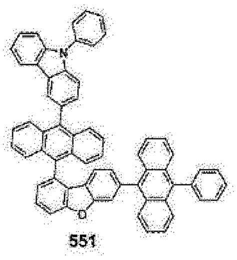
551

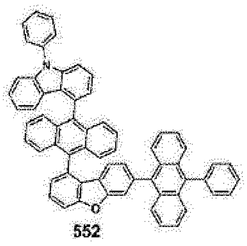
552

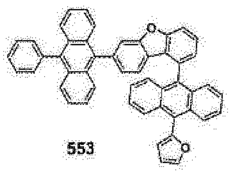
553

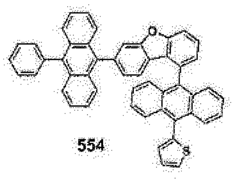
554

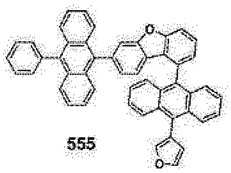
555

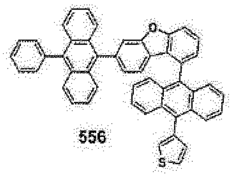
556

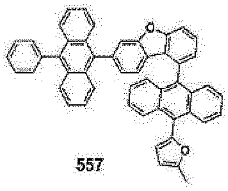
557

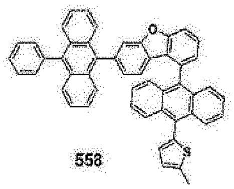
558

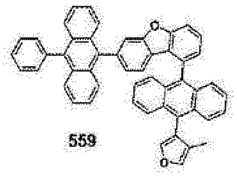
559

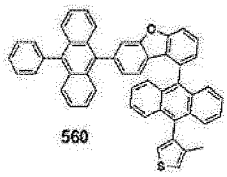
560

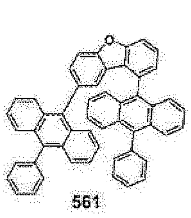
561

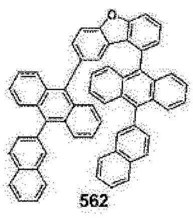
562

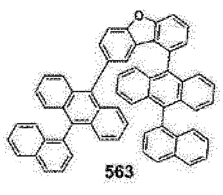
563

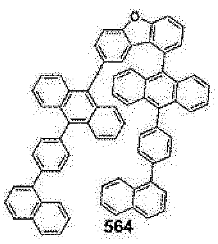
564

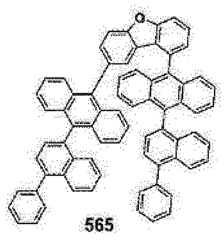
565

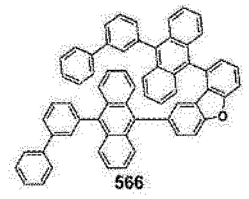
566

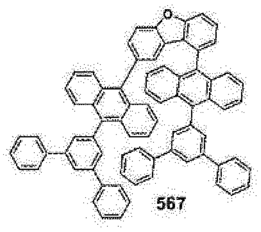
567

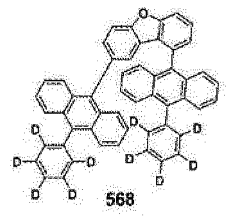
568

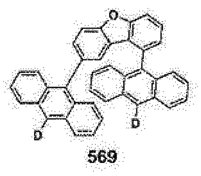
569

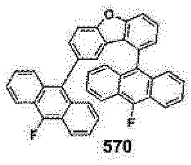
570

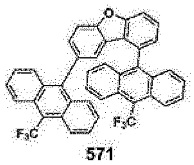
571

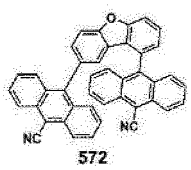
572

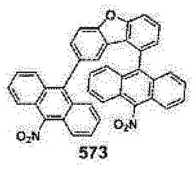
573

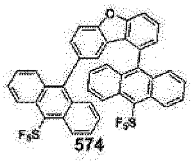
574

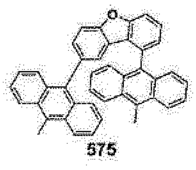
575

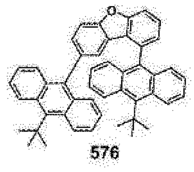
576

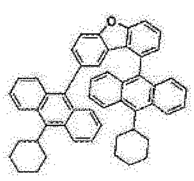
577

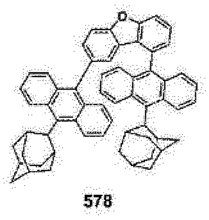
578

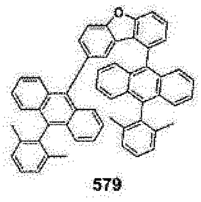
579

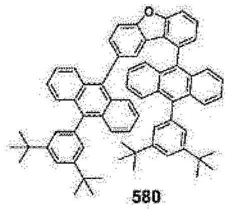
580

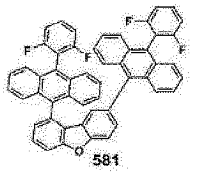
581

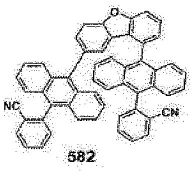
582

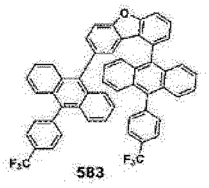
583

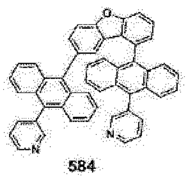
584

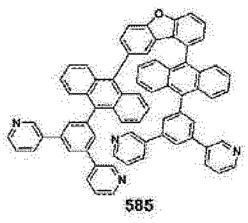
585

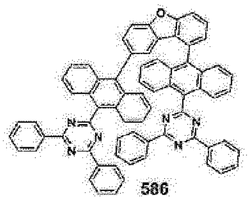
586

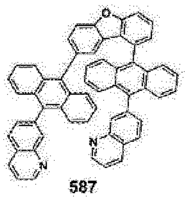
587

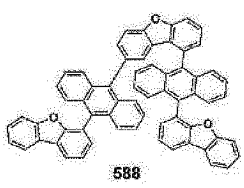
588

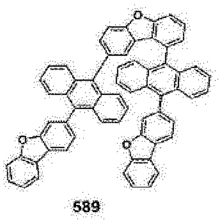
589

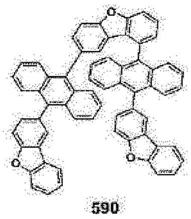
590

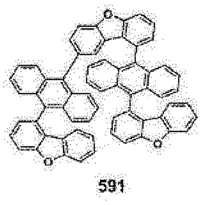
591

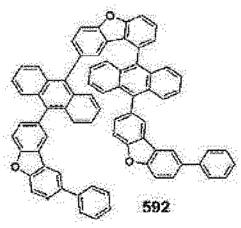
592

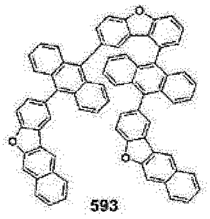
593

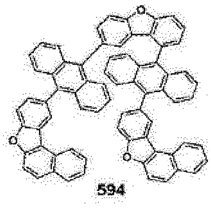
594

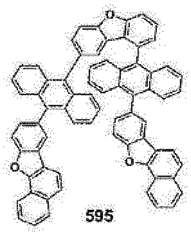
595

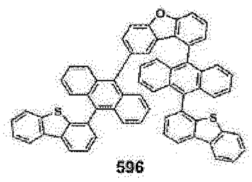
596

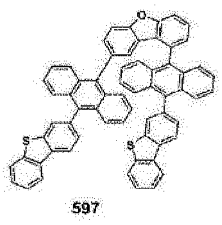
597

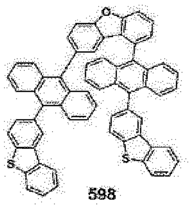
598

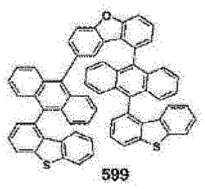
599

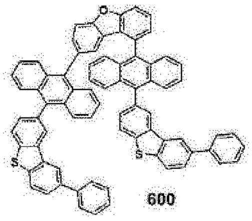
600

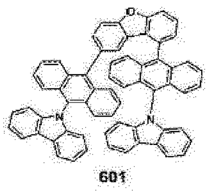
601

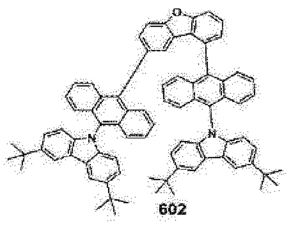
602

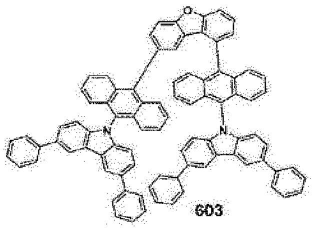
603

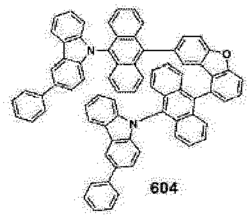
604

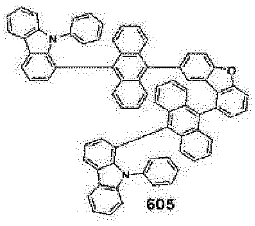
605

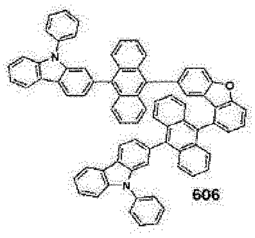
606

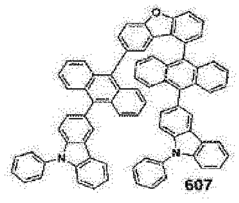
607

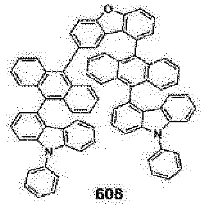
608

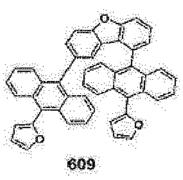
609

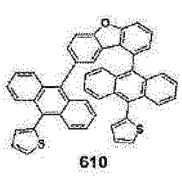
610

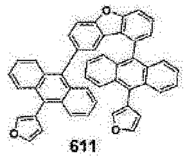
611

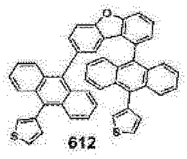
612

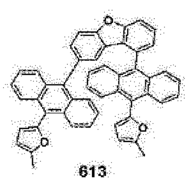
613

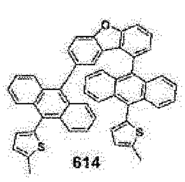
614

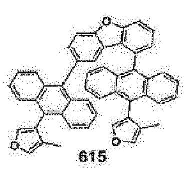
615

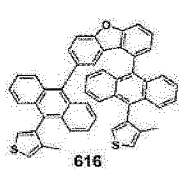
616

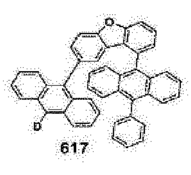
617

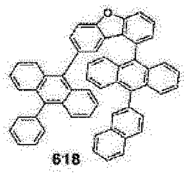
618

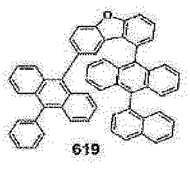
619

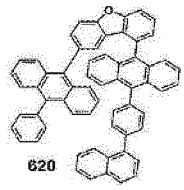
620

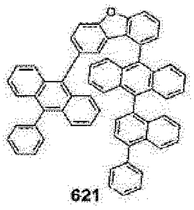
621

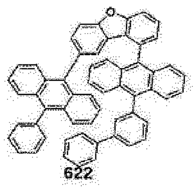
622

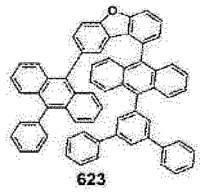
623

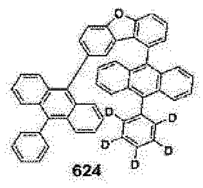
624

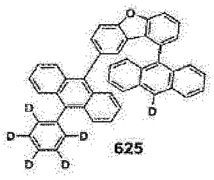
625

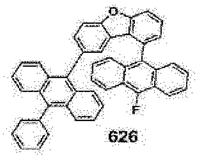
626

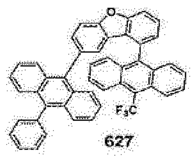
627

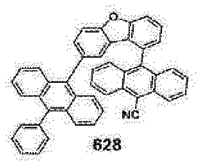
628

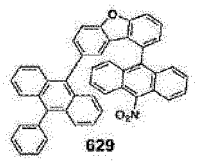
629

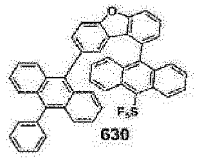
630

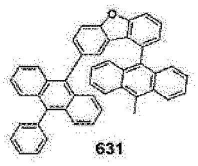
631

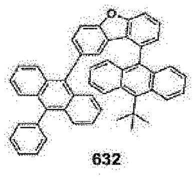
632

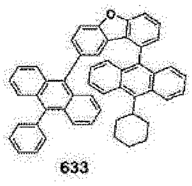
633

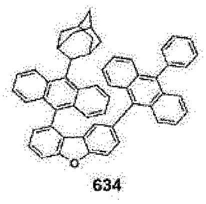
634

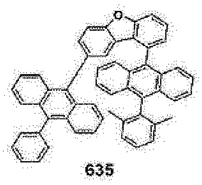
635

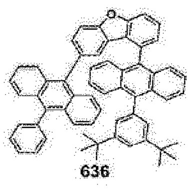
636

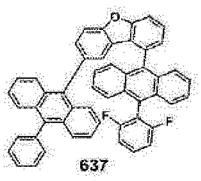
637

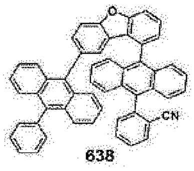
638

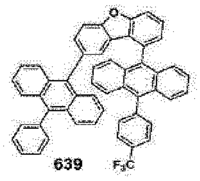
639

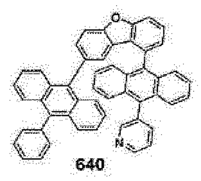
640

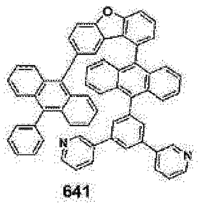
641

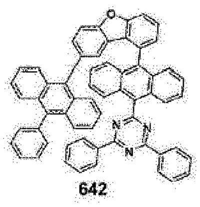
642

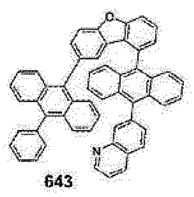
643

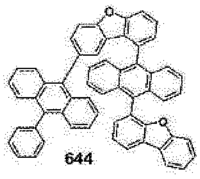
644

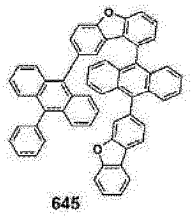
645

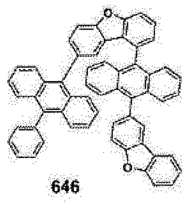
646

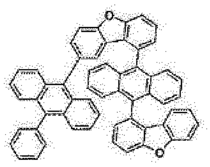
647

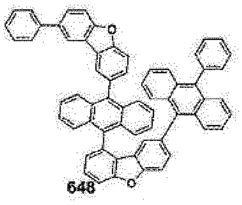
648

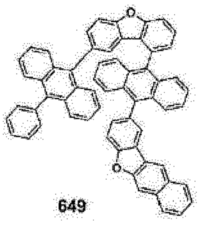
649

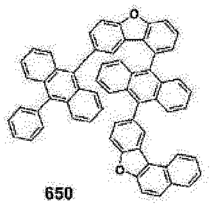
650

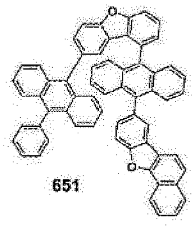
651

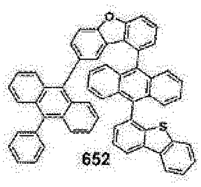
652

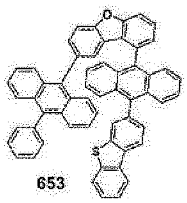
653

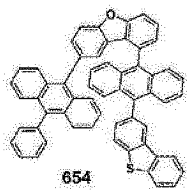
654

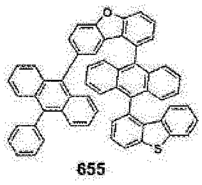
655

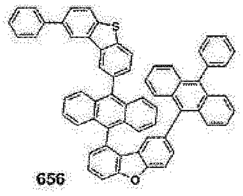
656

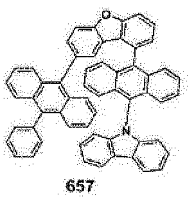
657

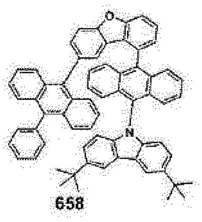
658

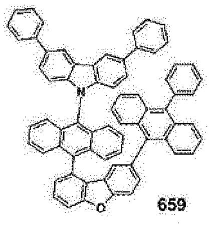
659

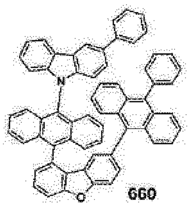
660

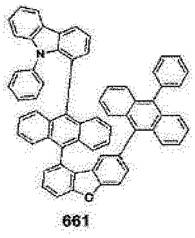
661

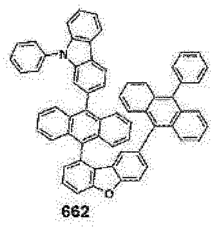
662

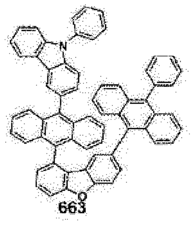
663

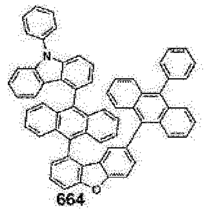
664

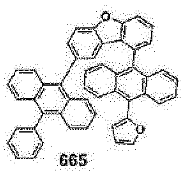
665

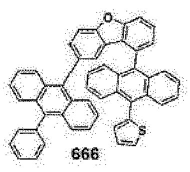
666

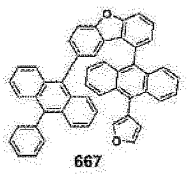
667

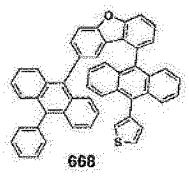
668

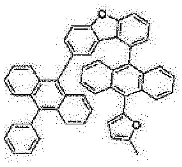
669

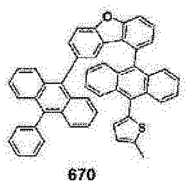
670

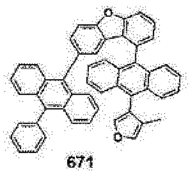
671

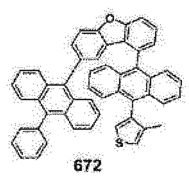
672

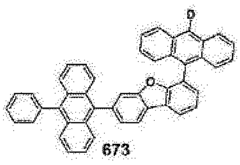
673
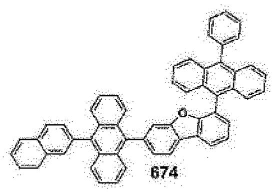
674
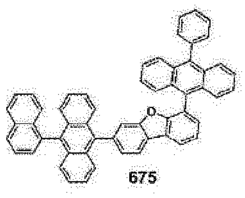
675
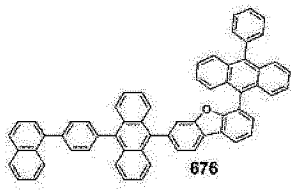
676

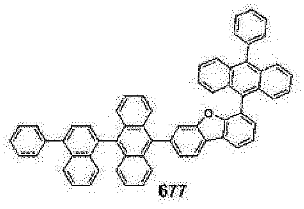
677
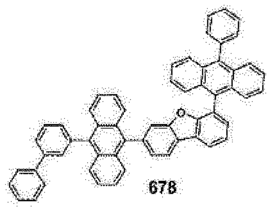
678
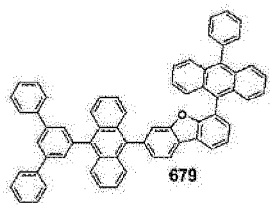
679
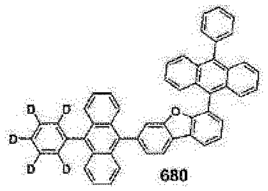
680

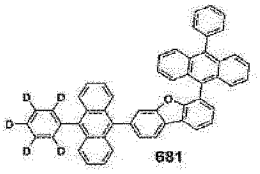
681
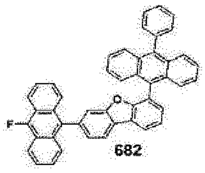
682
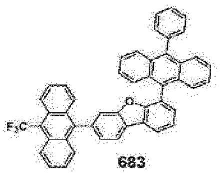
683
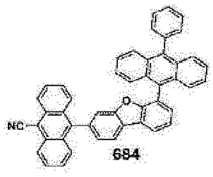
684

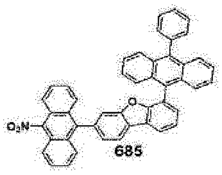
685
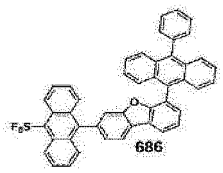
686
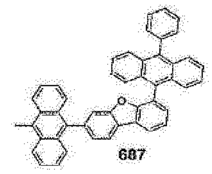
687
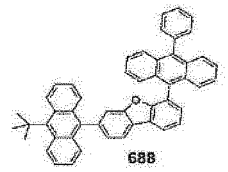
688

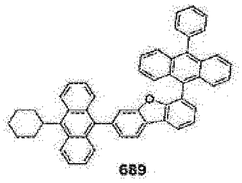
689
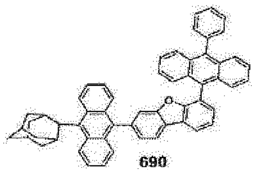
690
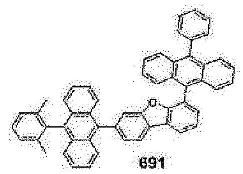
691
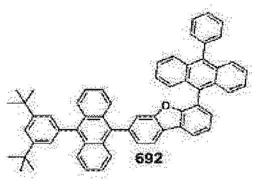
692

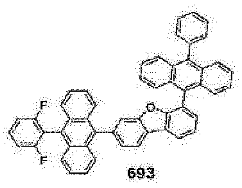
693
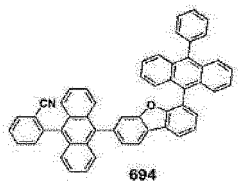
694
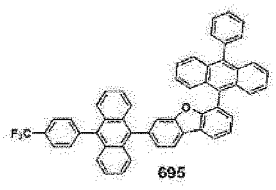
695
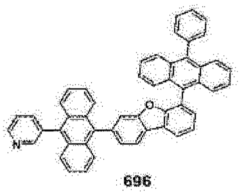
696

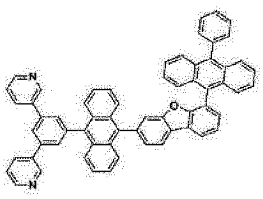
697
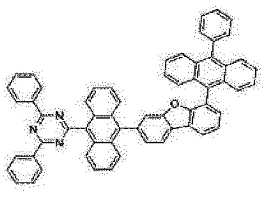
698
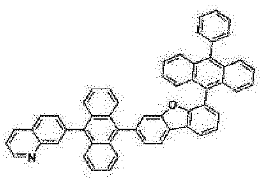
699
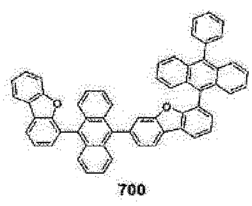
700

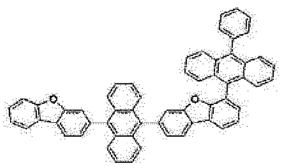
701

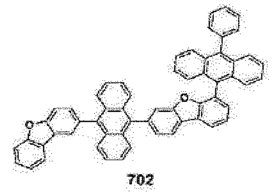
702

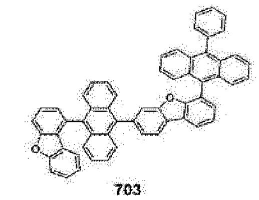
703

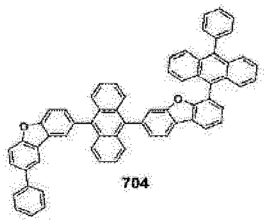
704

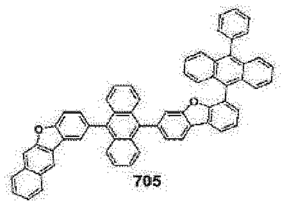
705

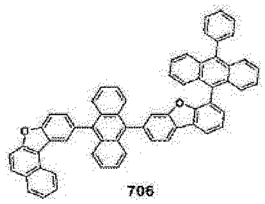
706

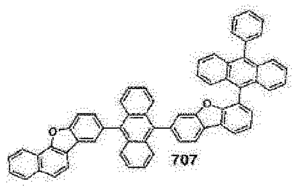
707

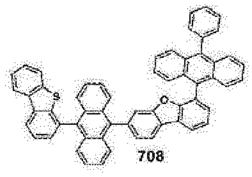
708

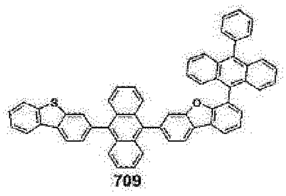
709

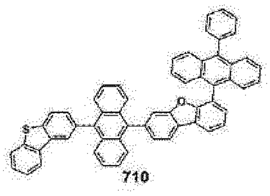
710

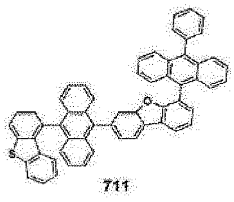
711

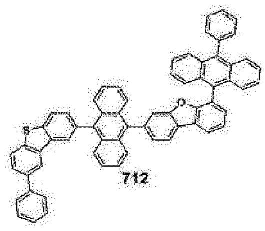
712

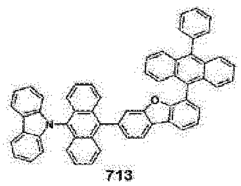
713

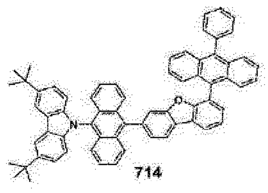
714

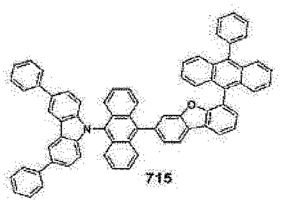
715

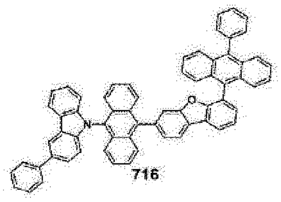
716

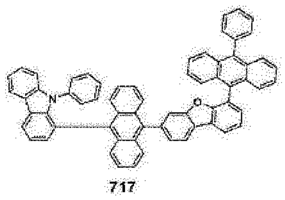
717

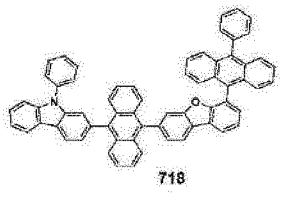
718

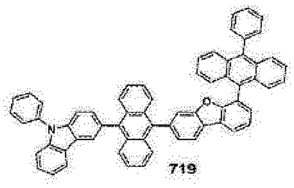
719

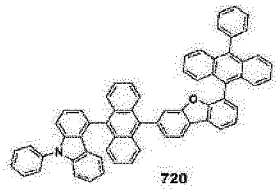
720

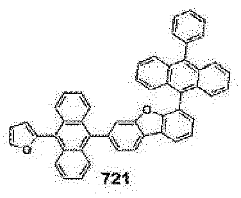
721

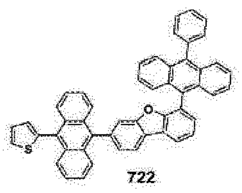
722

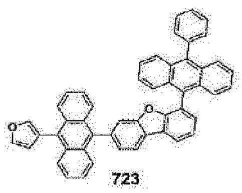
723

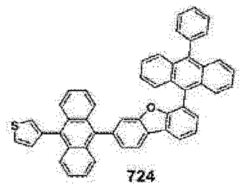
724

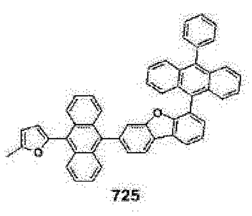
725

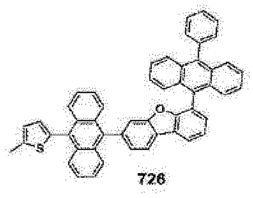
726

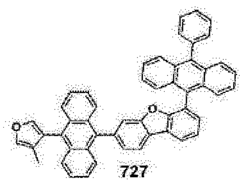
727

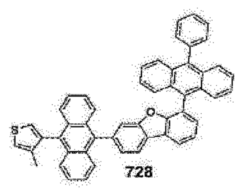
728

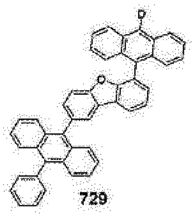
729

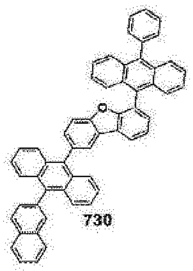
730

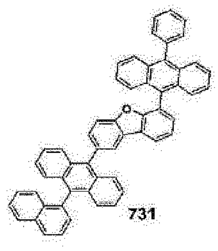
731

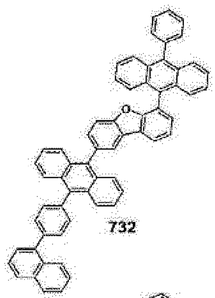
732

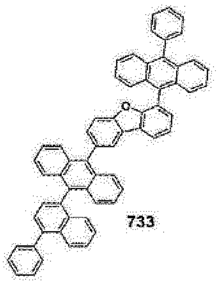
733

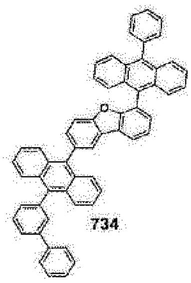
734

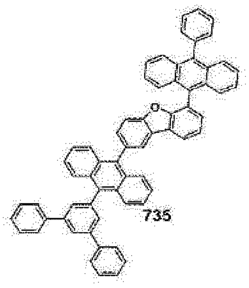
735

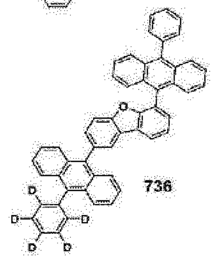
736

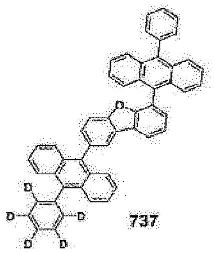
737

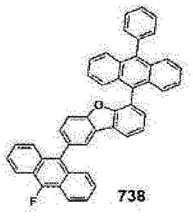
738

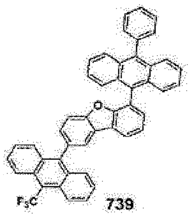
739

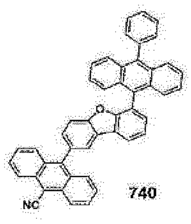
740

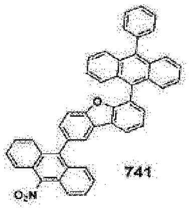
741

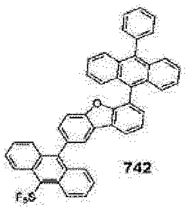
742

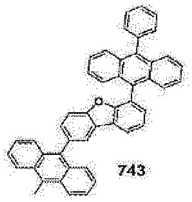
743

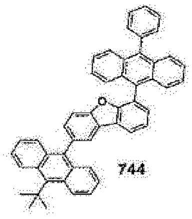
744

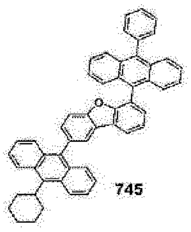
745

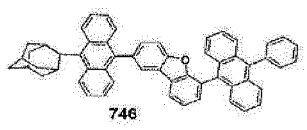
746

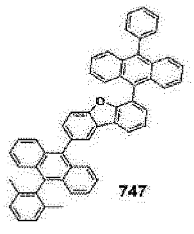
747

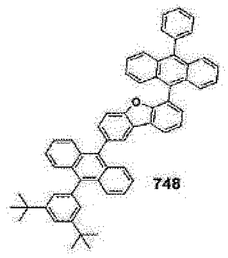
748

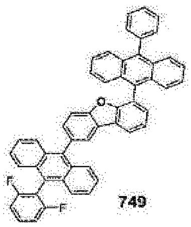
749

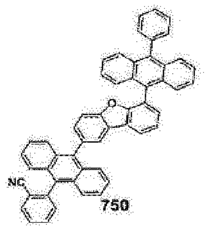
750

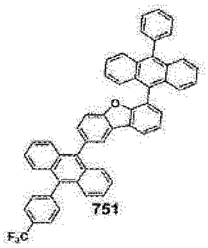
751

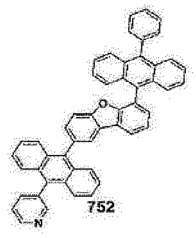
752

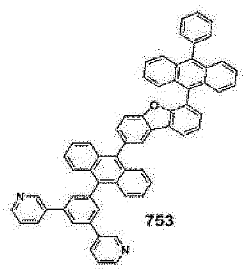
753

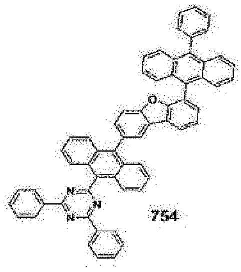
754

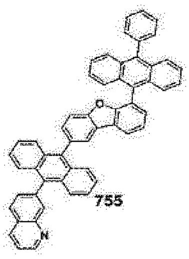
755

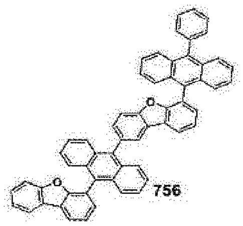
756

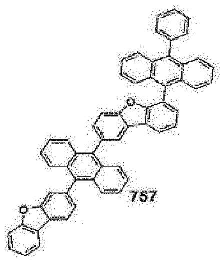
757

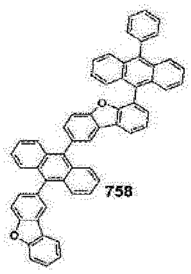
758

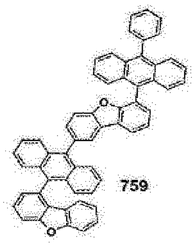
759

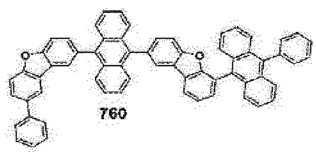
760

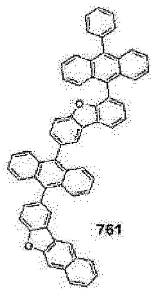
761

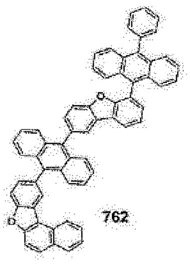
762

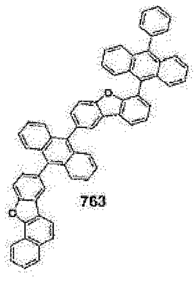
763

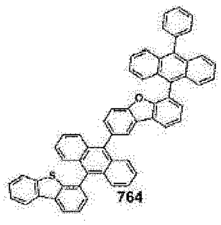
764

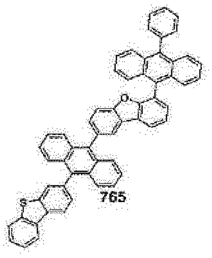
765

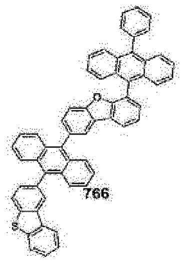
766

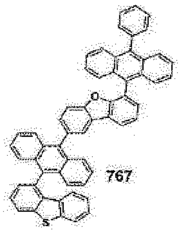
767

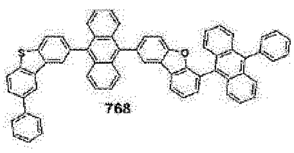
768

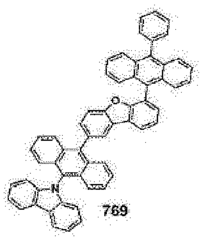
769

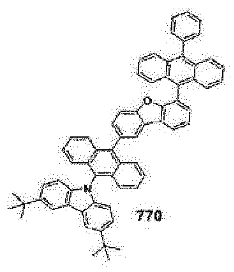
770

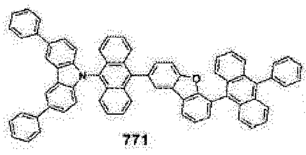
771

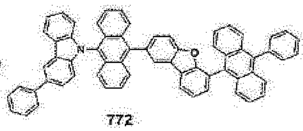
772

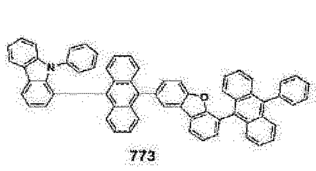
773

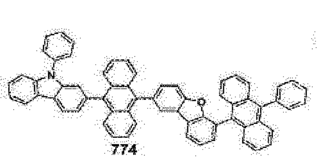
774

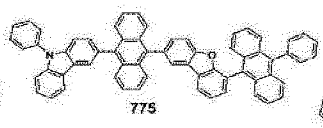
775

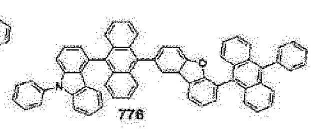
776

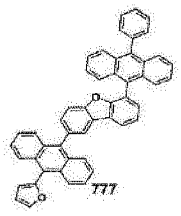
777

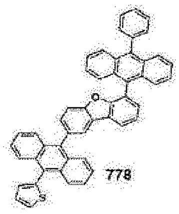
778

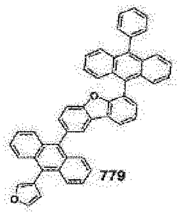
779

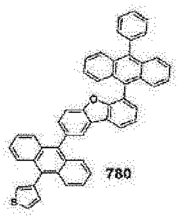
780

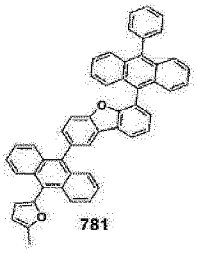
781

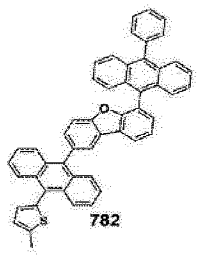
782

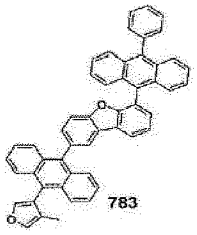
783

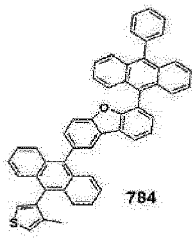
784

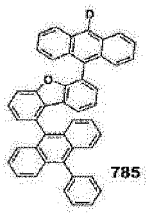
785

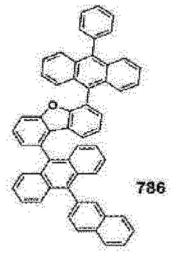
786

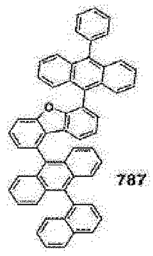
787

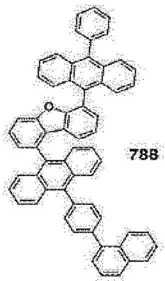
788

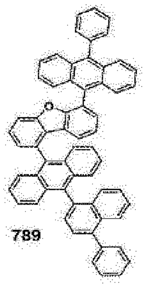
789

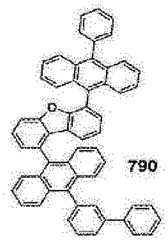
790

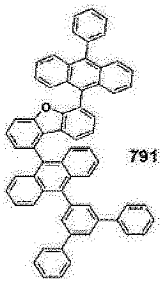
791

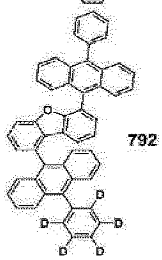
792

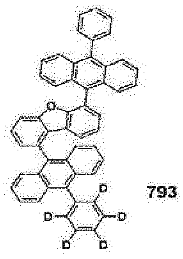
793

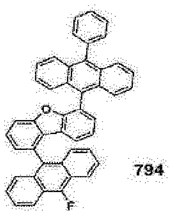
794

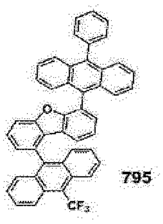
795

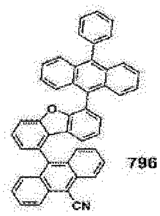
796

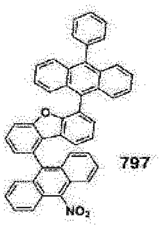
797

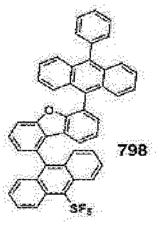
798

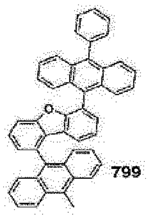
799

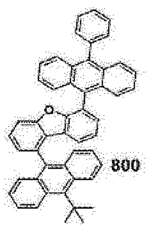
800

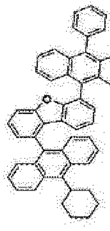
801

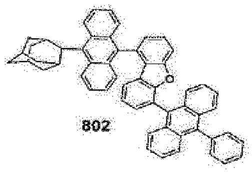
802

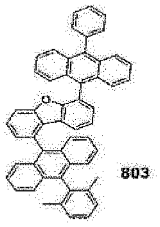
803

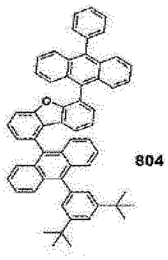
804

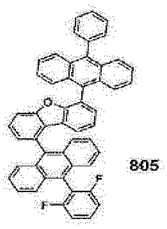
805

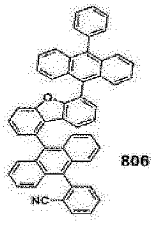
806

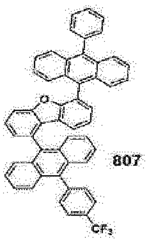
807

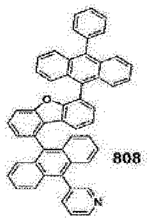
808

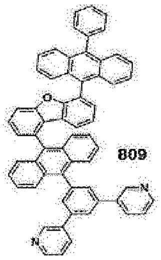
809

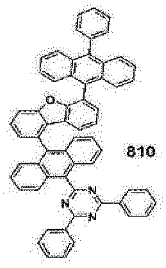
810

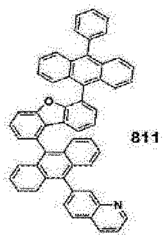
811

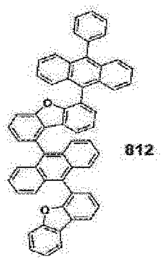
812

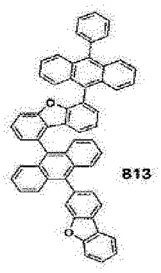
813

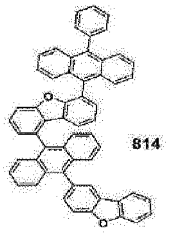
814

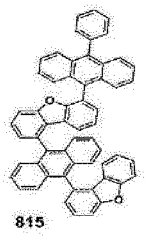
815

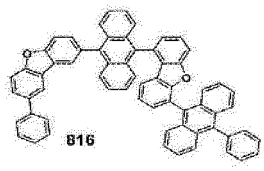
816

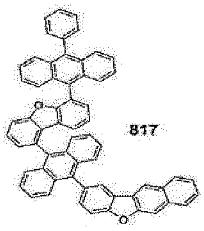
817

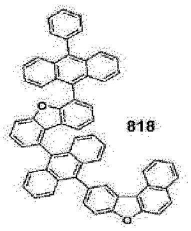
818

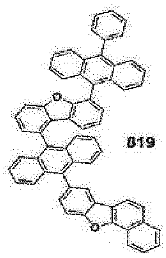
819

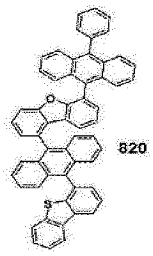
820

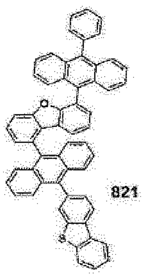
821

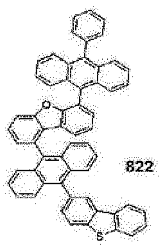
822

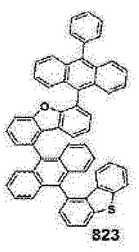
823

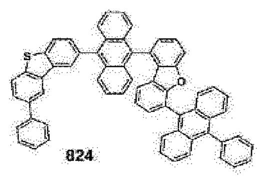
824

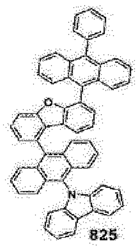
825

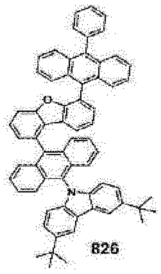
826

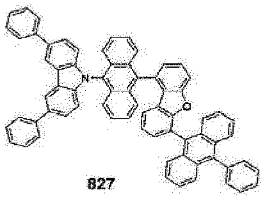
827

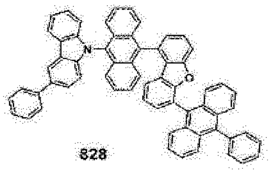
828

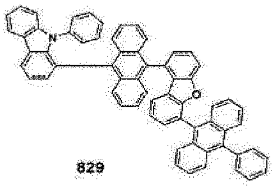
829

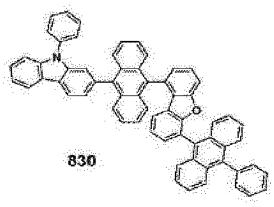
830

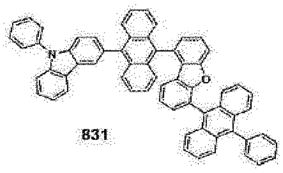
831

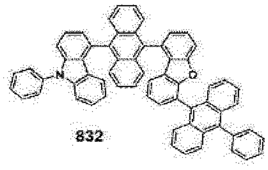
832

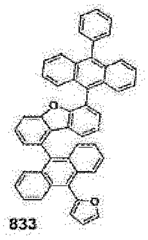
833

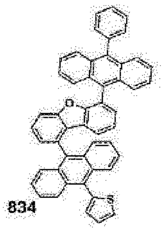
834

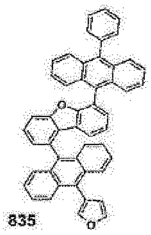
835

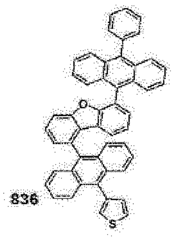
836

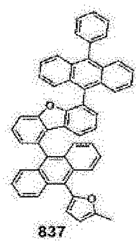
837

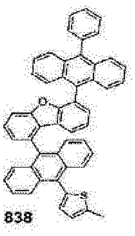
838

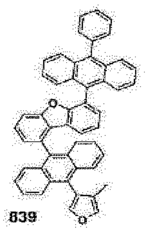
839

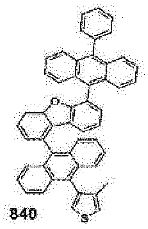
840

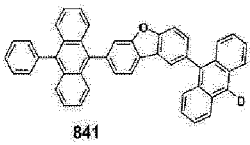
841

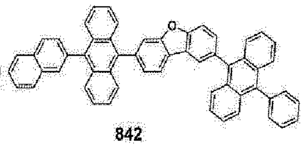
842

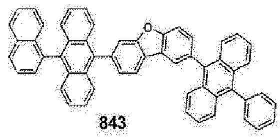
843

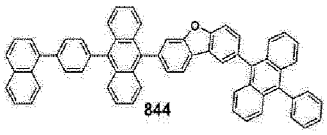
844

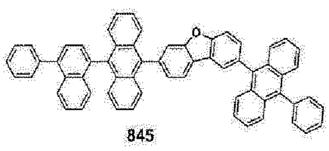
845

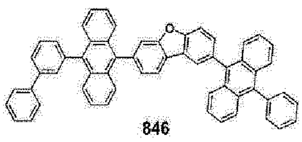
846

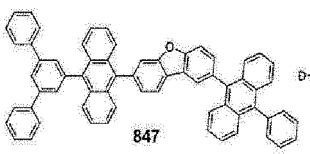
847

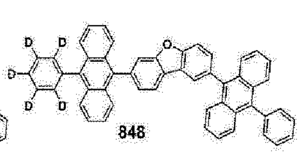
848

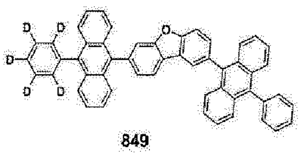
849

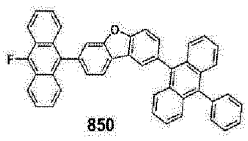
850

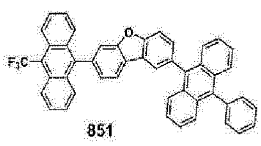
851

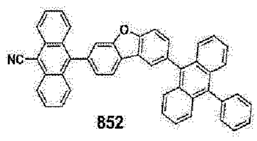
852

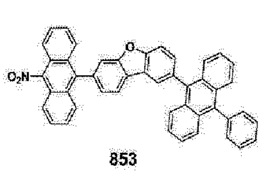
853

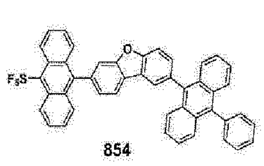
854

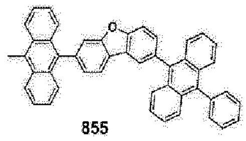
855

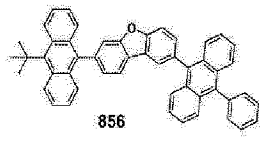
856

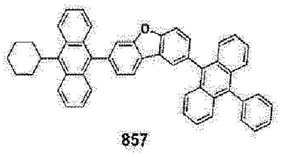
857

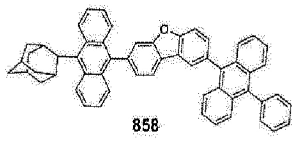
858

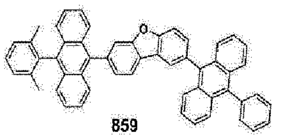
859

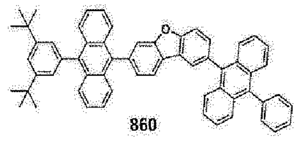
860

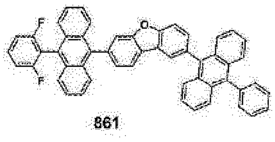
861

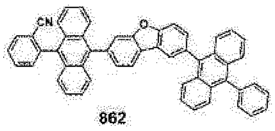
862

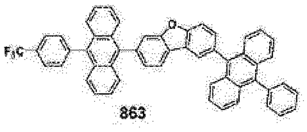
863

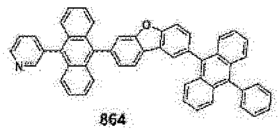
864

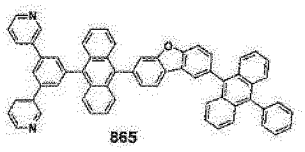
865

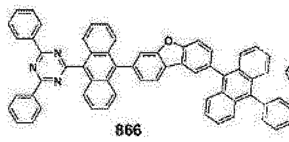
866

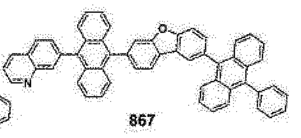
867

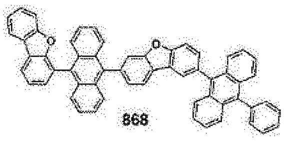
868

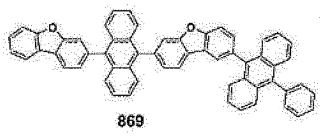
869

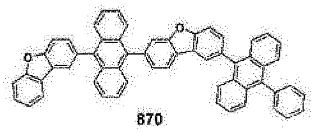
870

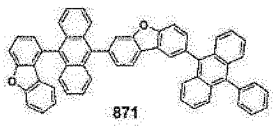
871

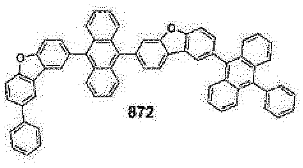
872

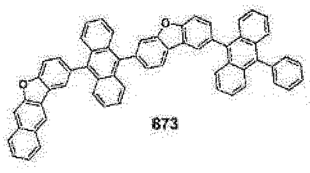
873

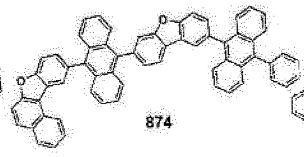
874

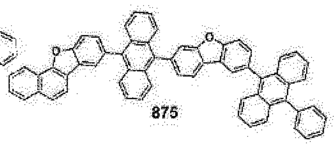
875

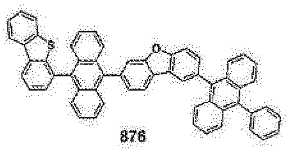
876

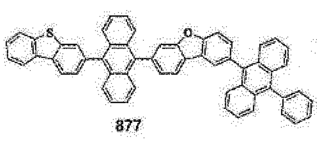
877

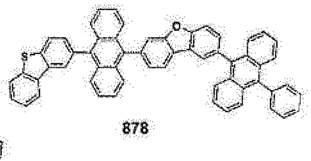
878

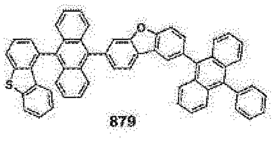
879

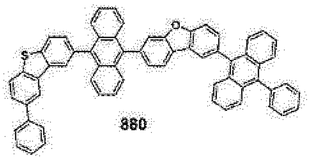
880

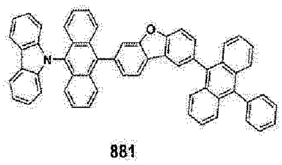
881

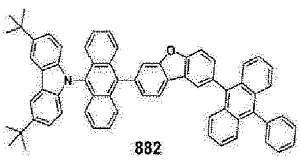
882

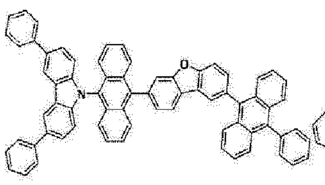
883

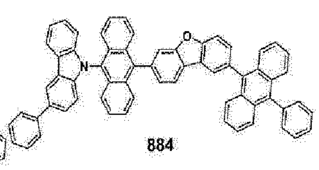
884

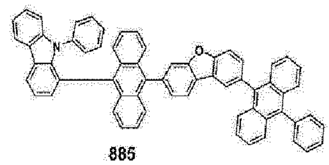

885

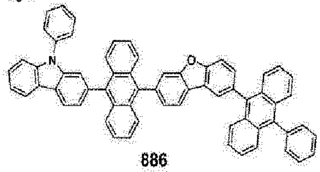

886

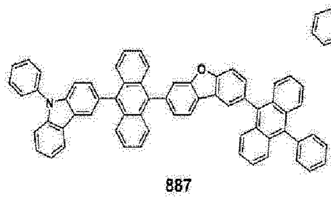

887

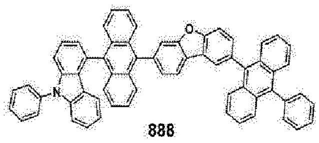

888

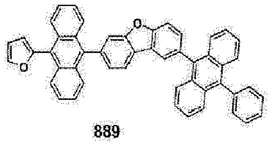

889

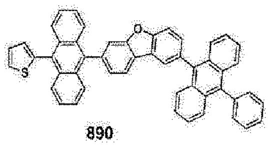

890

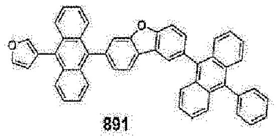

891

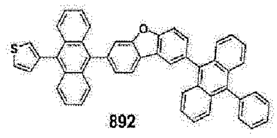

892

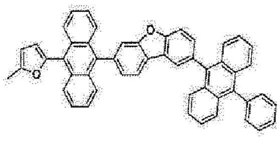

893

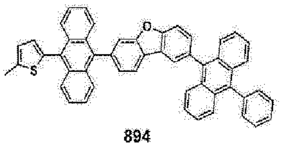

894

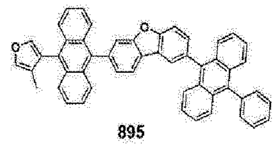

895

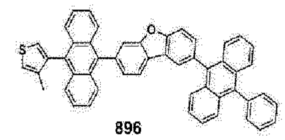

896

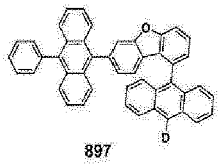

897

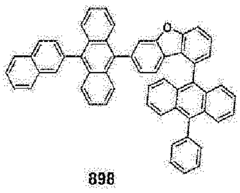

898

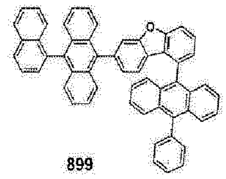

899

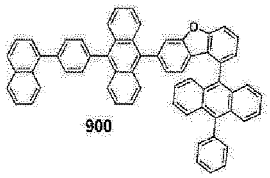

900

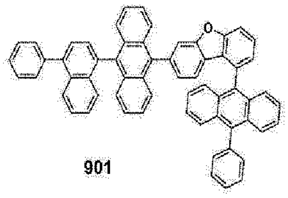

901

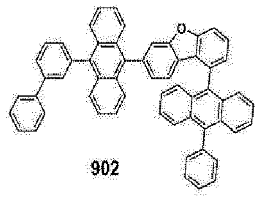

902

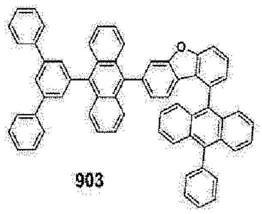

903

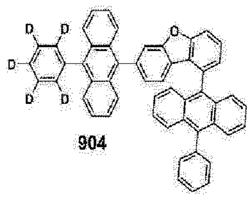

904

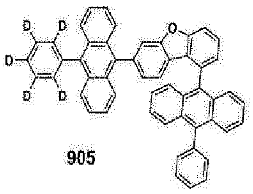

905

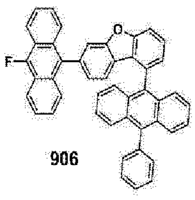

906

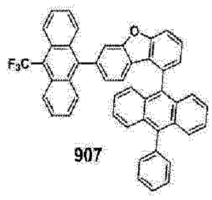

907

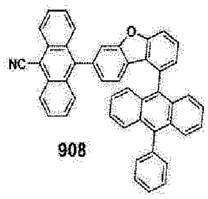

908

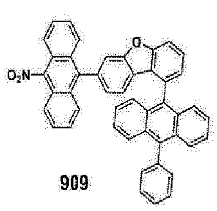

909

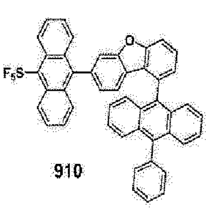

910

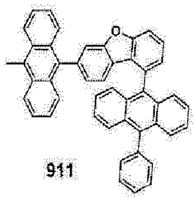

911

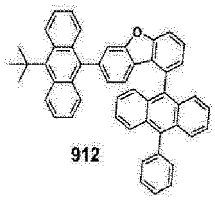

912

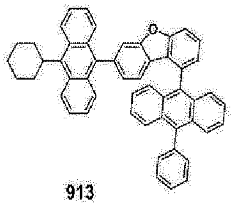
913

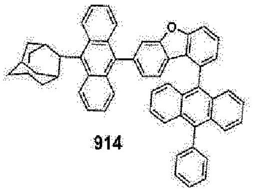
914

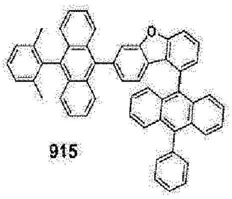
915

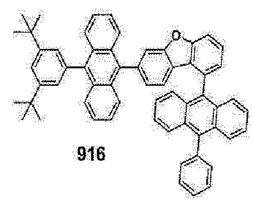
916

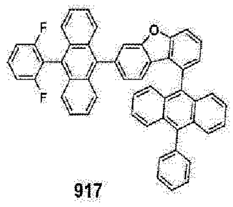
917

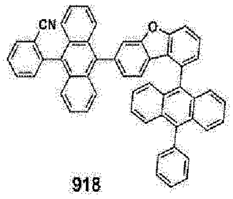
918

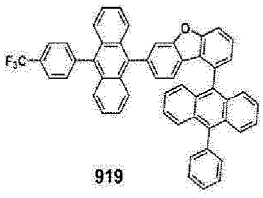
919

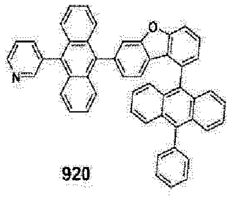
920

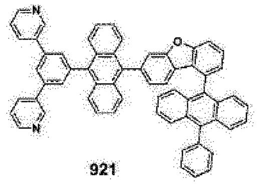
921

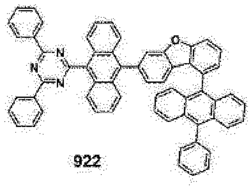
922

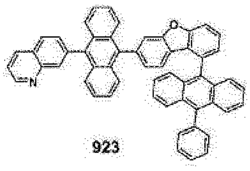
923

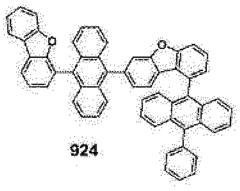
924

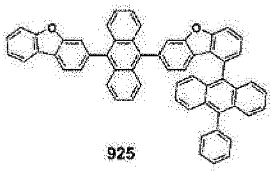
925

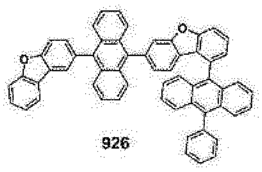
926

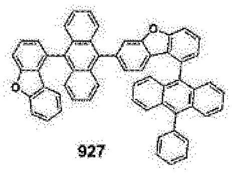
927

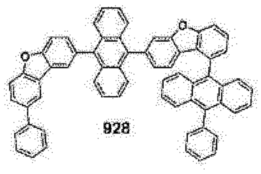
928

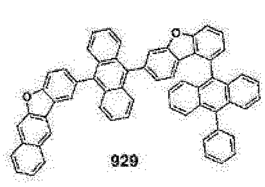
929

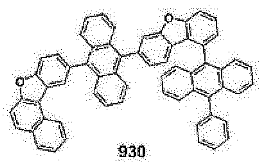
930

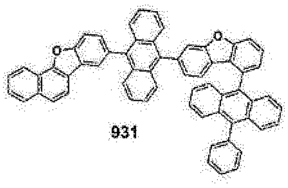
931

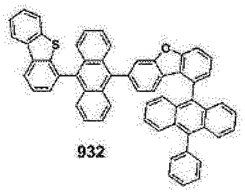
932

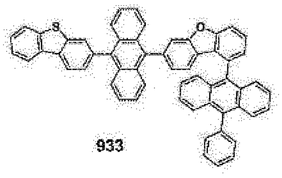
933

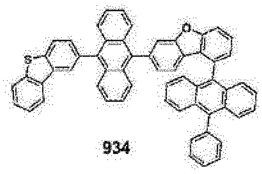
934

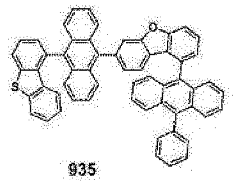
935

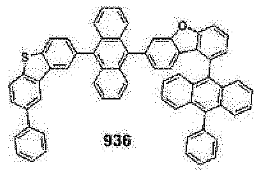
936

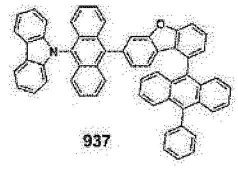
937

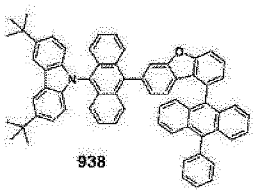
938

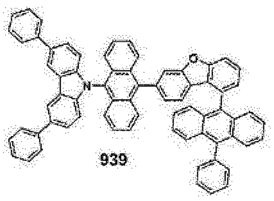
939

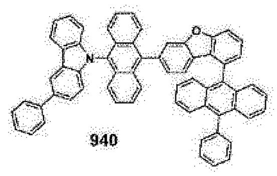
940

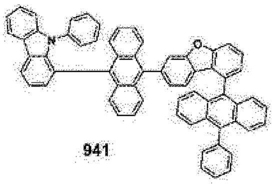
941

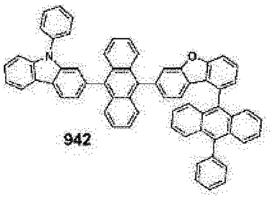
942

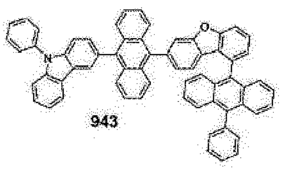
943

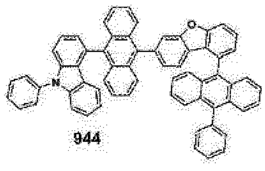
944

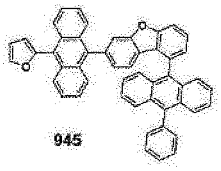
945

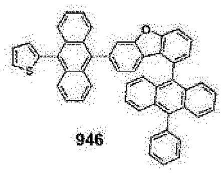
946

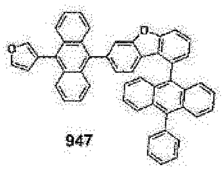
947

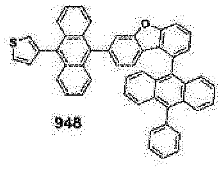
948

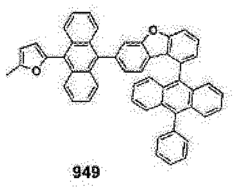
949

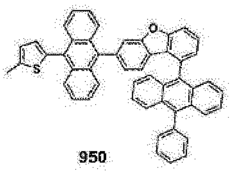
950

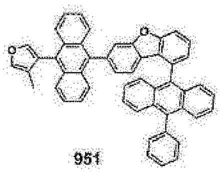
951

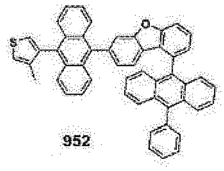
952

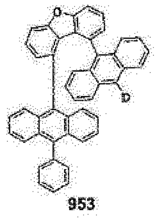
953

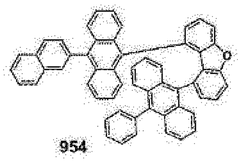
954

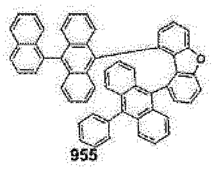
955

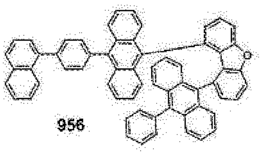
956

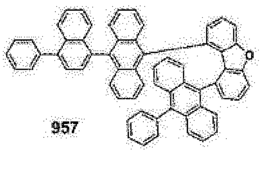
957

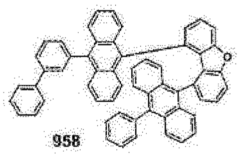
958

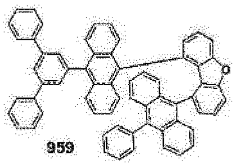
959

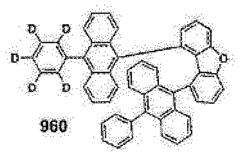
960

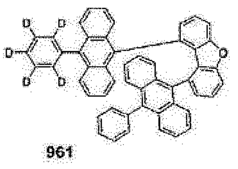
961

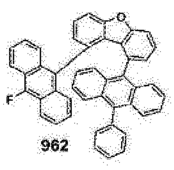
962

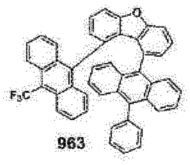
963

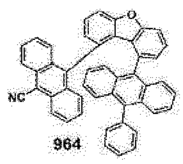
964

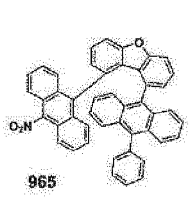
965

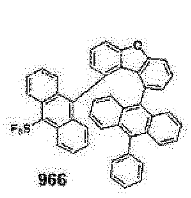
966

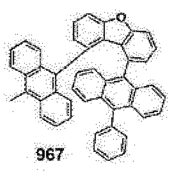
967

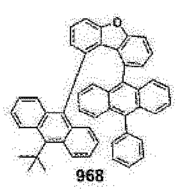
968

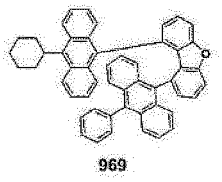
969
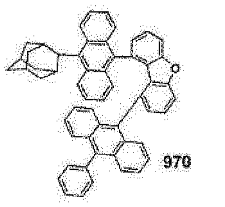
970
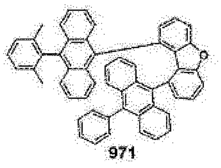
971
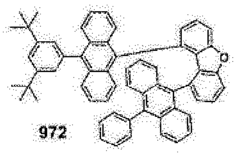
972

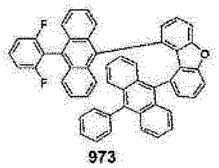
973
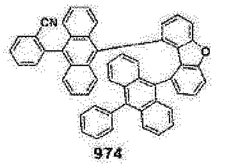
974
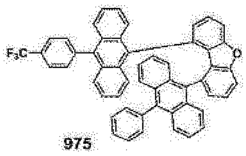
975
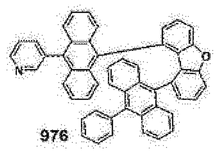
976

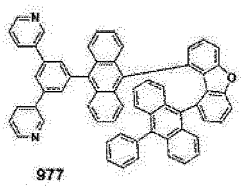
977
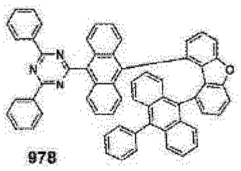
978
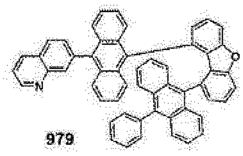
979
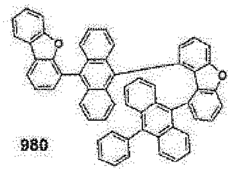
980

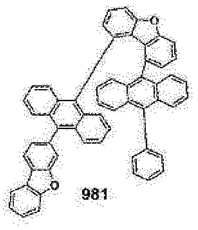
981
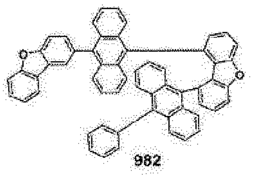
982
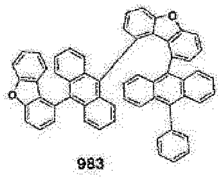
983
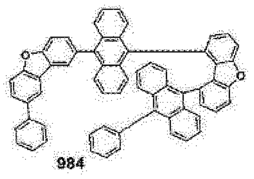
984

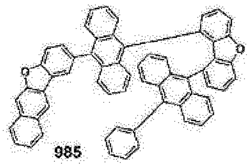
985
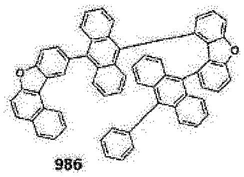
986
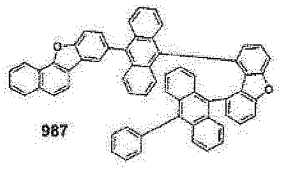
987
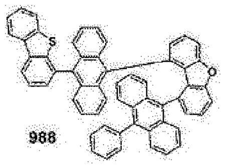
988

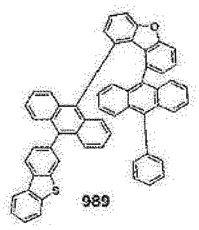
989
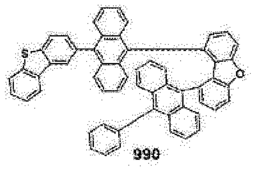
990
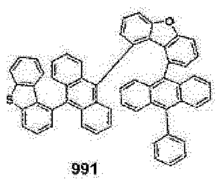
991
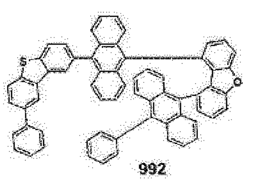
992

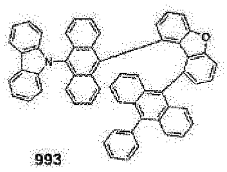
993
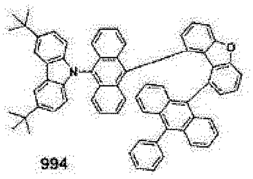
994
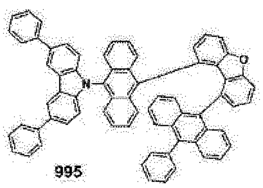
995
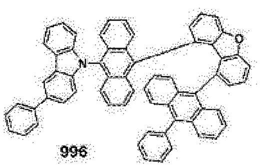
996

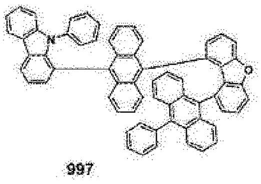
997

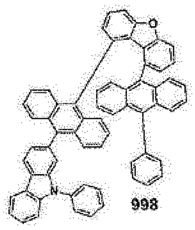
998

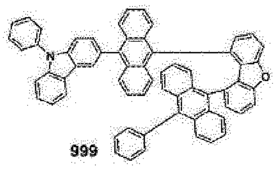
999

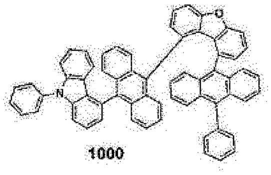
1000

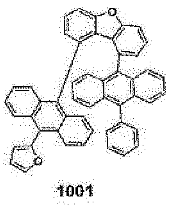
1001

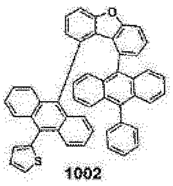
1002

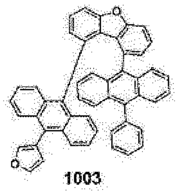
1003

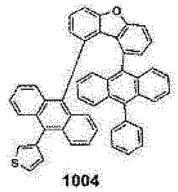
1004

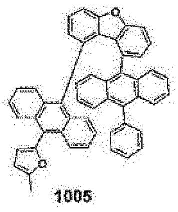
1005

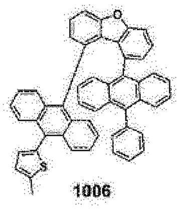
1006

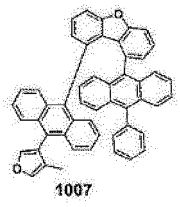
1007

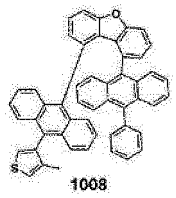
1008

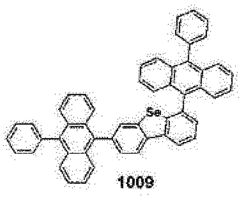
1009

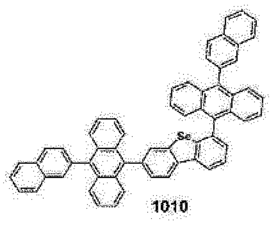
1010

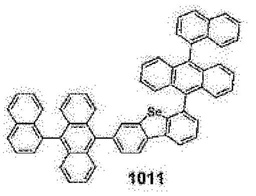
1011

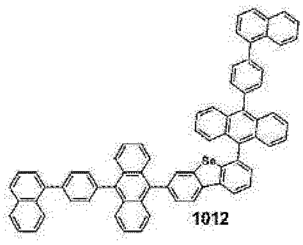
1012

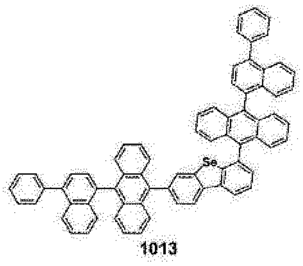
1013

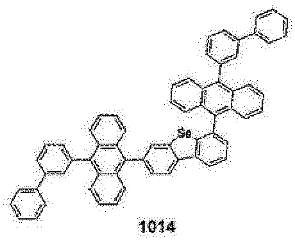
1014

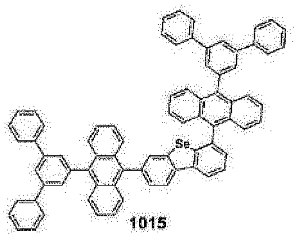
1015

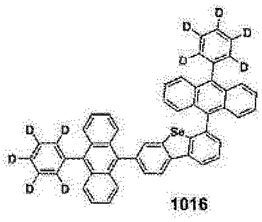
1016

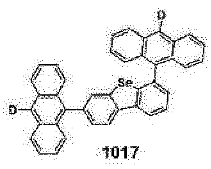
1017

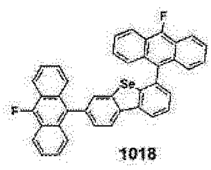
1018

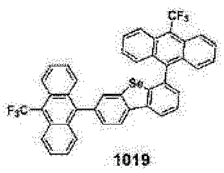
1019

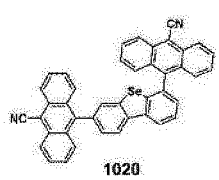
1020

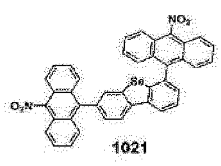
1021

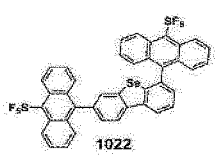
1022

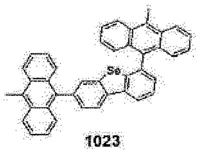
1023

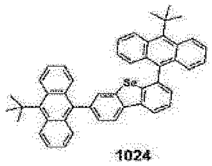
1024

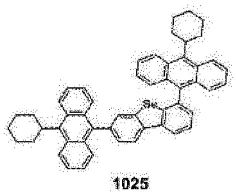
1025

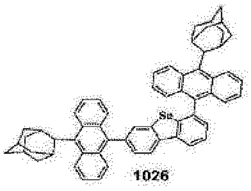
1026

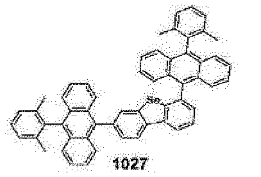
1027

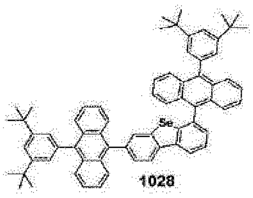
1028

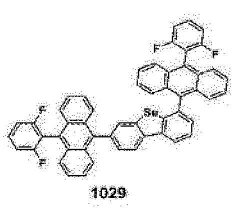
1029

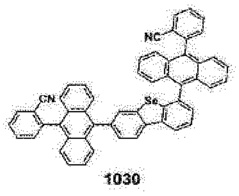
1030

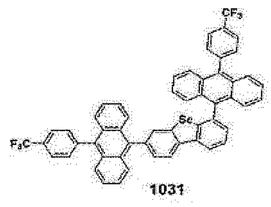
1031

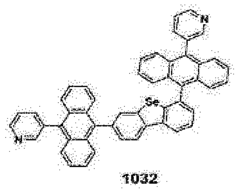
1032

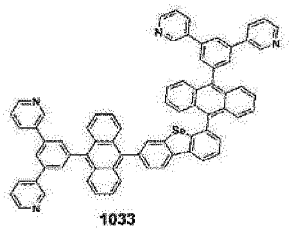
1033

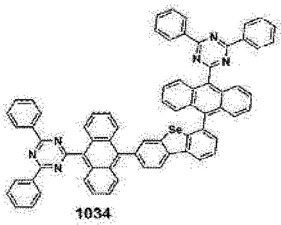
1034

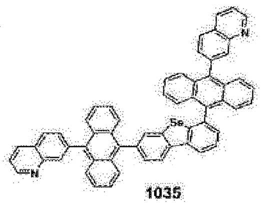
1035

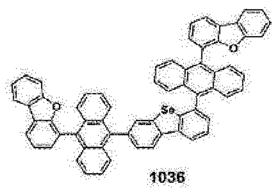
1036

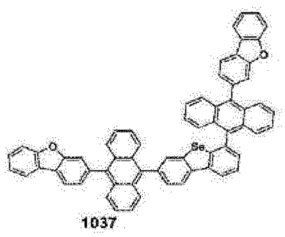
1037

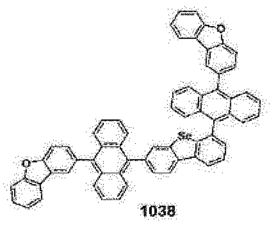
1038

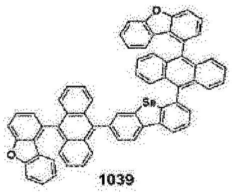
1039

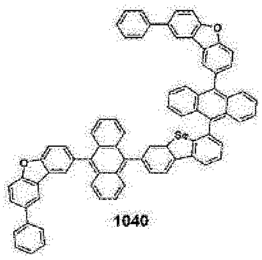
1040

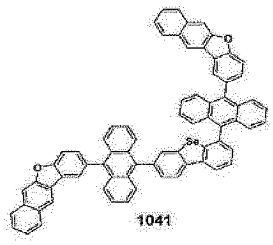
1041

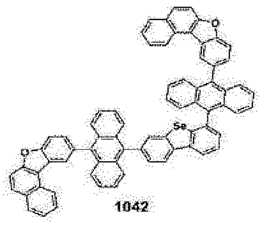
1042

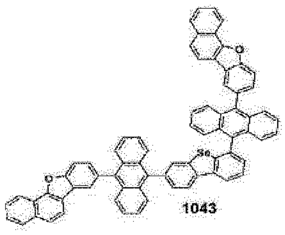
1043

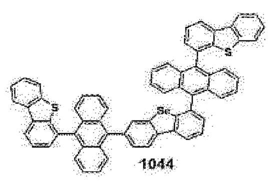
1044

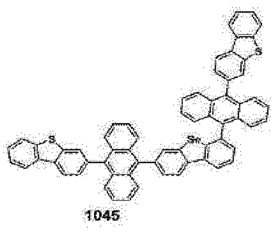
1045

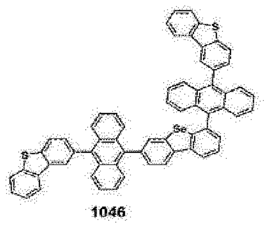
1046

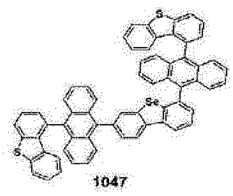
1047

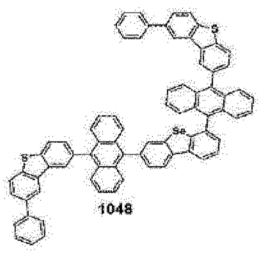
1048

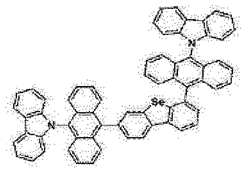
1049

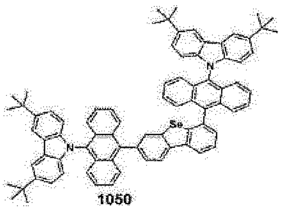
1050

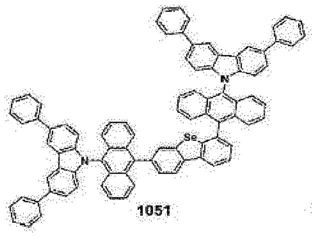
1051

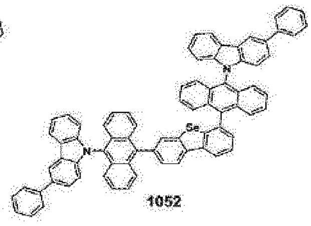
1052

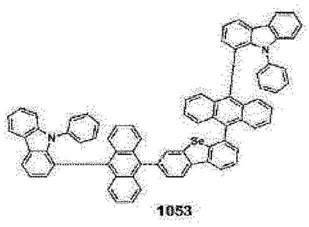
1053

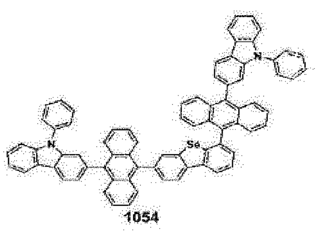
1054

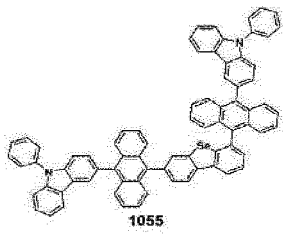
1055

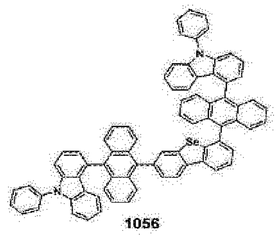
1056

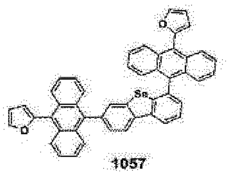
1057

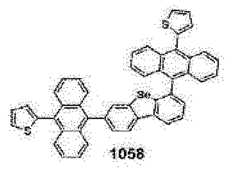
1058

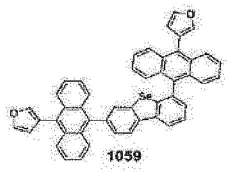
1059

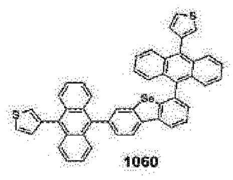
1060

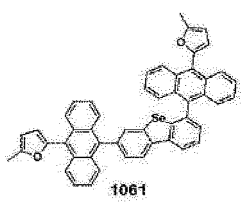
1061

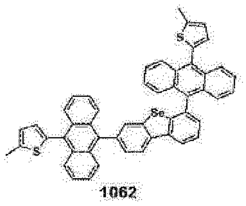
1062

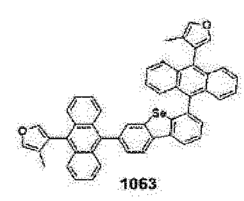
1063

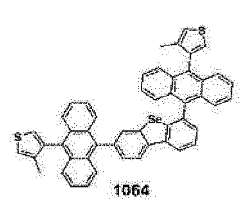
1064

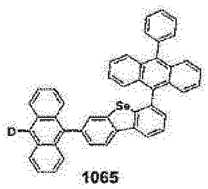
1065

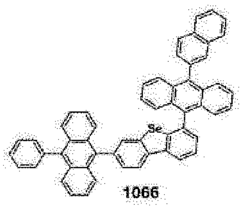
1066

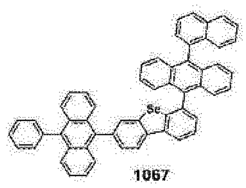
1067

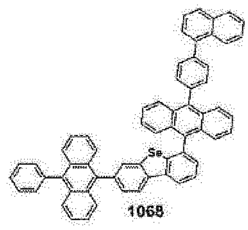
1068

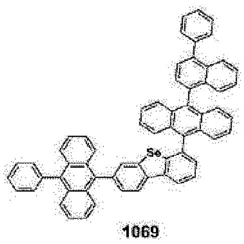
1069

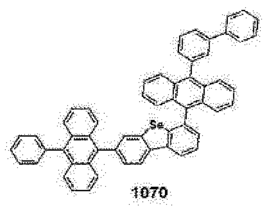
1070

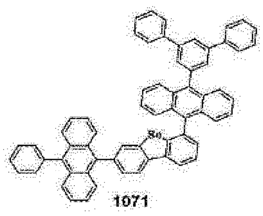
1071

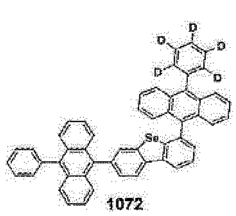
1072

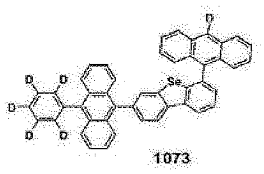
1073

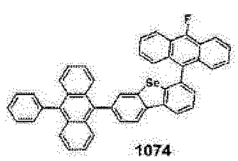
1074

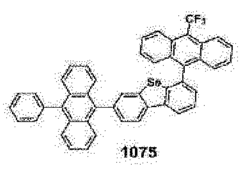
1075

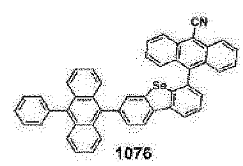
1076

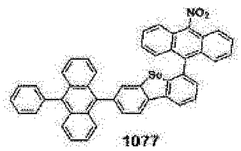
1077

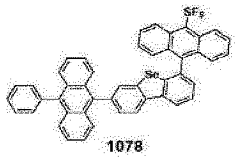
1078

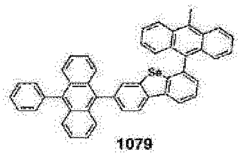
1079

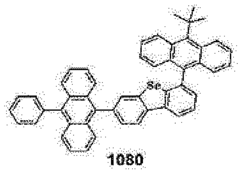
1080

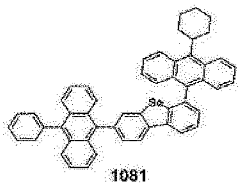
1081

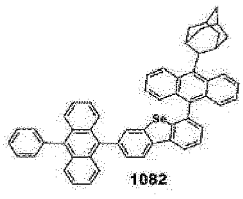
1082

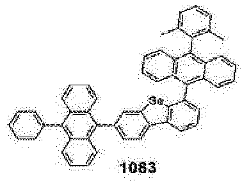
1083

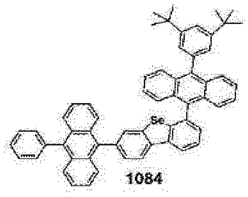
1084

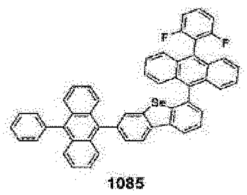
1085

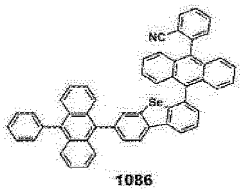
1086

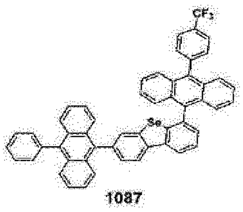
1087

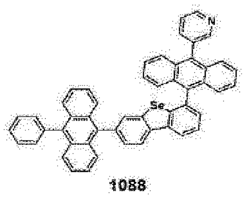
1088

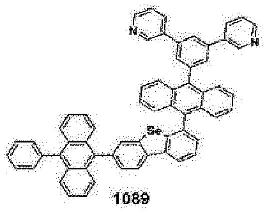
1089

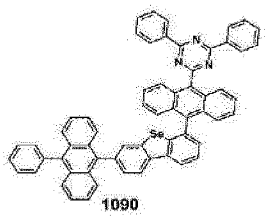
1090

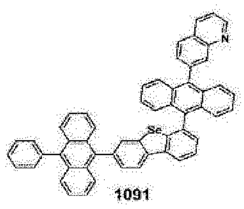
1091

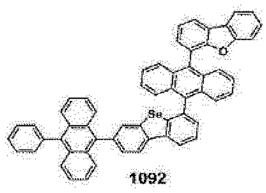
1092

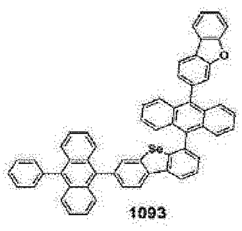
1093

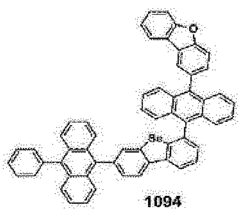
1094

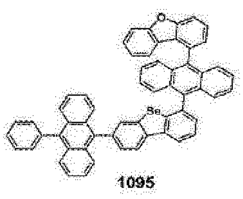
1095

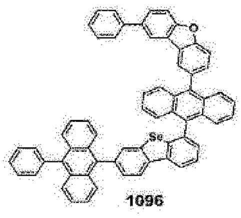
1096

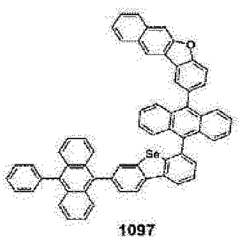
1097

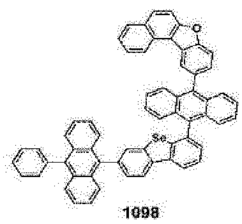
1098

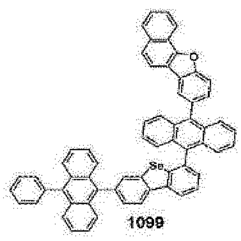
1099

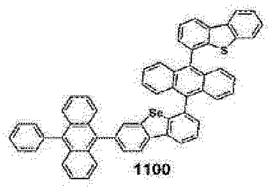
1100

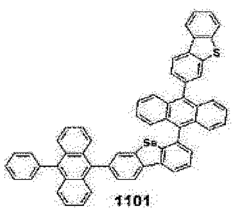
1101

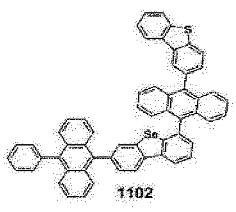
1102

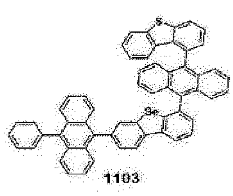
1103

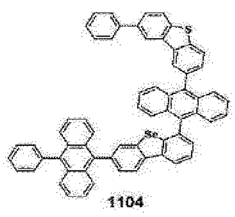
1104

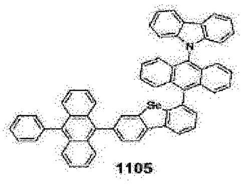

1105

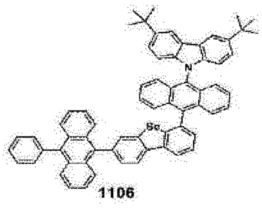

1106

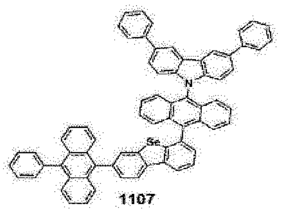

1107

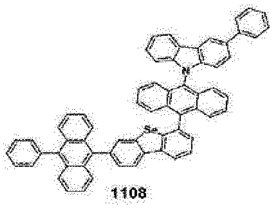

1108

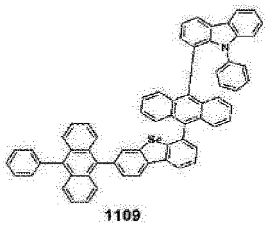

1109

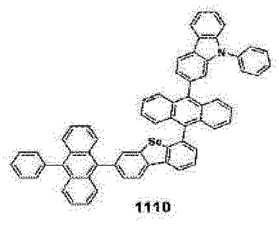

1110

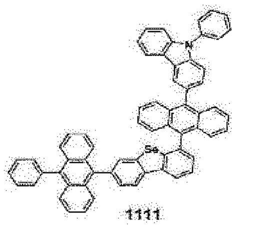

1111

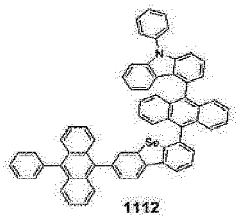

1112

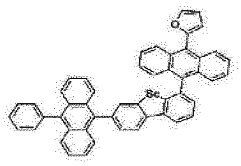

1113

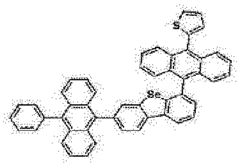

1114

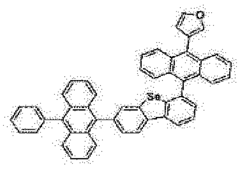

1115

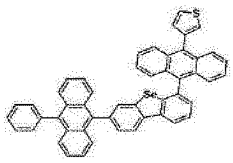

1116

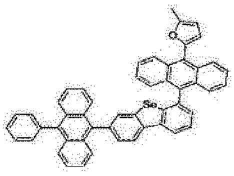

1117

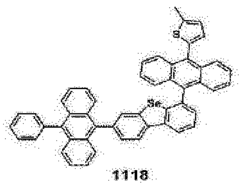

1118

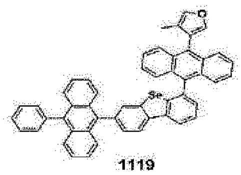

1119

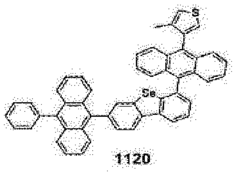

1120

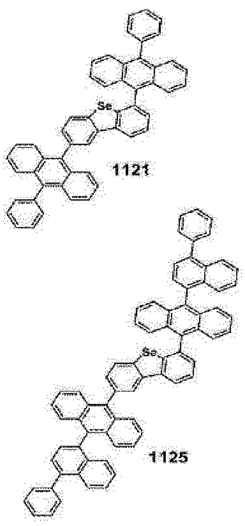

1121

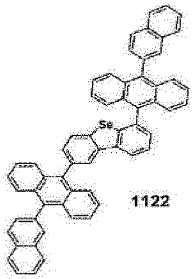

1122

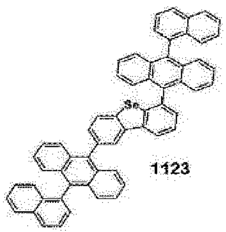

1123

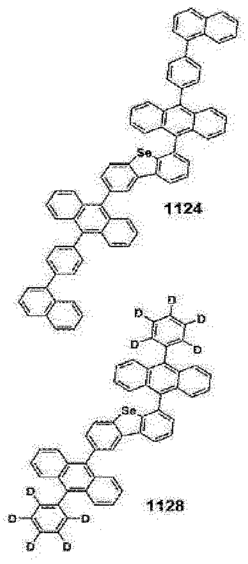

1124

1125

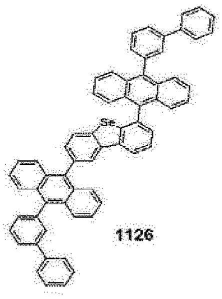

1126

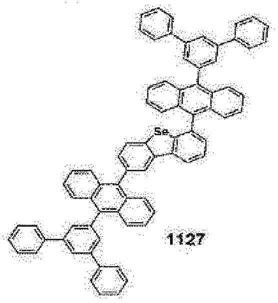

1127

1128

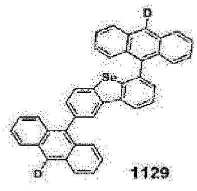
1129

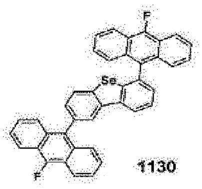
1130

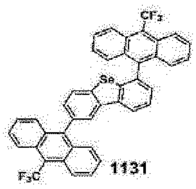
1131

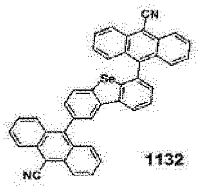
1132

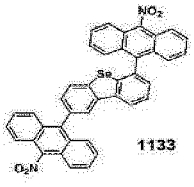
1133

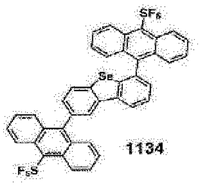
1134

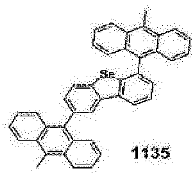
1135

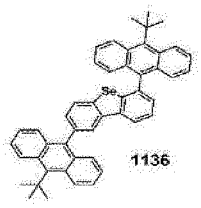
1136

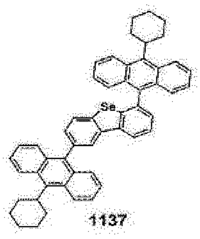
1137

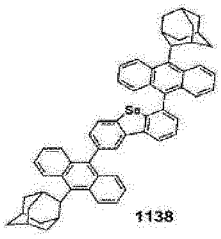
1138

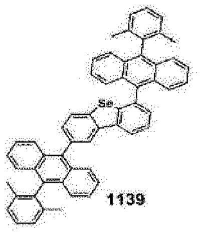
1139

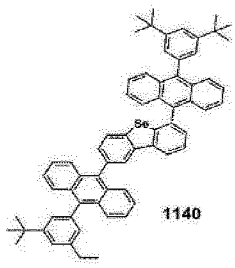
1140

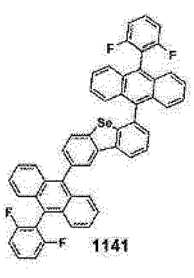
1141

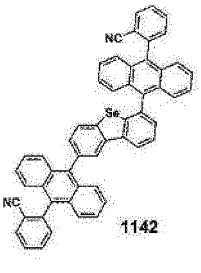
1142

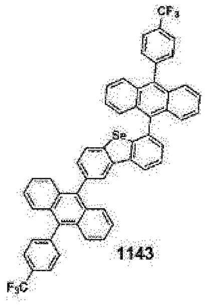
1143

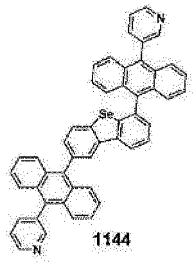
1144

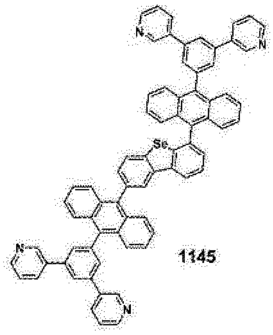
1145

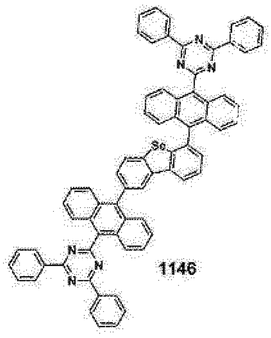
1146

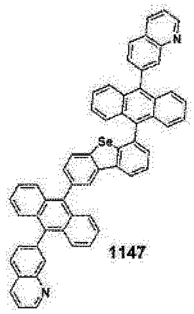
1147

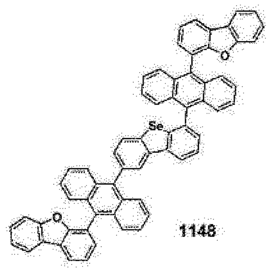
1148

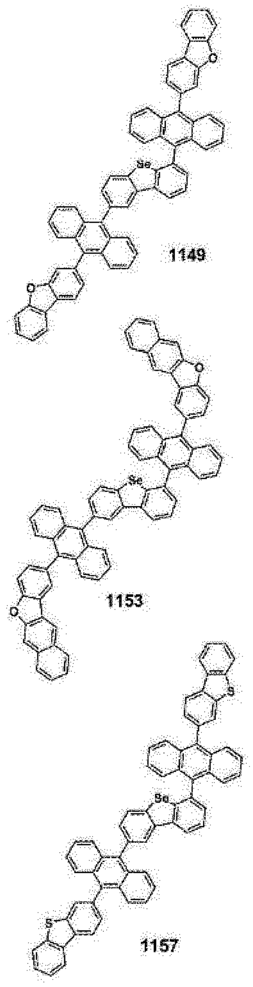

1149

1153

1157

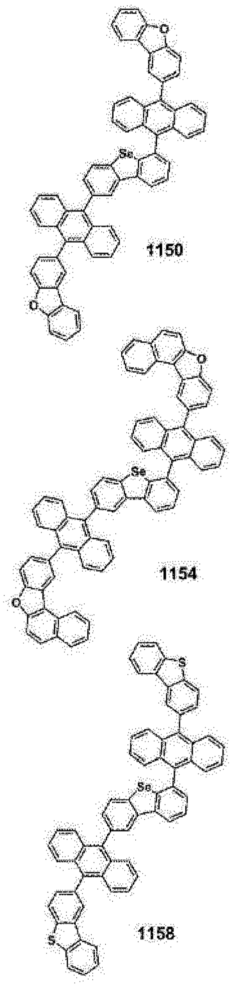

1150

1154

1158

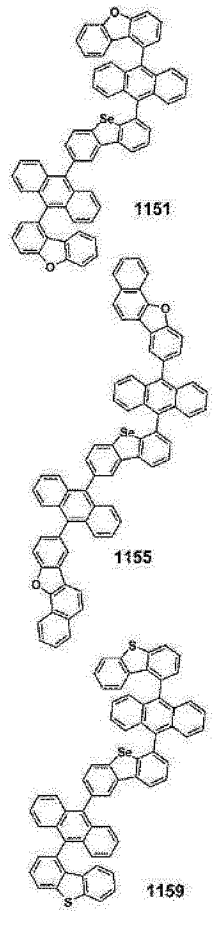

1151

1155

1159

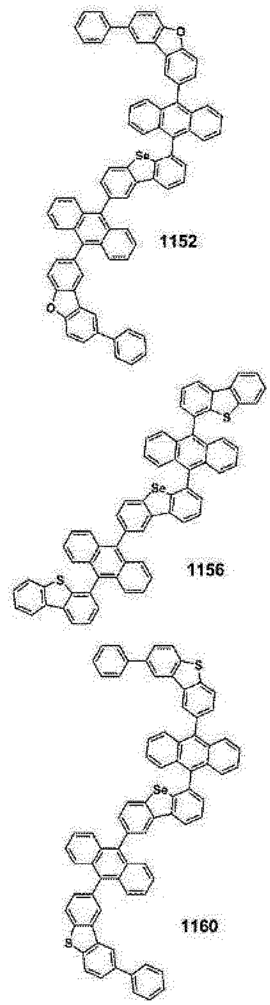

1152

1156

1160

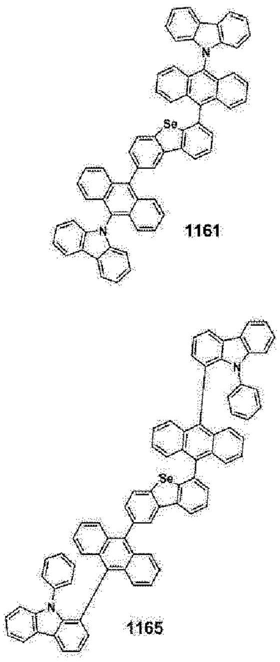

1161

1165

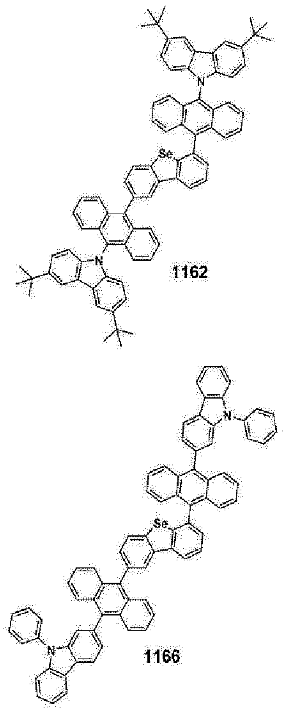

1162

1166

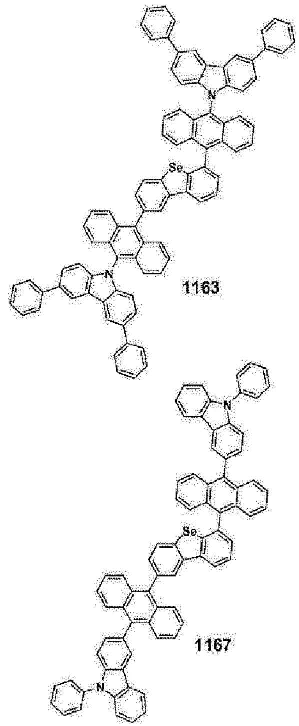

1163

1167

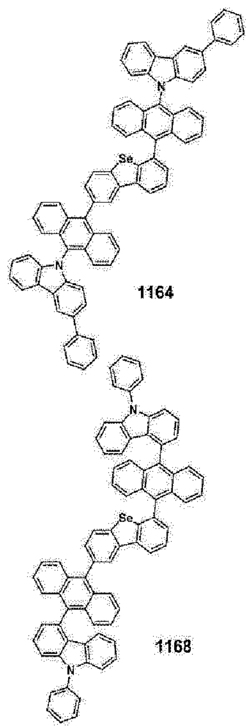

1164

1168

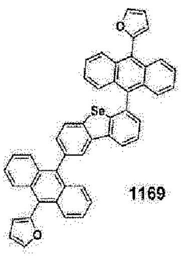
1169

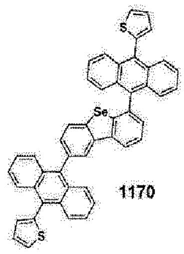
1170

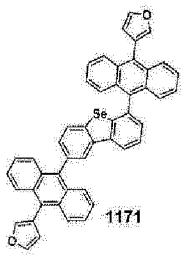
1171

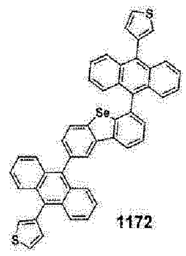
1172

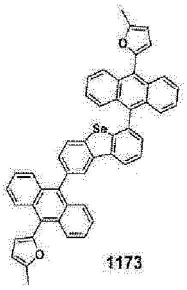
1173

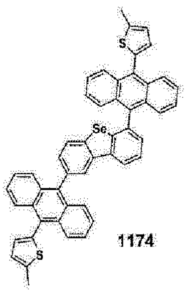
1174

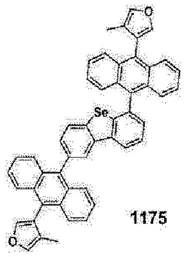
1175

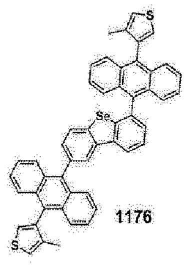
1176

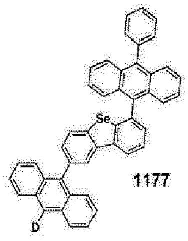
1177

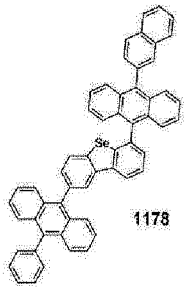
1178

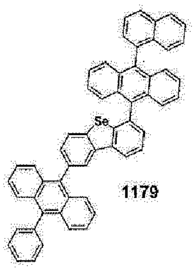
1179

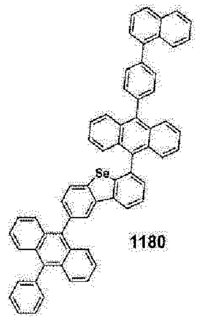
1180

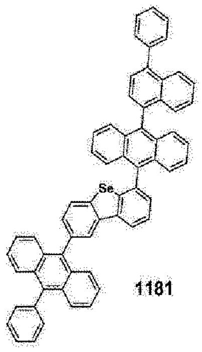
1181

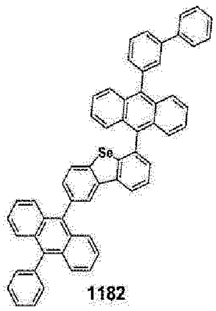
1182

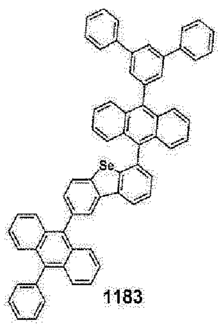
1183

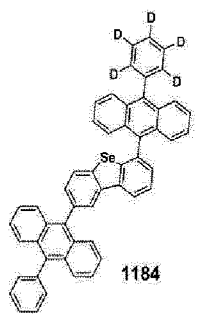
1184

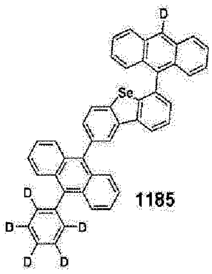
1185

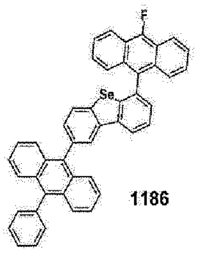
1186

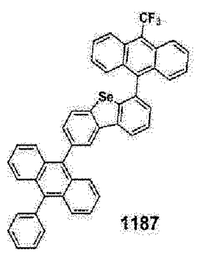
1187

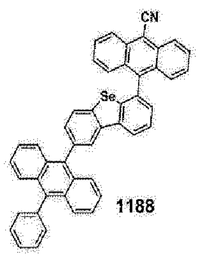
1188

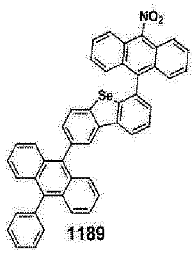
1189

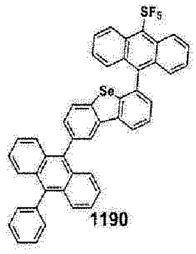
1190

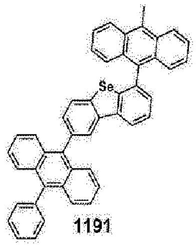
1191

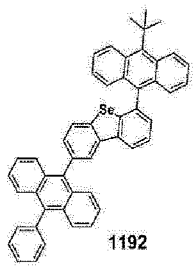
1192

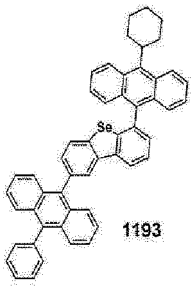
1193

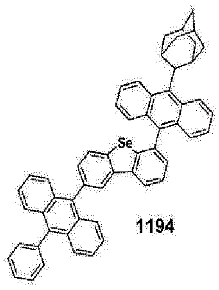
1194

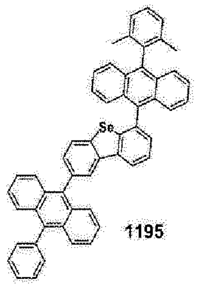
1195

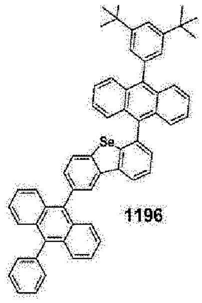
1196

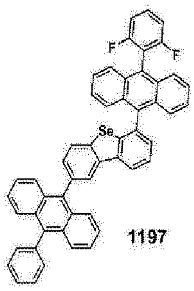
1197

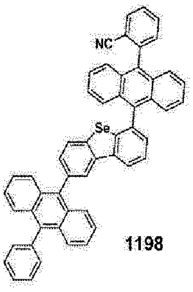
1198

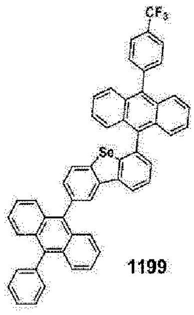
1199

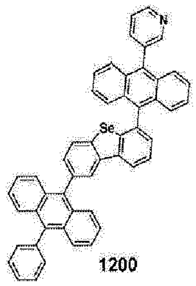
1200

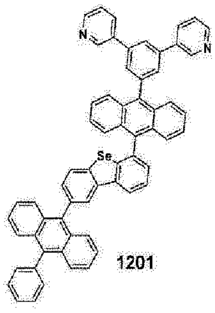
1201

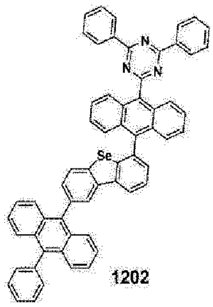
1202

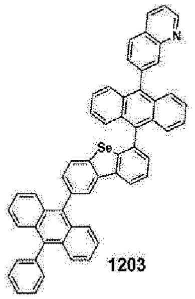
1203

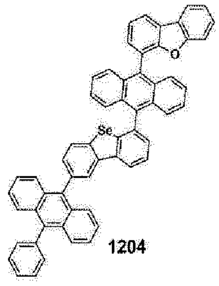
1204

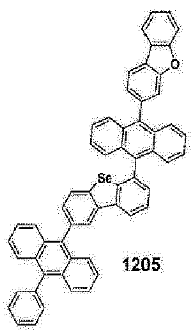
1205

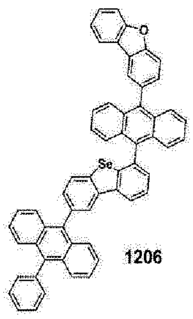
1206

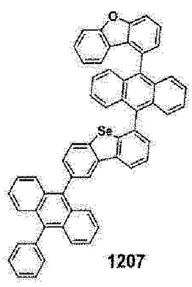
1207

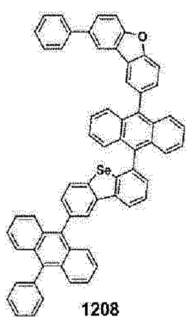
1208

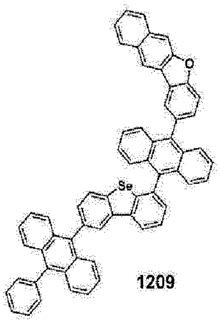
1209

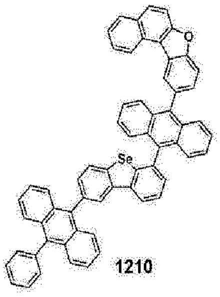
1210

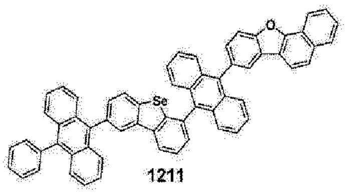
1211

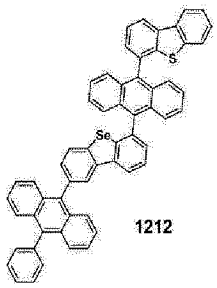
1212

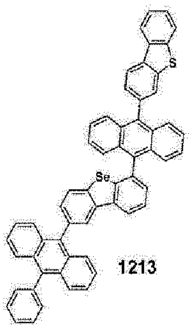
1213

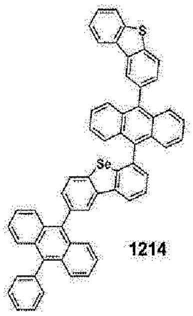
1214

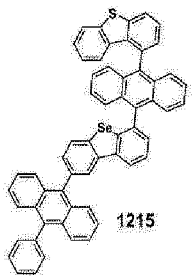
1215

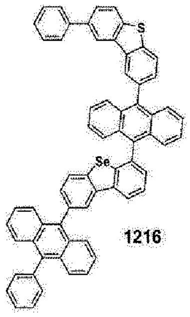
1216

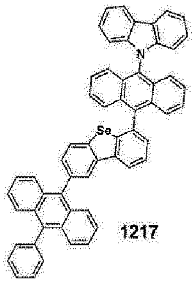
1217

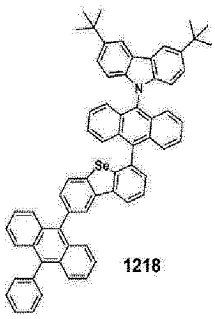
1218

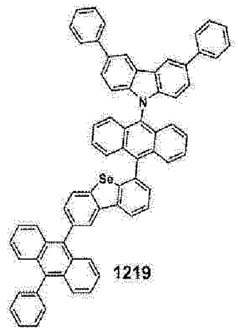
1219

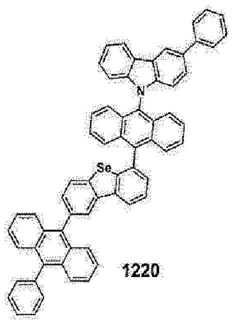
1220

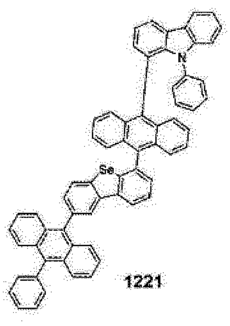
1221

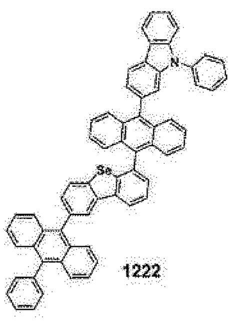
1222

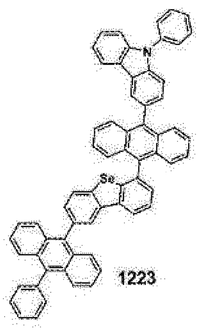
1223

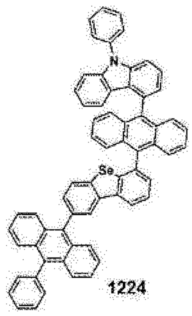
1224

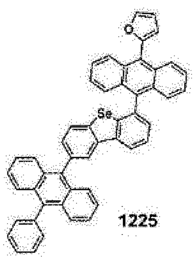
1225

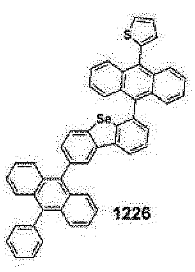
1226

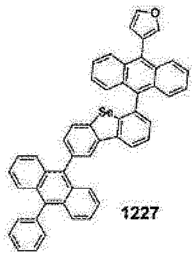
1227

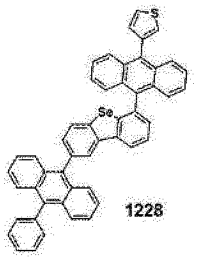
1228

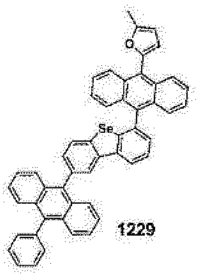
1229

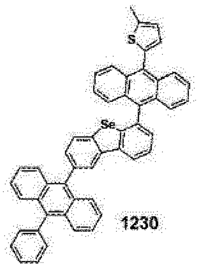
1230

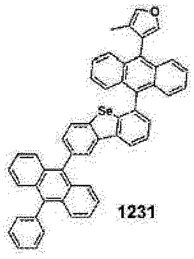
1231

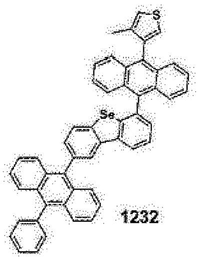
1232

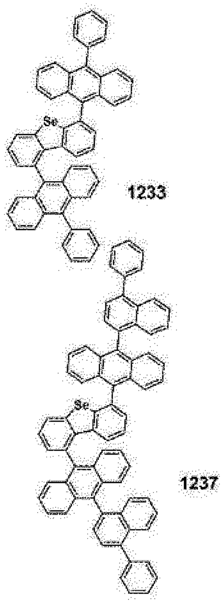
1233

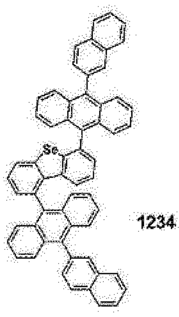
1234

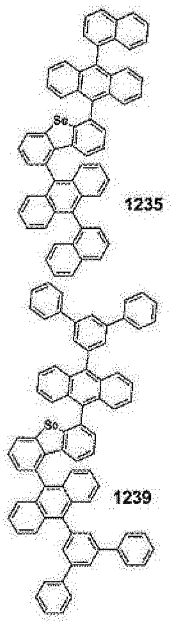
1235

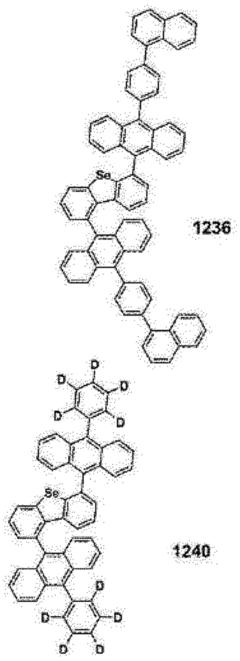
1236

1237

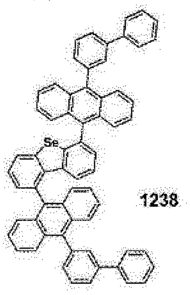
1238

1239

1240

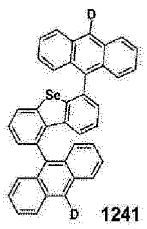
1241

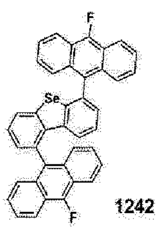
1242

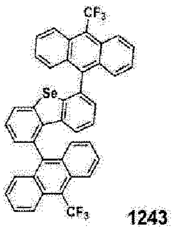
1243

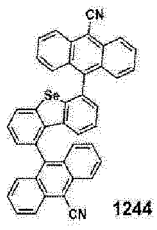
1244

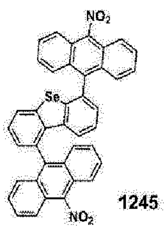
1245

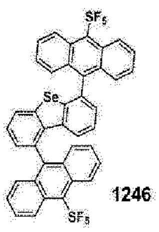
1246

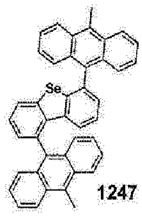
1247

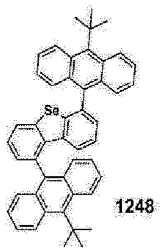
1248

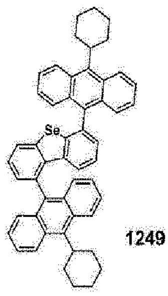
1249

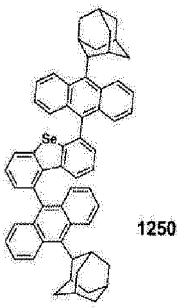
1250

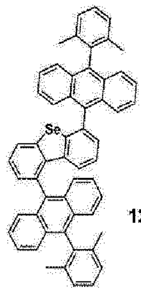
1251

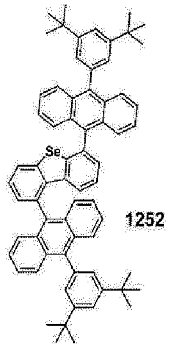
1252

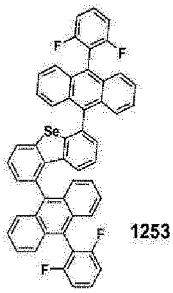
1253

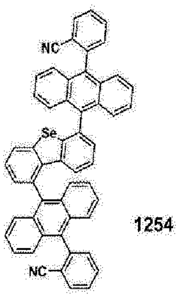
1254

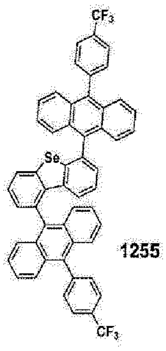
1255

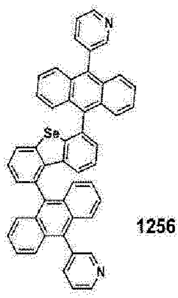
1256

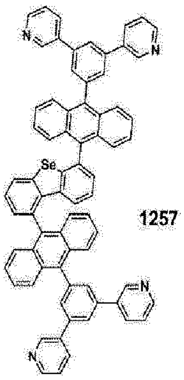
1257

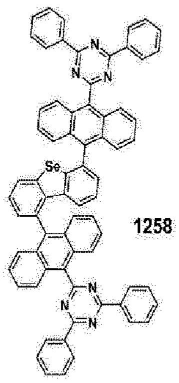
1258

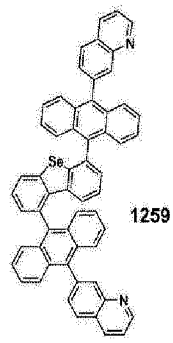
1259

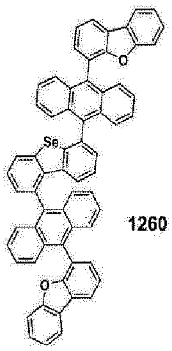
1260

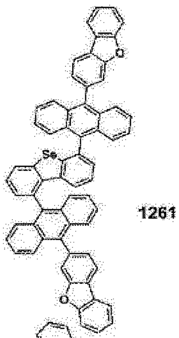
1261

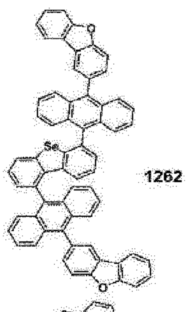
1262

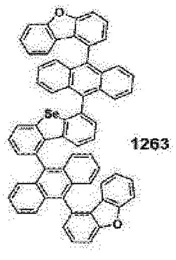
1263

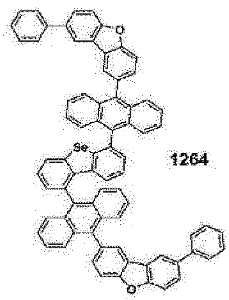
1264

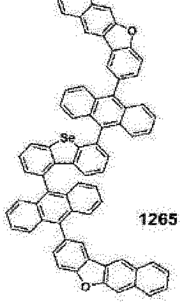
1265

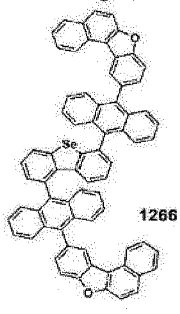
1266

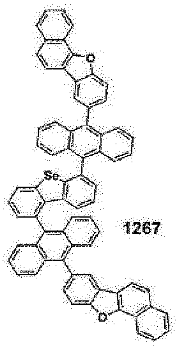
1267

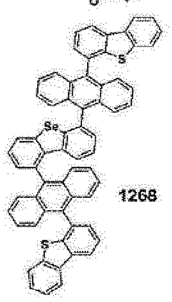
1268

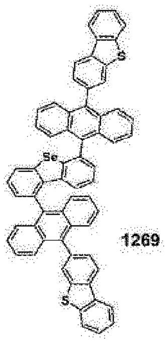
1269

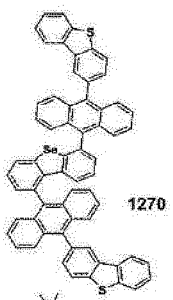
1270

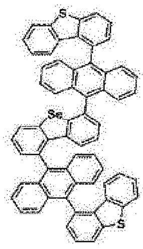
1271

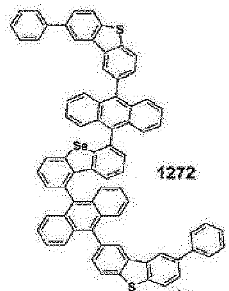
1272

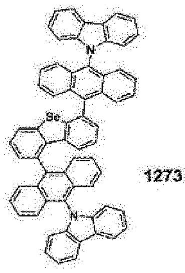
1273

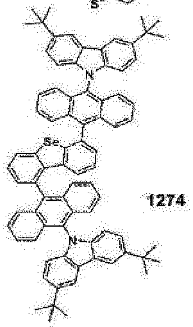
1274

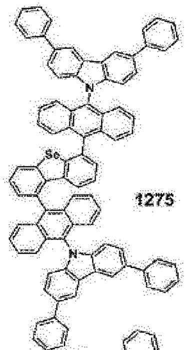
1275

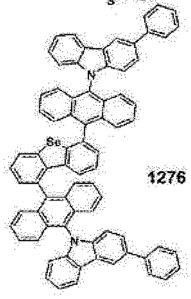
1276

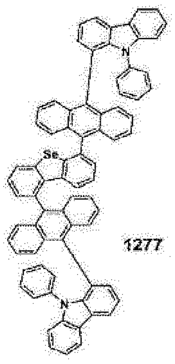
1277

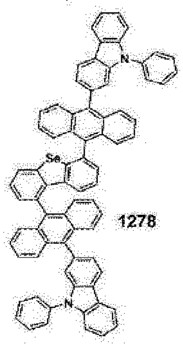
1278

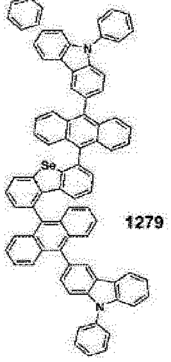
1279

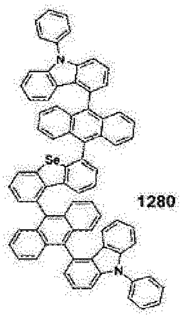
1280

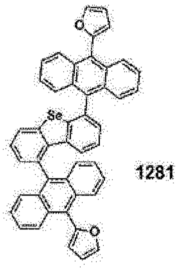
1281

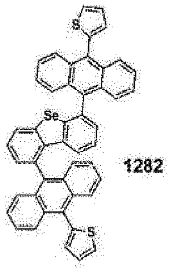
1282

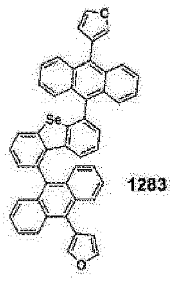
1283

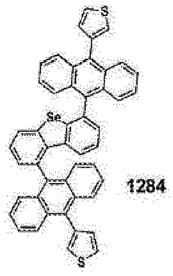
1284

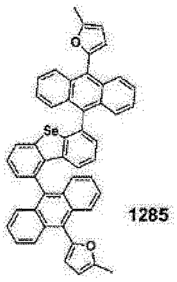
1285

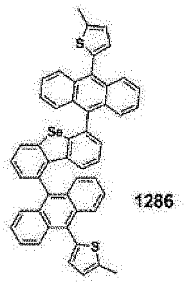
1286

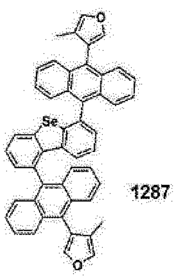
1287

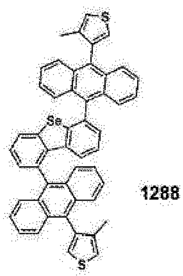
1288

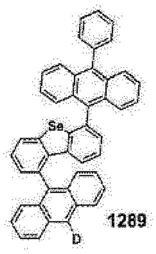
1289

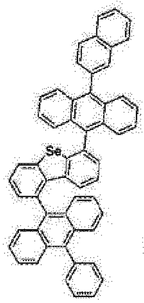
1290

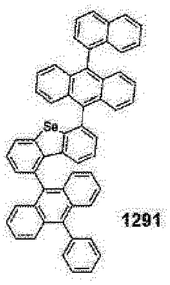
1291

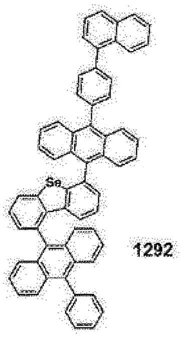
1292

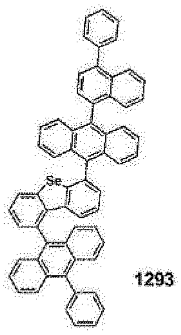
1293

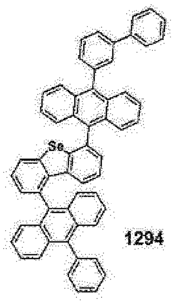
1294

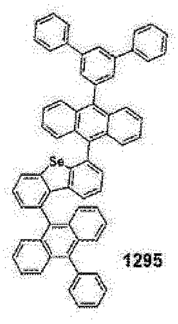
1295

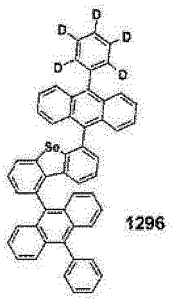
1296

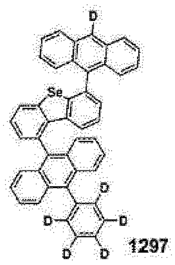
1297

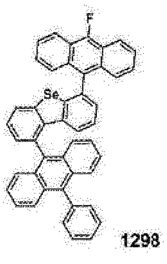
1298

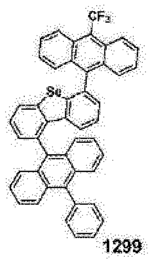
1299

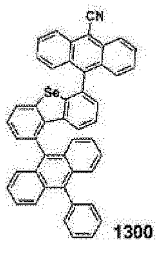
1300

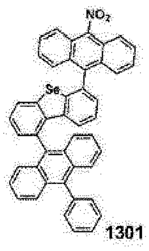
1301

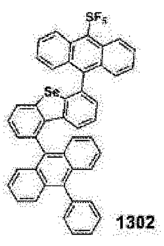
1302

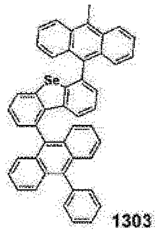
1303

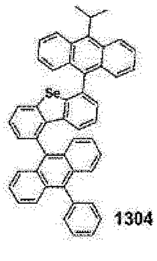
1304

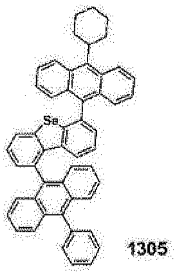
1305

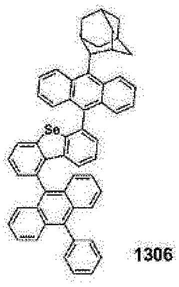
1306

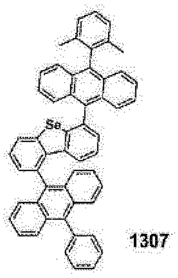
1307

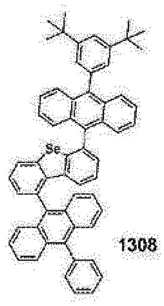
1308

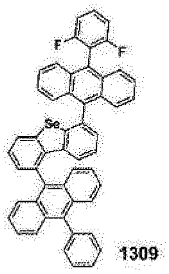
1309

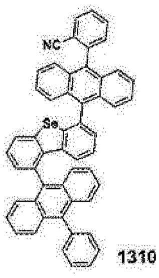
1310

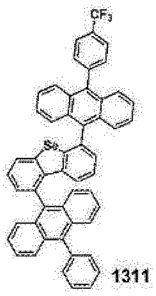
1311

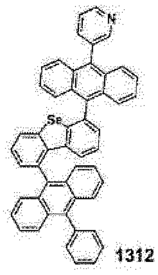
1312

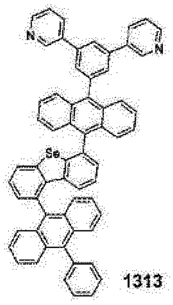
1313

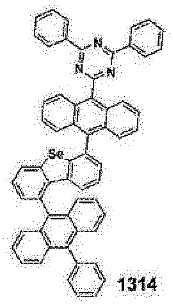
1314

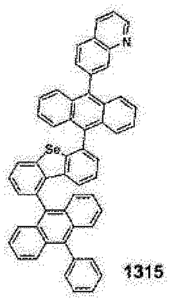
1315

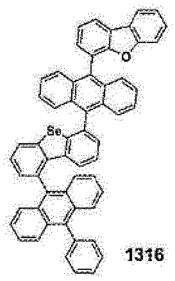
1316

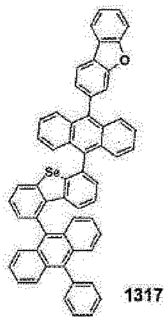
1317

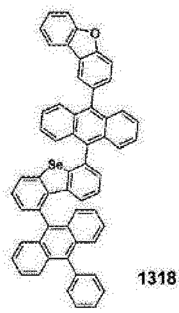
1318

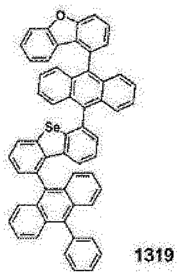
1319

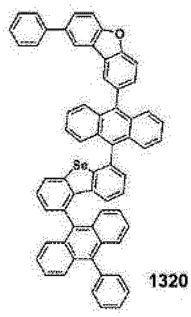
1320

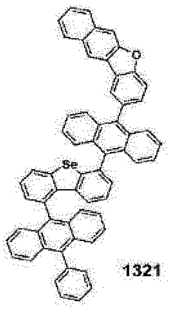
1321

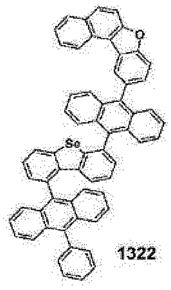
1322

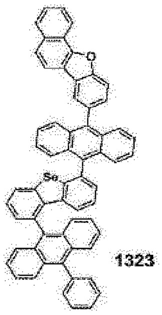
1323

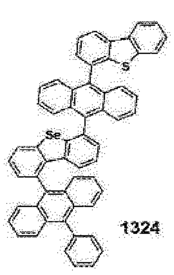
1324

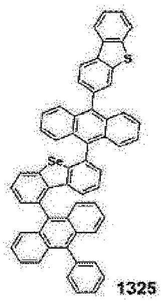
1325

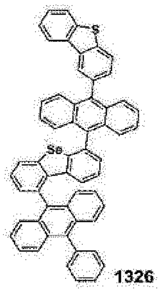
1326

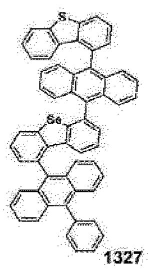
1327

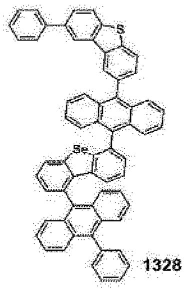
1328

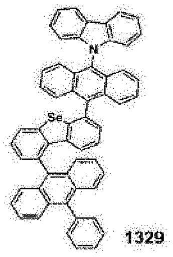
1329

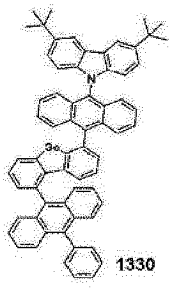
1330

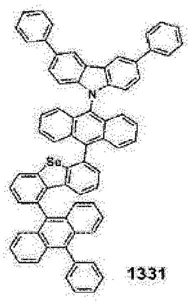
1331

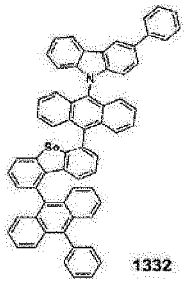
1332

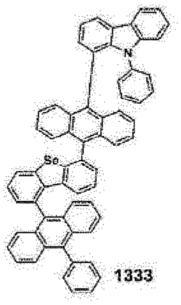
1333

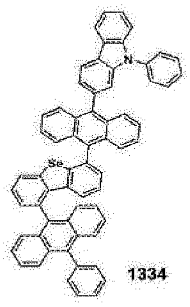
1334

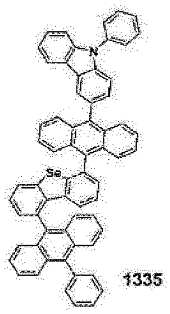
1335

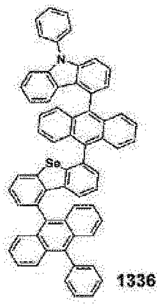
1336

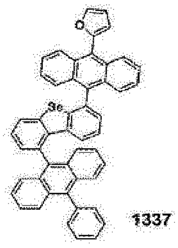
1337

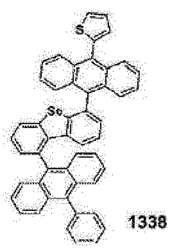
1338

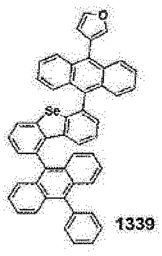
1339

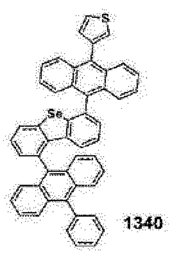
1340

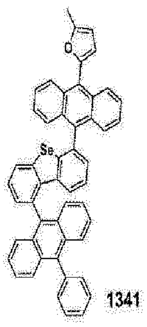
1341

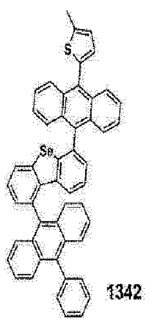
1342

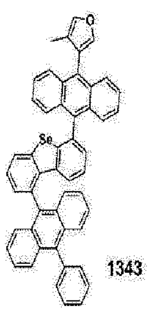
1343

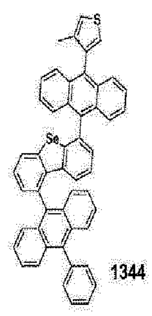
1344

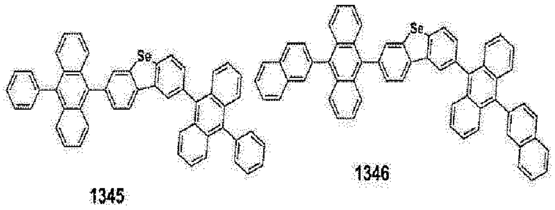
1345

1346

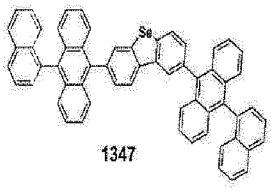
1347

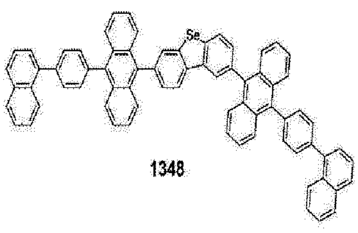
1348

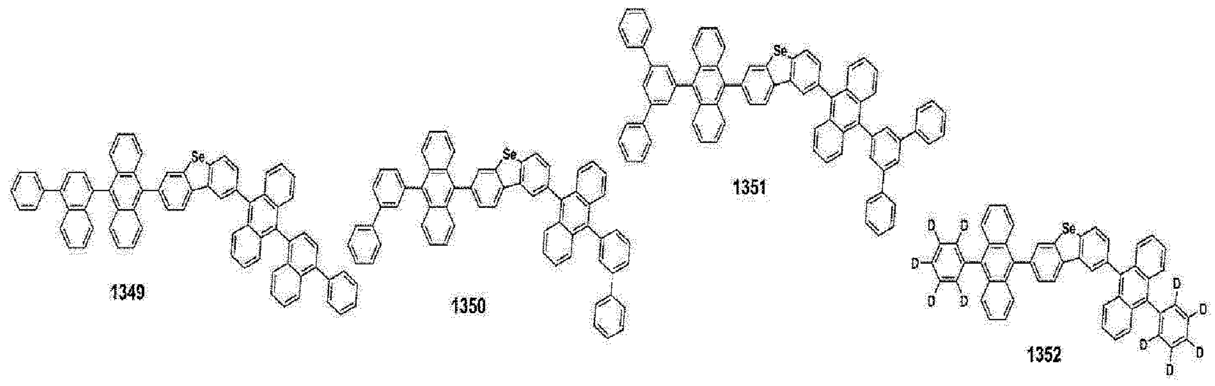

1349 1350 1351 1352

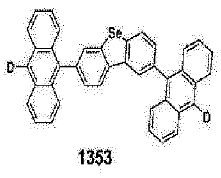

1353

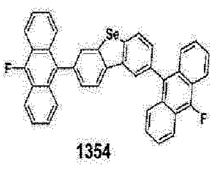

1354

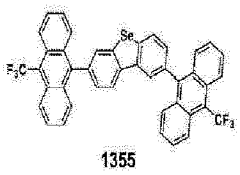

1355

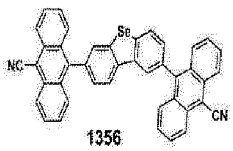

1356

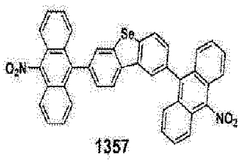

1357

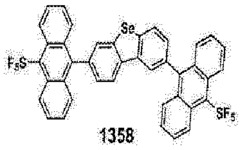

1358

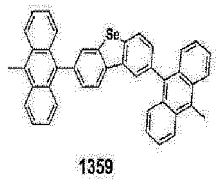

1359

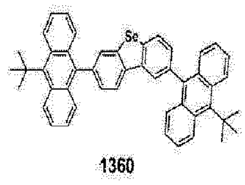

1360

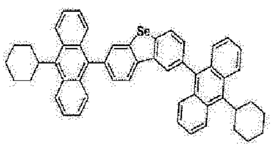

1361

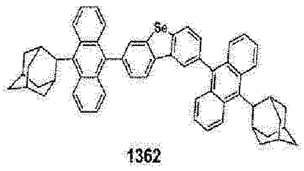

1362

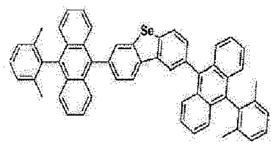

1363

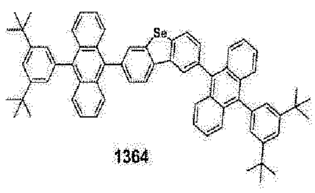

1364

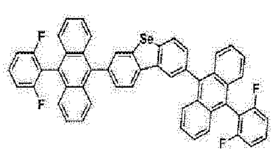

1365

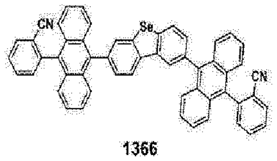

1366

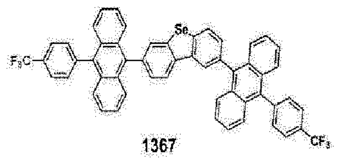

1367

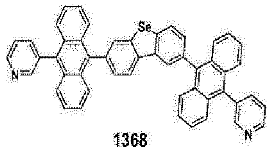

1368

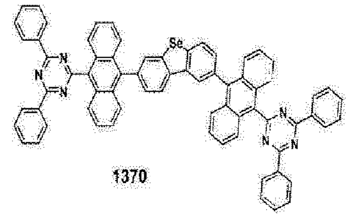

1370

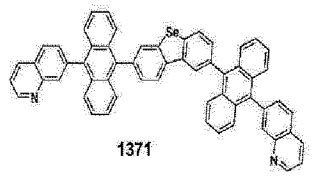

1371

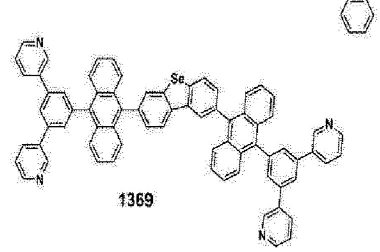

1369

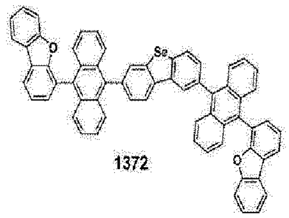

1372

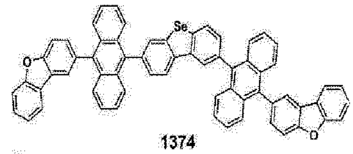

1374

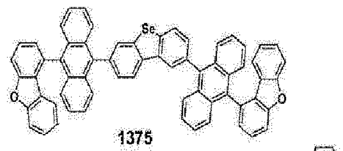

1375

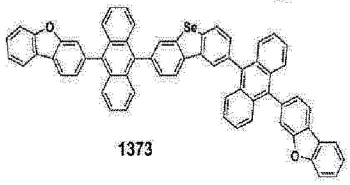

1373

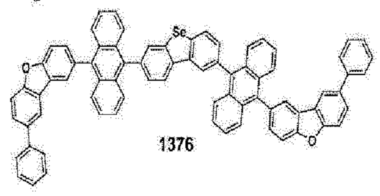

1376

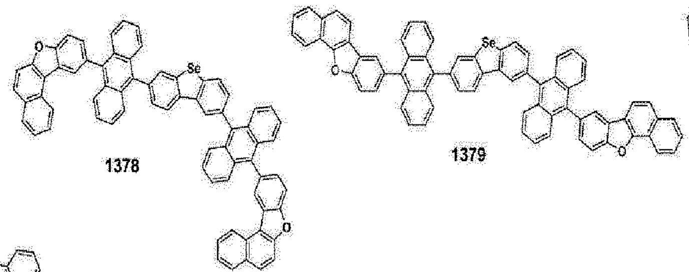

1378

1379

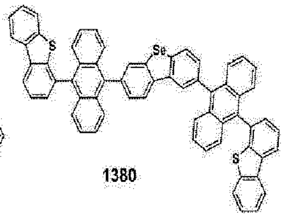

1380

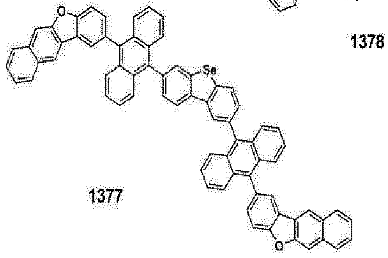

1377

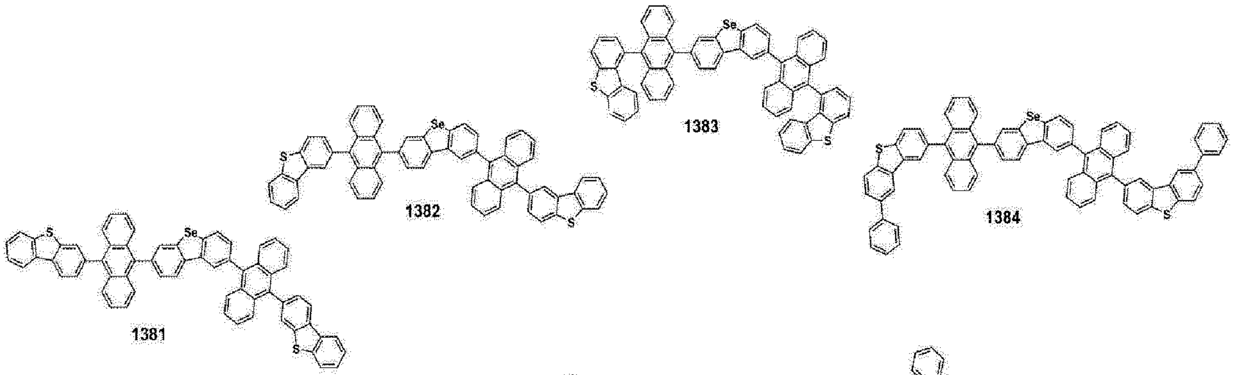

1383

1382

1384

1381

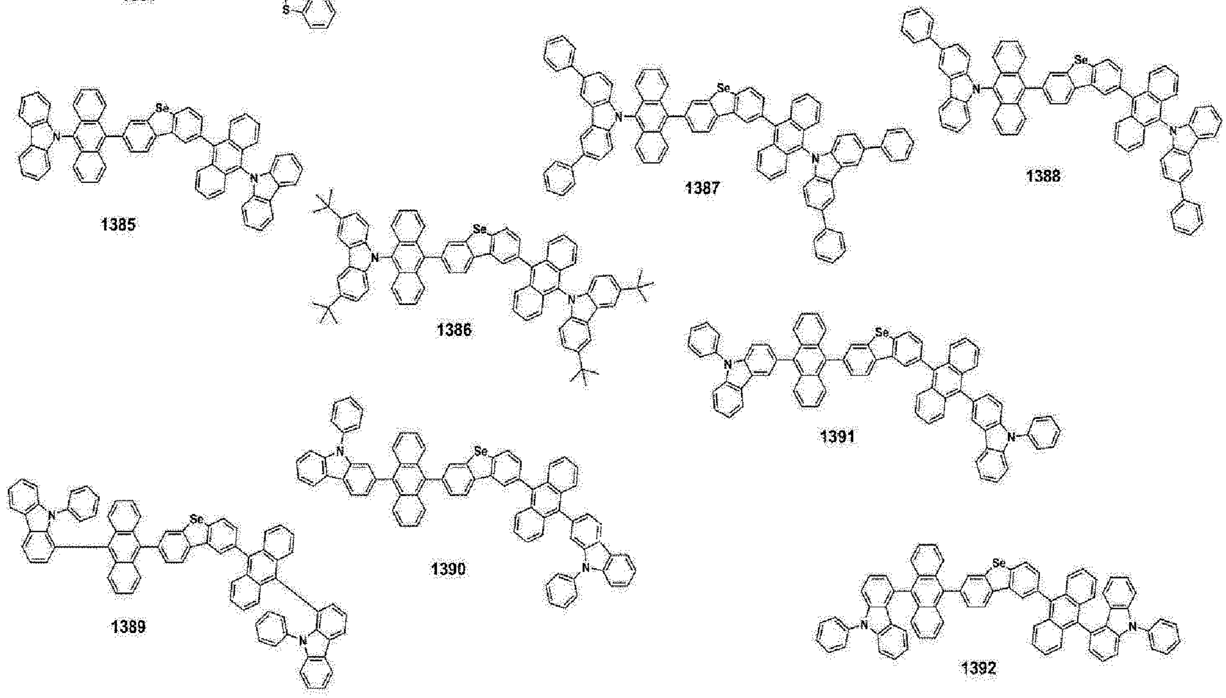

1387

1388

1385

1386

1391

1390

1389

1392

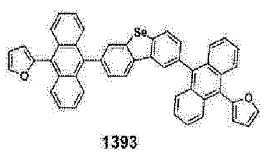

1393

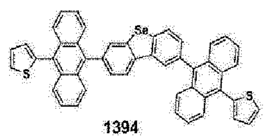

1394

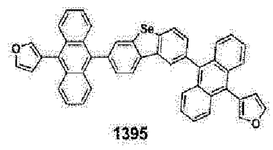

1395

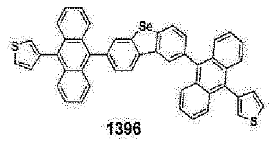

1396

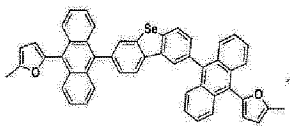

1397

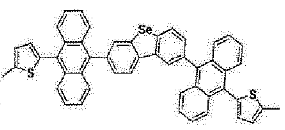

1398

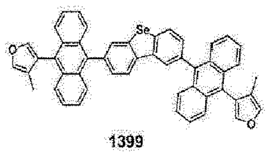

1399

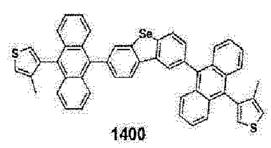

1400

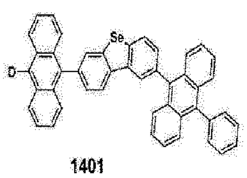

1401

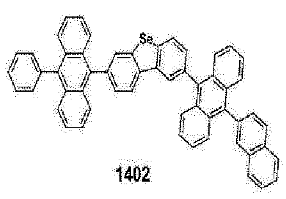

1402

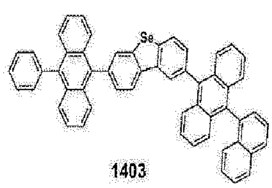

1403

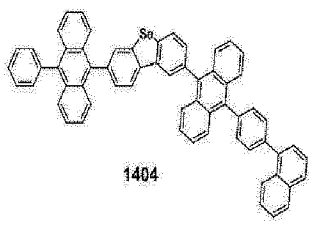

1404

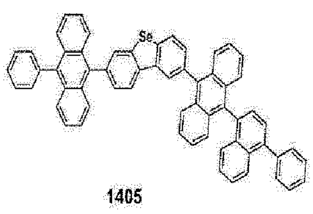

1405

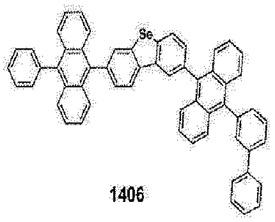

1406

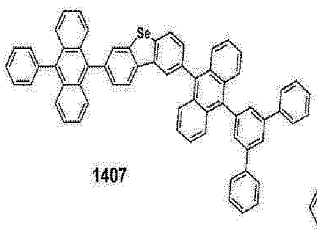

1407

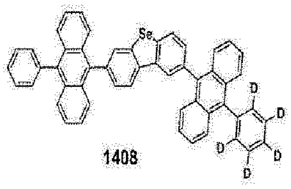

1408

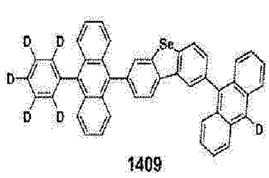

1409

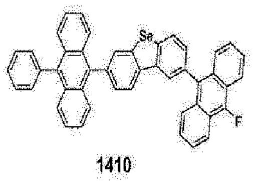

1410

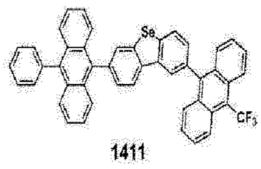

1411

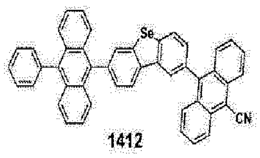

1412

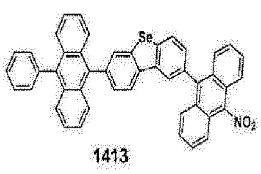

1413

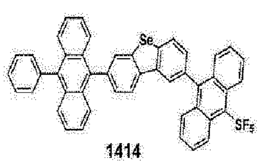

1414

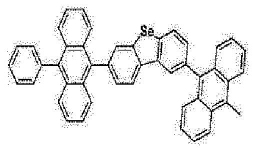

1415

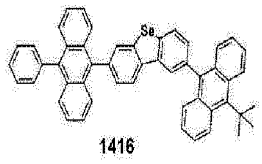

1416

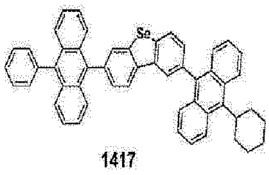

1417

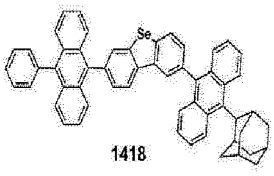

1418

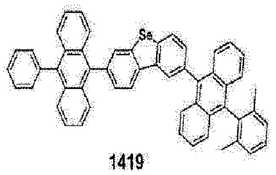

1419

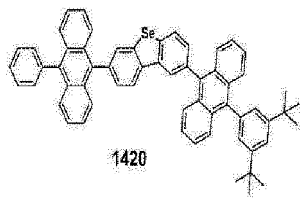

1420

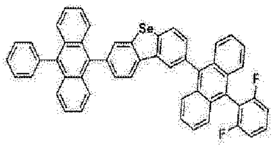

1421

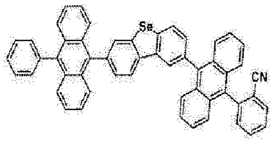

1422

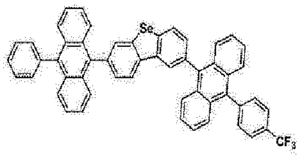

1423

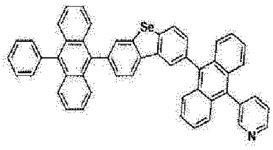

1424

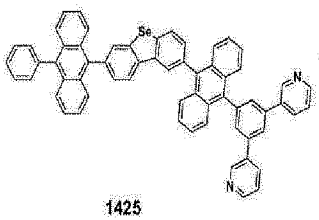

1425

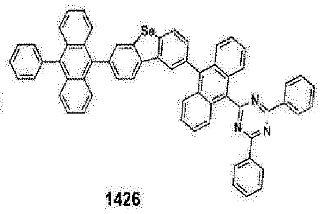

1426

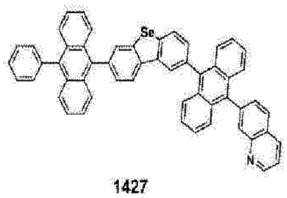

1427

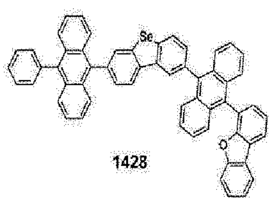

1428

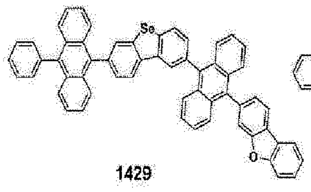
1429

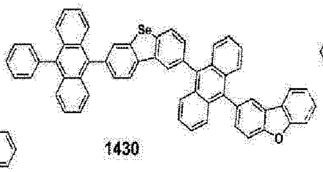
1430

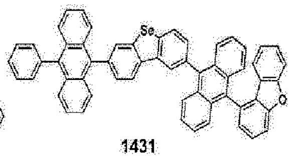
1431

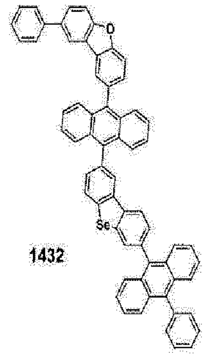
1432

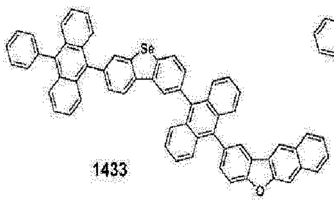
1433

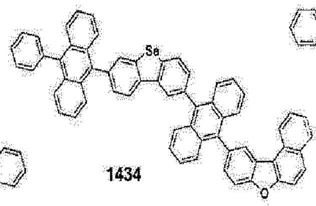
1434

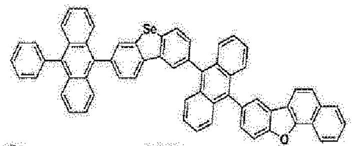
1435

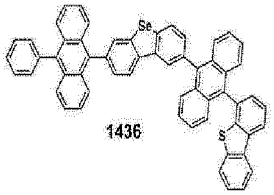
1436

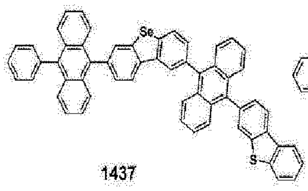
1437

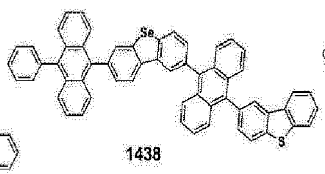
1438

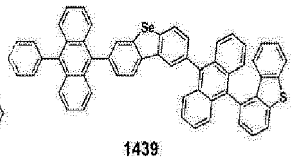
1439

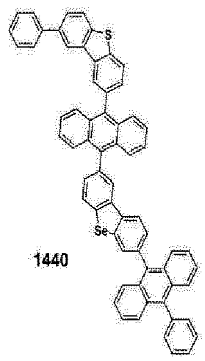
1440

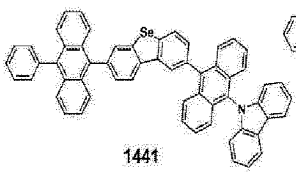
1441

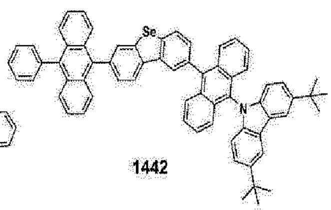
1442

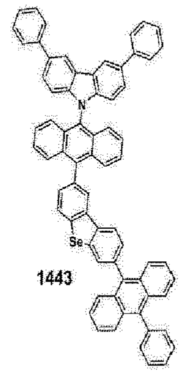
1443

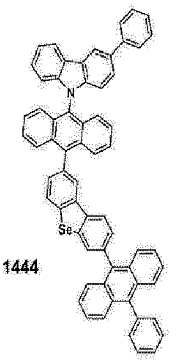
1444

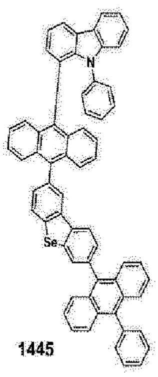
1445

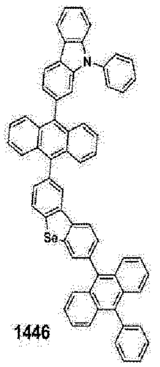
1446

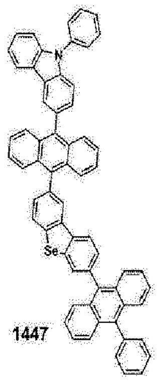
1447

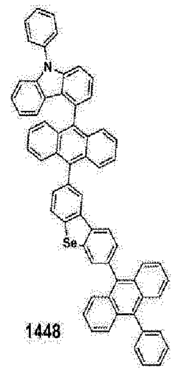
1448

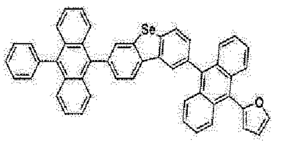
1449

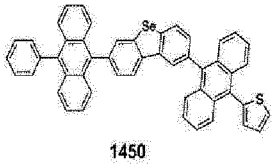
1450

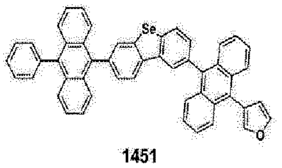
1451

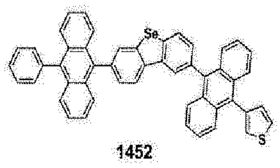
1452

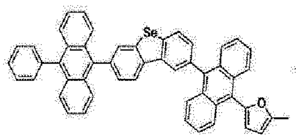
1453

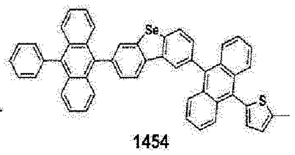
1454

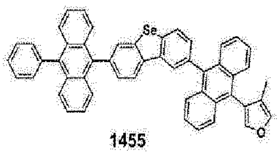
1455

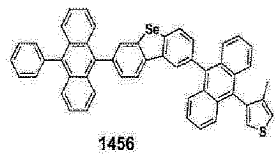
1456

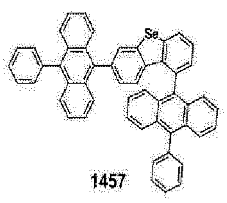
1457

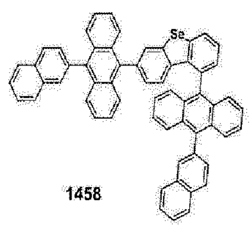
1458

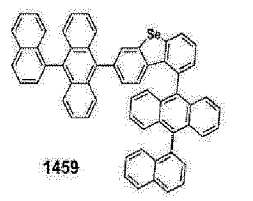
1459

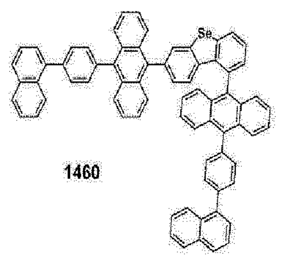
1460

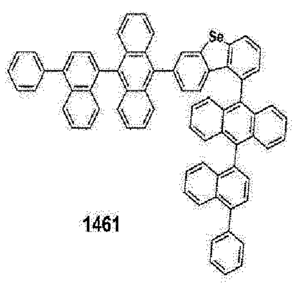
1461

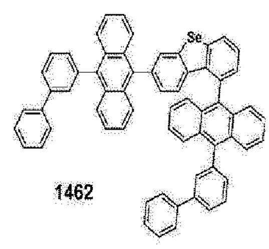
1462

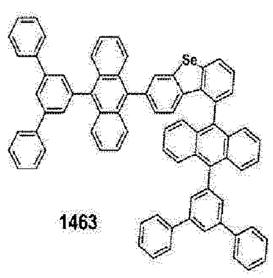
1463

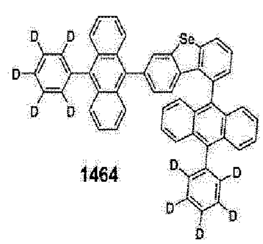
1464

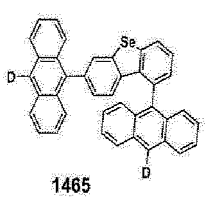
1465

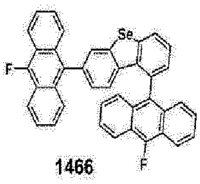
1466

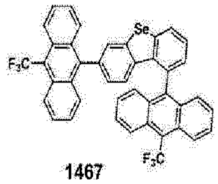
1467

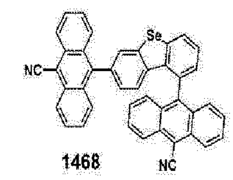
1468

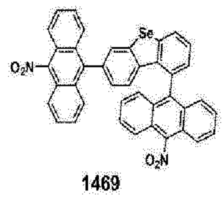

1469

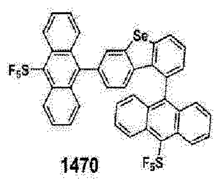

1470

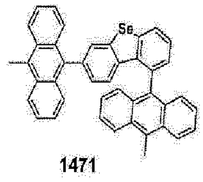

1471

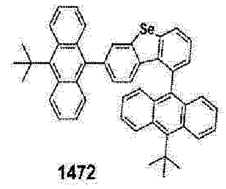

1472

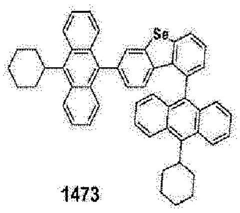

1473

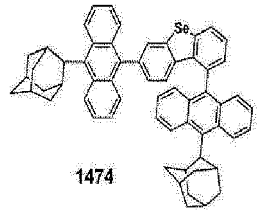

1474

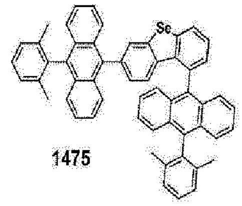

1475

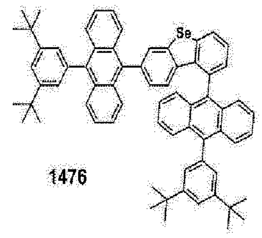

1476

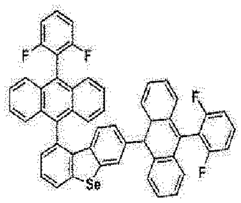

1477

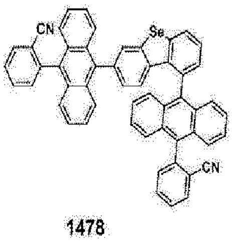

1478

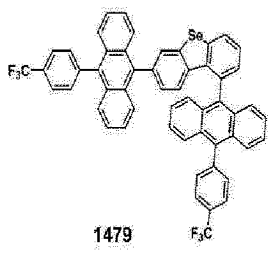

1479

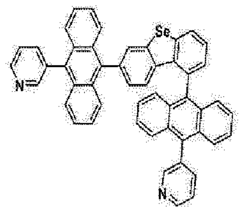

1480

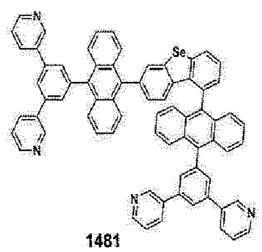

1481

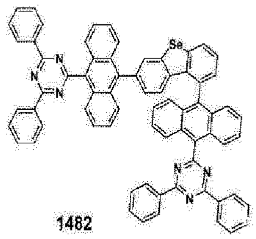

1482

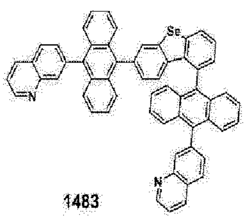

1483

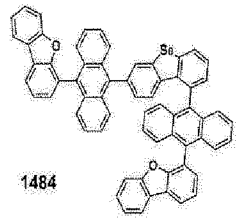

1484

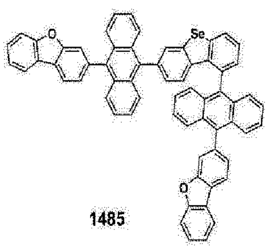

1485

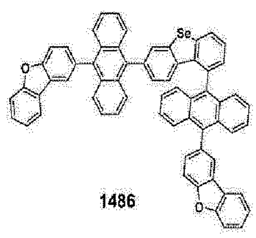

1486

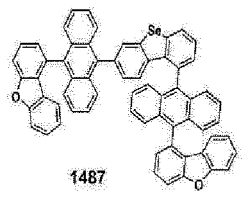

1487

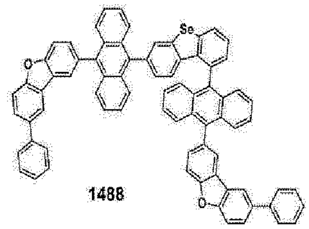

1488

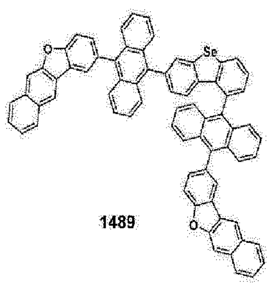

1489

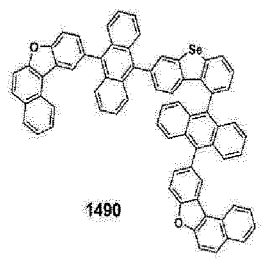

1490

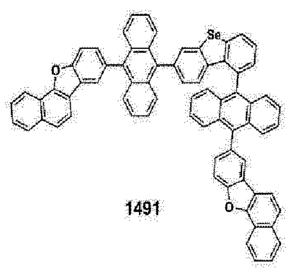

1491

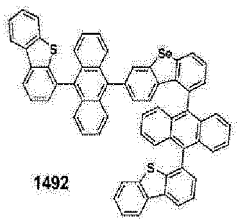

1492

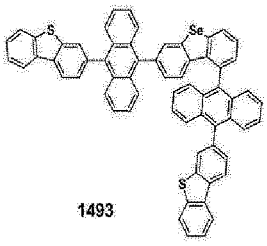
1493

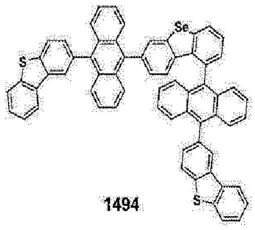
1494

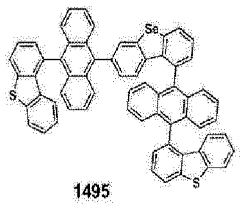
1495

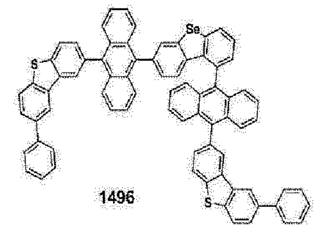
1496

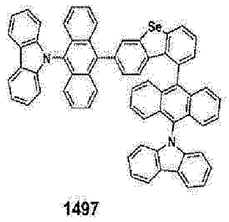
1497

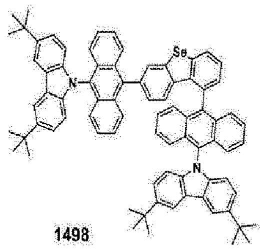
1498

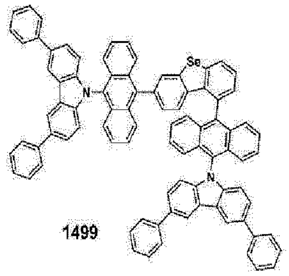
1499

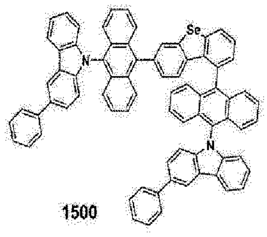
1500

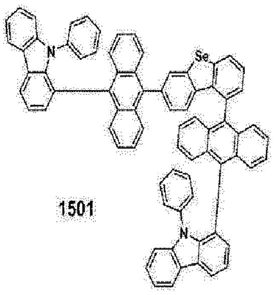
1501

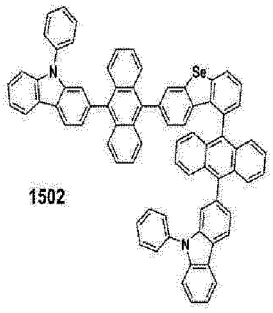
1502

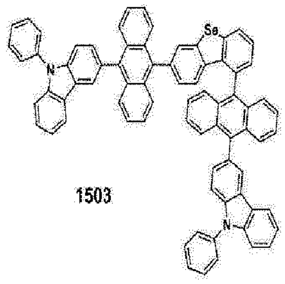
1503

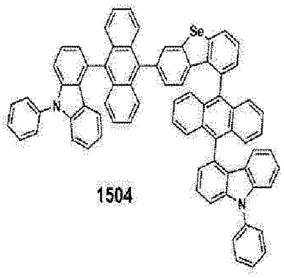
1504

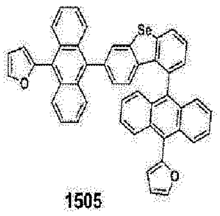
1505

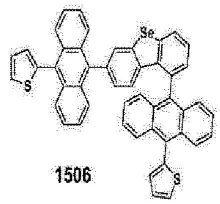
1506

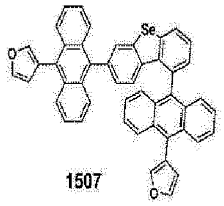
1507

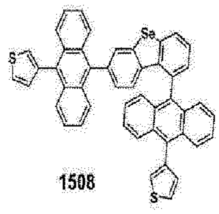
1508

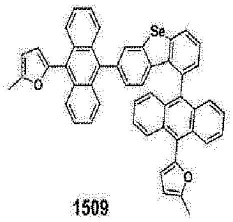
1509

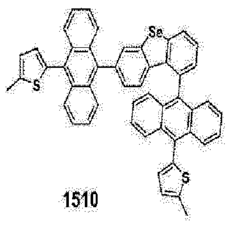
1510

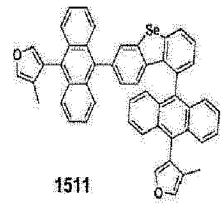
1511

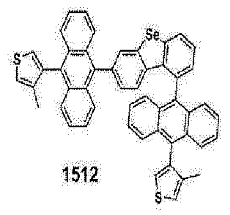
1512

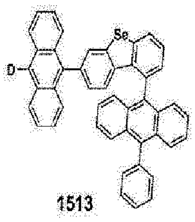
1513

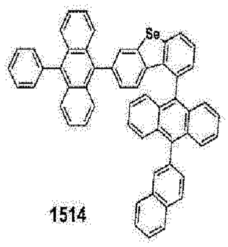
1514

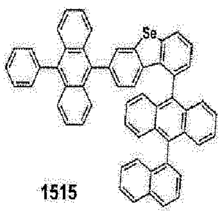
1515

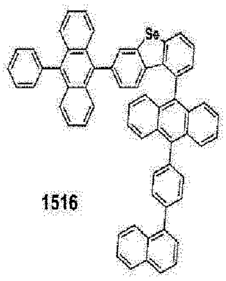
1516

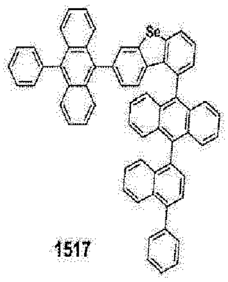
1517

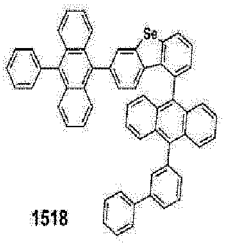
1518

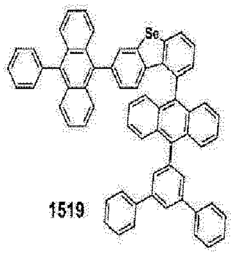
1519

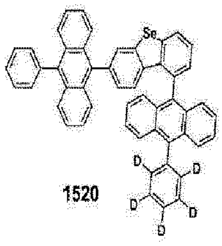
1520

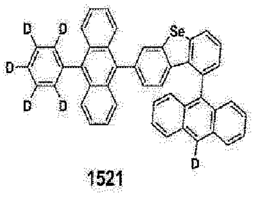
1521

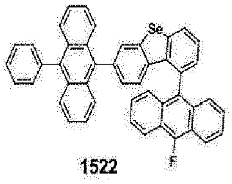
1522

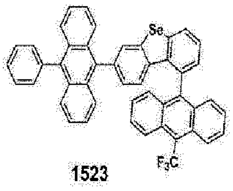
1523

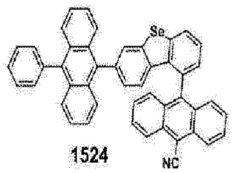
1524

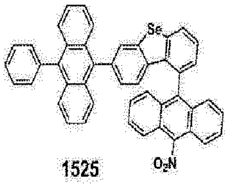
1525

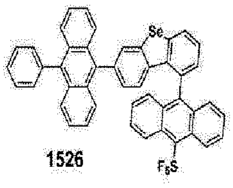
1526

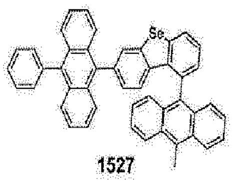
1527

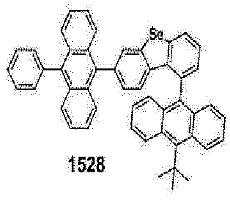
1528

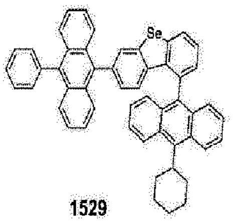
1529

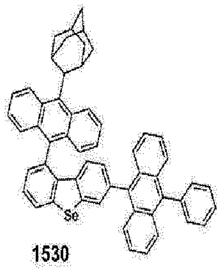
1530

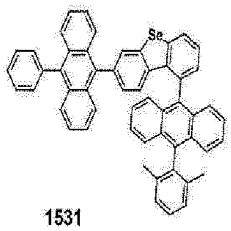
1531

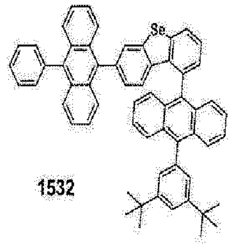
1532

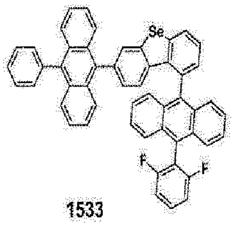
1533

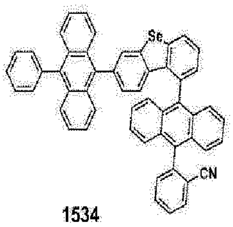
1534

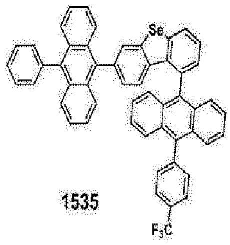
1535

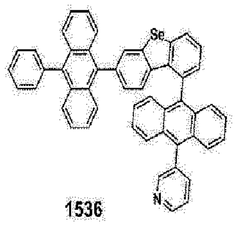
1536

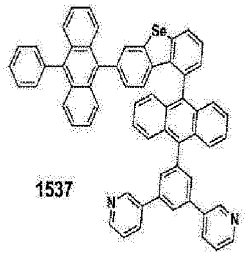
1537

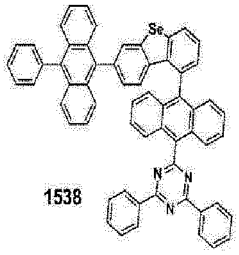
1538

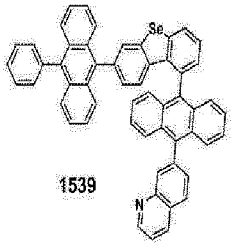
1539

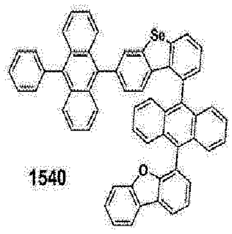
1540

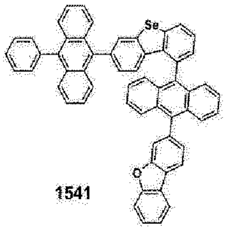

1541

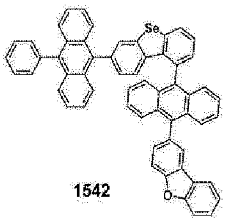

1542

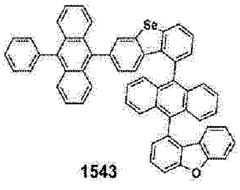

1543

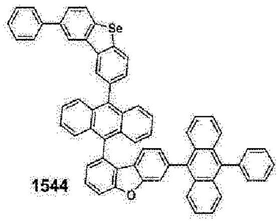

1544

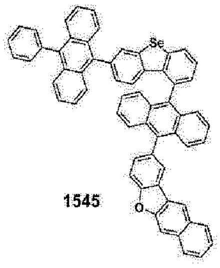

1545

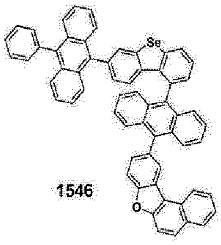

1546

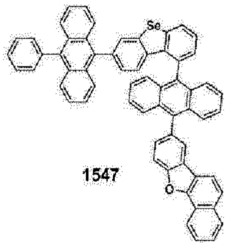

1547

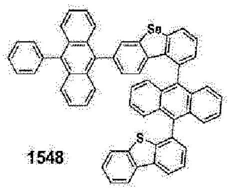

1548

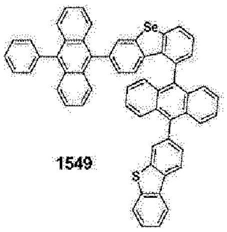

1549

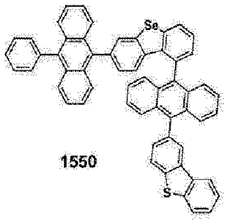

1550

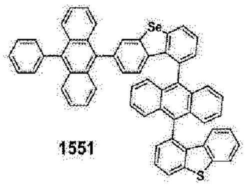

1551

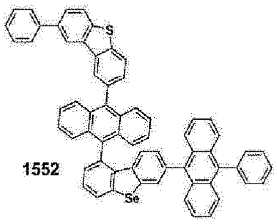

1552

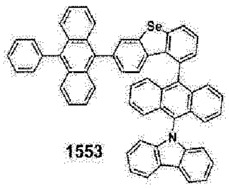

1553

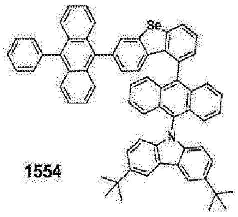

1554

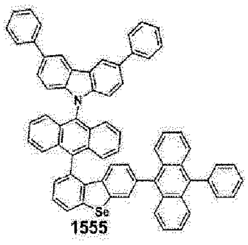

1555

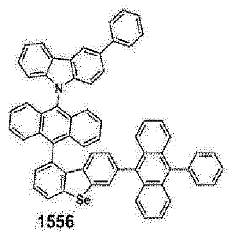

1556

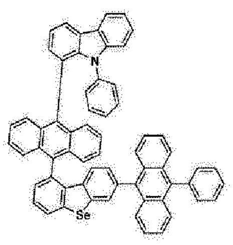

1557

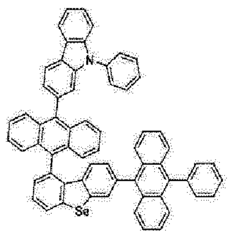

1558

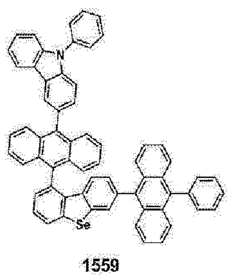

1559

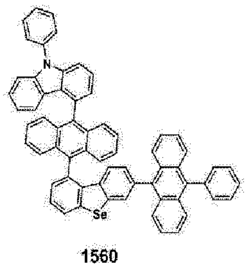

1560

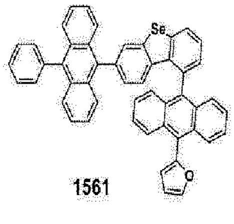

1561

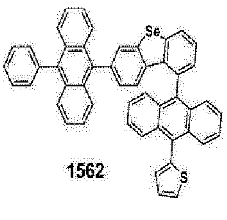

1562

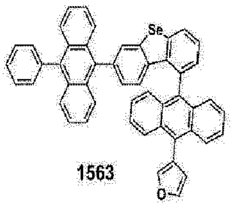

1563

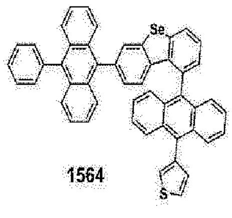

1564

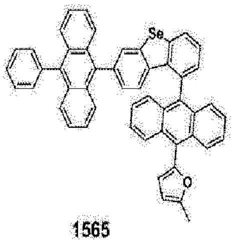
1565

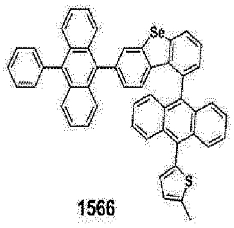
1566

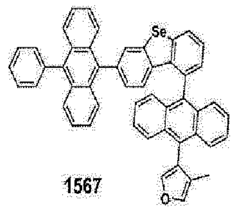
1567

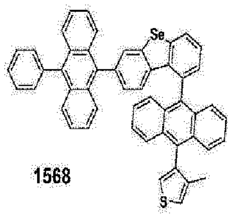
1568

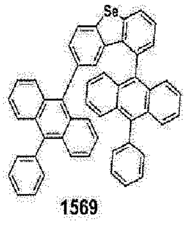
1569

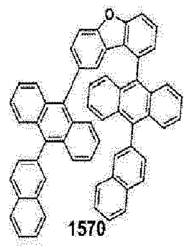
1570

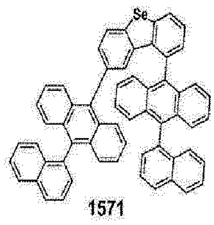
1571

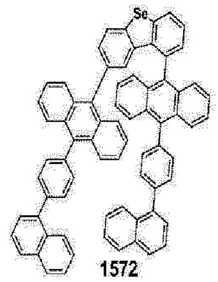
1572

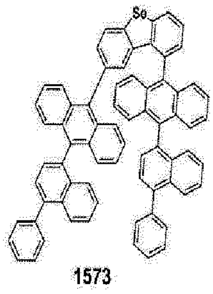
1573

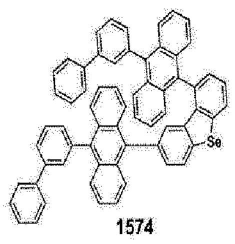
1574

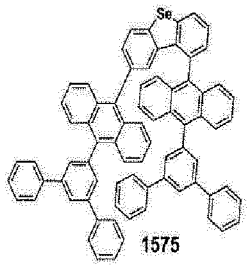
1575

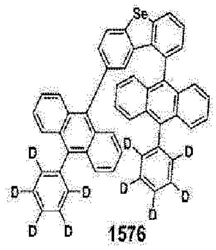
1576

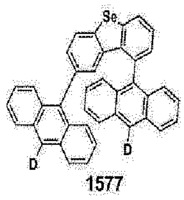
1577

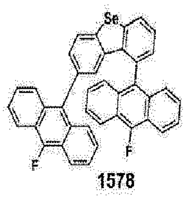
1578

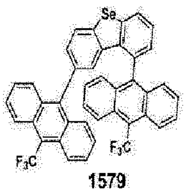
1579

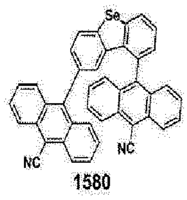
1580

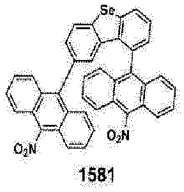
1581

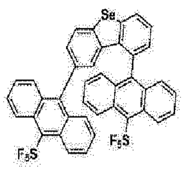
1582

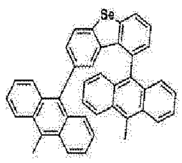
1583

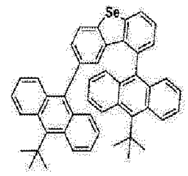
1584

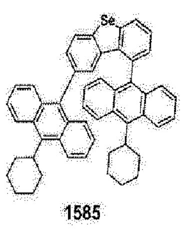
1585

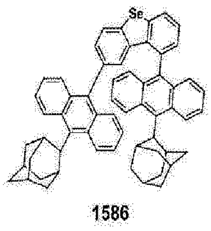
1586

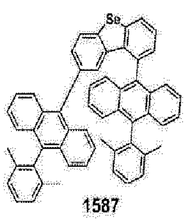
1587

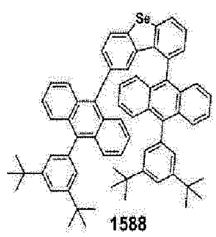
1588

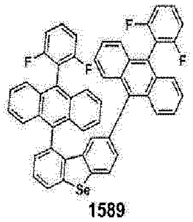
1589

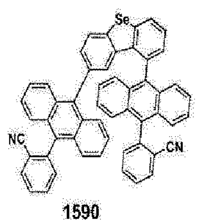
1590

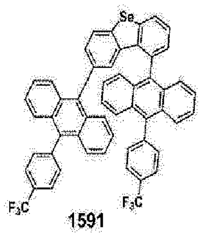
1591

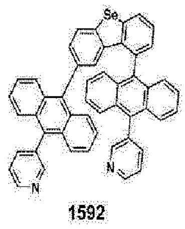
1592

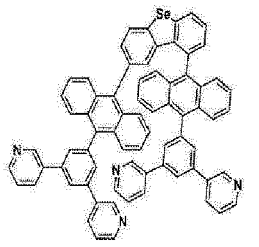
1593

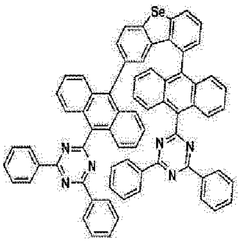
1594

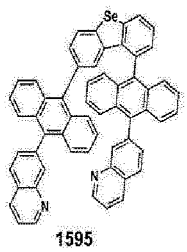
1595

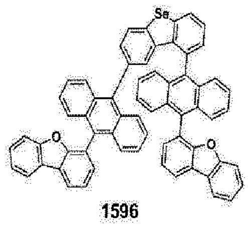
1596

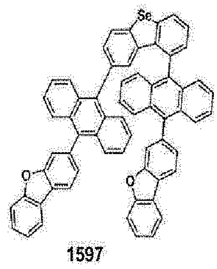
1597

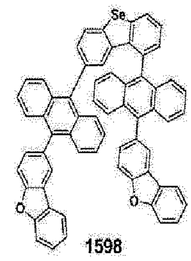
1598

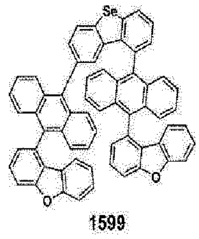
1599

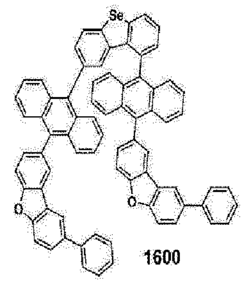
1600

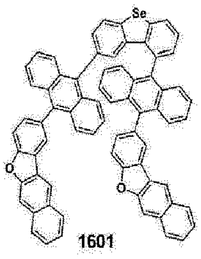
1601

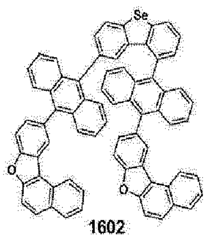
1602

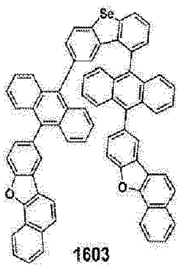
1603

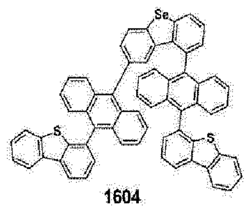
1604

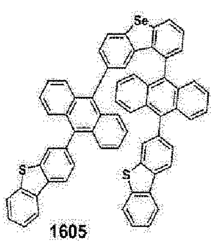
1605

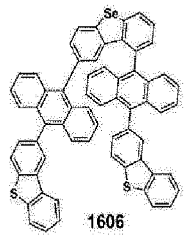
1606

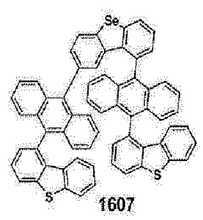
1607

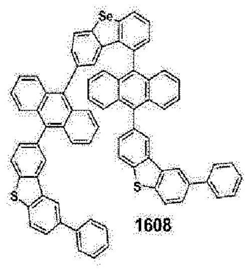
1608

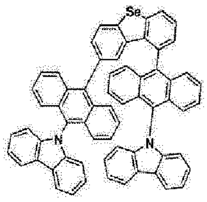
1609

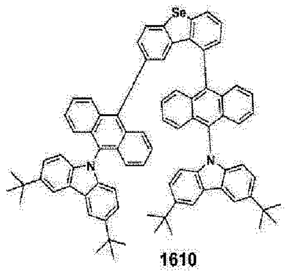
1610

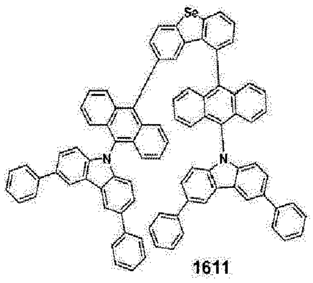
1611

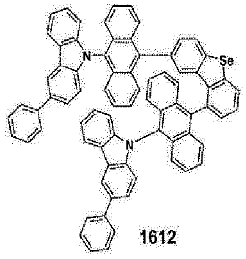
1612

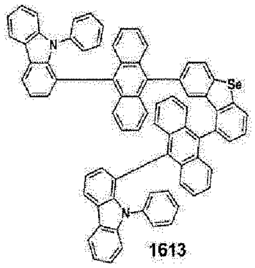
1613

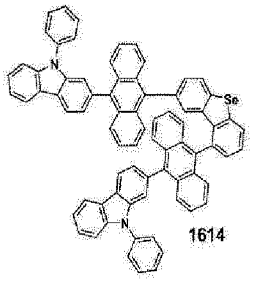
1614

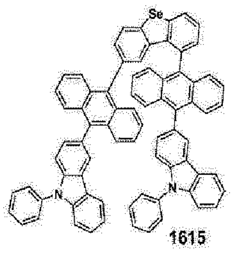
1615

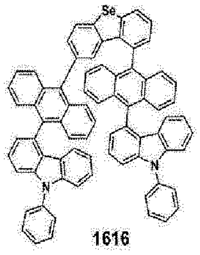
1616

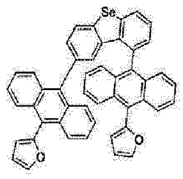
1617

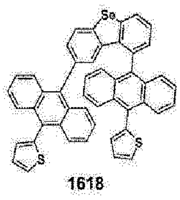
1618

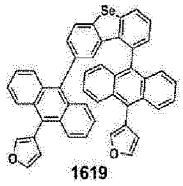
1619

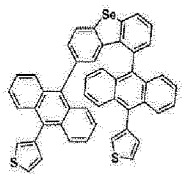
1620

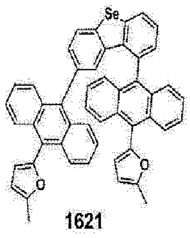
1621

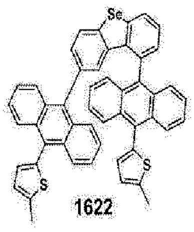
1622

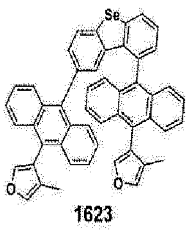
1623

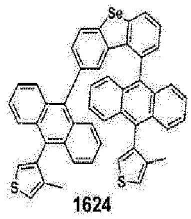
1624

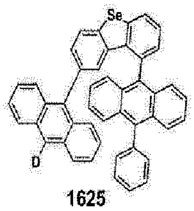
1625

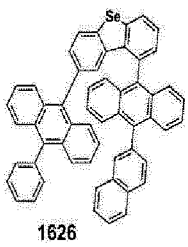
1626

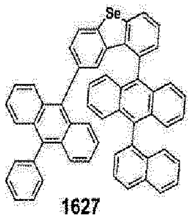
1627

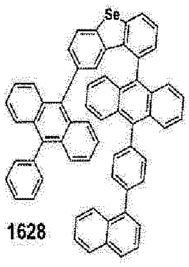
1628

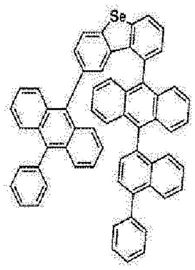

1629

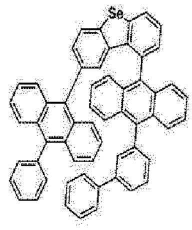

1630

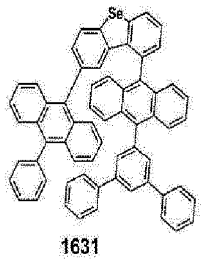

1631

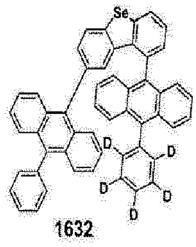

1632

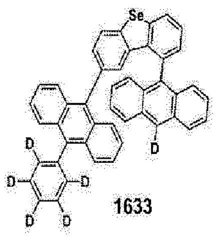

1633

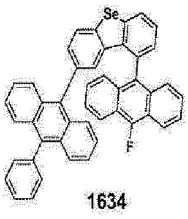

1634

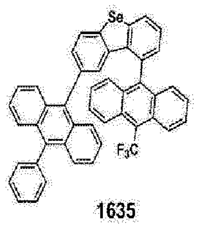

1635

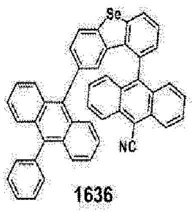

1636

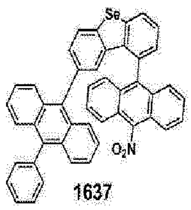

1637

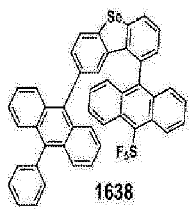

1638

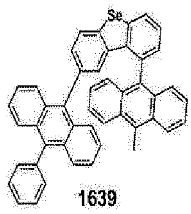

1639

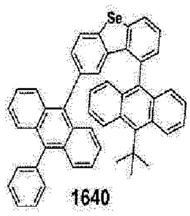

1640

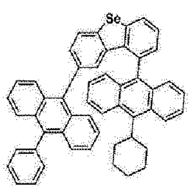

1641

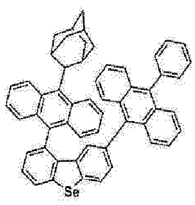

1642

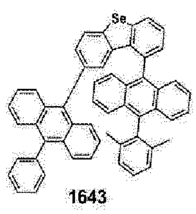

1643

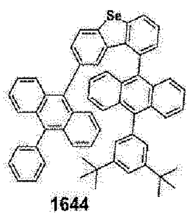

1644

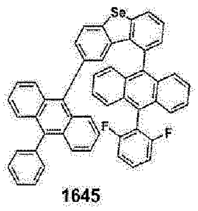

1645

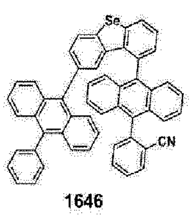

1646

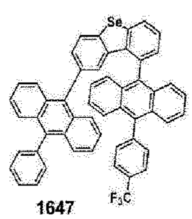

1647

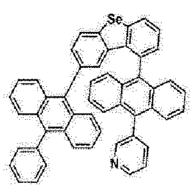

1648

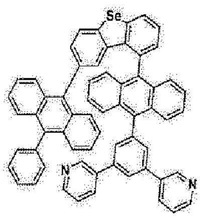

1649

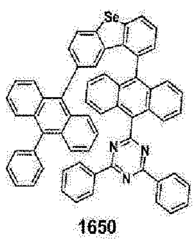

1650

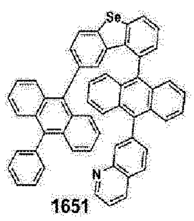

1651

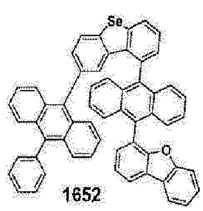

1652

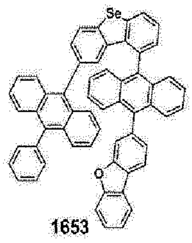
1653

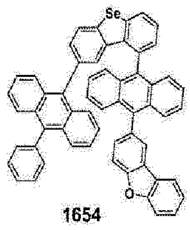
1654

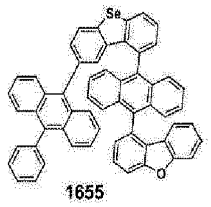
1655

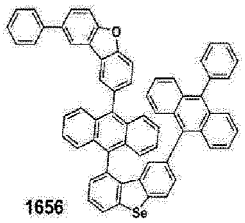
1656

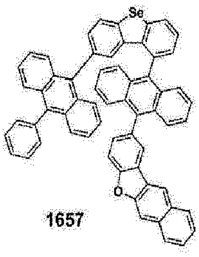
1657

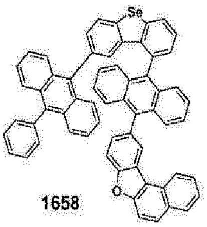
1658

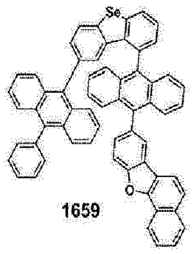
1659

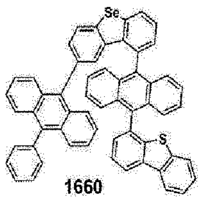
1660

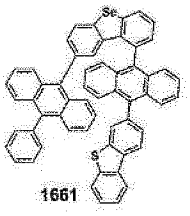
1661

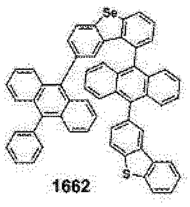
1662

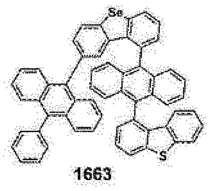
1663

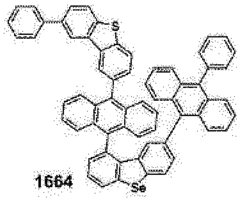
1664

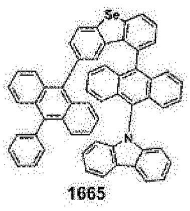
1665

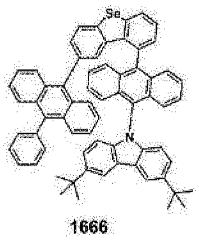
1666

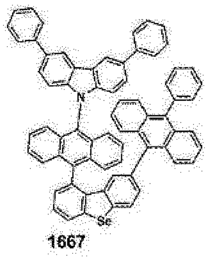
1667

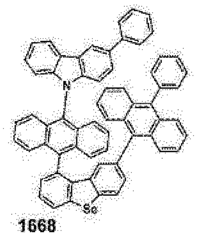
1668

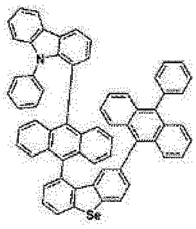
1669

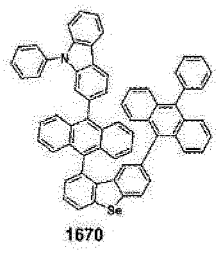
1670

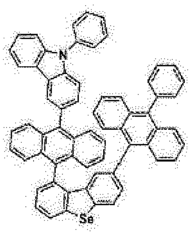
1671

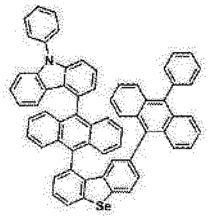
1672

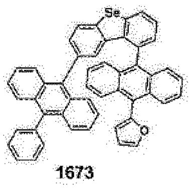
1673

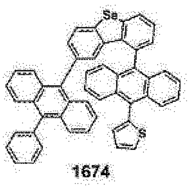
1674

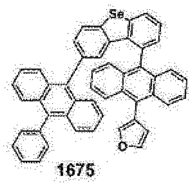
1675

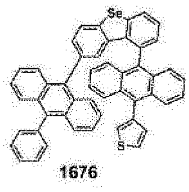
1676

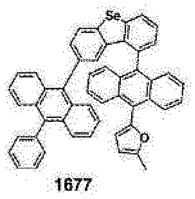
1677

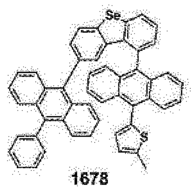
1678

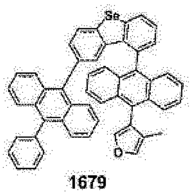
1679

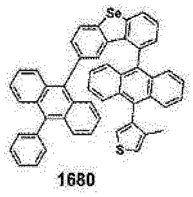
1680

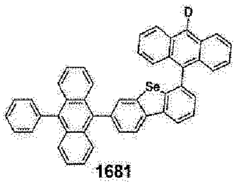
1681

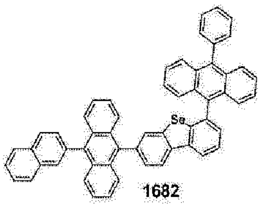
1682

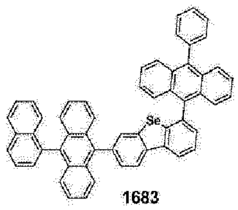
1683

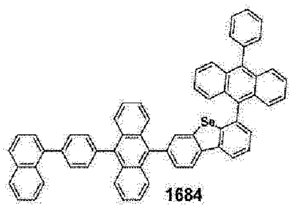
1684

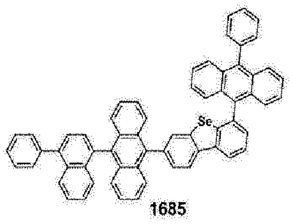
1685

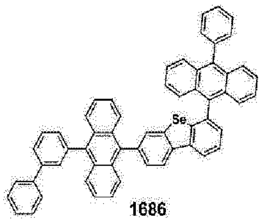
1686

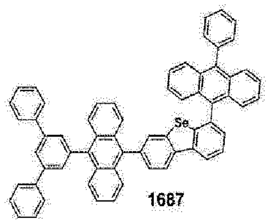
1687

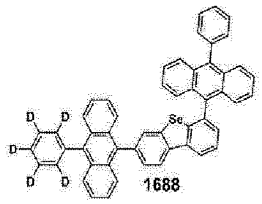
1688

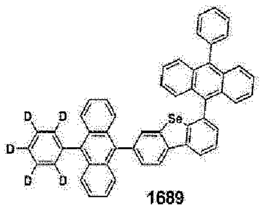
1689

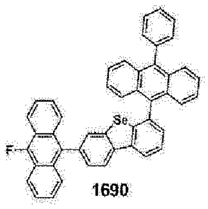
1690

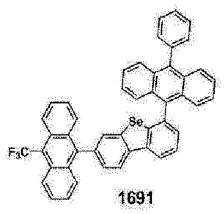
1691

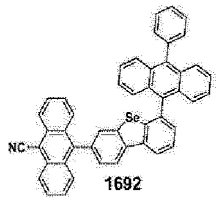
1692

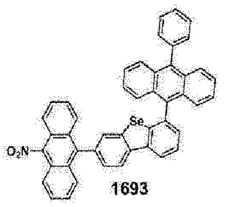
1693

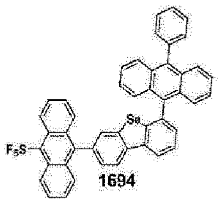
1694

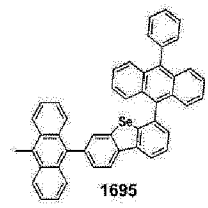
1695

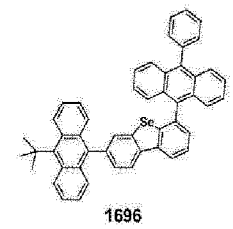
1696

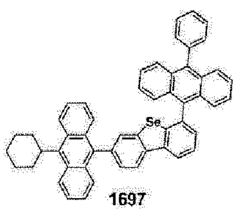
1697

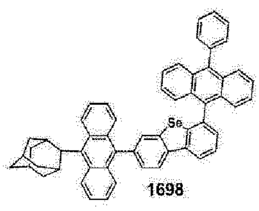
1698

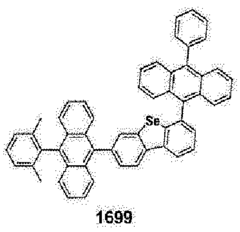
1699

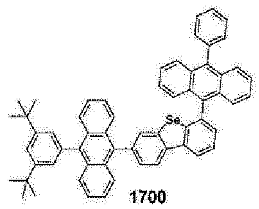
1700

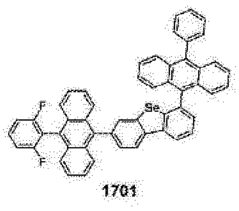
1701
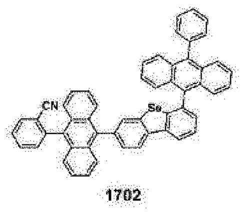
1702
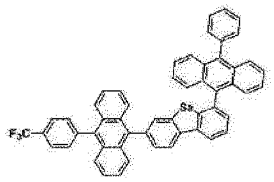
1703
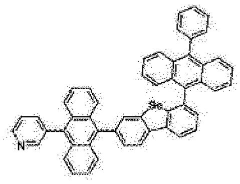
1704
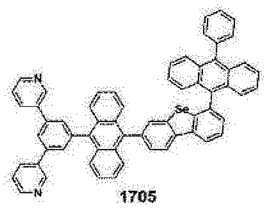
1705
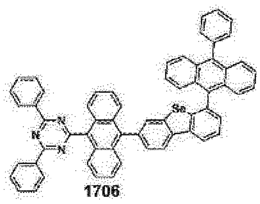
1706
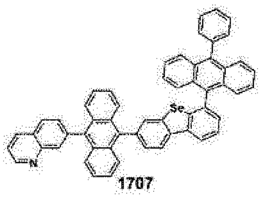
1707
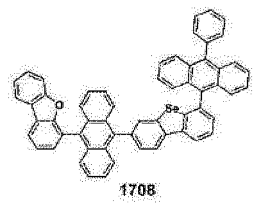
1708
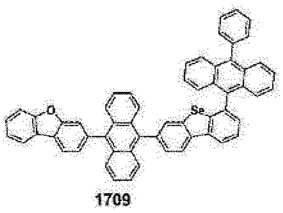
1709
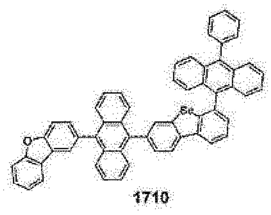
1710
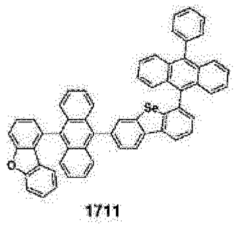
1711
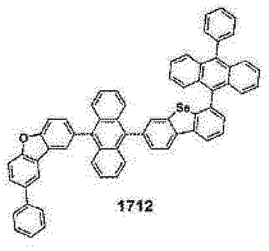
1712
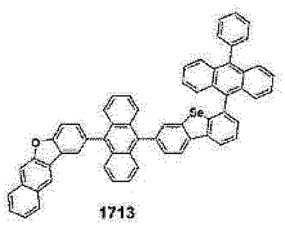
1713
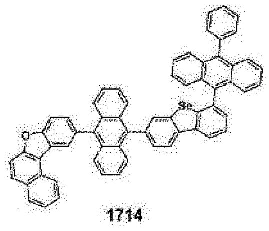
1714
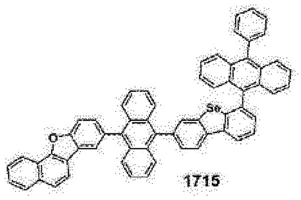
1715
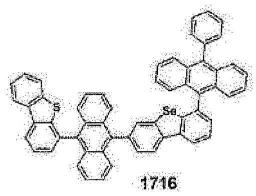
1716
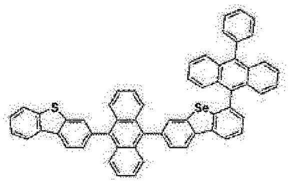
1717
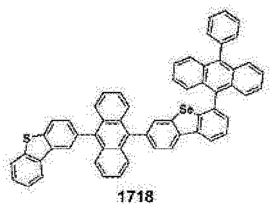
1718
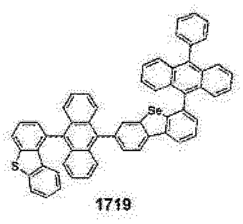
1719
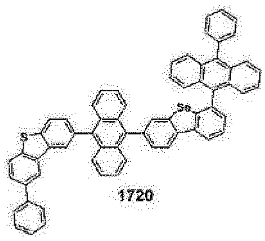
1720
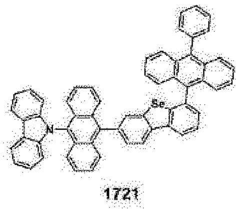
1721
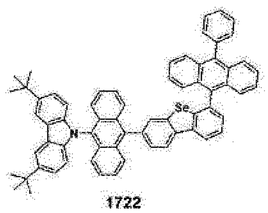
1722
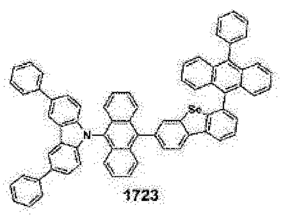
1723
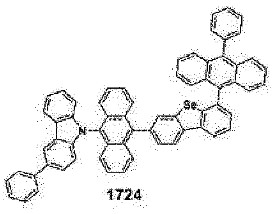
1724

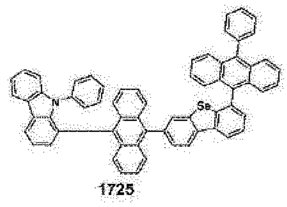
1725

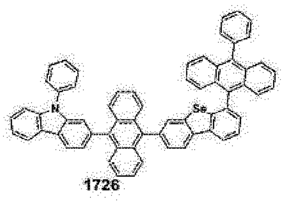
1726

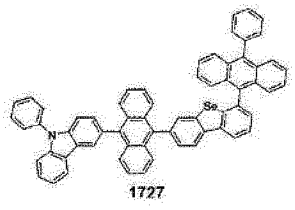
1727

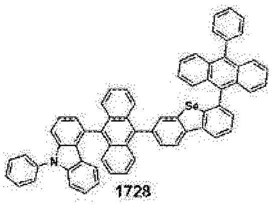
1728

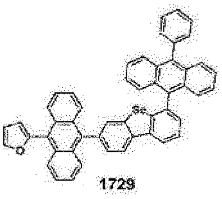
1729

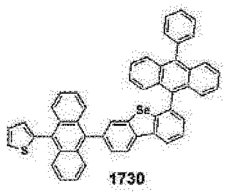
1730

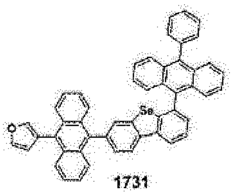
1731

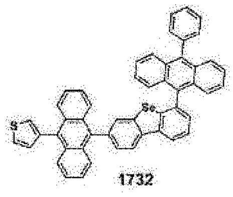
1732

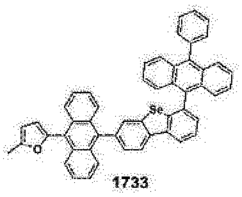
1733

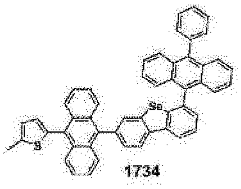
1734

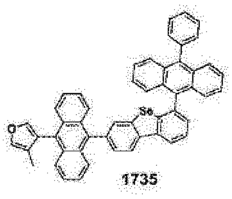
1735

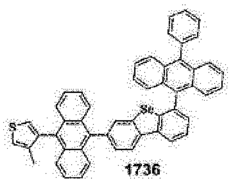
1736

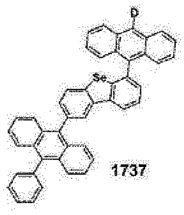
1737

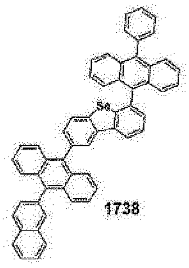
1738

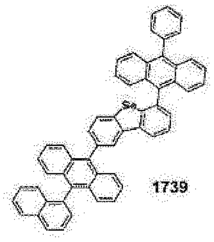
1739

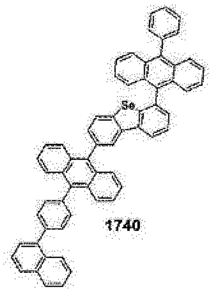
1740

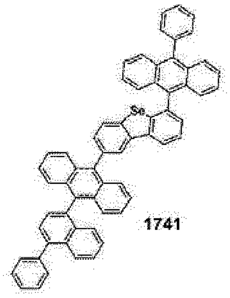
1741

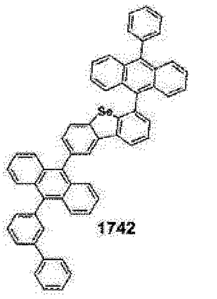
1742

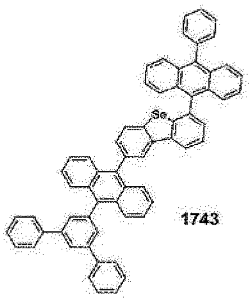
1743

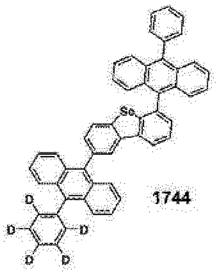
1744

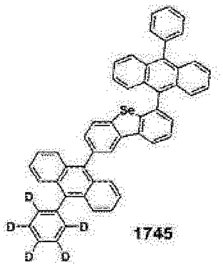
1745

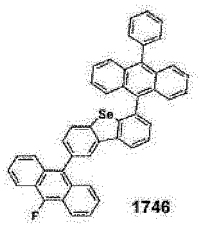
1746

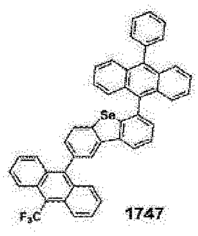
1747

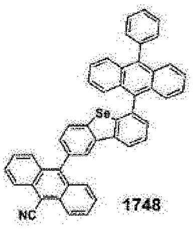
1748

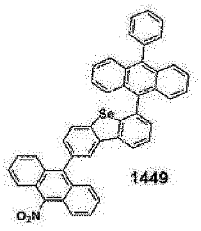
1449

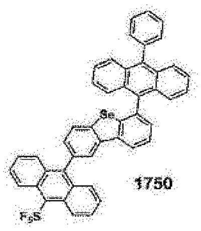
1750

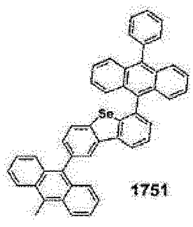
1751

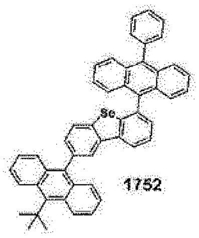
1752

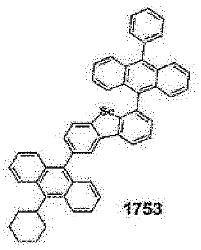
1753

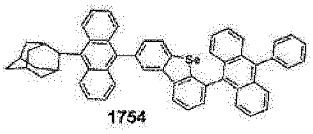
1754

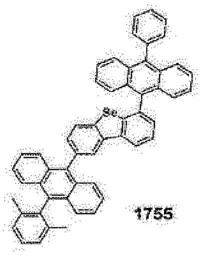
1755

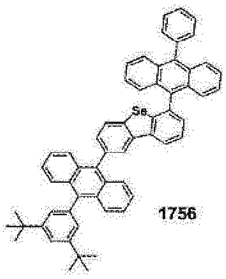
1756

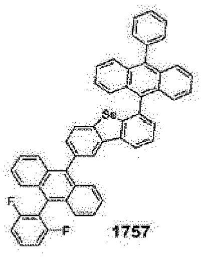
1757

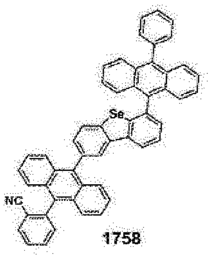
1758

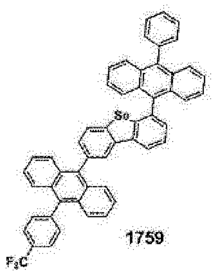
1759

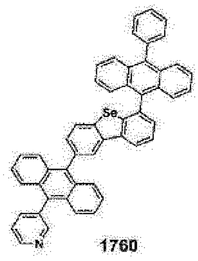
1760

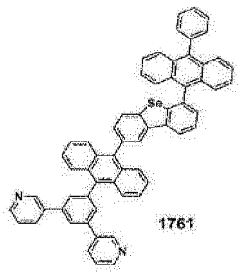
1761

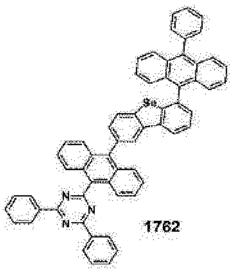
1762

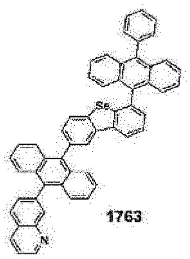
1763

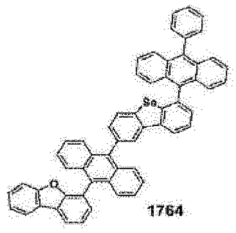
1764

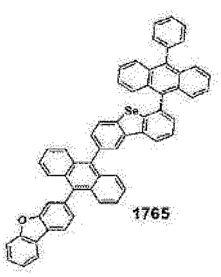
1765

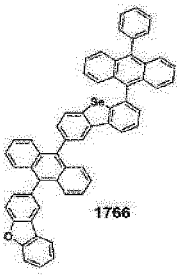
1766

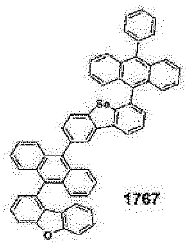
1767

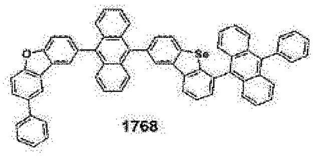
1768

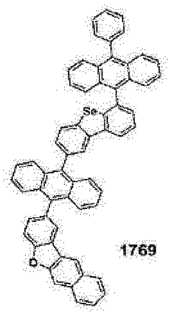
1769

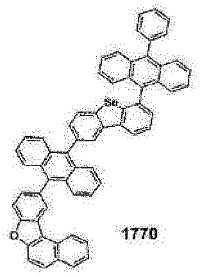
1770

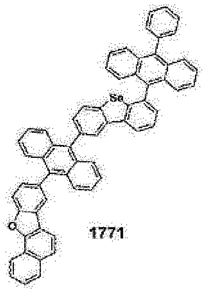
1771

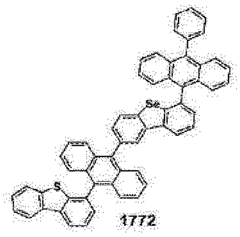
1772

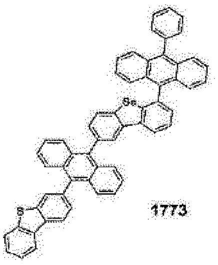
1773

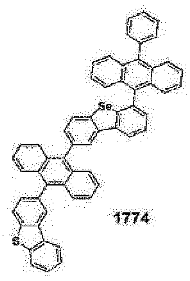
1774

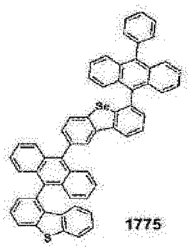
1775

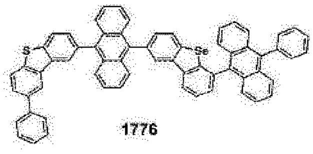
1776

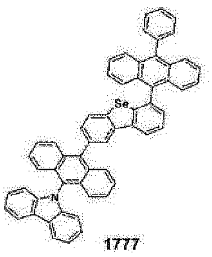
1777

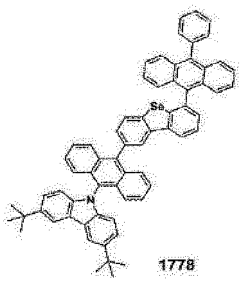
1778

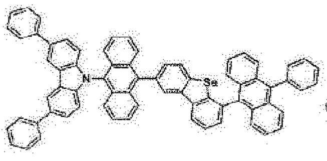
1779

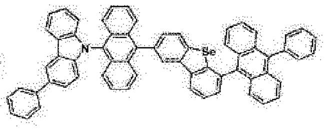
1780

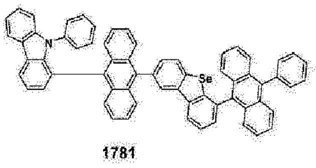
1781

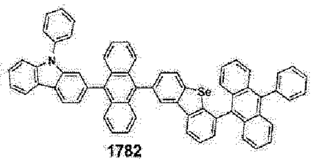
1782

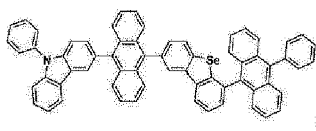
1783

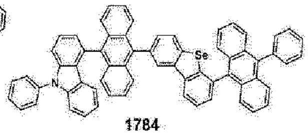
1784

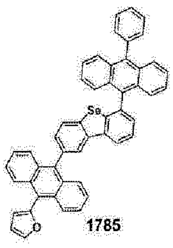
1785

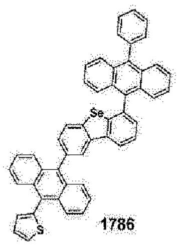
1786

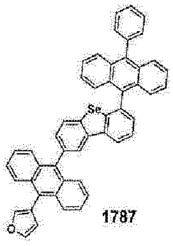
1787

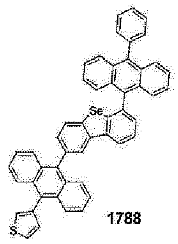
1788

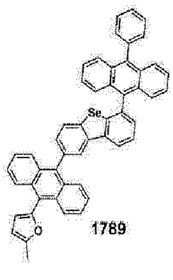
1789

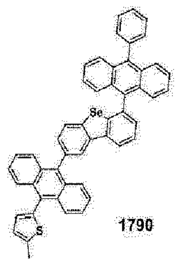
1790

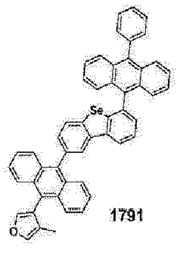
1791

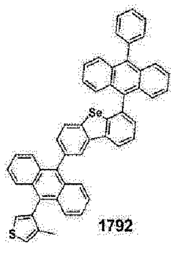
1792

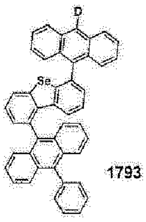
1793

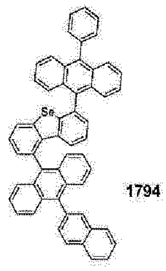
1794

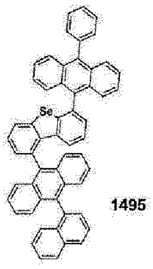
1495

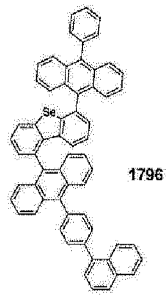
1796

1797
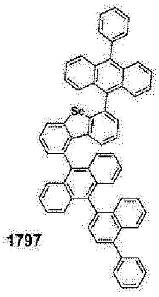

1798
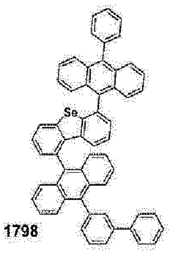

1799
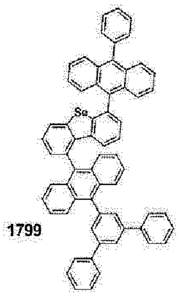

1800
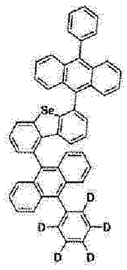

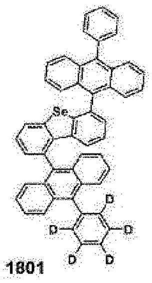
1801

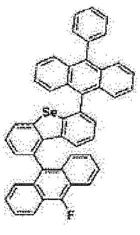
1802

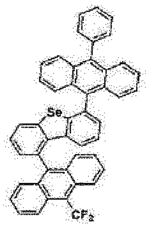
1803

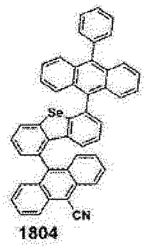
1804

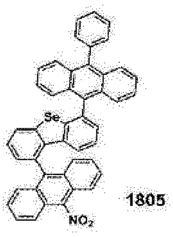
1805

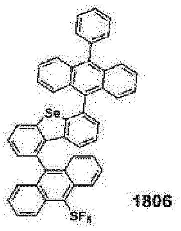
1806

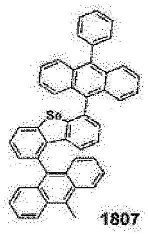
1807

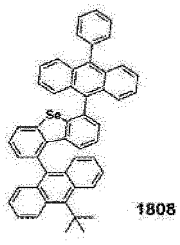
1808

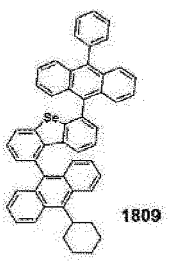
1809

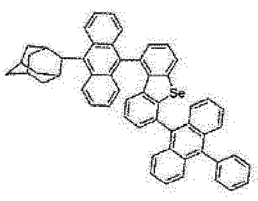
1810

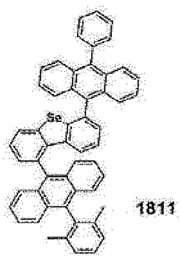
1811

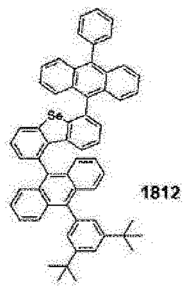
1812

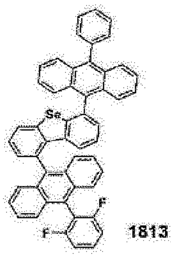
1813

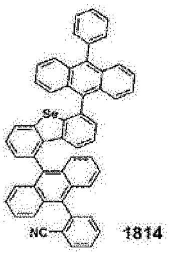
1814

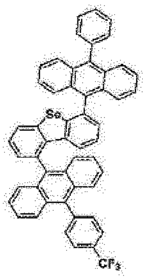
1815

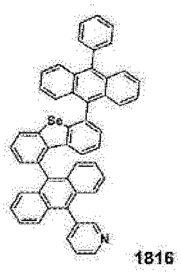
1816

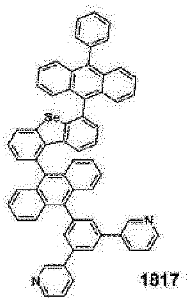
1817

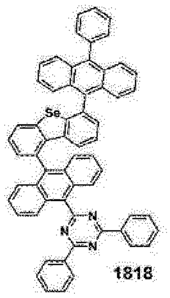
1818

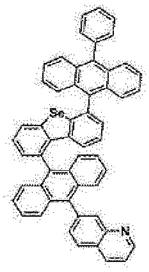
1819

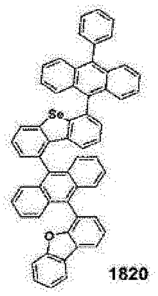
1820

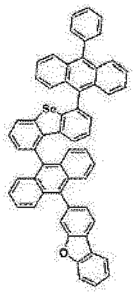
1821

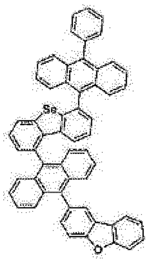
1822

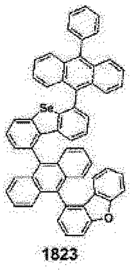
1823

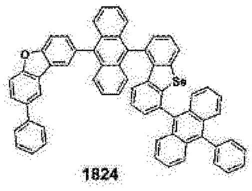
1824

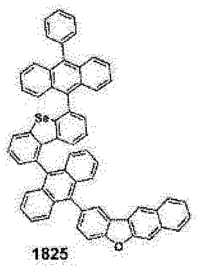
1825

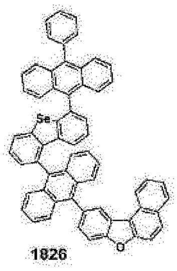
1826

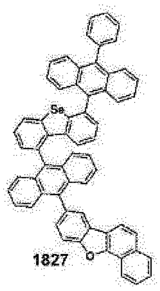
1827

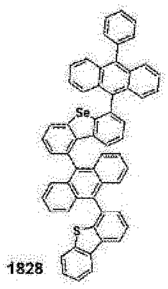
1828

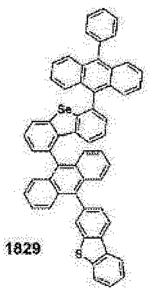
1829

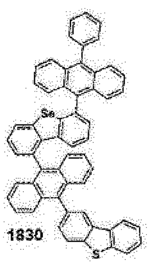
1830

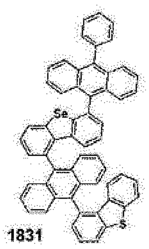
1831

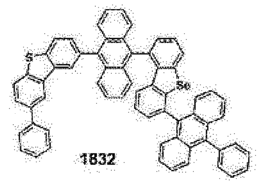
1832

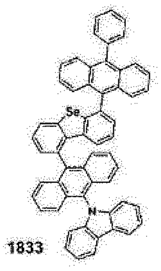
1833
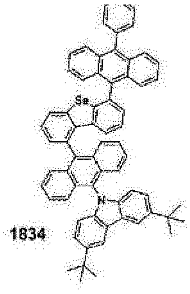
1834
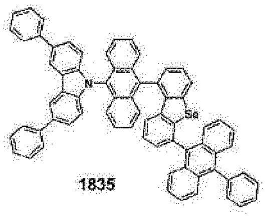
1835
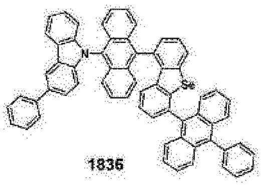
1836

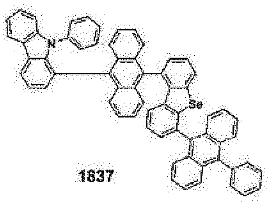
1837
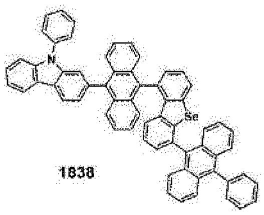
1838
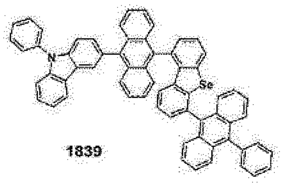
1839
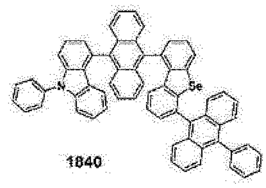
1840

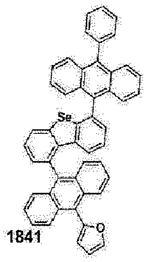
1841
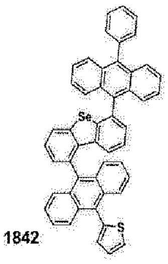
1842
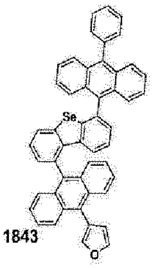
1843
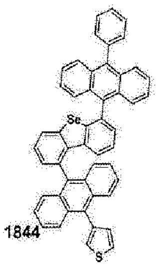
1844

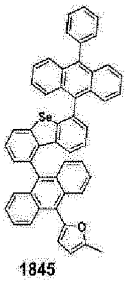
1845
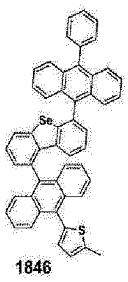
1846
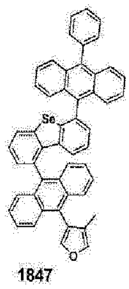
1847
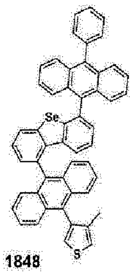
1848

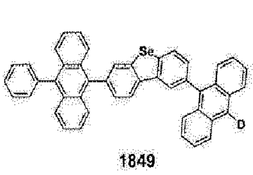
1849
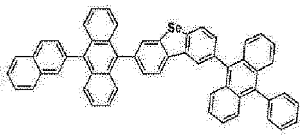
1850
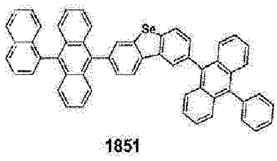
1851
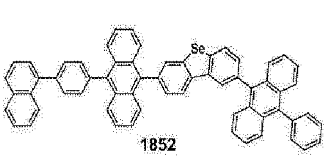
1852

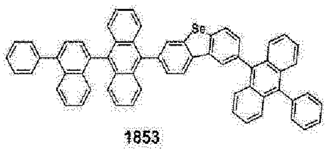
1853
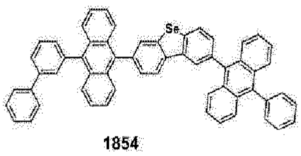
1854
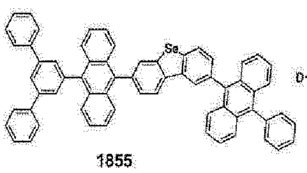
1855
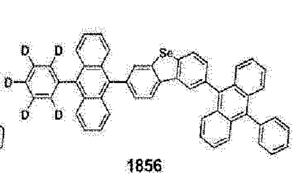
1856

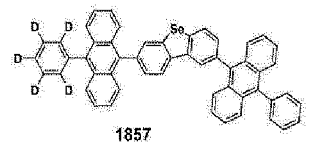
1857

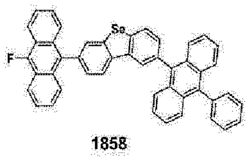
1858

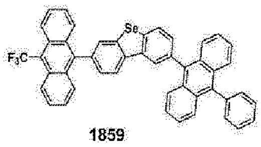
1859

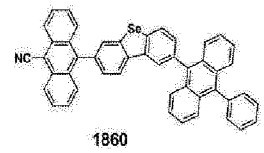
1860

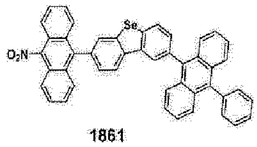
1861

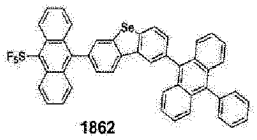
1862

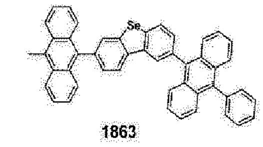
1863

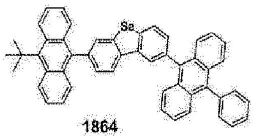
1864

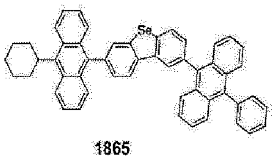
1865

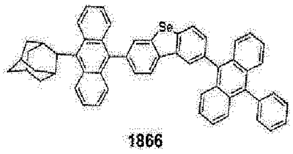
1866

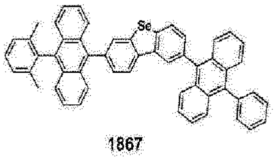
1867

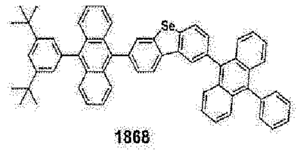
1868

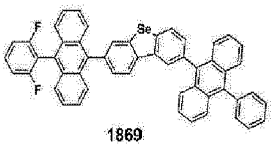
1869

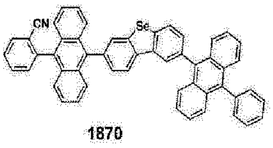
1870

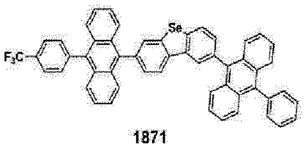
1871

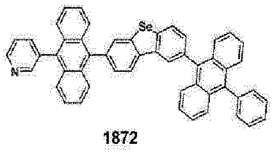
1872

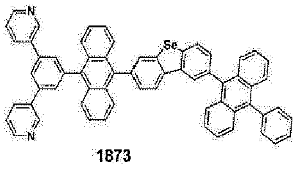
1873

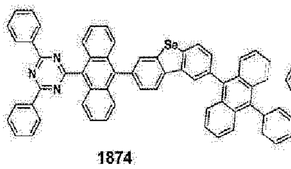
1874

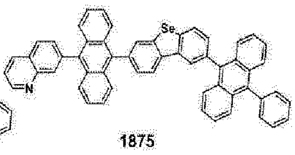
1875

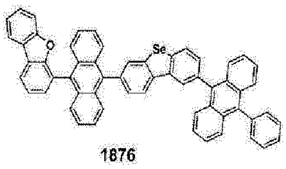
1876

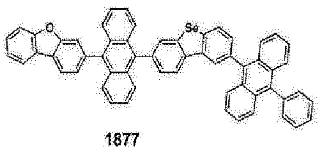
1877

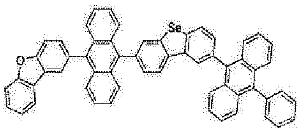
1878

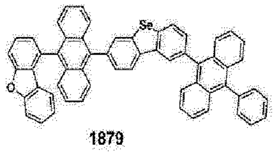
1879

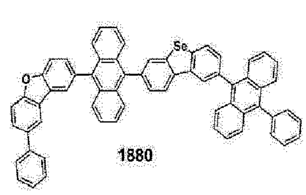
1880

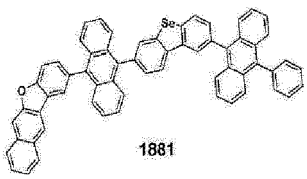
1881

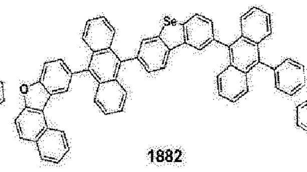
1882

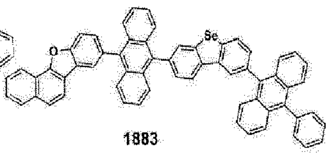
1883

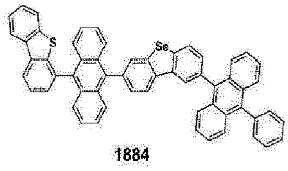
1884

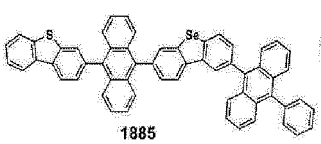
1885

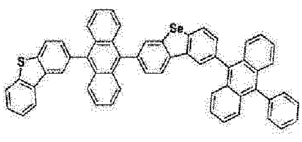
1886

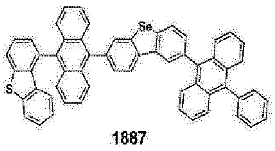
1887

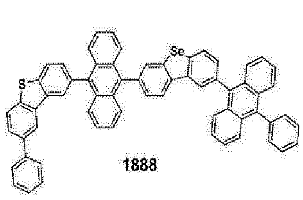
1888

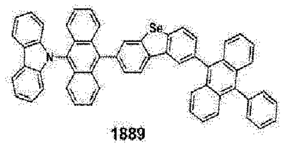
1889

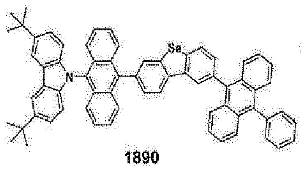
1890

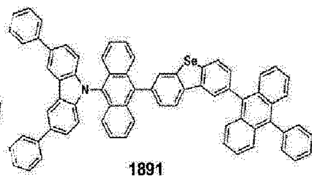
1891

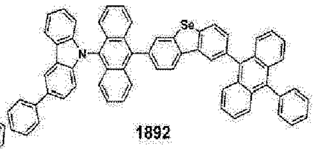
1892

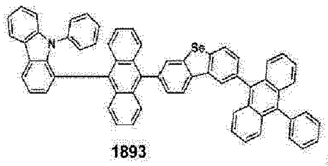
1893

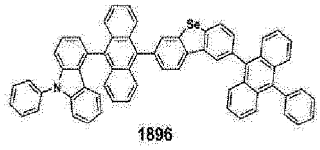
1896

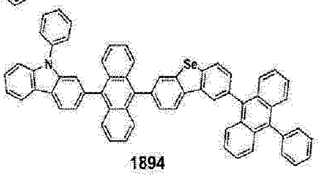
1894

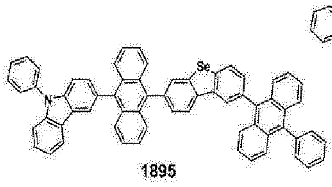
1895

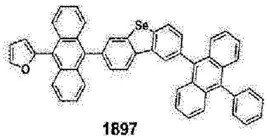
1897

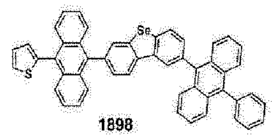
1898

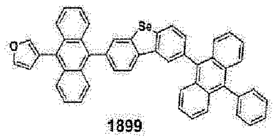
1899

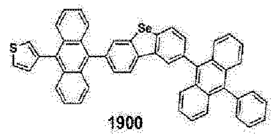
1900

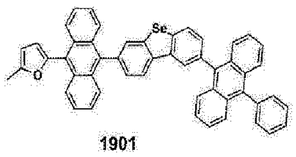
1901

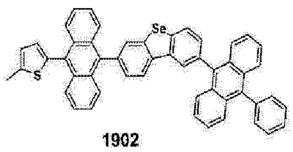
1902

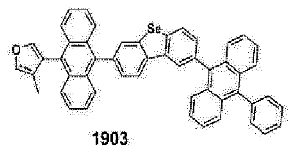
1903

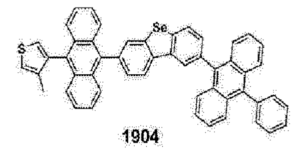
1904

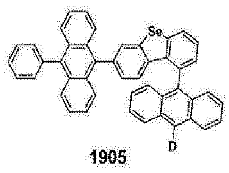
1905

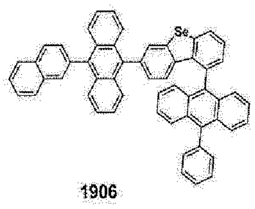
1906

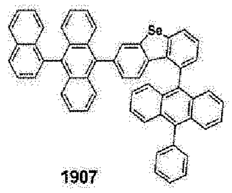
1907

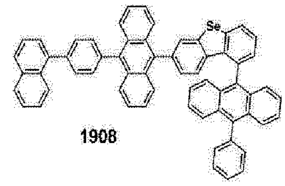
1908

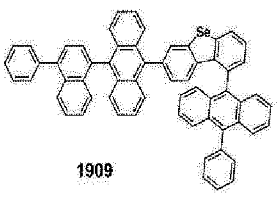
1909

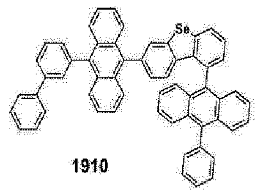
1910

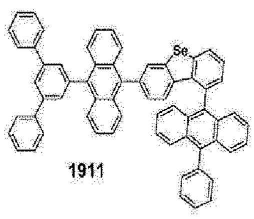
1911

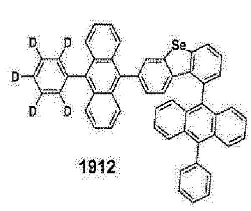
1912

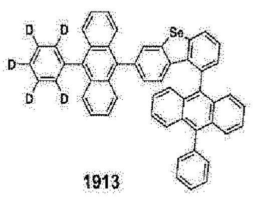
1913

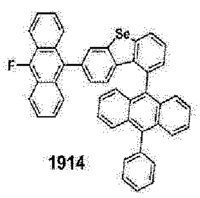
1914

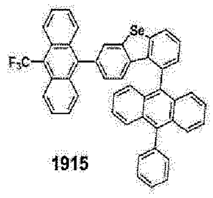
1915

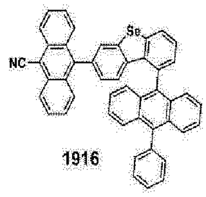
1916

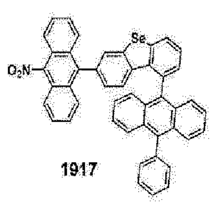
1917

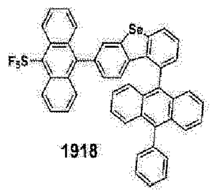
1918

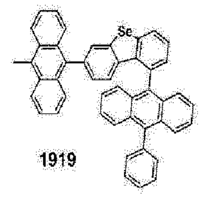
1919

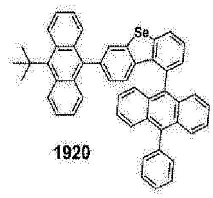
1920

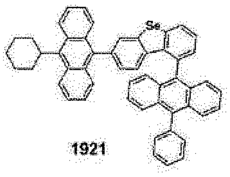
1921

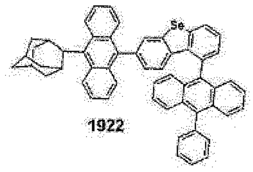
1922

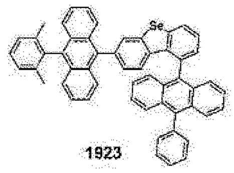
1923

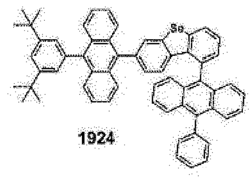
1924

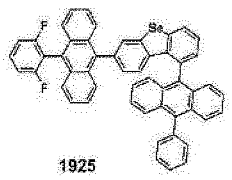
1925

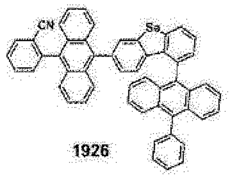
1926

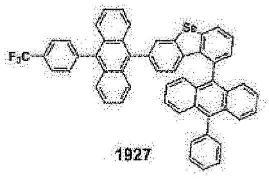
1927

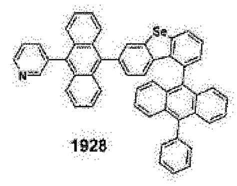
1928

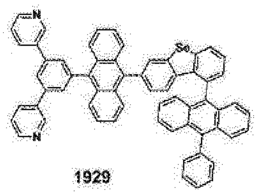
1929

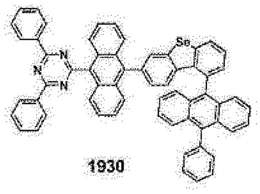
1930

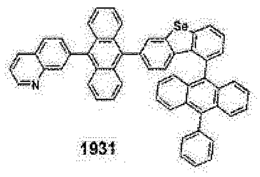
1931

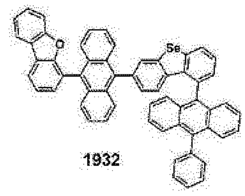
1932

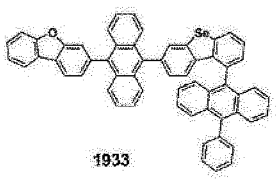
1933

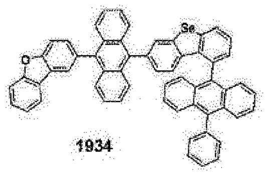
1934

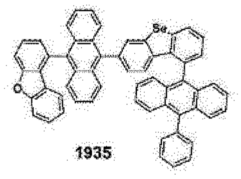
1935

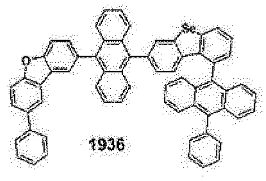
1936

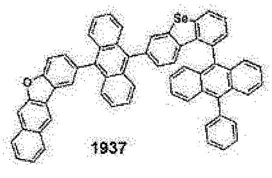
1937

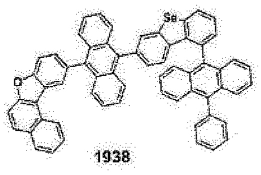
1938

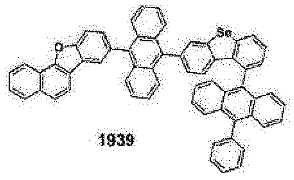
1939

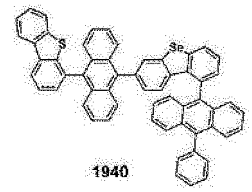
1940

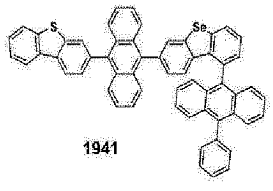
1941

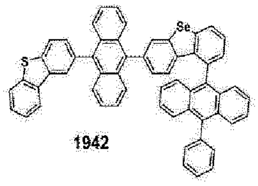
1942

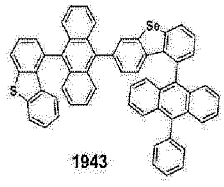
1943

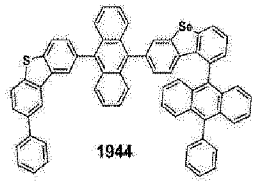
1944

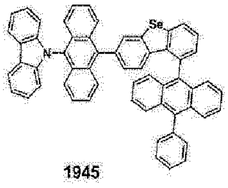
1945
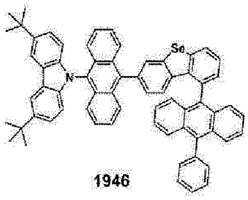
1946
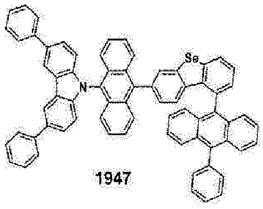
1947
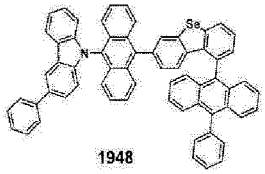
1948
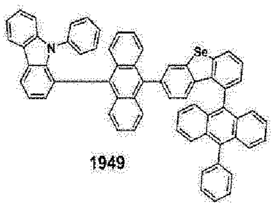
1949
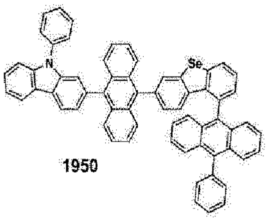
1950
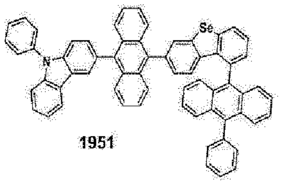
1951
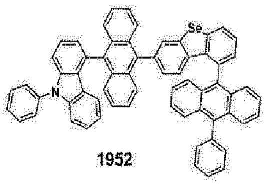
1952
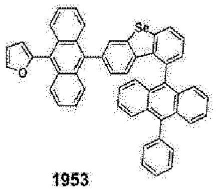
1953
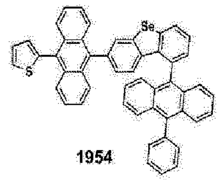
1954
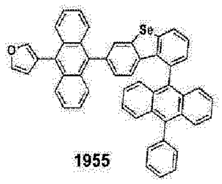
1955
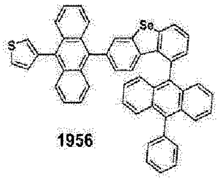
1956
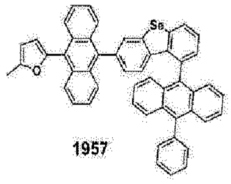
1957
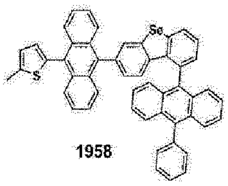
1958
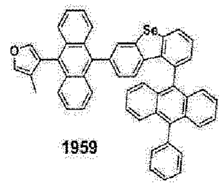
1959
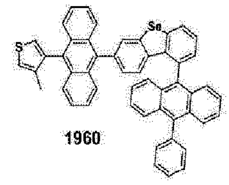
1960
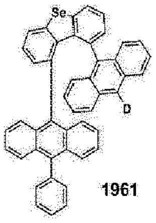
1961
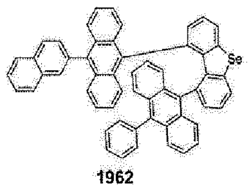
1962
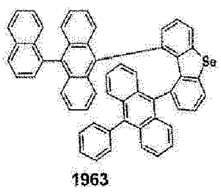
1963
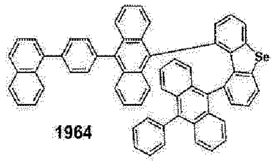
1964
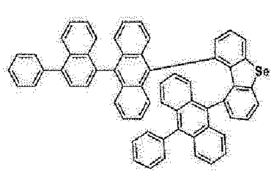
1965
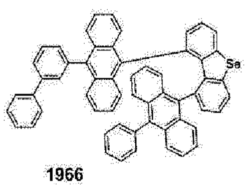
1966
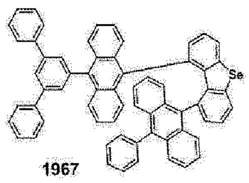
1967
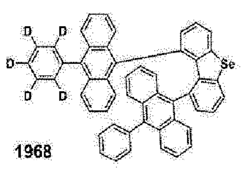
1968
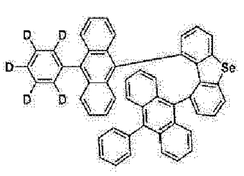
1969
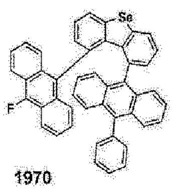
1970
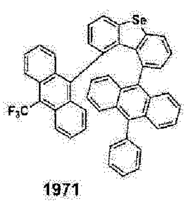
1971
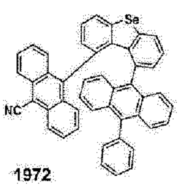
1972

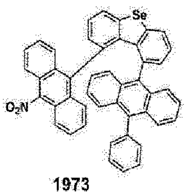
1973

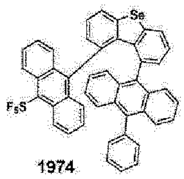
1974

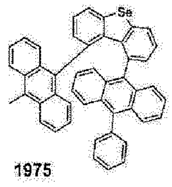
1975

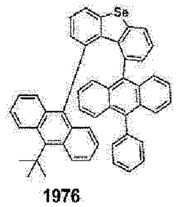
1976

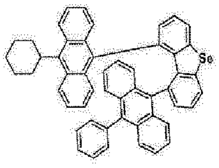
1977

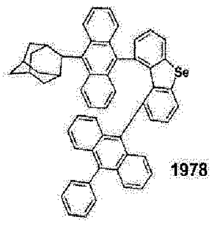
1978

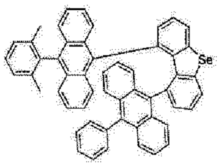
1979

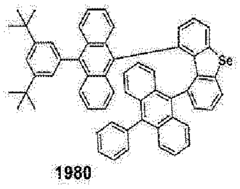
1980

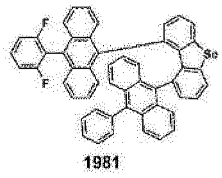
1981

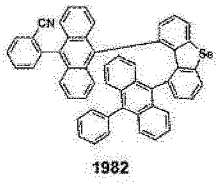
1982

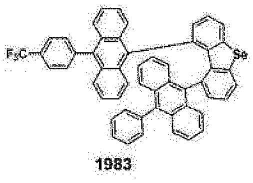
1983

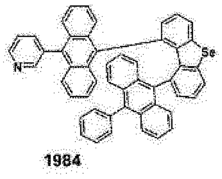
1984

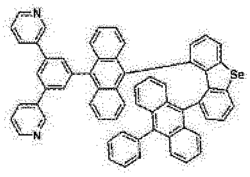
1985

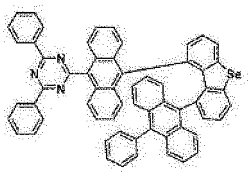
1986

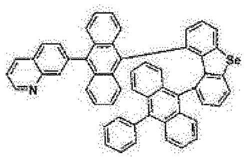
1987

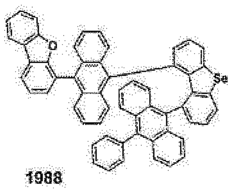
1988

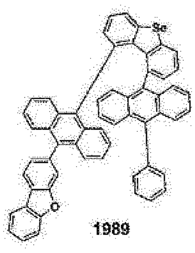
1989

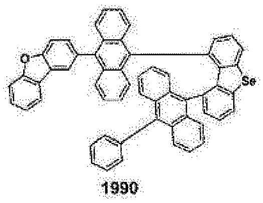
1990

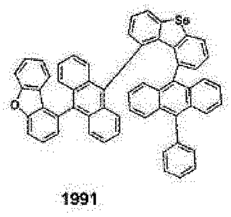
1991

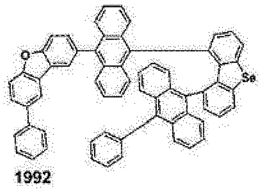
1992

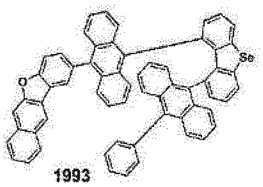
1993

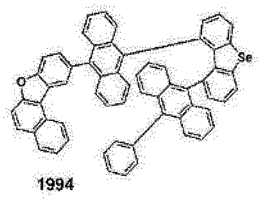
1994

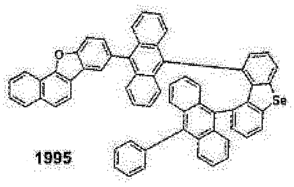
1995

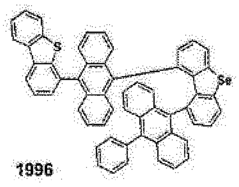
1996

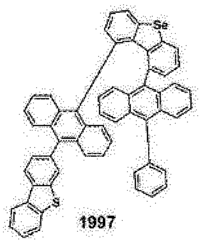
1997

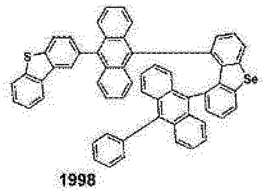
1998

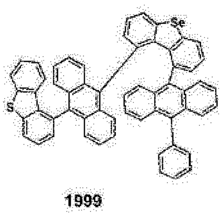
1999

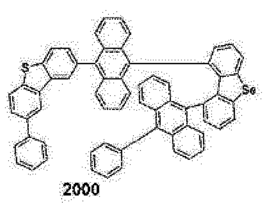
2000

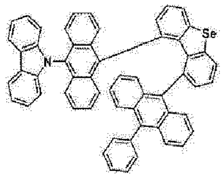
2001

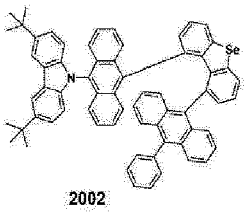
2002

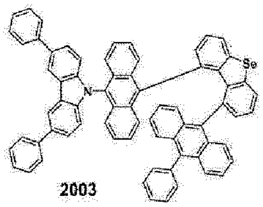
2003

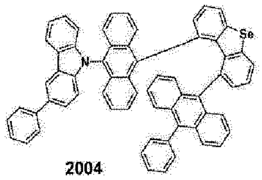
2004

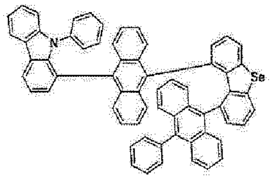
2005

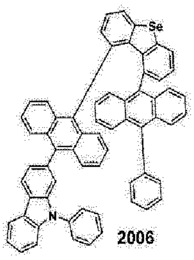
2006

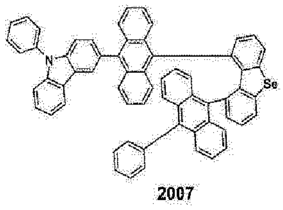
2007

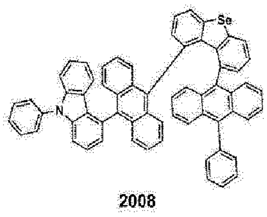
2008

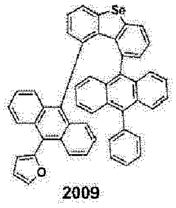
2009

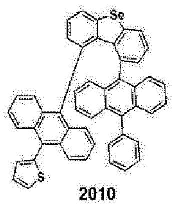
2010

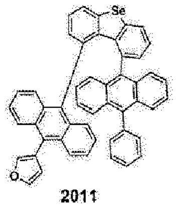
2011

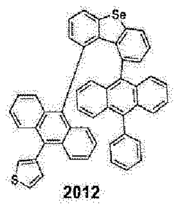
2012

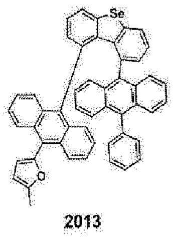
2013

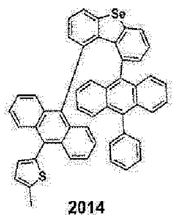
2014

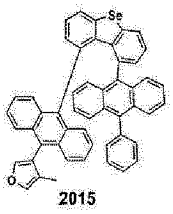
2015

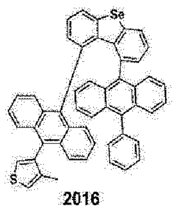
2016

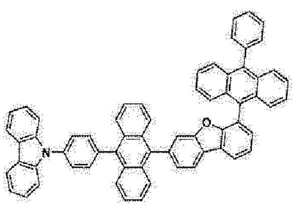
2017

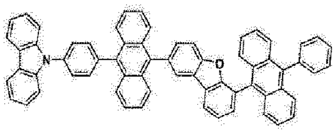
2018

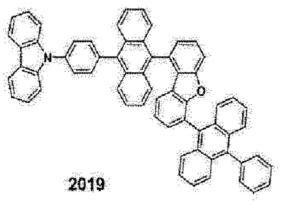
2019

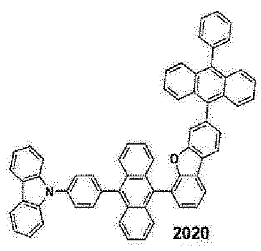
2020

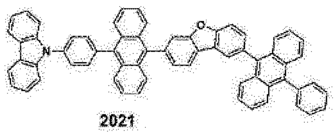
2021

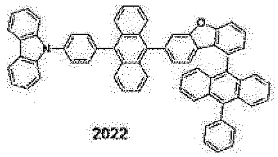
2022

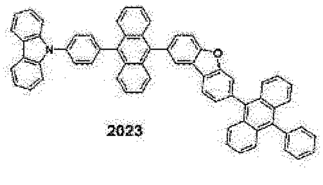
2023

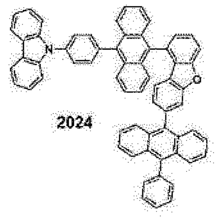
2024

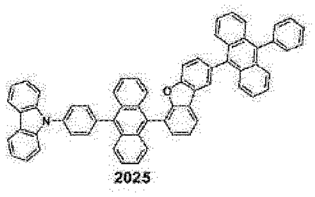
2025

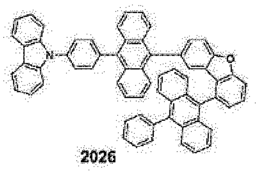
2026

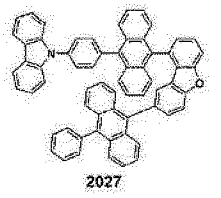
2027

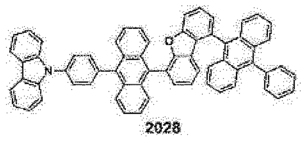
2028

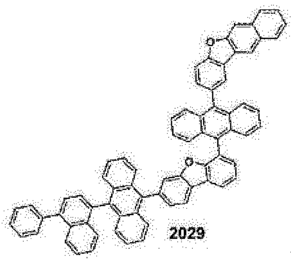
2029

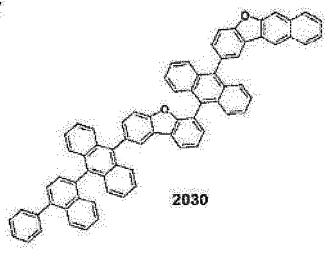
2030

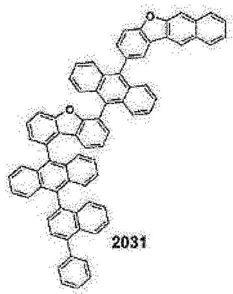
2031

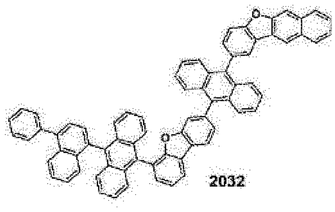
2032

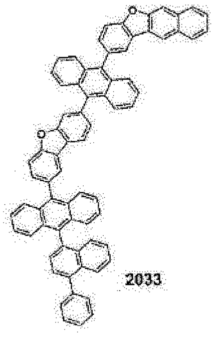
2033

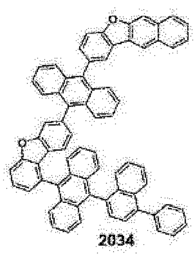
2034

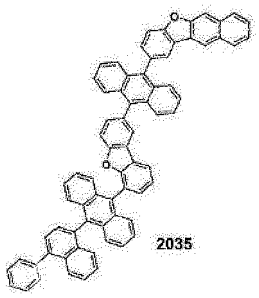
2035

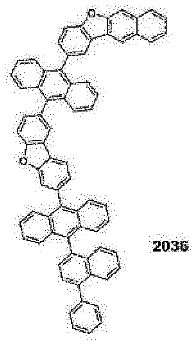
2036

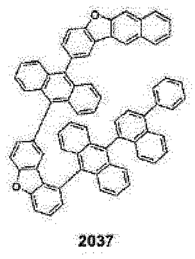
2037

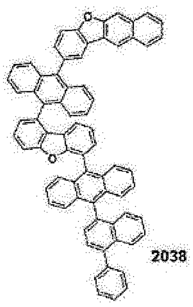
2038

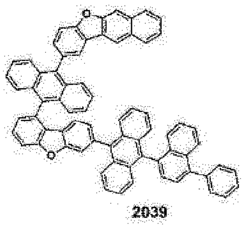
2039

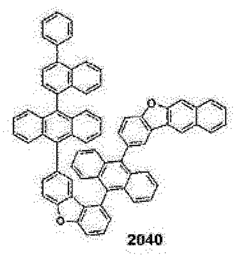
2040

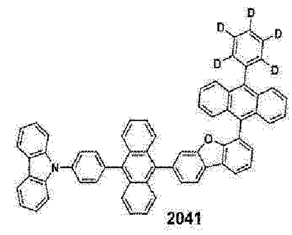
2041

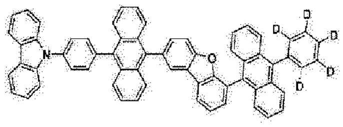
2042

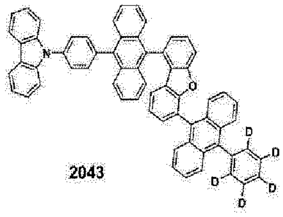
2043

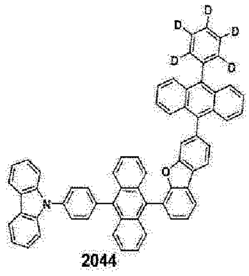
2044

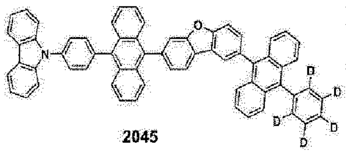
2045

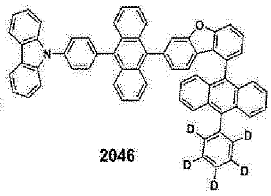
2046

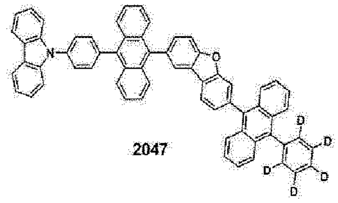
2047

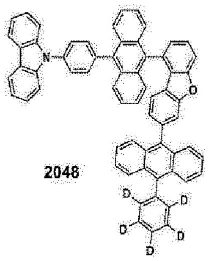
2048

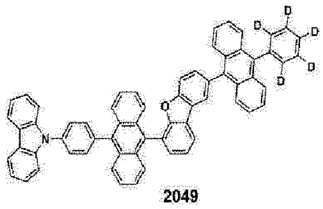
2049

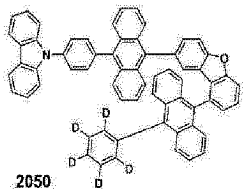
2050

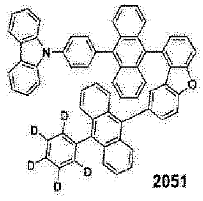
2051

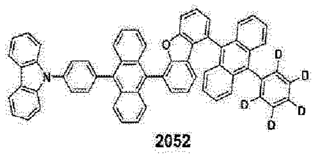
2052

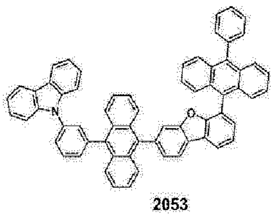
2053

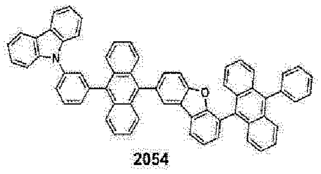
2054

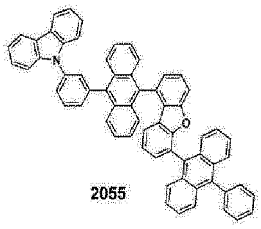
2055

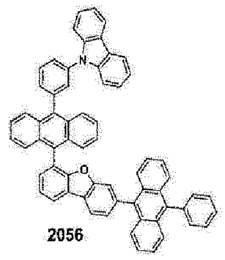
2056

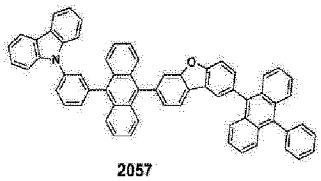
2057

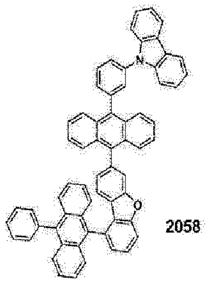
2058

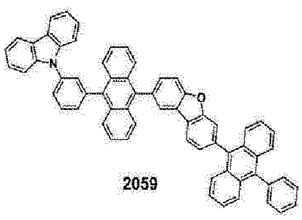
2059

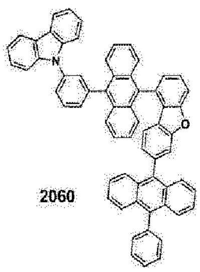
2060

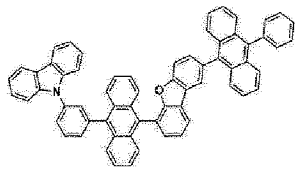
2061

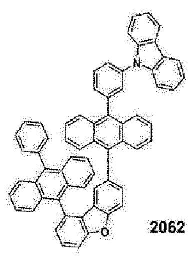
2062

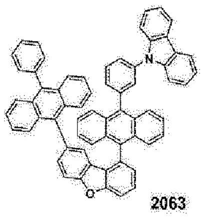
2063

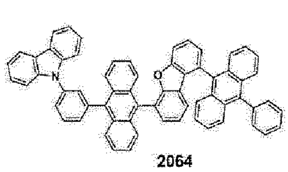
2064

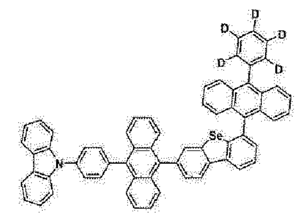
2065

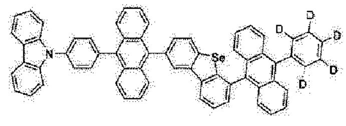
2066

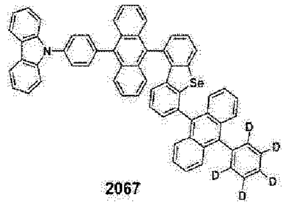
2067

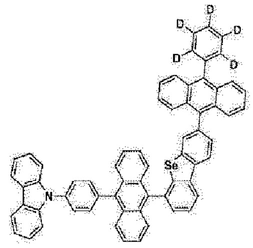
2068

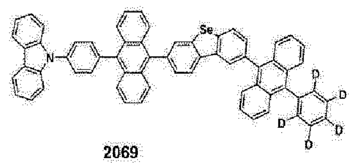
2069

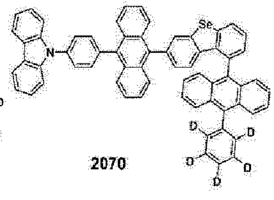
2070

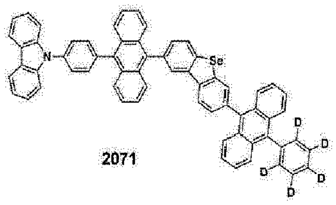
2071

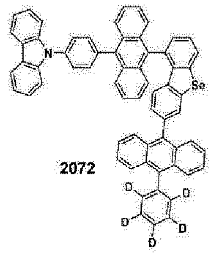
2072

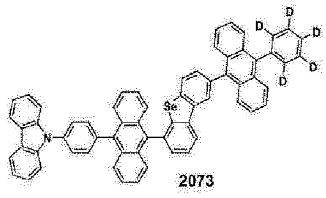
2073

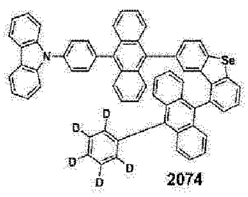
2074

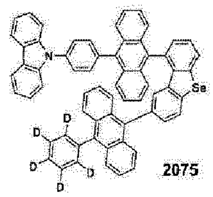
2075

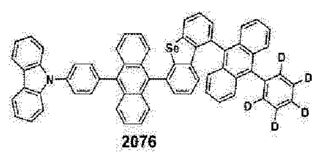
2076

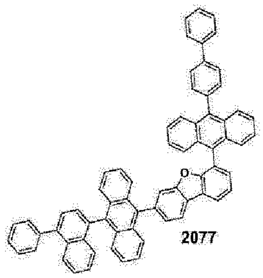
2077

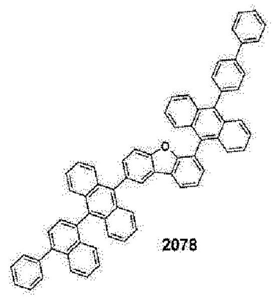
2078

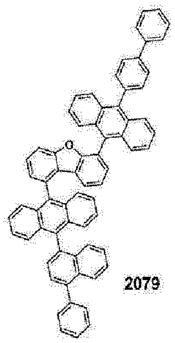
2079

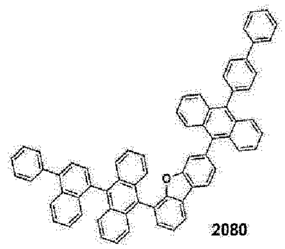
2080

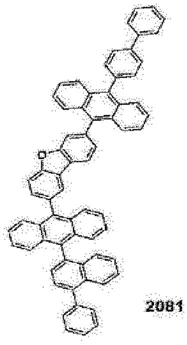
2081

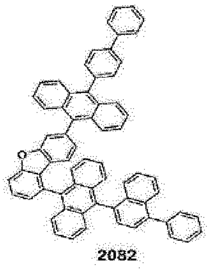
2082

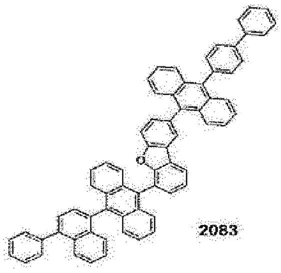
2083

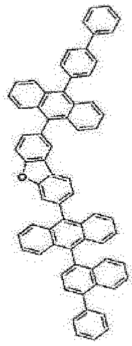
2084

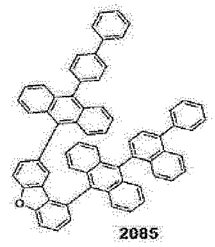
2085

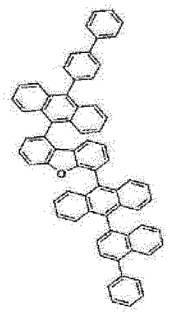
2086

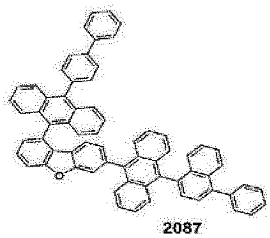
2087

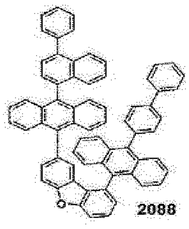
2088

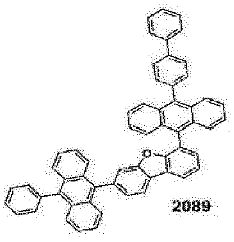
2089

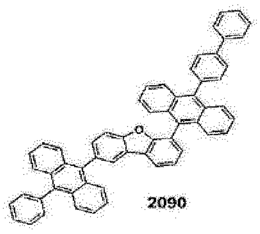
2090

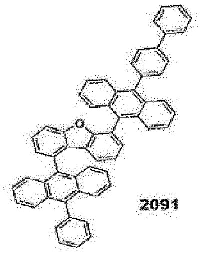
2091

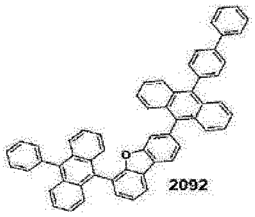
2092

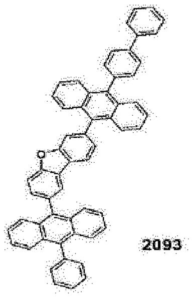
2093

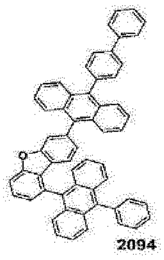
2094

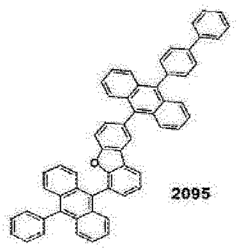
2095

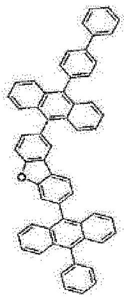
2096

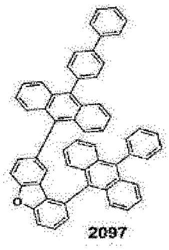
2097

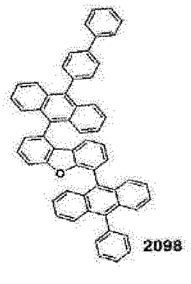
2098

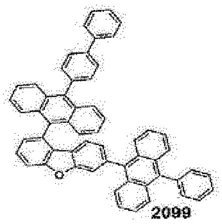
2099

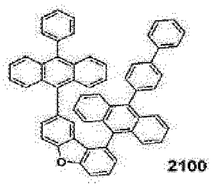
2100

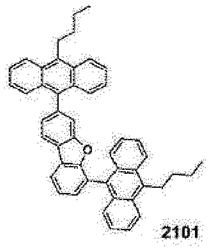
2101

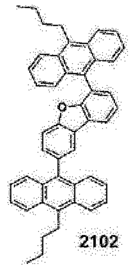
2102

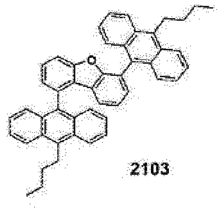
2103

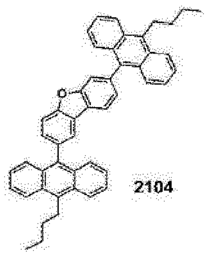
2104

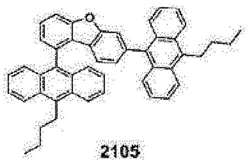
2105

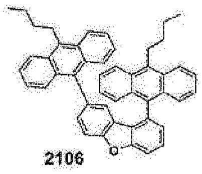
2106

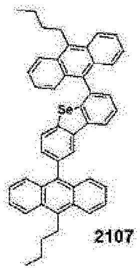
2107

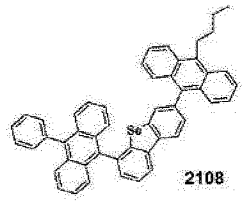
2108

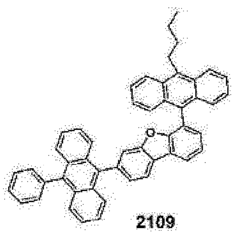
2109

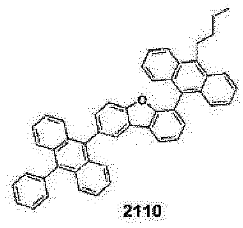
2110

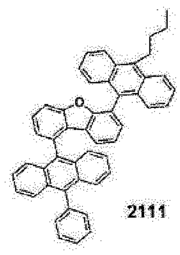
2111

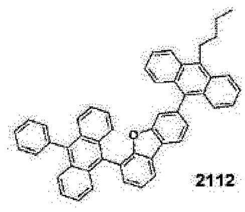
2112

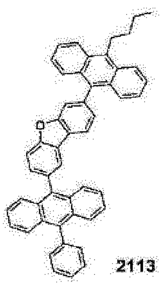
2113

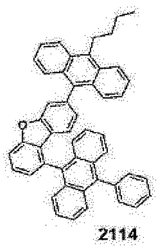
2114

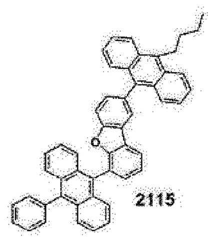
2115

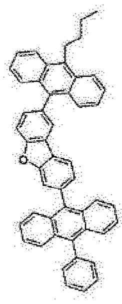
2116

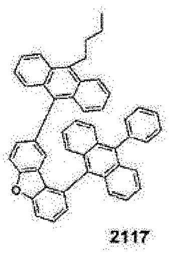
2117

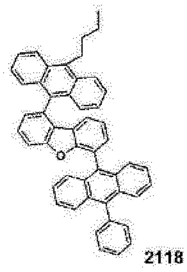
2118

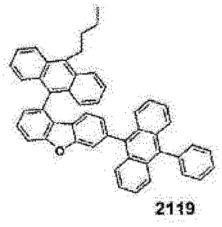
2119

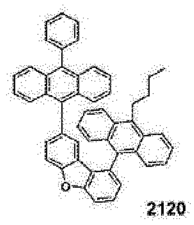
2120 .

The heterocyclic compound represented by one of Formulae 1-1 to 1-6 has an asymmetric structure, and thus, the crystallinity thereof may be decreased. Accordingly, in the heterocyclic compound, close packing between molecules may be prevented, and thus, the heterocyclic compound may have the effect of obtaining relatively excellent amorphous thin-film characteristics.

As described above, the heterocyclic compound may have such electrical characteristics suitable for use as a material for an organic light-emitting device, for example, a host material in the emission layer, a hole transport material, an electron transport material, and the like. Accordingly, an organic light-emitting device using the heterocyclic compound may have high efficiency and/or long lifespan.

In one or more embodiments, the heterocyclic compound satisfies Equation 1:

Equation 1

$$E(T1) < E(S1) < 2 \times E(T1).$$

In Equation 1, E(T1) indicates the lowest excitation triplet energy level of the heterocyclic compound and E(S1) indicates the lowest excitation singlet energy level of the heterocyclic compound.

In one or more embodiments, the heterocyclic compound satisfies Equation 2:

Equation 2

$$[\, 2 \times E(T1) \,] - E(S1) < 0.5 \text{ eV}.$$

In Equation 2, E(T1) indicates the lowest excitation triplet energy level of the heterocyclic compound and E(S1) indicates the lowest excitation singlet energy level of the heterocyclic compound.

Since the heterocyclic compound satisfies Equation 1 and/or Equation 2, the triplet-triplet fusion (TTF) phenomenon in which triplet excitons are fused to generate singlet excitons may highly likely occur. Accordingly, when the heterocyclic compound is applied to an organic light-emitting device, fluorescent emission may occur from singlet excitons generated by the TTF phenomenon, thereby improving emission efficiency and lifetime of a device.

For example, the highest occupied molecular orbital (HOMO), lowest unoccupied molecular orbital (LUMO) and triplet energy level (E(T1)), singlet (E (S1)) energy levels of Compounds 1, 113, 134, 171, 1121, and 2102 are measured using the DFT method of the Gaussian program (B3LYP, structurally optimized at the level of 6-31G (d, p)). The evaluation results are shown in Table 1 below.

Table 1

| Compound No. | HOMO (eV) | LUMO (eV) | E(S1) (eV) | E(T1) (eV) | 2E(T1) - E(S1) (eV) |
|---|---|---|---|---|---|
| Compound 1 | -5.12 | -1.64 | 3.13 | 1.73 | 0.33 |
| Compound 113 | -5.13 | -1.64 | 3.15 | 1.74 | 0.33 |
| Compound 134 | -5.29 | -1.82 | 3.14 | 1.73 | 0.32 |
| Compound 171 | -5.13 | -1.67 | 3.13 | 1.73 | 0.33 |
| Compound 1121 | -5.13 | -1.70 | 3.12 | 1.73 | 0.34 |
| Compound 2102 | -5.08 | -1.63 | 3.11 | 1.71 | 0.31 |

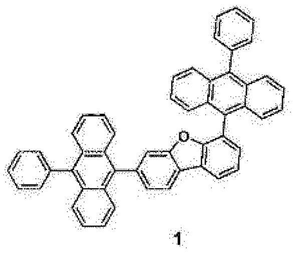

1

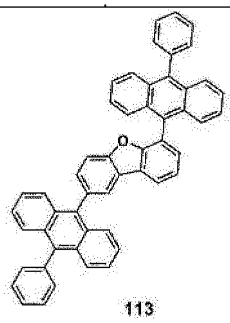

113

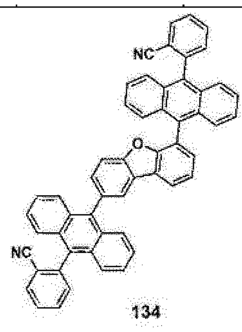

134

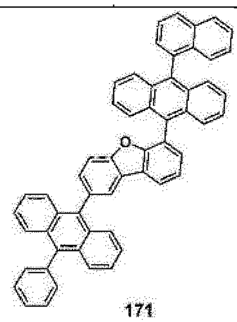

171

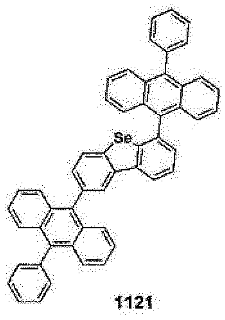

1121

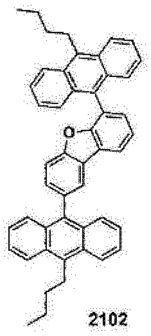

2102

From the Table 1, it can be seen that the heterocyclic compound has electric characteristics suitable for use in an electronic device, for example, as a material for an emission layer of an organic light-emitting device.

Synthesis method of the heterocyclic compound may be recognized by those skilled in the art with reference to the following Synthesis Examples.

The heterocyclic compound may be suitable for use in an organic layer, for example, an emission layer, a hole transport region material, and/or an electron transport region material of an organic layer. Accordingly, an embodiment of the invention provides an organic light-emitting device including: a first electrode; a second electrode; and an organic layer located between the first electrode and the second electrode and including an emission layer, wherein the organic layer includes at least one heterocyclic compound represented by one of Formula 1-1 to 1-6.

The organic light-emitting device may have a low driving voltage, high efficiency, high luminance, high quantum emission efficiency, and long lifespan, due to the inclusion of an organic layer including the heterocyclic compound as described above.

In one or more embodiments, in the organic light-emitting device,

the first electrode is an anode and the second electrode is a cathode,
the organic layer further includes a hole transport region between the first electrode and the emission layer and an electron transport region between the emission layer and the second electrode,
the hole transport region may include a hole injection layer, a hole transport layer, an electron blocking layer, or any combination thereof, and
the electron transport region may include a hole blocking layer, an electron transport layer, an electron injection layer, or any combination thereof, but embodiments of the present disclosure are not limited thereto.

For example, the emission layer of the organic light-emitting device may include at least one heterocyclic compound represented by Formulae 1-1 to 1-6.

In one or more embodiments, the emission layer in the organic light-emitting device may include a host and a dopant, and the host may include at least one heterocyclic compound represented by Formulae 1-1 to 1-6, and the dopant may include a phosphorescent dopant or a fluorescent dopant. For example, the dopant may include a phosphorescent dopant (for example, an organometallic compound represented by Formula 81). The host may further include any host, in addition to the heterocyclic compound represented by Formulae 1-1 to 1-6.

The emission layer may emit red light, green light, or blue light.

In one or more embodiments, the emission layer may include a fluorescent dopant, but embodiments of the present disclosure are not limited thereto.

In one or more embodiments, the emission layer may include a phosphorescent dopant, but embodiments of the present disclosure are not limited thereto.

In one or more embodiments, the heterocyclic compound may be included in the hole transport region of the organic light-emitting device.

For example, the hole transport region of the organic light-emitting device includes at least one of a hole injection layer, a hole transport layer an electron blocking layer, or any combination thereof and at least one of the hole injection layer, the hole transport layer, the electron blocking layer, or any combination thereof may include the heterocyclic compound.

In one or more embodiments, the heterocyclic compound may be included in the electron transport region of the organic light-emitting device.

For example, the electron transport region of the organic light-emitting device includes at least one of a hole blocking layer, an electron transport layer, an electron injection layer, or any combination thereof and at least one of the hole blocking layer, the electron transport layer, the electron injection layer, or any combination thereof may include the heterocyclic compound.

In one or more embodiments, the hole transport region of the organic light-emitting device may include an electron blocking layer, and the heterocyclic compound may be included in the electron blocking layer. The electron blocking layer may be in direct contact with the emission layer.

In one or more embodiments, the electron transport region of the organic light-emitting device may include a hole blocking layer, and the heterocyclic compound may be included in the hole blocking layer. The hole blocking layer may be in direct contact with the emission layer.

In one or more embodiments, the organic layer of the organic light-emitting device may further include a fluorescent dopant in addition to the heterocyclic compound.

For example, the fluorescent dopant may be a condensation polycyclic compound or a styryl compound.

For example, the fluorescent dopant may include one of a naphthalene-containing core, a fluorene-containing core, a spiro-bifluorene-containing core, a benzofluorene-containing core, a dibenzofluorene-containing core, a phenanthrene-containing core, an anthracene-containing core, a fluoranthene-containing core, a triphenylene-containing core, a pyrene-containing core, a chrysene-containing core, a naphthacene-containing core, a picene-containing core, a perylene-containing core, a pentaphene-containing core, an indenoanthracene-containing core, a tetracene-containing core, a bisanthracene-containing core, a core represented by Formulae 501-1 to 501-18, or any combination thereof, but embodiments of the present disclosure are not limited thereto:

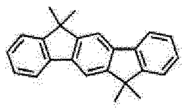
501-1

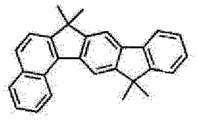
501-2

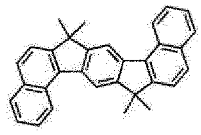
501-3

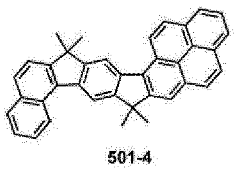
501-4

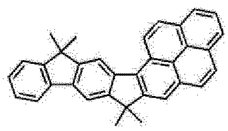
501-5

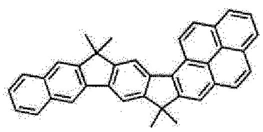
501-6

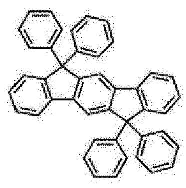
501-7

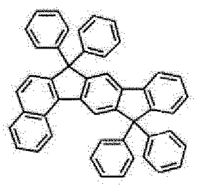
501-8

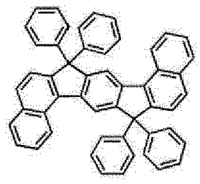
501-9

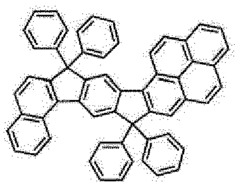
501-10

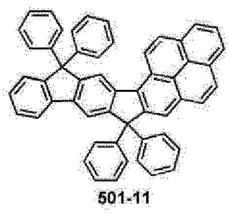
501-11

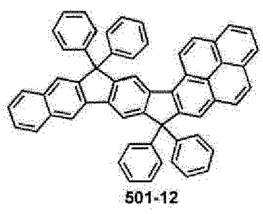
501-12

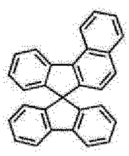
501-13

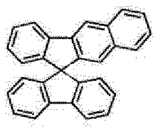
501-14

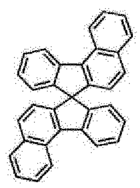
501-15

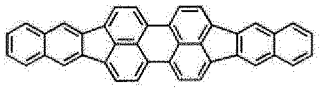

501-16

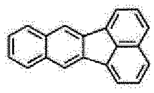

501-17

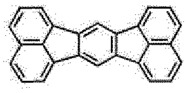

501-18

In one or more embodiments, the fluorescent dopant may be a styryl-amine-based compound or a styryl-carbazole-based compound, but embodiments of the present disclosure are not limited thereto.

In one or more embodiments, the fluorescent dopant may be a compound represented by Formula 501:

Formula 501

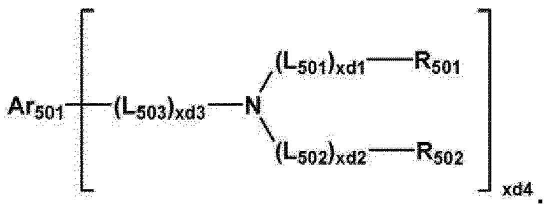

In Formula 501,
$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by Formulae 501-1 to 501-18; or

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by Formulae 501-1 to 501-18, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group - $Si(Q_{501})(Q_{502})(Q_{503})$ (wherein $Q_{501}$ to $Q_{503}$ may each independently be hydrogen, $C_1$-$C_{60}$ alkyl group, a $C_1$-$C_{60}$ alkoxy group, a $C_6$-$C_{60}$ aryl group, a $C_1$-$C_{60}$ heteroaryl group, a monovalent non-aromatic condensed polycyclic group, a monovalent non-aromatic condensed heteropolycyclic group, or any combination thereof), or any combination thereof;

$L_{501}$ to $L_{503}$ may each independently be a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ arylene group, a substituted or unsubstituted $C_1$-$C_{60}$ heteroarylene group, a substituted or unsubstituted divalent non-aromatic condensed polycyclic group, a substituted or unsubstituted divalent non-aromatic condensed heteropolycyclic group, or any combination thereof,

$R_{501}$ and $R_{502}$ may each independently be:

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, a dibenzothiophenyl group, or any combination thereof; or

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, or any combination thereof, each substituted with at least

one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, or any combination thereof,

xd1 to xd3 may each independently be 0, 1, 2, or 3, and

xd4 may be 0, 1, 2, 3, 4, 5, or 6.

For example, in Formula 501,

$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by Formulae 501-1 to 501-18; or

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, a group represented by Formula 501-1 to 501-18, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a dibenzofuranyl group, a dibenzothiophenyl group, a carbazolyl group, a pyridinyl group, a pyrimidinyl group, a triazinyl group, a quinolinyl group, an isoquinolinyl group, -Si($Q_{501}$)($Q_{502}$)($Q_{503}$) ($Q_{501}$ to $Q_{503}$ may each independently be hydrogen, $C_1$-$C_{20}$ alkyl group, a $C_1$-$C_{20}$ alkoxy group, a phenyl group, a biphenyl group, a terphenyl group, a naphthyl group, or any combination thereof), or any combination thereof,

$L_{501}$ to $L_{503}$ are the same as described in connection with $L_{21}$,

xd1 to xd3 may each independently be 0, 1, or 2, and

xd4 may be 0, 1, 2, or 3, but embodiments of the present disclosure are not limited thereto.

In one or more embodiments, the fluorescent dopant may include a compound represented by one of Formulae 502-1 to 502-5:

Formula 502-1

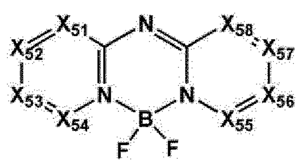

Formula 502-2

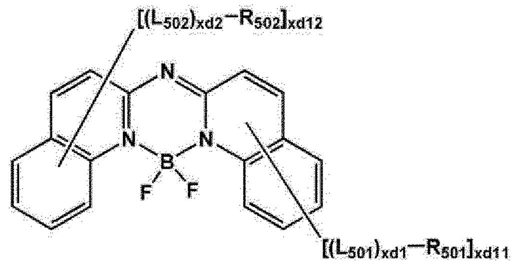

Formula 502-3

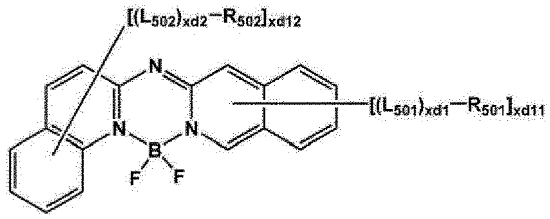

Formula 502-4

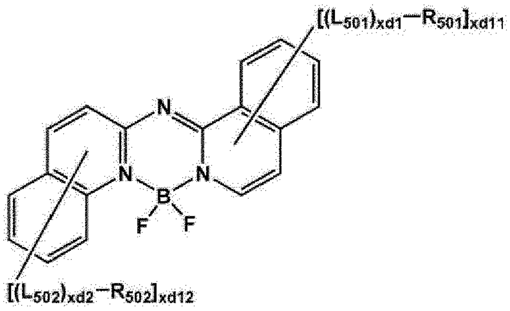

Formula 502-5

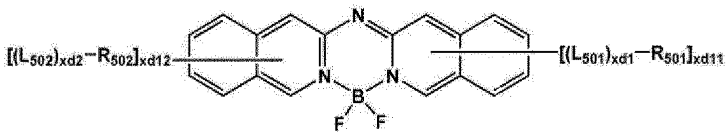

In Formulae 502-1 to 502-5,

$X_{51}$ may be N or C-[$(L_{501})_{xd1}$-$R_{501}$], $X_{52}$ may be N or C-[$(L_{502})_{xd2}$-$R_{502}$], $X_{53}$ may be N or C-[$(L_{503})_{xd3}$-$R_{503}$], $X_{54}$ may be N or C-[$(L_{504})_{xd4}$-$R_{504}$], $X_{55}$ may be N or C-[$(L_{505})_{xd5}$-$R_{505}$], $X_{56}$ may be N or C-[$(L_{506})_{xd6}$-$R_{506}$], $X_{57}$ may be N or C-[$(L_{507})_{xd7}$-$R_{507}$], and $X_{58}$ may be N or C-[$(L_{508})_{xd8}$-$R_{508}$],

$L_{501}$ to $L_{508}$ are each the same as described in connection with $L_{501}$ in Formula 501,
xd1 to xd8 are each the same as described in connection with xd1 in Formula 501,
$R_{501}$ to $R_{508}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, or any combination thereof,
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, a dibenzothiophenyl group, or any combination thereof; or
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, a dibenzothiophenyl group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl

group, a quinoxalinyl group, a quinazolinyl group, a carbazolyl group, a triazinyl group, a dibenzofuranyl group, a dibenzothiophenyl group, or any combination thereof,

xd11 and xd12 may each independently be an integer from 0 to 5, and

two of $R_{501}$ to $R_{504}$ may optionally be linked together to form a saturated or unsaturated ring, and

two of $R_{505}$ to $R_{508}$ may optionally be linked together to form a saturated or unsaturated ring.

The fluorescent dopant may include at least one compound, for example, the following compounds FD(1) to FD(16) and FD1 to FD14:

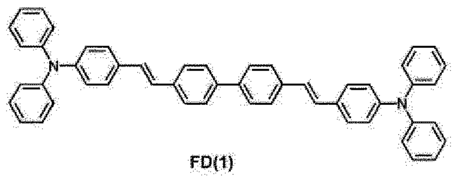

FD(1)

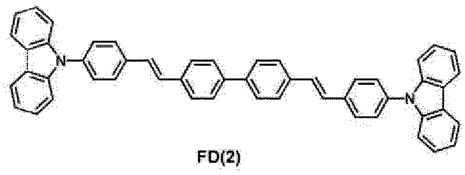

FD(2)

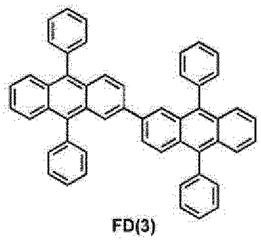

FD(3)

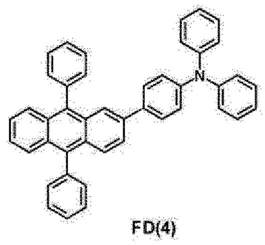

FD(4)

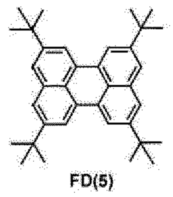

FD(5)

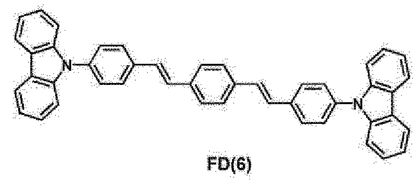

FD(6)

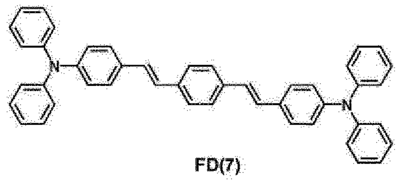

FD(7)

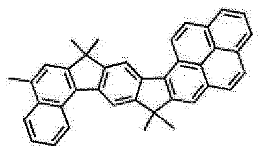

FD(8)

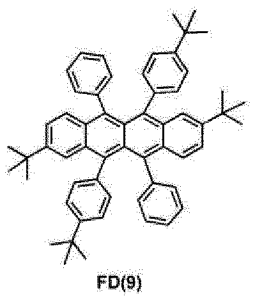

FD(9)

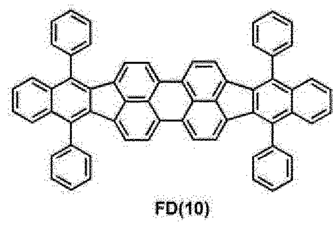

FD(10)

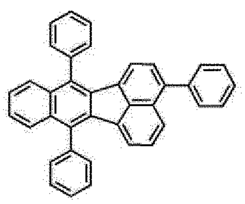

FD(11)

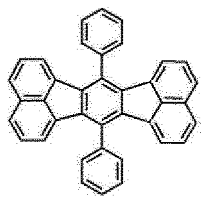

FD(12)

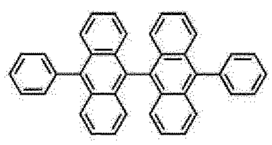

FD(13)

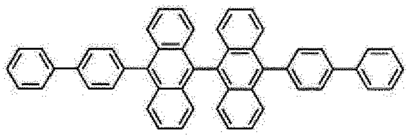

FD(14)

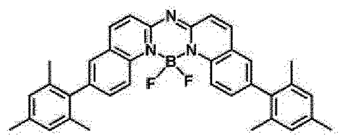

FD(15)

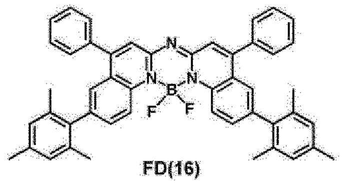

FD(16)

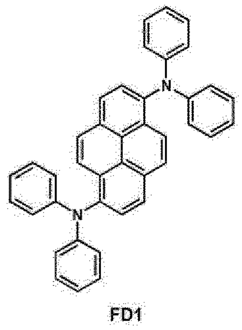

FD1

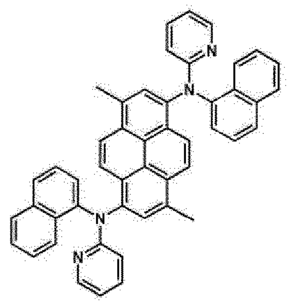

FD2

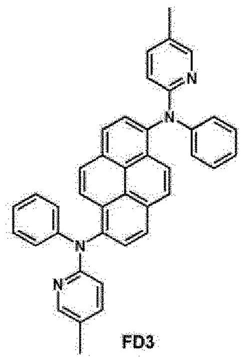

FD3

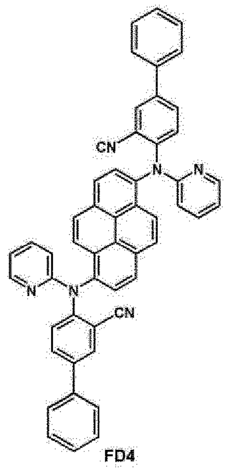

FD4

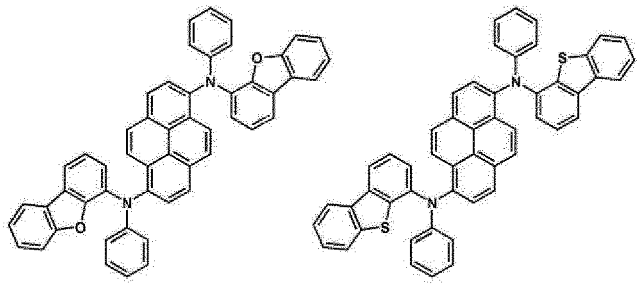

FD5

FD6

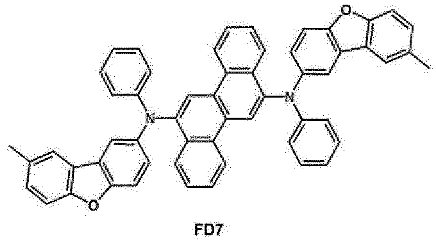

FD7

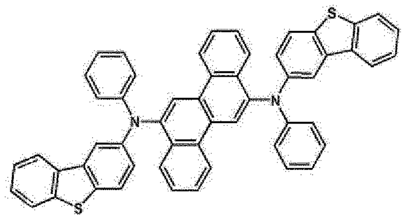

FD8

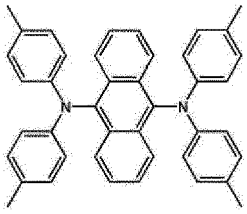

FD9

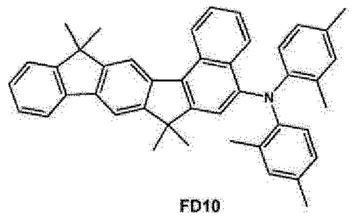

FD10

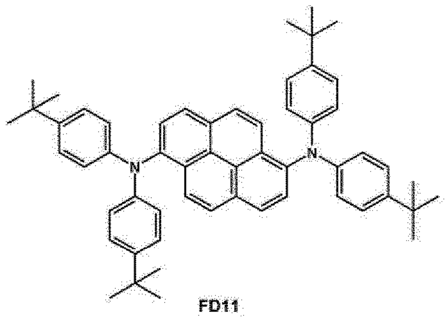

FD11

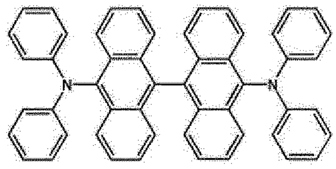

FD12

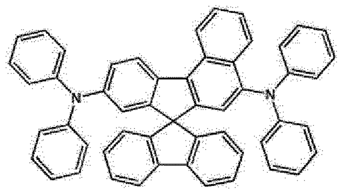

FD13

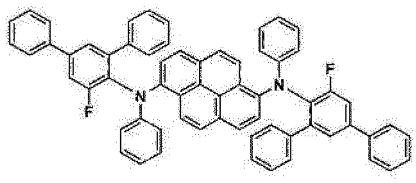

FD14

In one or more embodiments, the organic layer of the organic light-emitting device may further include a phosphorescent dopant in addition to the heterocyclic compound.

For example, the phosphorescent dopant may further include an organometallic compound represented by Formula 81:

Formula 81 $$M(L_{81})_{n81}(L_{82})_{n82}$$

Formula 81A

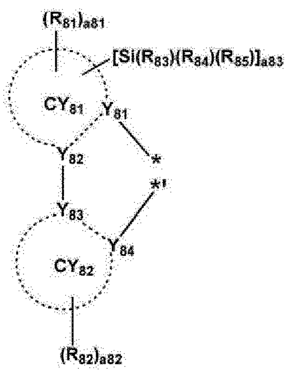

In Formulae 81 and 81A,

M may be iridium (Ir), platinum (Pt), osmium (Os), titanium (Ti), zirconium (Zr), hafnium (Hf), europium (Eu), terbium (Tb), thulium (Tm), or rhodium (Rh),

$L_{81}$ may be a ligand represented by Formula 81A, n81 may be an integer from 1 to 3, and when n81 is 2 or more, two or

more of $L_{81}$(s) may be identical to or different from each other,

$L_{82}$ may be an organic ligand, n82 may be an integer from 0 to 4, and when n82 is 2 or more, two or more of $L_{82}$(s) may be identical to or different from each other,

$Y_{81}$ to $Y_{84}$ may each independently be carbon (C) or nitrogen (N),

$Y_{81}$ and $Y_{82}$ may be linked to each other via a single bond or a double bond, and $Y_{83}$ and $Y_{84}$ may be linked to each other via a single bond or a double bond,

$CY_{81}$ and $CY_{82}$ may each independently be a $C_5$-$C_{30}$ carbocyclic group, a $C_2$-$C_{30}$ heterocarbocyclic group, or any combination thereof,

additionally, $CY_{81}$ and $CY_{82}$ may optionally be linked to each other via an organic linking group,

$R_{81}$ to $R_{85}$ may each independently be hydrogen, deuterium, -F, -Cl, -Br, - I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, -SFs, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, -Si($Q_{81}$)($Q_{82}$)($Q_{83}$), -N($Q_{84}$)($Q_{85}$), - B($Q_{86}$)($Q_{87}$), -P(=O)($Q_{88}$)($Q_{89}$), or any combination thereof,

a81 to a83 may each independently be an integer from 0 to 5,

when a81 is two or more, two or more $R_{81}$(s) may be identical to or different from each other,

when a82 is two or more, two or more $R_{82}$(s) may be identical to or different from each other,

when a81 is 2 or more, neighboring two $R_{81}$(s) may optionally be linked to form a saturated or unsaturated $C_2$-$C_{30}$ ring (for example, a benzene ring, a cyclopentane ring, a cyclohexane ring, a cyclopentene ring, a cyclohexene ring, a norbornane ring, a bicyclo[2.2.1]heptane ring, a naphthalene ring, a benzoindene ring, a benzoindole ring, a benzofuran ring, a benzothiophene ring, a pyridine ring, a pyrimidine ring, or a pyrazine ring) or a saturated or unsaturated $C_2$-$C_{30}$ ring substituted with at least one $R_{88}$(for example, a benzene ring, cyclopentane ring, a cyclohexane ring, cyclopentene ring, a cyclohexene ring, norbornane ring, a bicyclo[2.2.1]heptane ring, a naphthalene ring, benzoindene ring, benzoindole ring, a benzofuran ring, a benzothiophene ring, a pyridine ring, a pyrimidine ring, or a pyrazine ring, each substituted with at least one $R_{88}$),

when a82 is 2 or more, neighboring two $R_{82}$(s) may optionally be linked to form a saturated or unsaturated $C_2$-$C_{30}$ ring (for example, a benzene ring, a cyclopentane ring, a cyclohexane ring, a cyclopentene ring, a cyclohexene ring, a norbornane ring, a bicyclo[2.2.1]heptane ring, a naphthalene ring, a benzoindene ring, a benzoindole ring, a benzofuran ring, a benzothiophene ring, a pyridine ring, a pyrimidine ring, or a pyrazine ring) or a saturated or unsaturated $C_2$-$C_{30}$ ring substituted with at least one $R_{89}$ (for example, a benzene ring, cyclopentane ring, a cyclohexane ring, cyclopentene ring, a cyclohexene ring, norbornane ring, a bicyclo[2.2.1]heptane ring, a naphthalene ring, benzoindene ring, benzoindole ring, a benzofuran ring, a benzothiophene ring, a pyridine ring, a pyrimidine ring, or a pyrazine ring, each substituted with at least one $R_{89}$),

$R_{88}$ may be the same as explained in connection with $R_{81}$,

$R_{89}$ may be the same as explained in connection with $R_{82}$,

* and *' in Formula 81A each indicates a binding site to M in Formula 81, and

at least one substituent of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_2$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and substituted monovalent non-aromatic condensed heteropolycyclic group may be deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, -Si($Q_{91}$)($Q_{92}$)($Q_{93}$), or any combination thereof,

wherein $Q_{81}$ to $Q_{89}$ and $Q_{91}$ to $Q_{93}$ may each independently be hydrogen, deuterium, a $C_1$-$C_{60}$ alkyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_1$-$C_{60}$ heteroaryl group, a monovalent non-aromatic condensed

polycyclic group, a monovalent non-aromatic condensed heteropolycyclic group, or any combination thereof.

In one or more embodiments, in Formula 81A,

a83 may be 1 or 2,
$R_{83}$ to $R_{85}$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CH_3$, $-CD_2CD_3$, $-CD_2CD_2H$, $-CD_2CDH_2$, or any combination thereof;
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof; or
an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof, each substituted with at least one deuterium, a $C_1$-$C_{10}$ alkyl group, a phenyl group, or any combination thereof, but embodiments of the present disclosure are not limited thereto.

In one or more embodiments, in Formula 81A,

$Y_{81}$ may be nitrogen, $Y_{82}$ and $Y_{83}$ may be carbon, $Y_{84}$ may be nitrogen or carbon,
$CY_{81}$ and $CY_{82}$ may each independently be a cyclopentadiene group, a benzene group, a heptalene group, an indene group, a naphthalene group, an azulene group, a heptalene group, an indacene group, acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, a hexacene group, a pentacene group, a rubicene group, a coronene group, an ovalene group, a pyrrole group, an isoindole group, an indole group, an indazole group, a pyrazole group, an imidazole group, a triazole group, an oxazole group, an isoxazole group, an oxadiazole group, a thiazole group, an isothiazole group, a thiadiazole group, a purine group, a furan group, a thiophene group, a pyridine group, a pyrimidine group, a quinoline group, an isoquinoline group, a benzoquinoline group, a phthalazine group, a naphthyridine group, a quinoxaline group, a quinazoline group, a cinnoline group, a phenanthrididine group, an acridine group, a phenanthroline group, a phenazine group, a benzimidazole group, a benzofuran group, a benzothiophene group, an isobenzothiazole group, a benzoxazole group, an isobenzoxazole group, a benzocarbazole group, a dibenzocarbazole group, an imidazopyridine group, an imidazopyrimidine group, a dibenzofuran group, a dibenzothiophene group, a dibenzothiophene sulfone group, a carbazole group, a dibenzosilole group, a 2,3-dihydro-1H-imidazole group, or any combination thereof.

In one or more embodiments, in Formula 81A, $Y_{81}$ may be nitrogen, $Y_{82}$ to $Y_{84}$ may each be carbon, $CY_{81}$ may be 5-membered rings in which two nitrogen atoms are ring-forming atoms, and $CY_{82}$ may be a benzene group, a naphthalene group, a fluorene group, a dibenzofuran group, or a dibenzothiophene group, but embodiments of the present disclosure are not limited thereto.

In one or more embodiments, in Formula 81A, $Y_{81}$ may be nitrogen, $Y_{82}$ to $Y_{84}$ may each be carbon, $CY_{81}$ may be an imidazole group or a 2,3-dihydro-1H-imidazole group, and $CY_{82}$ may be a benzene group, a naphthalene group, a fluorene group, a dibenzofuran group, or a dibenzothiophene group, but embodiments of the present disclosure are not limited thereto.

In one or more embodiments, in Formula 81A,

$Y_{81}$ may be nitrogen and $Y_{82}$ to $Y_{84}$ may be carbon,
$CY_{81}$ may be a pyrrole group, a pyrazole group, an imidazole group, a triazole group, an oxazole group, an isoxazole group, an oxadiazole group, a thiazole group, an isothiazole group, a thiadiazole group, a pyridine group, a pyrimidine group, a quinoline group, an isoquinoline group, a benzoquinoline group, a phthalazine group, a naphthyridine group, a quinoxaline group, a quinazoline group, a cinnoline group, a benzimidazole group, an isobenzothiazole group, a benzoxazole group, or an isobenzoxazole group, and
$CY_{82}$ may be a cyclopentadiene group, a benzene group, a naphthalene group, a fluorene group, a benzofluorene group, a dibenzofluorene group, a phenanthrene group, an anthracene group, a triphenylene group, a pyrene group, a chrysene group, a perylene group, a benzofuran group, a benzothiophene group, a benzocarbazole group, a dibenzocarbazole group, a dibenzofuran group, a dibenzothiophene group, a dibenzothiophene sulfone group, a carbazole group, or a dibenzosilole group.

In one or more embodiments, in Formula 81A
$R_{81}$ and $R_{82}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SFs, $C_1$-$C_{20}$ alkyl group, a $C_1$-$C_{20}$ alkoxy group, or any combination thereof;
a $C_1$-$C_{20}$ alkyl group, a $C_1$-$C_{20}$ alkoxy group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof; or
$-B(Q_{86})(Q_{87})$, or $-P(=O)(Q_{88})(Q_{89})$, or any combination thereof,
wherein $Q_{86}$ to $Q_{89}$ may each independently be:

$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$, $-CHDCD_3$, $-CD_2CH_3$, $-CD_2CD_3$, $-CD_2CD_2H$, $-CD_2CDH_2$, or any combination thereof;
an n-propyl group, an isopropyl group, an n-butyl group, an isobutyl group, a sec-butyl group, a tert-butyl group, an

n-pentyl group, an isopentyl group, a sec-pentyl group, a tert-pentyl group, a phenyl group, a naphthyl group, or any combination thereof; or

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof, each substituted with at least one deuterium, a $C_1$ to $C_{10}$ alkyl group, a phenyl group, or any combination thereof.

In one or more embodiments, in Formula 81A, at least one $R_{81}$(s) in the number of a81 and $R_{82}$(s) in the number of a82 may be a cyano group.

In one or more embodiments, in Formula 81A, at least one $R_{82}$(s) in the number of a82 may be a cyano group.

In one or more embodiments, in Formula 81A, at least one $R_{81}$(s) in the number of a81 and $R_{82}$(s) in the number of a82 may be a deuterium.

In one or more embodiments, $L_{82}$ in Formula 81 may be a ligand represented by one of Formulae 3-1(1) to 3-1(69), 3-1(71) to 3-1(79), 3-1(81) to 3-1(88), 3-1(91) to 3-1(98), and 3-1(101) to 3-1(114):

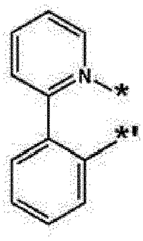
3-1(1)

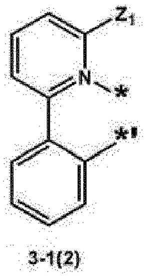
3-1(2)

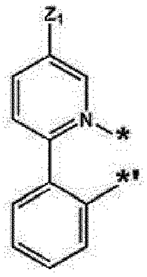
3-1(3)

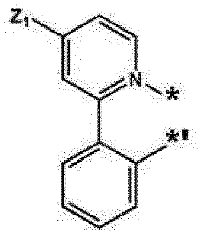
3-1(4)

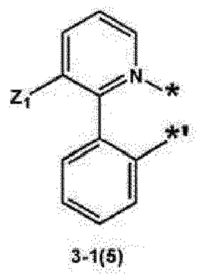
3-1(5)

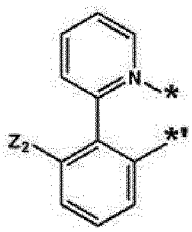
3-1(6)

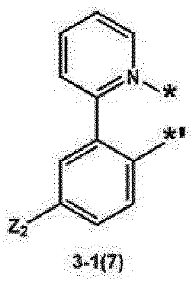
3-1(7)

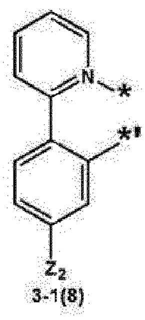
3-1(8)

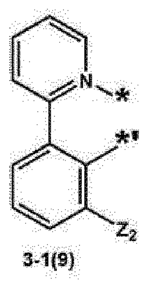
3-1(9)

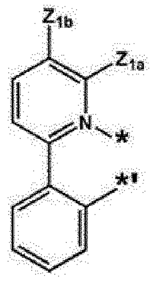
3-1(10)

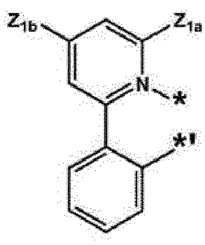
3-1(11)

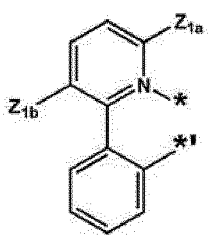
3-1(12)

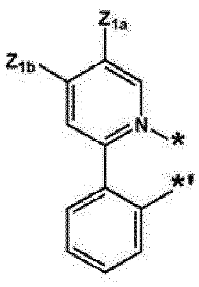
3-1(13)

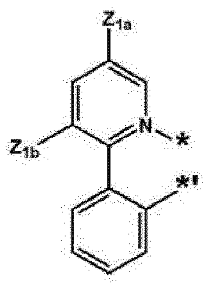
3-1(14)

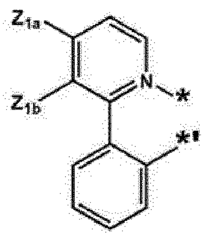
3-1(15)

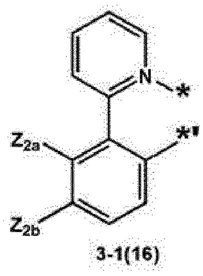
3-1(16)

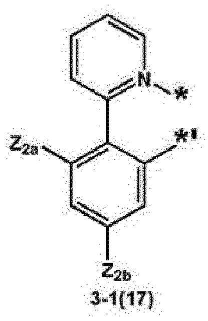
3-1(17)

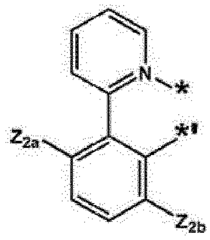
3-1(18)

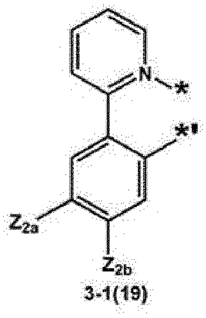
3-1(19)

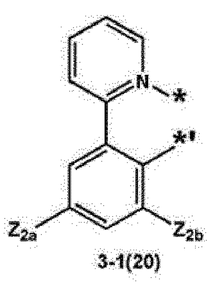
3-1(20)

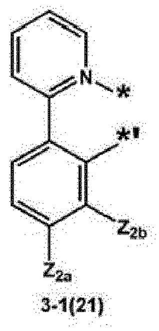
3-1(21)

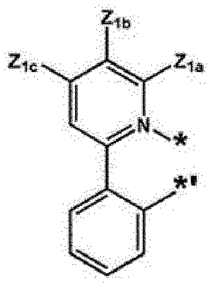
3-1(22)

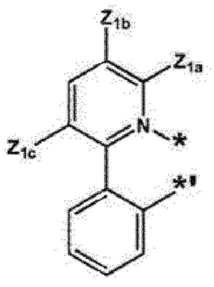
3-1(23)

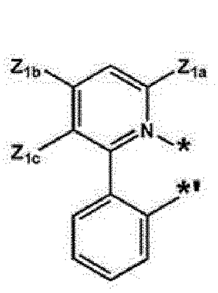
3-1(24)

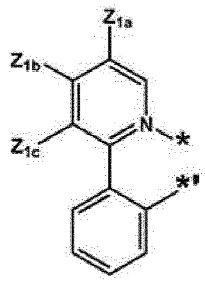
3-1(25)

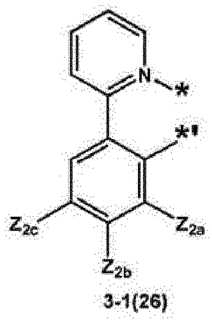
3-1(26)

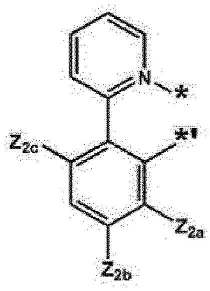
3-1(27)

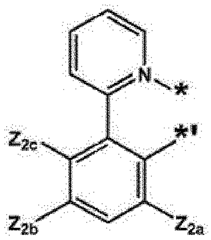
3-1(28)

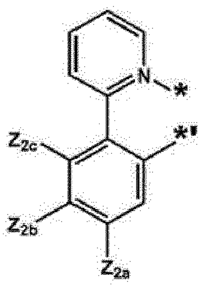
3-1(29)

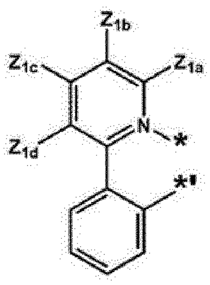
3-1(30)

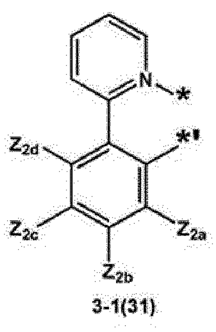
3-1(31)

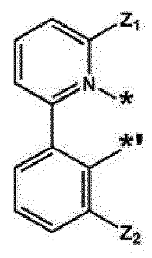
3-1(32)

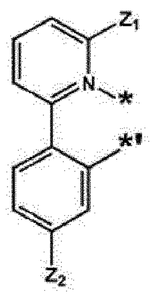
3-1(33)

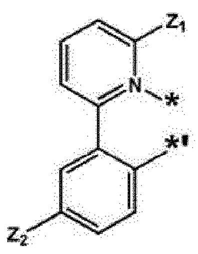
3-1(34)

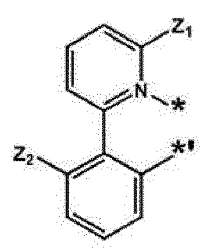
3-1(35)

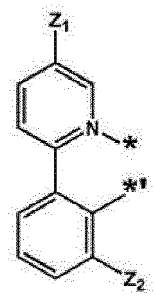

3-1(36)

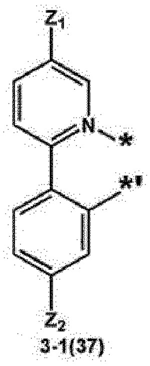

3-1(37)

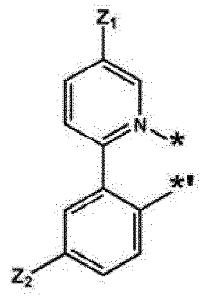

3-1(38)

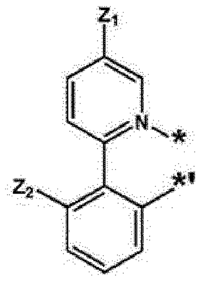

3-1(39)

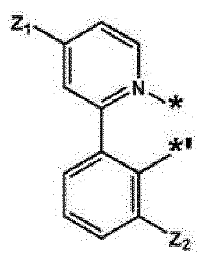

3-1(40)

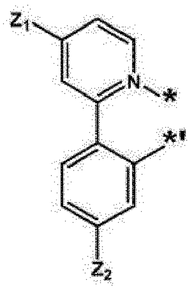

3-1(41)

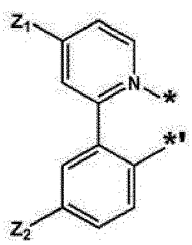

3-1(42)

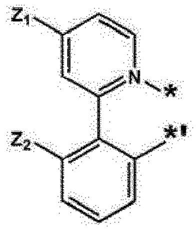

3-1(43)

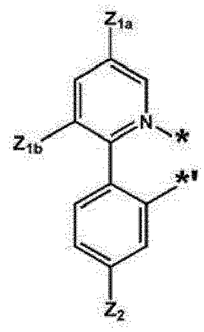

3-1(44)

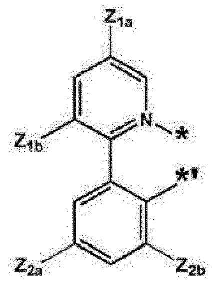

3-1(45)

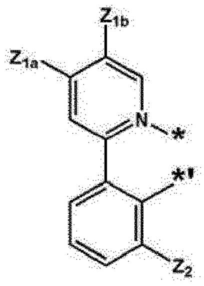

3-1(46)

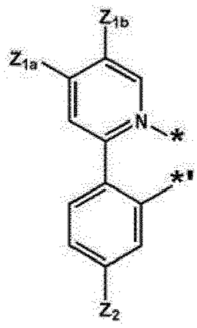

3-1(47)

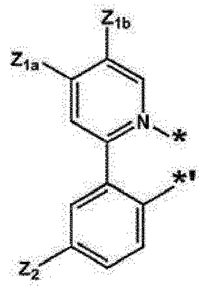

3-1(48)

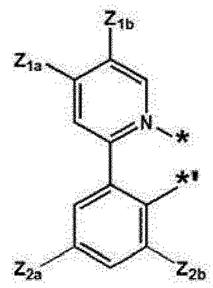

3-1(49)

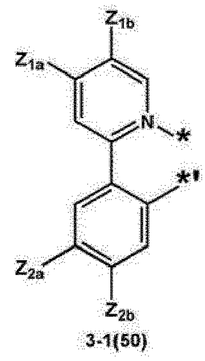

3-1(50)

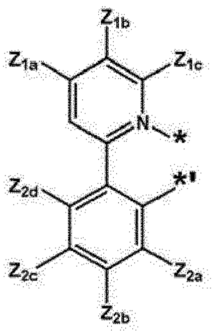

3-1(51)

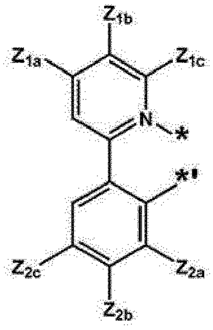

3-1(52)

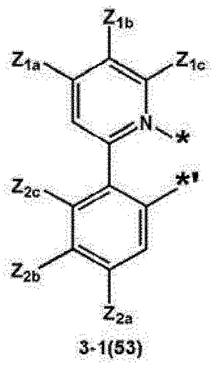

3-1(53)

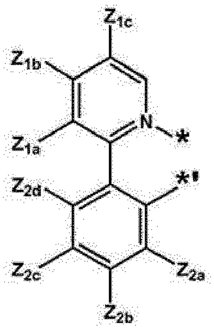

3-1(54)

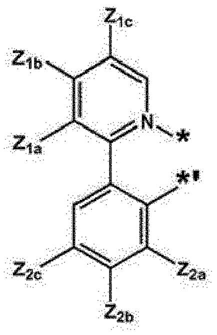

3-1(55)

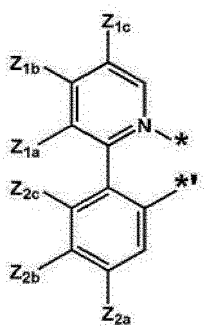
3-1(56)

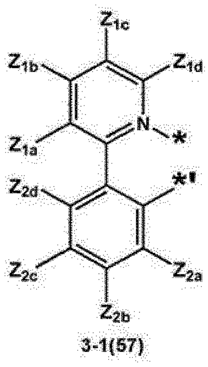
3-1(57)

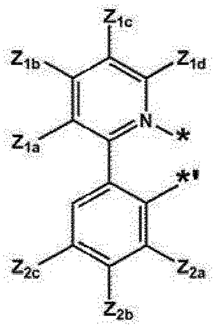
3-1(58)

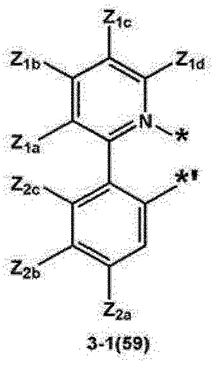
3-1(59)

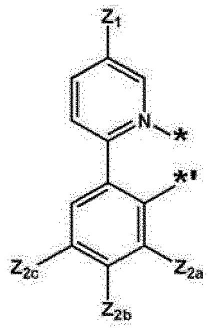
3-1(60)

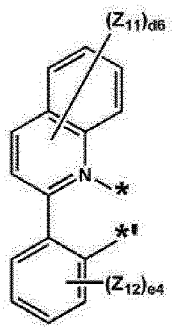
3-1(61)

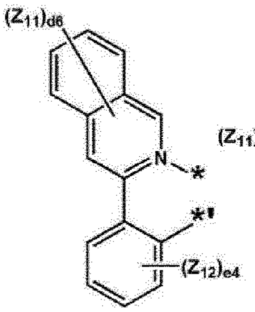
3-1(62)

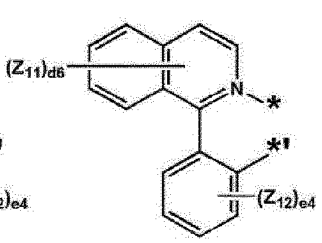
3-1(63)

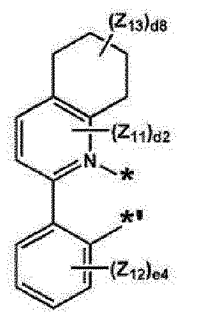
3-1(64)

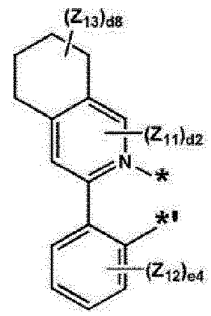
3-1(65)

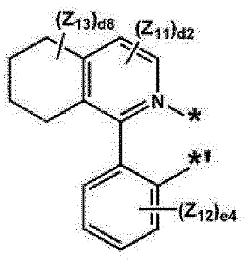
3-1(66)

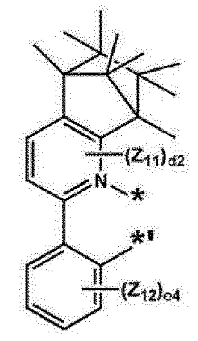
3-1(67)

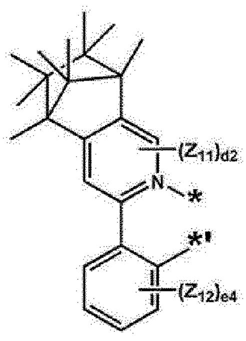
3-1(68)

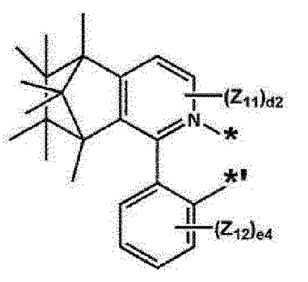
3-1(69)

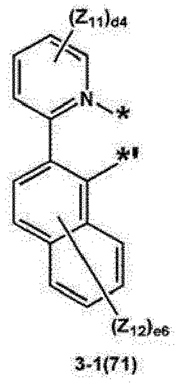
3-1(71)

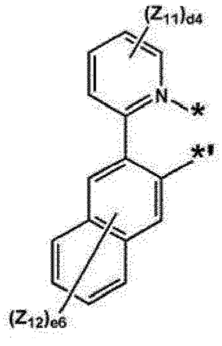
3-1(72)

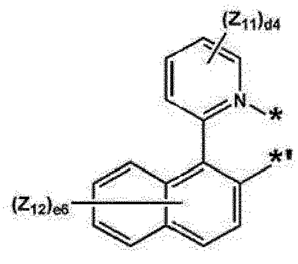
3-1(73)

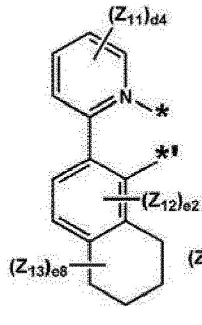
3-1(74)

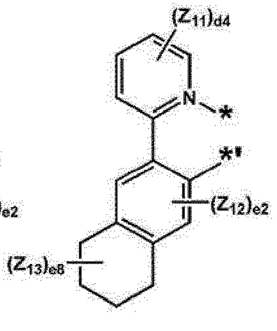
3-1(75)

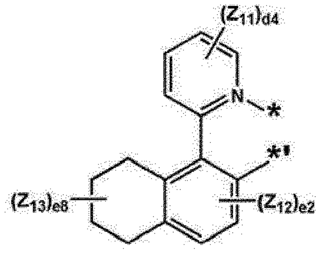

3-1(76)

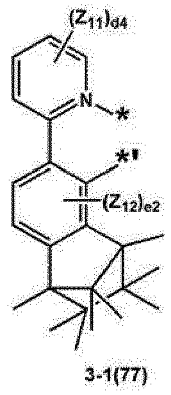

3-1(77)

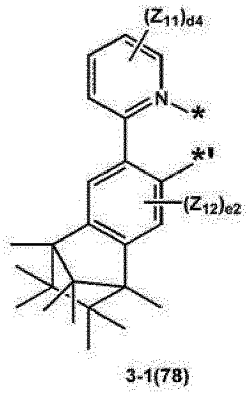

3-1(78)

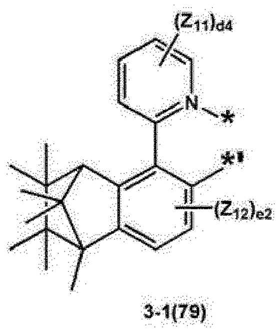

3-1(79)

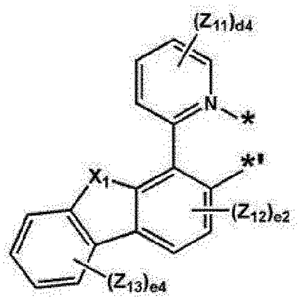

3-1(81)

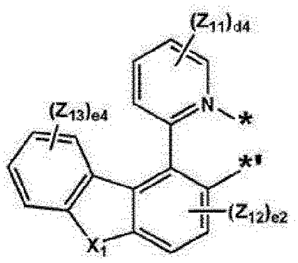

3-1(82)

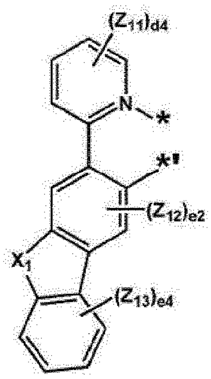

3-1(83)

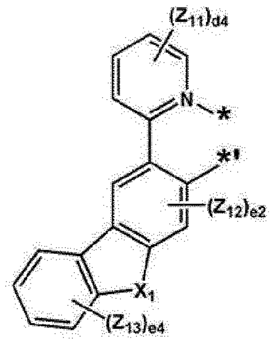

3-1(84)

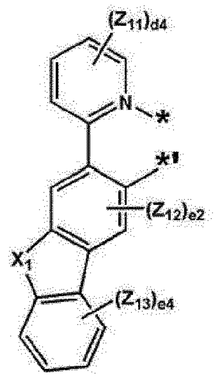

3-1(85)

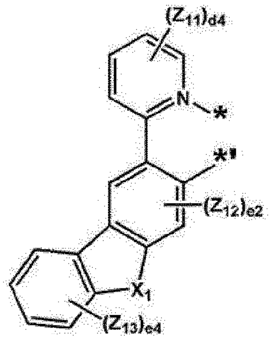

3-1(86)

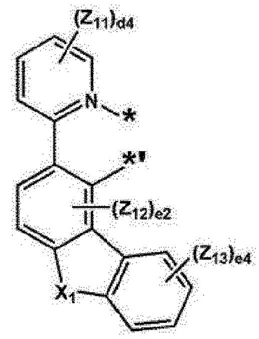

3-1(87)

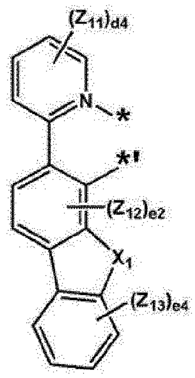

3-1(88)

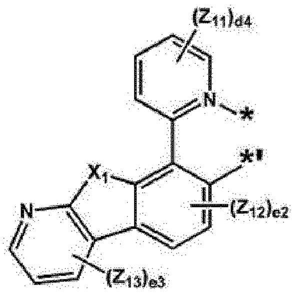

3-1(91)

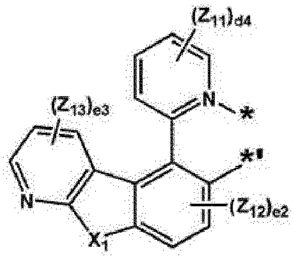

3-1(92)

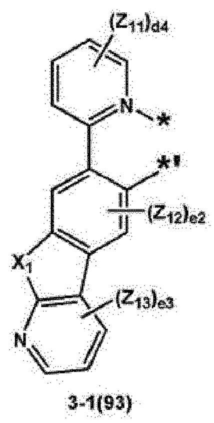

3-1(93)

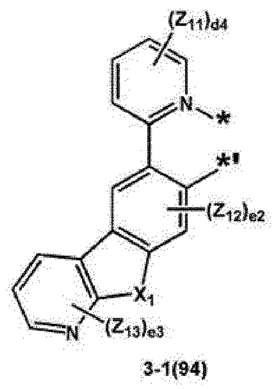

3-1(94)

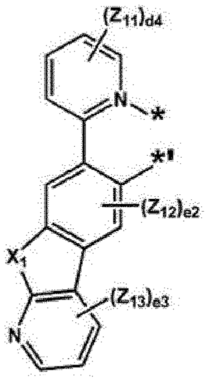

3-1(95)

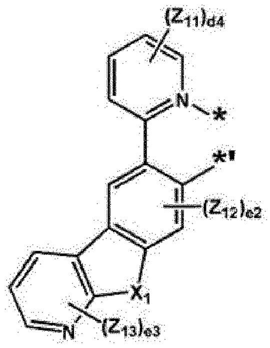

3-1(96)

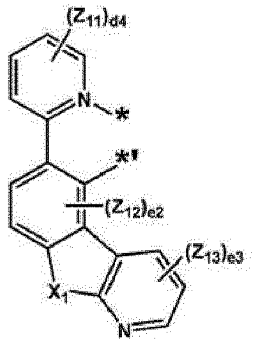

3-1(97)

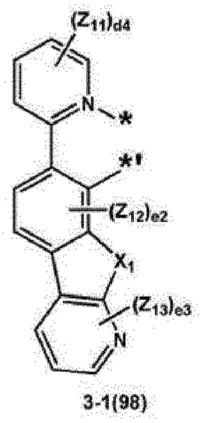

3-1(98)

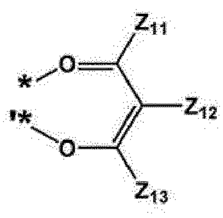

3-1(101)

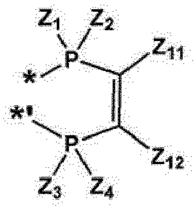

3-1(102)

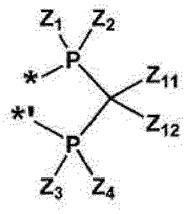

3-1(103)

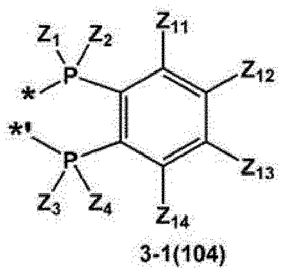

3-1(104)

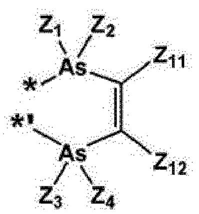

3-1(105)

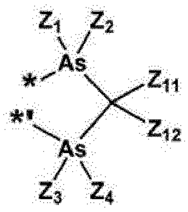

3-1(106)

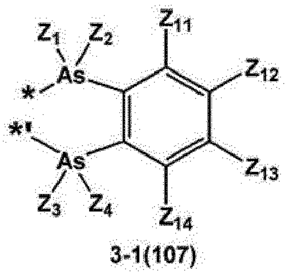

3-1(107)

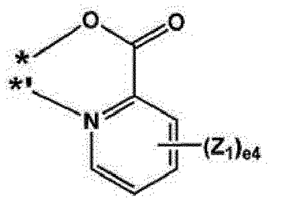

3-1(108)

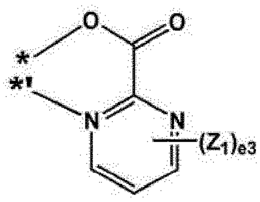

3-1(109)

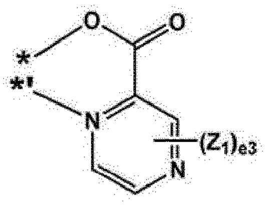

3-1(110)

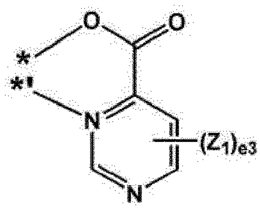

3-1(111)

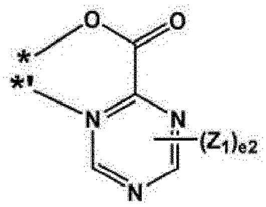

3-1(112)

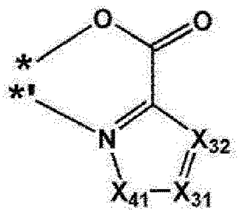

3-1(113)

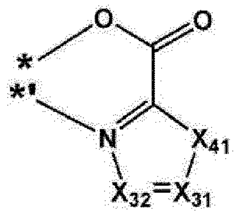

3-1(114)

.

In Formulae 3-1(1) to 3-1(69), 3-1(71) to 3-1(79), 3-1(81) to 3-1(88), 3-1(91) to 3-1(98), and 3-1(101) to 3-1(114),

$X_1$ may be O, S, $C(Z_{21})(Z_{22})$, or $N(Z_{23})$,

$X_{31}$ may be N or $C(Z_{1a})$ and $X_{32}$ may be N or $C(Z_{1b})$,
$X_{41}$ may be O, S, $N(Z_{1a})$, or $C(Z_{1a})(Z_{1b})$,
$Z_1$ to $Z_4$, $Z_{1a}$, $Z_{1b}$, $Z_{1c}$, $Z_{1d}$, $Z_{2a}$, $Z_{2b}$, $Z_{2c}$, $Z_{2d}$, $Z_{11}$ to $Z_{14}$ and $Z_{21}$ to $Z_{23}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SFs, $C_1$-$C_{20}$ alkyl group, a $C_1$-$C_{20}$ alkoxy group, or any combination thereof;
a $C_1$-$C_{20}$ alkyl group, a $C_1$-$C_{20}$ alkoxy group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof; or
$-B(Q_{86})(Q_{87})$, $-P(=O)(Q_{88})(Q_{89})$, or any combination thereof,
wherein $Q_{86}$ to $Q_{89}$ may each independently be:
$-CH_3$, $-CD_3$, $-CD_2H$, $-CDH_2$, $-CH_2CH_3$, $-CH_2CD_3$, $-CH_2CD_2H$, $-CH_2CDH_2$, $-CHDCH_3$, $-CHDCD_2H$, $-CHDCDH_2$,

$-CHDCD_3$, $-CD_2CH_3$, $-CD_2CD_3$, $-CD_2CD_2H$, $-CD_2CDH_2$, or any combination thereof;

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, a naphthyl group, or any combination thereof; or

an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, and a naphthyl group, each substituted with at least one deuterium, a $C_1$ to $C_{10}$ alkyl group, a phenyl group, or any combination thereof;

d2 and e2 may each independently be 0 or 2,

e3 may be an integer from 0 to 3,

d4 and e4 may each independently be an integer from 0 to 4,

d6 and e6 may each independently be an integer from 0 to 6,

d8 and e8 may each independently be an integer from 0 to 8,

* and *' each indicate a binding site to M in Formula 81.

In one or more embodiments, in Formula 81, M may be Ir and the sum of n81 and n82 may be 3; or M may be Pt and the sum of n81 and n82 may be 2.

In one or more embodiments, the organometallic compound represented by Formula 81 may be electrically neutral rather than a salt consisting of the pair of a cation and an anion.

In one or more embodiments, the organometallic compound represented by Formula 81 may include at least one compound of PD1 to PD78 and $FIr_6$, but embodiments of the present disclosure are not limited thereto.

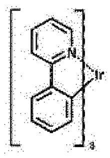
PD1

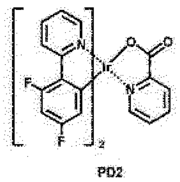
PD2

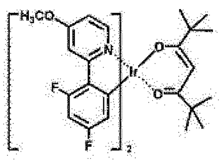
PD3

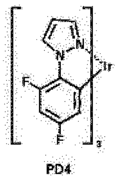
PD4

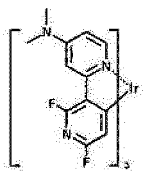
PD5

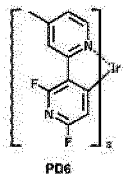
PD6

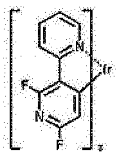
PD7

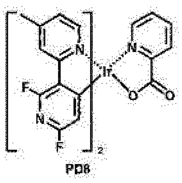
PD8

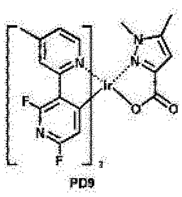
PD9

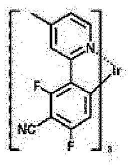
PD10

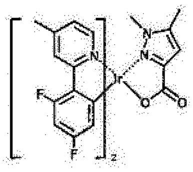
PD11

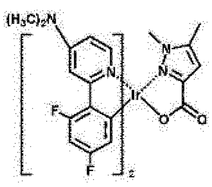
PD12

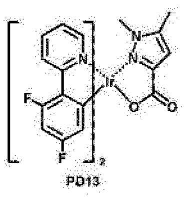
PD13

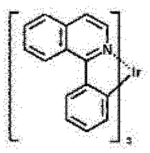
PD14

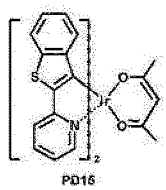
PD15

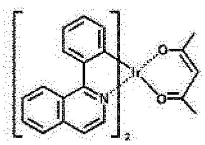
PD16

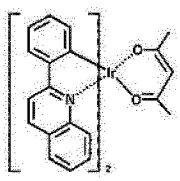
PD17

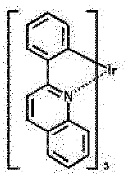
PD18

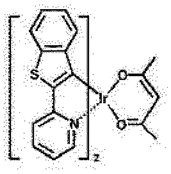
PD19

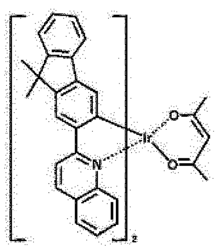
PD20

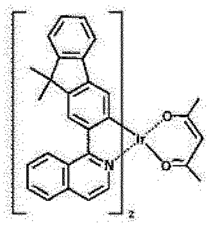
PD21

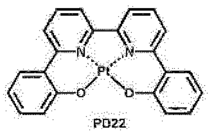
PD22

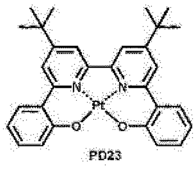
PD23

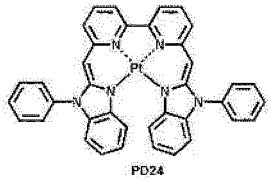
PD24

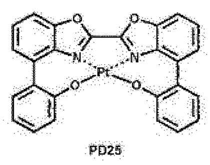
PD25

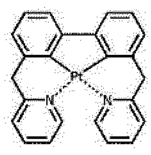
PD26

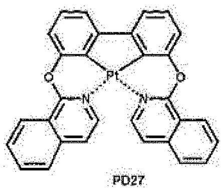
PD27

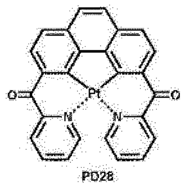
PD28

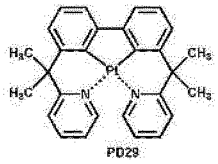
PD29

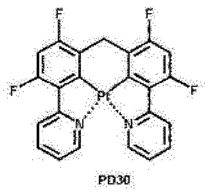
PD30

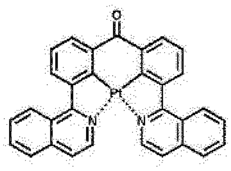
PD31

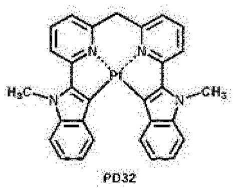
PD32

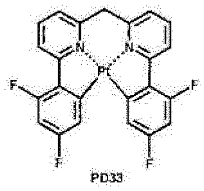
PD33

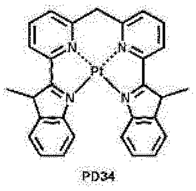
PD34

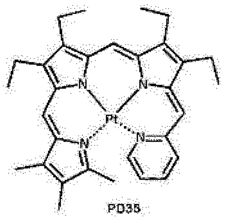
PD35

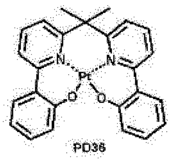
PD36

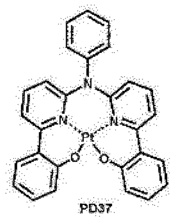
PD37

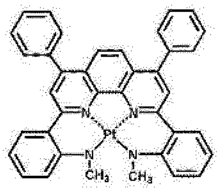
PD38

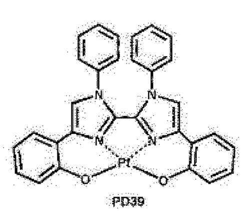
PD39

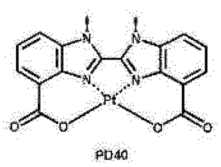
PD40

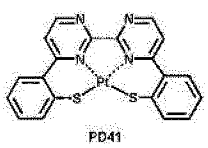
PD41

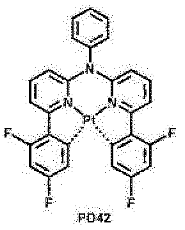
PD42

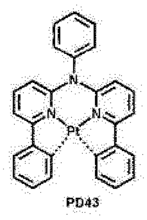
PD43

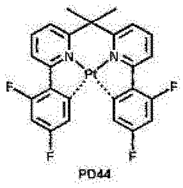
PD44

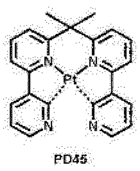
PD45

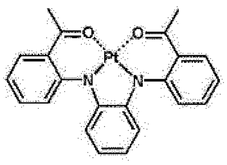
PD46

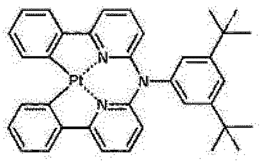
PD47

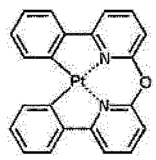
PD48

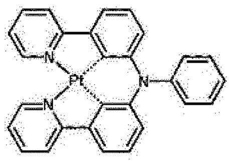
PD49

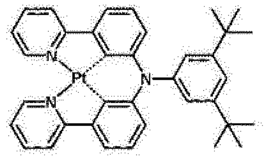
PD50

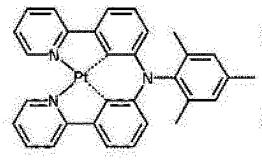
PD51

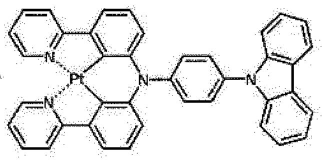
PD52

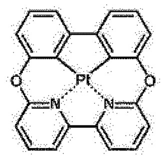
PD53

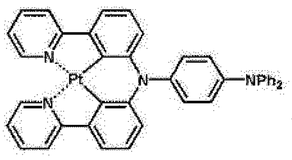
PD54

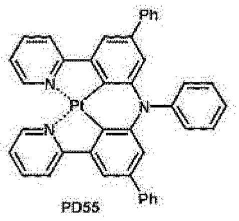
PD55

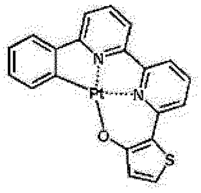
PD56

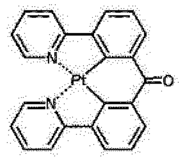
PD57

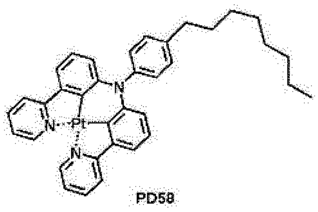
PD58

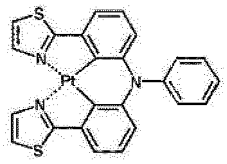
PD59

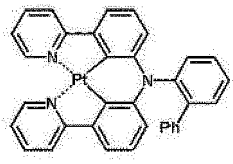
PD60

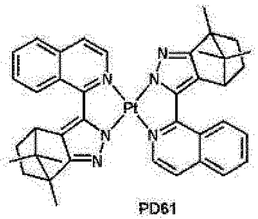
PD61

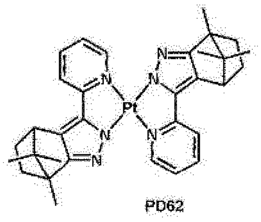
PD62

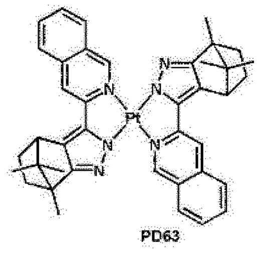
PD63

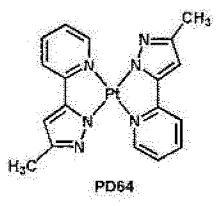
PD64

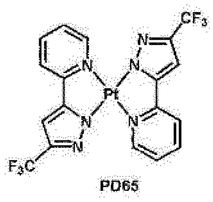
PD65

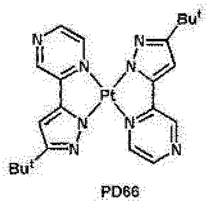
PD66

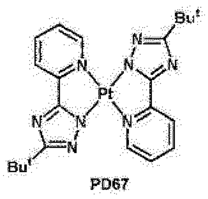
PD67

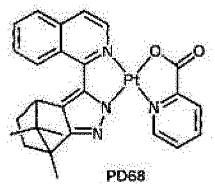
PD68

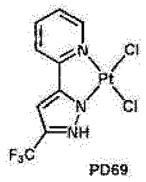
PD69

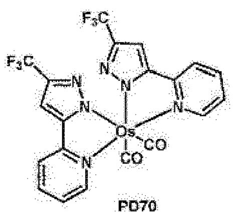
PD70

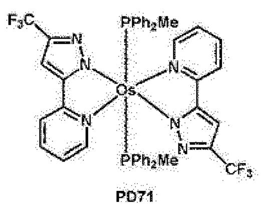
PD71

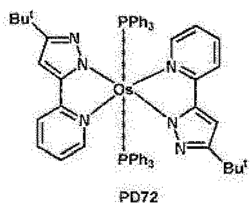
PD72

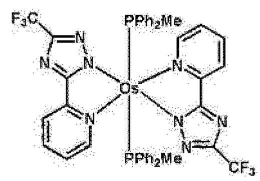
PD73

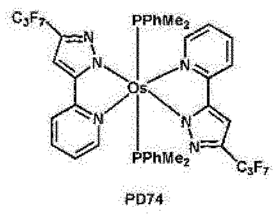
PD74

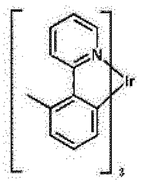
PD75

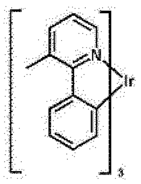
PD76

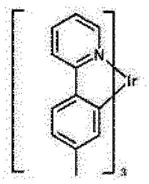
PD77

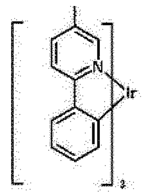
PD78

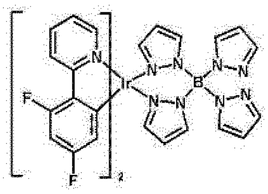
FIr6

The expression "(an organic layer) includes at least one heterocyclic compound" as used herein may include a case in which "(an organic layer) includes identical heterocyclic compounds represented by Formula 1-1 to 1-6" and a case in which "(an organic layer) includes two or more different heterocyclic compounds represented by Formula 1-1 to 1-6".

For example, the organic layer may include, as the heterocyclic compound, only Compound 1. In this regard, Compound 1 may exist only in the emission layer of the organic light-emitting device. In one or more embodiments, the organic layer may include, as the heterocyclic compound, Compound 1 and Compound 2. In this case, Compound 1 and Compound 2 may be present in an identical layer (for example, Compound 1 and Compound 2 may all be present in an emission layer), or different layers (for example, Compound 1 may be present in an emission layer and Compound 2 may be present in a hole blocking layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

The term "organic layer" used herein refers to a single layer and/or a plurality of layers between the first electrode and the second electrode of the organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment and a method of manufacturing an organic light-emitting device according to an embodiment will be described in connection with FIGURE. The organic light-emitting device 10 includes a first electrode 11, an organic layer 15, and a second electrode 19, which are sequentially stacked.

A substrate may be additionally located under the first electrode 11 or above the second electrode 19. For use as the substrate, any substrate that is used in organic light-emitting devices available in the art may be used, and the substrate may be a glass substrate or a transparent plastic substrate, each having excellent mechanical strength, thermal stability, transparency, surface smoothness, ease of handling, and water resistance.

In one or more embodiments, the first electrode 11 may be formed by depositing or sputtering a material for forming the first electrode11 on the substrate. The first electrode 11 may be an anode. The material for forming the first electrode 11 may be materials with a high work function to facilitate hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide ($SnO_2$), or zinc oxide (ZnO). In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is located on the first electrode 11.

The organic layer 15 may include a hole transport region, an emission layer, and an electron transport region.

The hole transport region may be between the first electrode 11 and the emission layer.

The hole transport region may include at least one a hole injection layer, a hole transport layer, an electron blocking layer, and a buffer layer.

The hole transport region may include only either a hole injection layer or a hole transport layer. In one or more embodiments, the hole transport region may have a hole injection layer/hole transport layer structure or a hole injection

layer/hole transport layer/electron blocking layer structure, wherein, for each structure, each layer is sequentially stacked in this stated order from the first electrode 11.

When the hole transport region includes a hole injection layer (HIL), the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about $10^{-8}$ torr to about $10^{-3}$ torr, and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include at least one m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or any combination thereof:

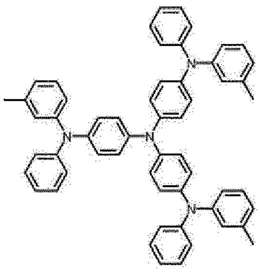

m-MTDATA

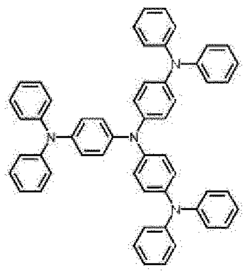

TDATA

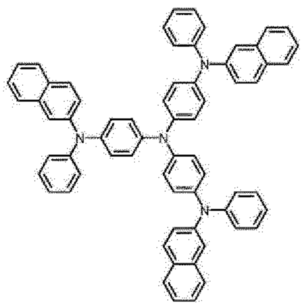

2-TNATA

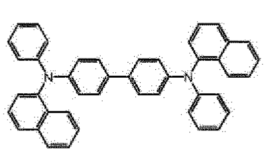

NPB

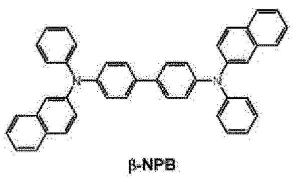

β-NPB

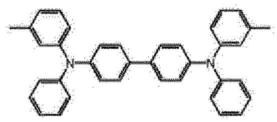

TPD

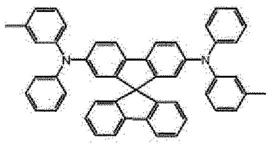

Spiro-TPD

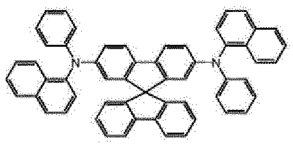

Spiro-NPB

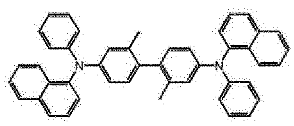

methylated NPB

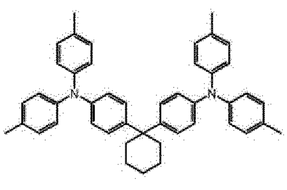

TAPC

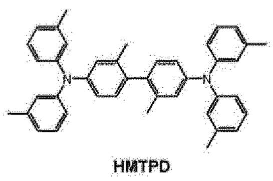

HMTPD

Formula 201

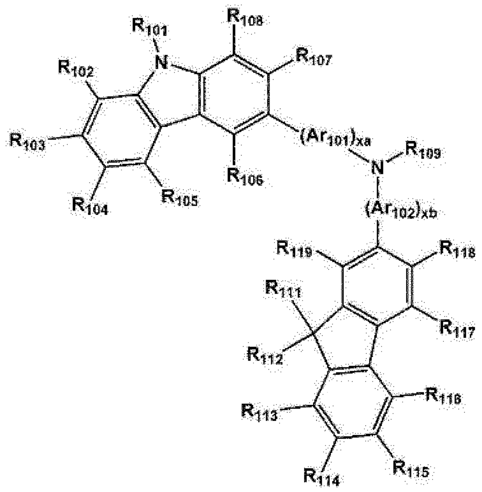

Formula 202

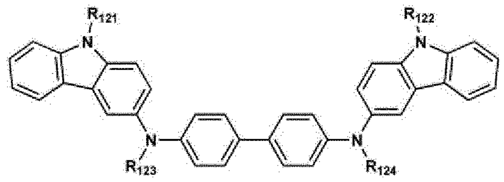

$Ar_{101}$ to $Ar_{102}$ in Formula 201 may each independently be:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof; or

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof.

xa and xb in Formula 201 may each independently be an integer from 0 to 5, or 0, 1, or 2. For example, xa may be 1 and xb may be 0, but xa and xb are not limited thereto.

$R_{101}$ to $R_{108}$, $R_{111}$ to $R_{119}$ and $R_{121}$ to $R_{124}$ in Formulae 201 and 202 may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, pentyl group, a hexyl group, etc.) a $C_1$-$C_{10}$ alkoxy group (for example, a methoxy group, an ethoxy group, a propoxy group, a butoxy group, a pentoxy group, etc.), or any combination thereof;

a $C_1$-$C_{10}$ alkyl group, a $C_1$-$C_{10}$ alkoxy group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid

group or a salt thereof, or any combination thereof;

a phenyl group, a naphthyl group, an anthracenyl group, a fluorenyl group, a pyrenyl group, or any combination thereof; or

a phenyl group, a naphthyl group, an anthracenyl group, a fluorenyl group, a pyrenyl group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group, or any combination thereof, but embodiments of the present disclosure are not limited thereto.

$R_{109}$ in Formula 201 may be:

a phenyl group, a naphthyl group, an anthracenyl group, or a pyridinyl group; or

a phenyl group, a naphthyl group, an anthracenyl group, or a pyridinyl group, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, an anthracenyl group, a pyridinyl group, or any combination thereof.

According to an embodiment, the compound represented by Formula 201 may be represented by Formula 201A below, but embodiments of the present disclosure are not limited thereto:

Formula 201A

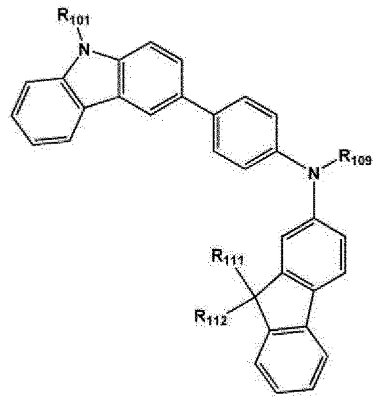

$R_{101}$, $R_{111}$, $R_{112}$, and $R_{109}$ in Formula 201A may be understood by referring to the description provided herein.

For example, the compound represented by Formula 201, and the compound represented by Formula 202 may include compounds HT1 to HT20 illustrated below, but are not limited thereto:

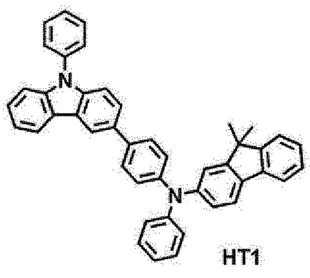

HT1

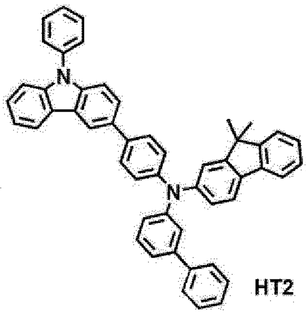

HT2

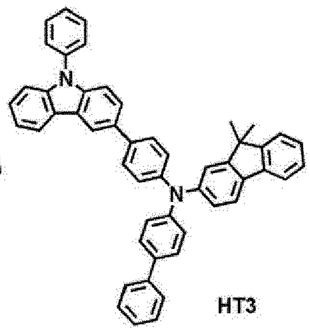

HT3

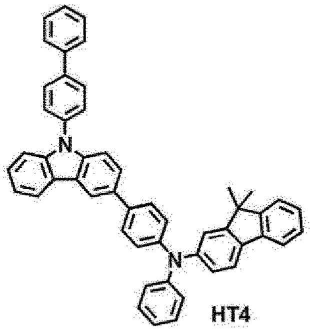

HT4

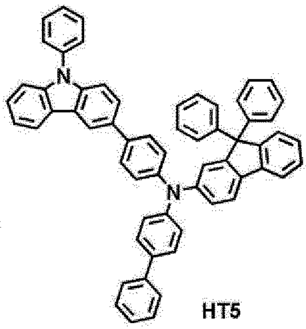

HT5

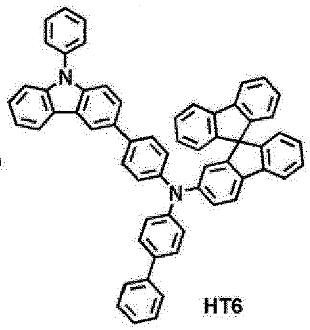

HT6

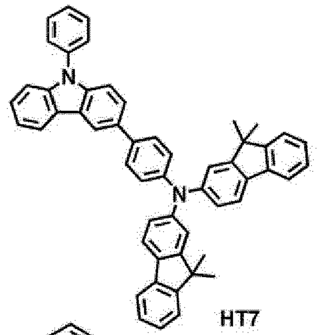

HT7

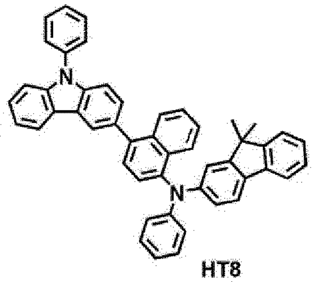

HT8

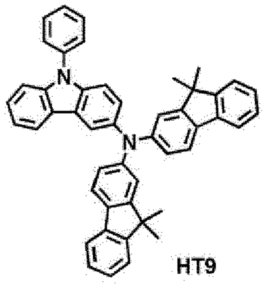

HT9

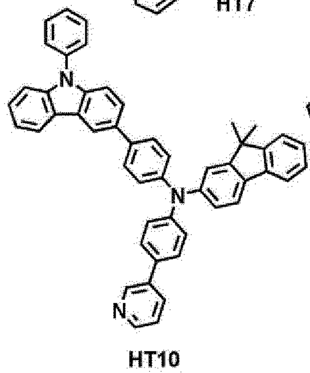

HT10

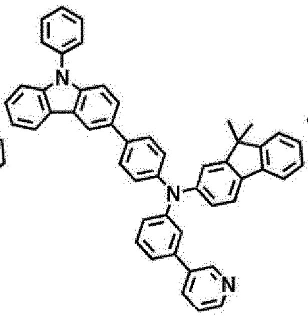

HT11

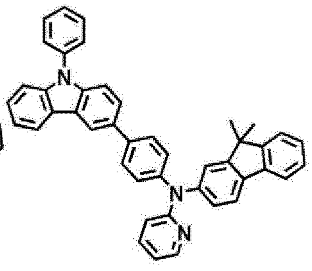

HT12

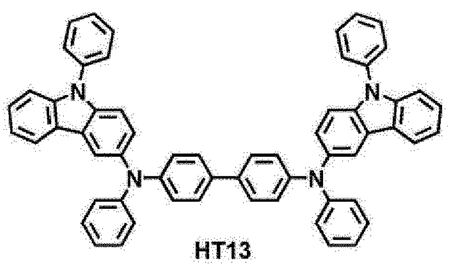

HT13

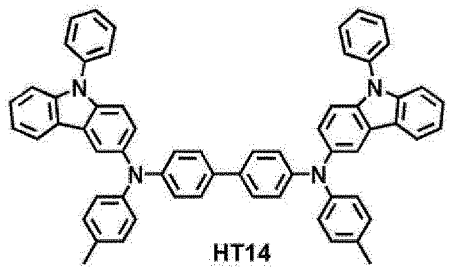

HT14

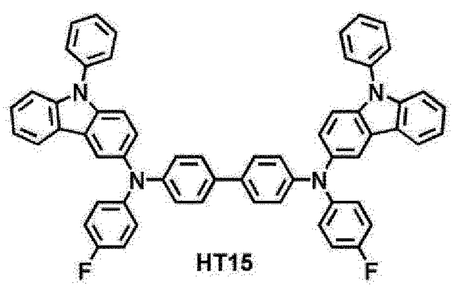

HT15

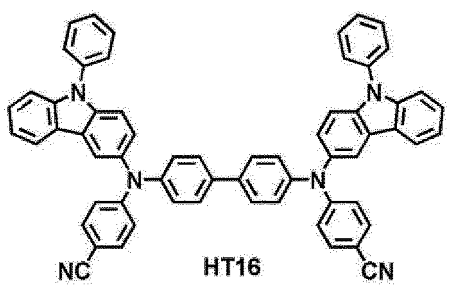

HT16

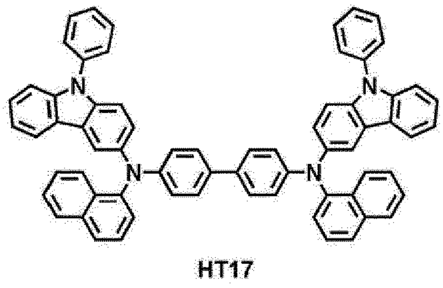

HT17

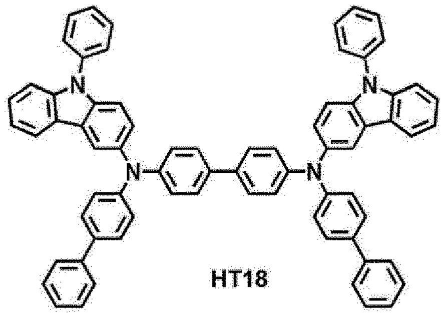

HT18

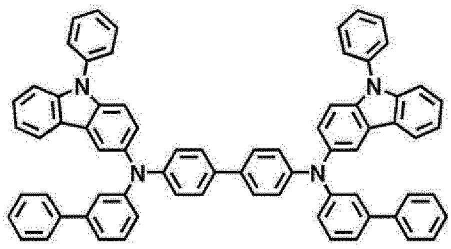

HT19

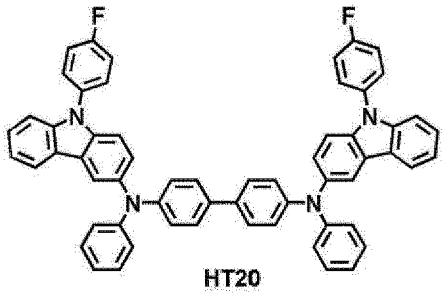

HT20

A thickness of the hole transport region may be in a range of about 100 Å to about 10,000 Å, for example, about 100 Å to about 1,000 Å. When the hole transport region includes at least one of a hole injection layer and a hole transport layer, a thickness of the hole injection layer may be in a range of about 100 Å to about 10,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region, the hole injection layer and the hole transport layer are within these ranges, satisfactory hole transporting characteristics may be obtained without a substantial increase in driving voltage.

The hole transport region may further include, in addition to these materials, a charge-generation material for improvement of conductive properties. The charge-generation material may be homogeneously or non-homogeneously dispersed in the hole transport region.

The charge-generation material may be, for example, a p-dopant. The p-dopant may be one of a quinone derivative, a metal oxide, and a cyano group-containing compound, but embodiments of the present disclosure are not limited thereto. Nonlimiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or Compound HT-D2 below, but are not limited thereto.

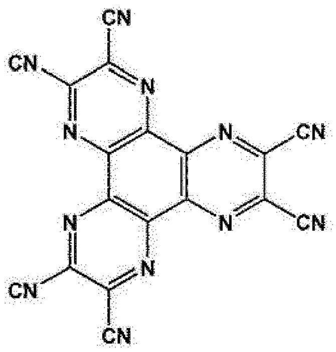

HT-D1

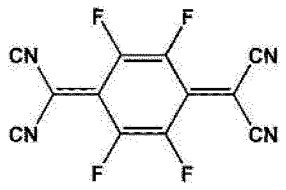

F4-TCNQ

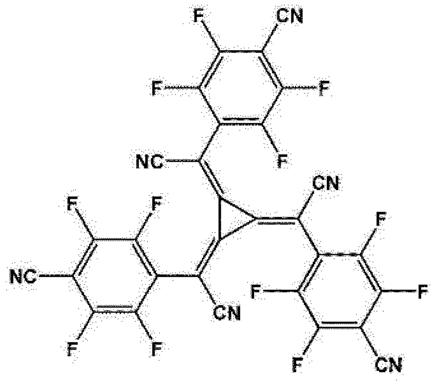

HT-D2

The hole transport region may include a buffer layer.

Also, the buffer layer may compensate for an optical resonance distance according to a wavelength of light emitted from the emission layer, and thus, efficiency of a formed organic light-emitting device may be improved.

Then, an emission layer (EML) may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a material that is used to form the hole transport layer.

The hole transport region may further include an electron blocking layer. The electron blocking layer may include a material available in the art, for example, mCP, but embodiments of the present disclosure are not limited.

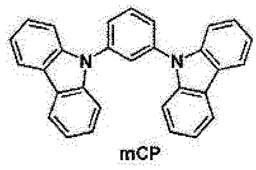

mCP

The thickness of the electron blocking layer may be about 50 Å to about 1,000 Å, for example about 70 Å to about 500 Å. When the thickness of the electron blocking layer is within the range described above, the electron blocking layer may have satisfactory electron blocking characteristics without a substantial increase in driving voltage.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

The emission layer may include the heterocyclic compound.

For example, the emission layer may include the heterocyclic compound alone.

In one or more embodiments, the emission layer may include the heterocyclic compound, and may further include: i) the second compound (for example, a compound different from the heterocyclic compound); ii) the organometallic compound represented by Formula 81; or iii) any combination thereof.

The heterocyclic compound, the second compound, and the organometallic compound represented by Formula 81 may each be the same as described above.

When the emission layer includes a host and a dopant, the amount of the dopant may be in the range of about 0.01 to about 20 parts by weight based on 100 parts by weight of the emission layer. However, the amount of the dopant included in the emission layer is not limited thereto. When the amount of the dopant satisfies the range, it may be possible to realize emission without extinction phenomenon.

When the emission layer includes the heterocyclic compound and the second compound, the weight ratio of the heterocyclic compound to the second compound may be in the range of about 1: 99 to about 99: 1, for example, about 70: 30 to about 30: 70. In one or more embodiments, the weight ratio of the heterocyclic compound and the second compound may be in the range of about 60:40 to about 40:60. When the weight ratio of the heterocyclic compound to the second compound in the emission layer is within this range, the charge transport balance in the emission layer may be effectively performed.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

Then, an electron transport region may be located on the emission layer.

The electron transport region may include at least one a hole blocking layer, an electron transport layer and an electron injection layer.

For example, the electron transport region may have a hole blocking layer/electron transport layer/electron injection layer structure or an electron transport layer/electron injection layer structure, and the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, Bphen, or any combination thereof, but embodiments of the present disclosure are not limited thereto.

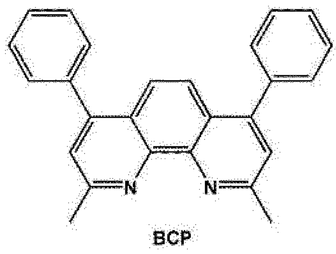

BCP

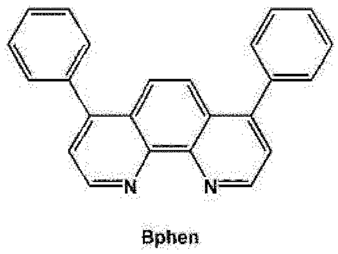

Bphen

In one or more embodiments, the hole blocking layer may include the heterocyclic compound.

A thickness of the hole blocking layer may be in a range of about 20 Å to about 1,000 Å, for example, about 30 Å to about 300 Å. When the thickness of the hole blocking layer is within these ranges, the hole blocking layer may have excellent hole blocking characteristics without a substantial increase in driving voltage.

The electron transport layer may further include at least one BCP, Bphen, $Alq_3$, BAlq, TAZ, NTAZ, or any combination thereof.

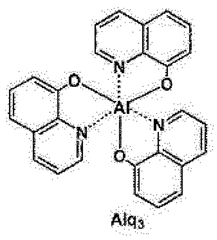

$Alq_3$

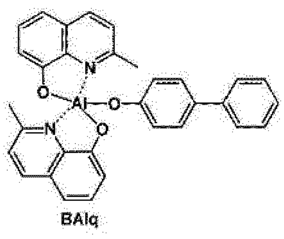

BAlq

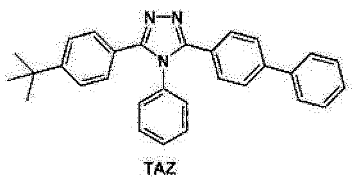

TAZ

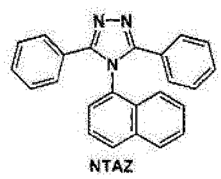

NTAZ

In one or more embodiments, the electron transport layer may include at least one of ET1, ET2, ET3, or any combination thereof, but are not limited thereto:

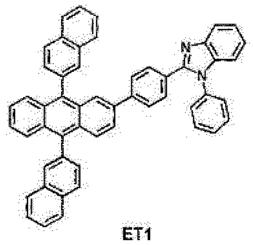

ET1

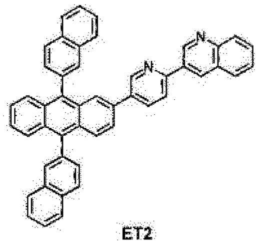

ET2

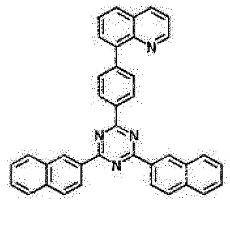

ET3

A thickness of the electron transport layer may be in a range of about 100 Å to about 1,000 Å, for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium quinolate, LiQ) or ET-D2:

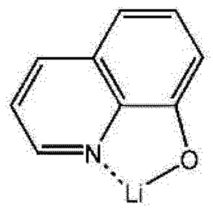

ET-D1

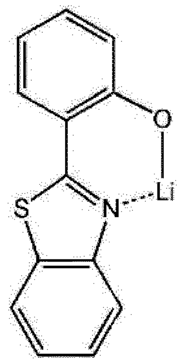

ET-D2

The electron transport region may include an electron injection layer (EIL) that promotes the flow of electrons from the second electrode 19 thereinto.

The electron injection layer may include at least one LiQ, LiF, NaCl, CsF, $Li_2O$, BaO, or any combination thereof.

A thickness of the electron injection layer may be in a range of about 1 Å to about 100 Å, and, for example, about 3 Å to about 90 Å. When the thickness of the electron injection layer is within the range described above, the electron injection layer may have satisfactory electron injection characteristics without a substantial increase in driving voltage.

The second electrode 19 is located on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as the material for forming the second electrode 19. To manufacture a top-emission type light-emitting device, a transmissive electrode formed using ITO or IZO may be used as the second electrode 19.

Hereinbefore, the organic light-emitting device has been described with reference to FIGURE, but embodiments of the present disclosure are not limited thereto.

According to one embodiment, provided is an electronic apparatus including a substrate, and an organic light-emitting device located on the substrate. The organic light-emitting device is the same as described above.

According to one embodiment, the electronic apparatus may further include a color conversion layer,
The color conversion layer may be located on at least one propagation direction of light emitted from the organic light-emitting device, and may include a quantum dot.

The quantum dot is a particle having a crystal structure of several to several tens of nanometers, and includes hundreds to thousands of atoms.

Since the quantum dot is very small in size, a quantum confinement effect may occur. The quantum confinement effect refers to a phenomenon in which a band gap of an object becomes large when the object becomes smaller than a nanometer size. Accordingly, when light having a wavelength having an energy intensity that is greater than the band gap of the quantum dot is irradiated to the quantum dot, the quantum dot is excited by absorbing the light and emits light having a specific wavelength and transits to the ground state. In this case, the wavelength of the emitted light has a value

corresponding to the band gap.

The quantum dot may be a semiconductor material. For example, the quantum dot may include a Group II-VI semiconductor compound, a Group III-V semiconductor compound, a Group IV-VI semiconductor compound, a Group IV element or compound, or a combination thereof. The Group II-VI semiconductor compound may be, for example, a binary compound which may be CdS, CdSe, CdTe, ZnS, ZnSe, ZnTe, ZnO, HgS, HgSe, HgTe, or a combination thereof; a ternary compound which may be CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, or a combination thereof; or a quaternary compound which may be CdHgZnTe, CdZnSeS, CdZnSeTe, CdZnSTe, CdHgSeS, CdHgSeTe, CdHgSTe, HgZnSeS, HgZnSeTe, HgZnSTe, or a combination thereof. The Group III-V semiconductor compound may be, for example, a binary compound which may be GaN, GaP, GaAs, GaSb, AlN, AlP, AlAs, AlSb, InN, InP, InAs, InSb, or a combination thereof; a ternary compound which may be GaNP, GaNAs, GaNSb, GaPAs, GaPSb, AlNP, AlNAs, AlNSb, AlPAs, AlPSb, InNP, InNAs, InNSb, InPAs, InPSb, GaAlNP, or a combination thereof; or a quaternary compound which may be GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, or a combination thereof. The Group IV-VI semiconductor compound may be, for example, a binary compound which may be SnS, SnSe, SnTe, PbS, PbSe, PbTe, or a combination thereof; a ternary compound which may be SnSeS, SnSeTe, SnSTe, PbSeS, PbSeTe, PbSTe, SnPbS, SnPbSe, SnPbTe, or a combination thereof; or a quaternary compound which may be SnPbSSe, SnPbSeTe, SnPbSTe, or a combination thereof. The Group IV element or compound may be, for example Si, Ge, SiC, SiGe, or a combination thereof.

The quantum dot may have a core structure or a core-shell structure, or a core-shell-shell structure. The quantum dot core may have a diameter of about 1 nm to several tens nm depending on a composition material. The quantum dot core-shell structure may be, for example, a CdSe/CdS structure or an InP/ZnS structure. The quantum dot core-shell-shell structure may be, for example, a CdSe/CdS/ZnS structure.

The quantum dot may adjust the color of emitted light according to the particle size. Therefore, the quantum dot may emit various emission colors such as blue, red, or green.

In addition, the form of the quantum dot is not particularly limited. For example, the quantum dot may be a spherical, cubic, pyramid, or multi-arm nanoparticle. In one or more embodiments, the quantum dot may have the form of nanotubes, nanowires, nanofibers, nanoplate particles, or the like.

The term "$C_1$-$C_{60}$ alkyl group" as used herein refers to a linear or branched saturated aliphatic hydrocarbon monovalent group having 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" used herein refers to a monovalent group represented by $-OA_{101}$ (wherein $A_{101}$ is the $C_1$-$C_{60}$ alkyl group), and examples thereof include a methoxy group, an ethoxy group, and an isopropyloxy group.

The term "$C_2$-$C_{60}$ alkenyl group" as used herein refers to a hydrocarbon group formed by substituting at least one carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a hydrocarbon group formed by substituting at least one carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_1$-$C_{10}$ heterocycloalkyl group" as used herein refers to a monovalent saturated monocyclic group having at least one N, O, P, Si, B, Se, Ge, Te, S, or any combination thereof as a ring-forming atom and 1 to 10 carbon atoms, and non-limiting examples thereof include a tetrahydrofuranyl group, and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_2$-$C_{10}$ heterocycloalkenyl group" as used herein refers to a monovalent monocyclic group that has at least one N, O, P, Si, B, Se, Ge, Te, S, or any combination thereof as a ring-forming atom, 2 to 10 carbon atoms, and at least

one (e.g., carbon-carbon) double bond in its ring. Examples of the $C_2$-$C_{10}$ heterocycloalkenyl group are a 2,3-dihydrofuranyl group, and a 2,3-dihydrothiophenyl group. The term "$C_2$-$C_{10}$ heterocycloalkenylene group" as used herein refers to a divalent group having the same structure as the $C_2$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other. The $C_7$-$C_{60}$ alkylaryl group refers to a $C_6$-$C_{60}$ aryl group substituted with at least one $C_1$-$C_{60}$ alkyl group.

The term "$C_1$-$C_{60}$ heteroaryl group" as used herein refers to a monovalent group having a carbocyclic aromatic system that has at least one heteroatom N, O, P, Si, B, Se, Ge, Te, S, or any combination thereof as a ring-forming atom, and 1 to 60 carbon atoms. The term "$C_1$-$C_{60}$ heteroarylene group" as used herein refers to a divalent group having a carbocyclic aromatic system that has at least one heteroatom N, O, P, B, Se, Ge, Te, S, or any combination thereof as a ring-forming atom, and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_6$-$C_{60}$ heteroaryl group and the $C_6$-$C_{60}$ heteroarylene group each include two or more rings, the rings may be fused to each other. The $C_2$-$C_{60}$ alkylheteroaryl group refers to a $C_1$-$C_{59}$ heteroaryl group substituted with at least one $C_1$-$C_{59}$ alkyl group.

The term "$C_6$-$C_{60}$ aryloxy group" as used herein indicates $-OA_{102}$ (wherein $A_{102}$ is the $C_6$-$C_{60}$ aryl group), and the term "$C_6$-$C_{60}$ arylthio group" as used herein indicates - $SA_{103}$ (wherein $A_{103}$ is the $C_6$-$C_{60}$ aryl group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom N, O, P, Si, B, Se, Ge, Te, S, or any combination thereof other than carbon atoms, as a ring-forming atom, and no aromaticity in its entire molecular structure. Examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "$C_5$-$C_{30}$ carbocyclic group" as used herein refers to a saturated or unsaturated cyclic group having, as a ring-forming atom, 5 to 30 carbon atoms only. The $C_5$-$C_{30}$ carbocyclic group may be a monocyclic group or a polycyclic group.

The term "$C_1$-$C_{30}$ heterocyclic group" as used herein refers to a saturated or unsaturated cyclic group having, as a ring-forming atom, at least one heteroatom N, O, Si, P, B, Se, Ge, Te, S, or any combination thereof other than 1 to 30 carbon atoms. The $C_1$-$C_{30}$ heterocyclic group may be a monocyclic group or a polycyclic group.

At least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_2$-$C_{10}$ heterocycloalkenyl group, the substituted $C_6$-$C_{60}$ aryl group, the substituted $C_7$-$C_{60}$ alkylaryl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, or any combination thereof;

a $C_1$-$C_{60}$ alkyl group, a $C_2$-$C_{60}$ alkenyl group, a $C_2$-$C_{60}$ alkynyl group, a $C_1$-$C_{60}$ alkoxy group, , or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a monovalent non-aromatic condensed polycyclic group, a monovalent non-aromatic condensed heteropolycyclic group, $-N(Q_{11})(Q_{12})$, - $Si(Q_{13})(Q_{14})(Q_{15})$, $-B(Q_{16})(Q_{17})$, $-P(=O)(Q_{18})(Q_{19})$, or

any combination thereof;
a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a monovalent non-aromatic condensed polycyclic group, a monovalent non-aromatic condensed heteropolycyclic group, or any combination thereof;
a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a monovalent non-aromatic condensed polycyclic group, a monovalent non-aromatic condensed heteropolycyclic group, or any combination thereof, each substituted with at least one deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_7$-$C_{60}$ alkylaryl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$, $-B(Q_{26})(Q_{27})$, $-P(=O)(Q_{28})(Q_{29})$, or any combination thereof; or

$-N(Q_{31})(Q_{32})$, $-Si(Q_{33})(Q_{34})(Q_{35})$, $-B(Q_{36})(Q_{37})$, $-P(=O)(Q_{38})(Q_{39})$, or any combination thereof,
wherein $Q_1$ to $Q_9$, $Q_{11}$ to $Q_{19}$, $Q_{21}$ to $Q_{29}$, and $Q_{31}$ to $Q_{39}$ may each independently be hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_1$-$C_{60}$ alkyl group substituted with at least one deuterium, a $C_1$-$C_{60}$ alkyl group, a $C_6$-$C_{60}$ aryl group, or any combination thereof, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a Ca-$C_{10}$ cycloalkenyl group, a $C_2$-$C_{10}$ heterocycloalkenyl group, a $C_6$-$C_{60}$ aryl group, a $C_6$-$C_{60}$ aryl group substituted with at least one deuterium, a $C_1$-$C_{60}$ alkyl group, a $C_6$-$C_{60}$ aryl group, or any combination thereof, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a monovalent non-aromatic condensed polycyclic group, a monovalent non-aromatic condensed heteropolycyclic group, or any combination thereof.

Hereinafter, a compound and an organic light-emitting device according to embodiments are described in detail with reference to Synthesis Example and Examples. However, the organic light-emitting device is not limited thereto. The wording "'B' was used instead of 'A'" used in describing Synthesis Examples means that an amount of 'A' used was identical to an amount of 'B' used, in terms of a molar equivalent.

Examples

Synthesis Example 1: Synthesis of Compound 1

Compound 1 was synthesized according to the following reaction scheme.

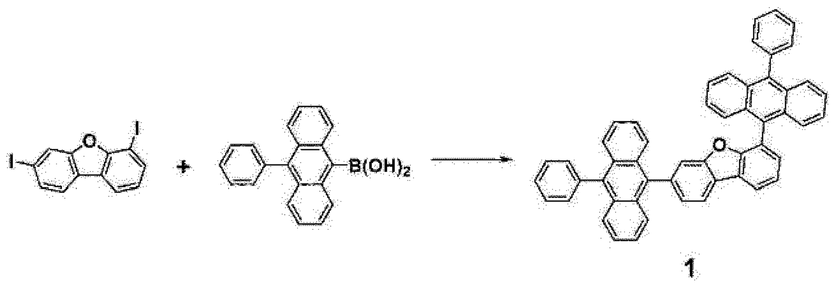

1

3,6-diiododibenzofuran (5 g, 11.9 mmol), (10-phenylanthracen-9-yl)boronic acid (7.8 g, 26.2 mmol), palladium tetrakistriphenylphosphine ($(Pd(PPh_3)_4$, 2.8 g, 2.4 mmol), and potassium phosphate tribasic ($K_3PO_4$, 15.2 g, 71.4 mmol) were added to 50 ml of toluene, 12 ml of ethanol, and 12 ml of distilled water, and the mixture was refluxed. After the reaction was completed, the reaction mixture was cooled to room temperature, the organic layer was extracted with toluene, dried using anhydrous sodium sulfate ($Na_2SO_4$), and filtered using a silica filter. The filtrate was concentrated and recrystallize with toluene and ethyl acetate to obtain Compound 1. (3.8 g, 5.6 mmol, yield 47 %)
LCMS (calculated: 672.25, found (M+1): 673.245 m/z)

Synthesis Example 2: Synthesis of Compound 113

Compound 113 was synthesized using the same method as in Synthesis Example 1, except that when Compound 1 was synthesized, 6-bromo-2-iododibenzofuran was used instead of 3,6-diiododibenzofuran. (Yield of 45 %) LCMS (calculated: 672.25, found(M+1): 673.245 m/z)

Synthesis Example 3: Synthesis of Compound 134

Compound 134 was synthesized in the same manner as used in Synthesis Example 1, except that when Compound 1 was synthesized, 6-bromo-2-iododibenzofuran was used instead of 3,6-diiododibenzofuran, and 2-(10-(4,4,5,5-tetramethyl-1,3,2-dioxaborolan-2-yl)anthracen-9-yl)benzonitrile was used instead of (10-phenylan anthracene-9-yl)boronic acid, (Yield of 35 %)
LCMS (calculated: 722.24, found(M+1): 723.241 m/z)

Synthesis Example 4: Synthesis of Compound 171

Compound 171 was synthesized according to the following reaction scheme.

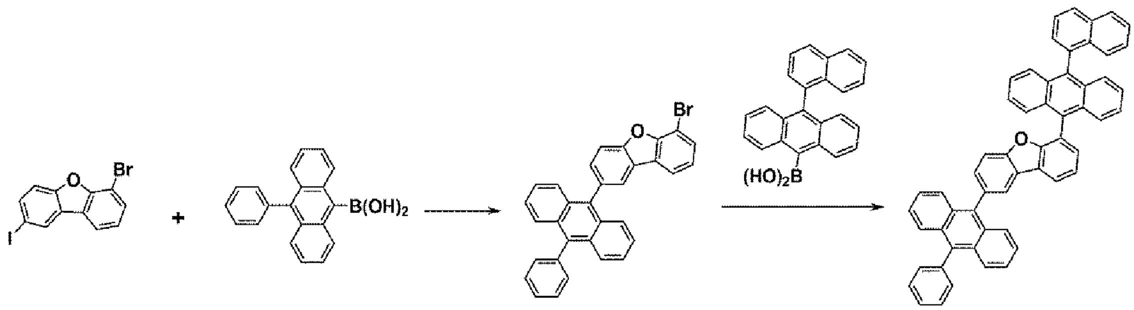

Intermediate (1)

171

(1) Synthesis of Intermediate (1)

6-bromo-2-iododibenzofuran (10 g, 26.8 mmol), (10-phenylanthracen-9-yl)boronic acid (8.8 g, 29.5 mmol), palladium tetrakistriphenylphosphine ($Pd(PPh_3)_4$, 6.2 g, 5.4 mmol), and potassium carbonate ($K_2CO_3$, 11.1 g, 80.4 mmol) were added to 120 ml of tetrahydrofuran (THF) and 40 ml of distilled water, and the mixture was refluxed. After the reaction was completed, the reaction mixture was cooled to room temperature, the organic layer was extracted with toluene, dried using anhydrous sodium sulfate ($Na_2SO_4$) and concentrated, and subjected to silica column chromatography, thereby obtaining Intermediate 1. (7.3 g, 14.6 mmol, yield 55 %)

(2) Synthesis of Compound 171

Compound 171 was synthesized using the same method as in Synthesis Example 1, except that when Compound 1 was synthesized, Intermediate 1 was used instead of 3,6-diiododibenzofuran, and 1.2 eq. of 10-(1-naphthyl)anthracene-9-boronic acid was used instead of (10-phenylanthracen-9-yl)boronic acid. (Yield of 52 %) LCMS (calculated: 722.26, found(M+1): 723.257 m/z)

Synthesis Example 5: Synthesis of Compound 1121

Compound 1121 was synthesized using the same method as in Synthesis Example 1, except that when Compound 1 was synthesized, 6-bromo-2-iododibenzoselenophene was used instead of 3,6-diiododibenzofuran. (Yield of 38 %)
LCMS (calculated: 736.17, found (M+1): 737.155 m/z)

Synthesis Example 6: Synthesis of Compound 2102

Compound 2102 was synthesized using the same method as in Synthesis Example 1, except that when Compound 1 was synthesized, 6-bromo-2-iododibenzofuran was used instead of 3,6-diiododibenzofuran and (10-butylanthracene-9-yl)boronic acid was used instead of (10-phenylanthracen-9-yl)boronic acid. (Yield of 46 %)

LCMS (calculated: 632.31, found(M+1): 633.307 m/z)

Synthesis Example 7: Synthesis of Compound A

Compound A was synthesized using the same method as in Synthesis Example 1, except that when Compound 1 was synthesized, 2,8-dibromodibenzofuran was used instead of 3,6-diiododibenzofuran. (Yield of 43 %)
LCMS (calculated: 672.25, found (M+1): 673.245 m/z)

Synthesis Example 8: Synthesis of Compound B

Compound B was synthesized using the same method as in Synthesis Example 1, except that when Compound 1 was synthesized, 6-bromo-2-iododibenzothiophene was used instead of 3,6-diiododibenzofuran. (Yield of 41 %)
LCMS (calculated: 688.22, found (M+1): 689.167 m/z)

Example 1

A patterned ITO glass substrate (50 mm x 50 mm x 0.7 mm) was ultrasonically cleaned in acetone, isopropyl alcohol, and distilled water, each for 20 minutes, and then, heat-treated at a temperature of 250 °C for 10 minutes.
Then, HATCN was deposited on the ITO electrode (anode) on the glass substrate at a deposition rate of 1Å/sec to form a hole injection layer having a thickness of 100 Å, and NPB was deposited on the hole injection layer at a deposition rate of 1Å/sec to form a hole transport layer having a thickness of 800 Å.
Then, mCP was deposited on the hole transport layer at a deposition rate of 1Å/sec to form an electron blocking layer having a thickness of 50 Å.
Compound 1 (host) and Compound D1 (dopant) were co-deposited on the electron blocking layer respectively at deposition rates of 0.97 Å/sec and 0.3 Å/sec to form an emission layer having a thickness of 200 Å.
DPEPO and LiQ (at the ratio of 1:1) were co-deposited on the emission layer at the deposition rate of 0.5Å/sec to form an electron transport layer having a thickness of 300 Å, and then LiQ was deposited on the electron transport layer at the deposition rate of 0.5 Å/sec to form an electron injection layer having a thickness of 10 Å, and then, Al was vacuum-deposited on the electron injection layer to form a second electrode(cathode) having a thickness of 1000 Å, thereby completing the manufacture of an organic light-emitting device having the structure of ITO/HATCN (100 Å)/NPB (800 Å)/mCP (50 Å)/Compound 1 + Compound D1 (3%) (200 Å)/DPEPO:LiQ (300 Å)/LiQ (10 Å)/Al (1000 Å).

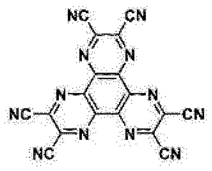
HATCN

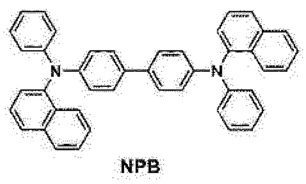
NPB

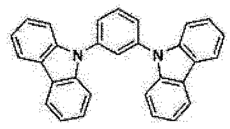
mCP

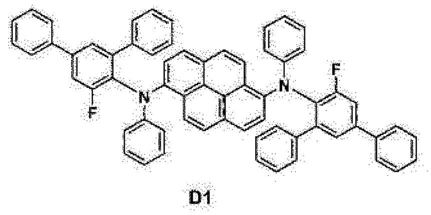
D1

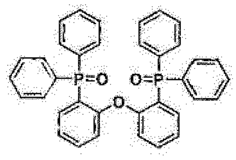
DPEPO

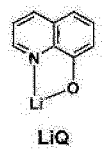
LiQ

Examples 2 to 5 and Comparative Examples 1 to 2

Organic light-emitting devices were manufactured in the same manner as in Example 1, except that Compounds shown in Table 2 were each used instead of Compound 1 as a host in forming an emission layer.

Evaluation Example 1: Characterization of organic light-emitting device

For each of the organic light-emitting devices manufactured according to Examples 1 to 5 and Comparative Examples 1 to 2, the photoluminescence quantum efficiency (PLQY), external quantum efficiency (EQE), TTF ratio and lifespan ($T_{95}$) were evaluated as relative values. The results are shown in Table 2. This evaluation was performed using a

current-voltage meter (Keithley 2400) and a luminescence meter (Minolta Cs-1,000A), and the lifespan ($T_{95}$)(at 6000nit) was evaluated as a relative value by measuring, as a relative value, the amount of time that elapsed until luminance was reduced to 95% of the initial brightness of 100%. The TTF ratio was obtained by taking the square of the inverse of the y-intercept value in the graph of, with respect to time, the 1/square root of the TrEL (1/sqrt (TrEL)) from 500 ns to 4000 ns after the decay of the transient electroluminescence (TrEL) was measured.

Table 2

| No. | Emission layer host | **EQE** (relative value) | lifespan($T_{95}$) (relative value) | TTF ratio (%) |
|---|---|---|---|---|
| Example 1 | Compound 1 | 100 | 100 | 23.8 |
| Example 2 | Compound 113 | 107.6 | 106.3 | 29.8 |
| Example 3 | Compound 171 | 107.9 | 116.3 | 28.5 |
| Example 4 | Compound 1121 | 113.4 | 53.2 | 26.3 |
| Example 5 | Compound 2102 | 117.3 | 60.0 | 24.2 |
| Comparative Example 1 | Compound A | 102.5 | 28.8 | 22 |
| Comparative Example 2 | Compound B | 111.8 | 52.8 | 22.8 |

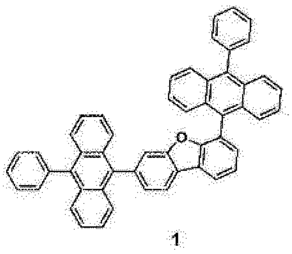
1

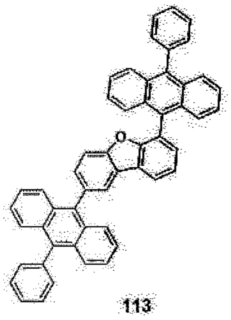
113

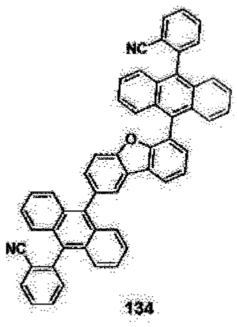
134

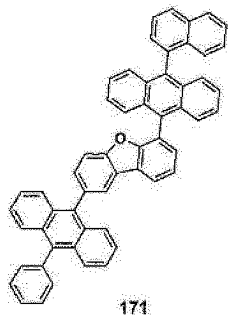
171

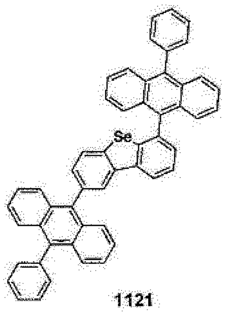
1121

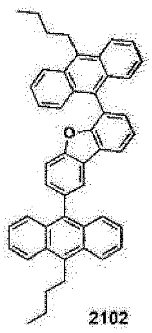
2102

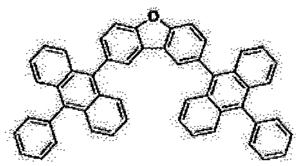
A

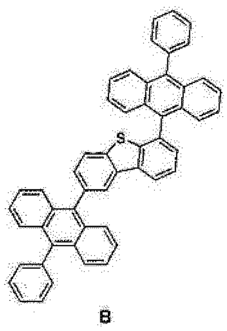
B

Table 2 shows that the organic light-emitting devices of Examples 1 to 5 have excellent external quantum efficiency and lifespan characteristics and high TTF ratios. In addition, the organic light-emitting devices of Example 1 to 5 showed better lifespan characteristics and higher TTF ratios than the organic light-emitting devices of Comparative Examples 1 and 2.

As described above, the heterocyclic compounds according to embodiments of the present disclosure have excellent electrical characteristics and thermal stability. Accordingly, an organic light-emitting device using the heterocyclic compound may have high efficiency, long lifespans, and high TTF ratios.

## Claims

**1.** A heterocyclic compound represented by one of Formulae 1-1 to 1-6:

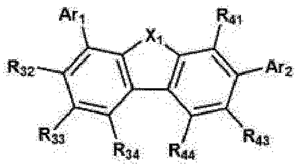
1-1

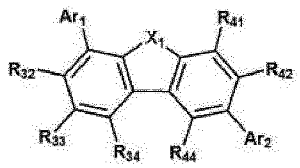
1-2

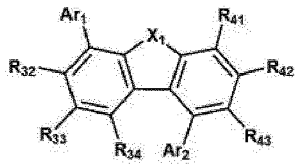
1-3

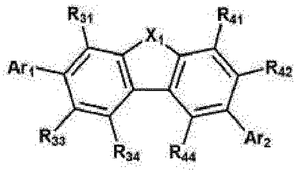
1-4

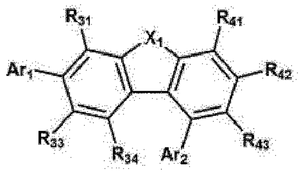
1-5

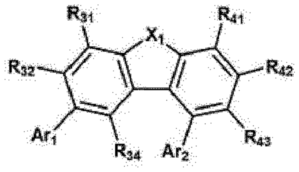
1-6

Formula 1A

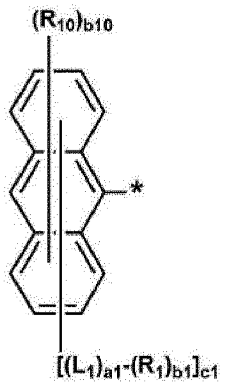

Formula 1B

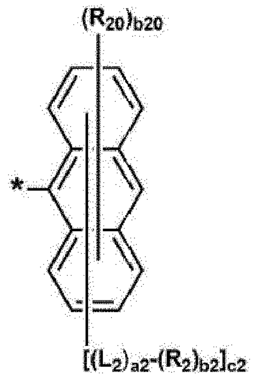

wherein, in Formulae 1-1 to 1-6, 1A, and 1B,

$X_1$ is O or Se,
$Ar_1$ is a group represented by Formula 1A, and $Ar_2$ is a group represented by Formula 1B,
$L_1$ and $L_2$ are each independently a substituted or unsubstituted $C_5$-$C_{30}$ carbocyclic group, a substituted or unsubstituted $C_1$-$C_{30}$ heterocyclic group, or any combination thereof,
a1 and a2 are each independently an integer from 0 to 3,
wherein when a1 is 2 or more, two or more of $L_1$(s) are identical to or different from each other, and when a2 is 2 or more, two or more of $L_2$(s) are identical to or different from each other,
$R_1$, $R_2$, $R_{10}$, $R_{20}$, $R_{31}$ to $R_{34}$, and $R_{41}$ to $R_{44}$ are each independently hydrogen, deuterium, -F, -Cl, -Br, -I, $-SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-B(Q_6)(Q_7)$, $-P(=O)(Q_8)(Q_9)$, or any combination thereof,
b1 and b2 are each independently an integer from 1 to 5,
wherein when b1 is 2 or more, two or more of $R_1$(s) are identical to or different from each other, and when b2 is 2 or more, two or more of $R_2$ are identical to or different from each other,
b10 and b20 are each independently an integer from 1 to 8,
wherein, when b10 is 2 or more, two or more of $R_{10}$(s) are identical to or different from each other, and when b20 is 2 or more, two or more of $R_{20}$(s) are identical to or different from each other,

c1 and c2 are each independently an integer from 1 to 8,
wherein, when c1 is 2 or more, two or more of $-(L_1)_{a1}-(R_1)_{b1}$ groups are identical to or different from each other, and when c2 is 2 or more, two or more of $-(L_2)_{a2}-(R_2)_{b2}$ groups are identical to or different from each other,
the sum of b10 and c1 is 9 and the sum of b20 and c2 is 9, and
at least one substituent of the substituted $C_5$-$C_{30}$ carbocyclic group, the substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_2$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_1$-$C_{60}$ heteroaryl group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, or any combination thereof;
a $C_1$-$C_{60}$ alkyl group, a $C_2$-$C_{60}$ alkenyl group, a $C_2$-$C_{60}$ alkynyl group, a $C_1$-$C_{60}$ alkoxy group, or any combination thereof, each substituted with at least one deuterium, - F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-Si(Q_{11})(Q_{12})(Q_{13})$, $-N(Q_{14})(Q_{15})$, $-B(Q_{16})(Q_{17})$, or any combination thereof;
a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof;
a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof, each substituted with at least one deuterium, -F, -Cl, -Br, -I, $-CD_3$, $- CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $- Si(Q_{21})(Q_{22})(Q_{23})$, $-N(Q_{24})(Q_{25})$, $-B(Q_{26})(Q_{27})$, or any combination thereof; or
$-Si(Q_{31})(Q_{32})(Q_{33})$, $-N(Q_{34})(Q_{35})$, $-B(Q_{36})(Q_{37})$, or any combination thereof,
wherein $Q_1$ to $Q_7$, $Q_{11}$ to $Q_{17}$, $Q_{21}$ to $Q_{27}$, and $Q_{31}$ to $Q_{37}$ are each independently hydrogen, deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_2$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted monovalent non-aromatic condensed polycyclic group, a substituted or unsubstituted monovalent non-aromatic condensed heteropolycyclic group, or any combination thereof.

**2.** The heterocyclic compound of claim 1, wherein $Ar_1$ is represented by one of Formulae 1A-1 to 1A-5:

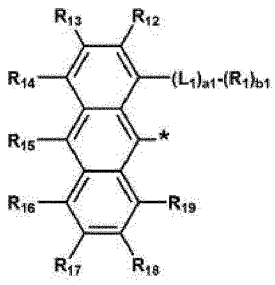
1A-1

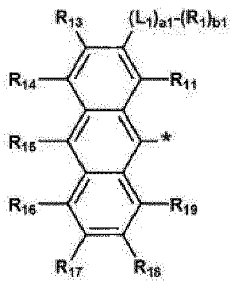
1A-2

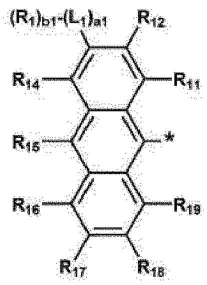
1A-3

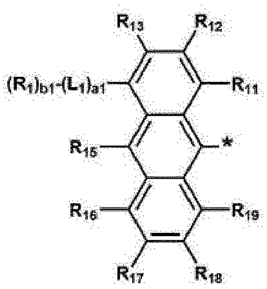
1A-4

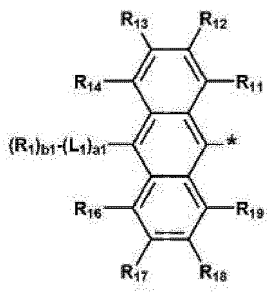
1A-5

wherein, in Formulae 1A-1 to 1A-5,

$L_1$, a1, $R_1$, and b1 are the same as described in connection with claim 1,
$R_{11}$ to $R_{19}$ are the same as described in connection with $R_{10}$ in claim 1, and
* indicates a binding site to a neighboring atom; and/or

wherein $Ar_2$ is represented by one of Formulae 2A-1 to 2A-5:

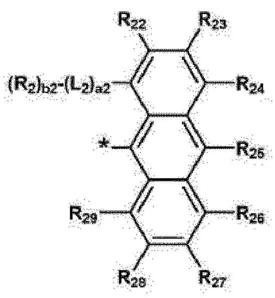
2A-1

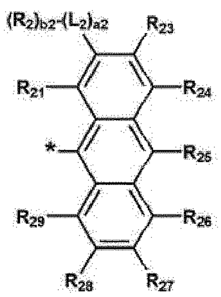
2A-2

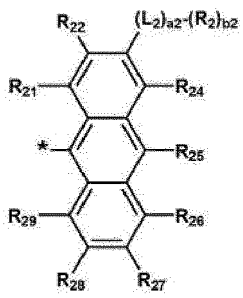
2A-3

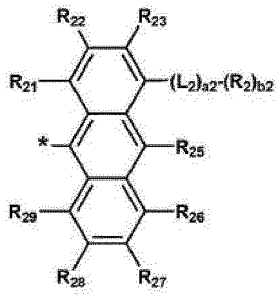
2A-4

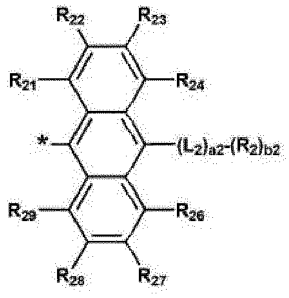
2A-5

wherein, in Formulae 2A-1 to 2A-5,

$L_2$, a2, $R_2$, and b2 are the same as described in connection with claim 1,
$R_{21}$ to $R_{29}$ are the same as described in connection with $R_{20}$ in claim 1, and
* indicates a binding site to a neighboring atom.

**3.** The heterocyclic compound of any of claims 1 or 2, wherein $L_1$ and $L_2$ are each independently:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof; or
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, a pentacenylene group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_2$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, or any combination thereof; and/or
wherein $R_1$ and $R_2$ are each independently deuterium, -F, a cyano group, a group represented by Formulae 9-1 to 9-19, a group represented by Formulae 10-1 to 10-208, or any combination thereof:

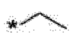
9-1
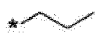
9-2
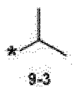
9-3
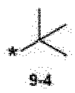
9-4
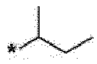
9-5
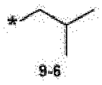
9-6
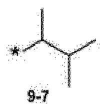
9-7

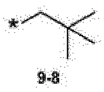
9-8
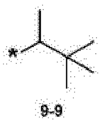
9-9
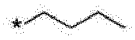
9-10
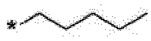
9-11
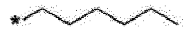
9-12

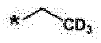
9-13
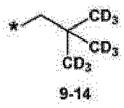
9-14
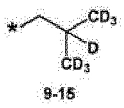
9-15
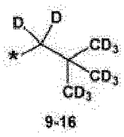
9-16
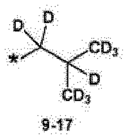
9-17
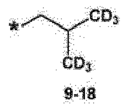
9-18

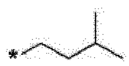
9-19

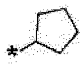
10-1
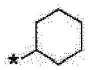
10-2
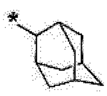
10-3
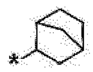
10-4
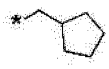
10-5
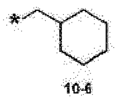
10-6
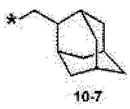
10-7

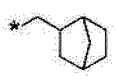
10-8
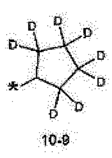
10-9
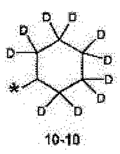
10-10
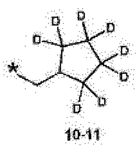
10-11
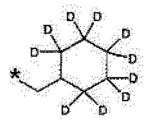
10-12
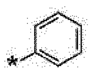
10-13

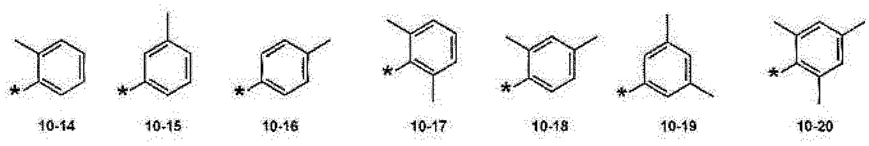

10-14 10-15 10-16 10-17 10-18 10-19 10-20

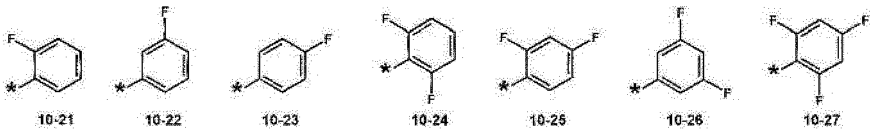

10-21 10-22 10-23 10-24 10-25 10-26 10-27

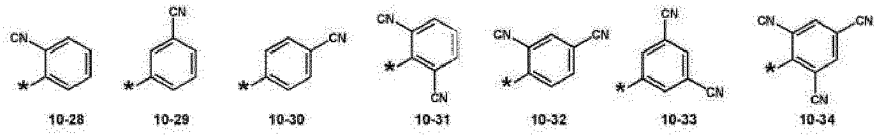

10-28 10-29 10-30 10-31 10-32 10-33 10-34

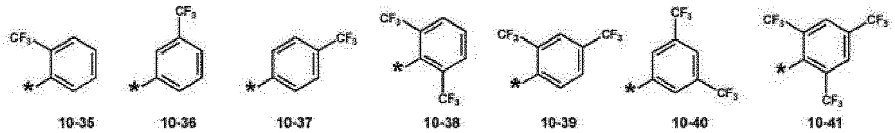

10-35 10-36 10-37 10-38 10-39 10-40 10-41

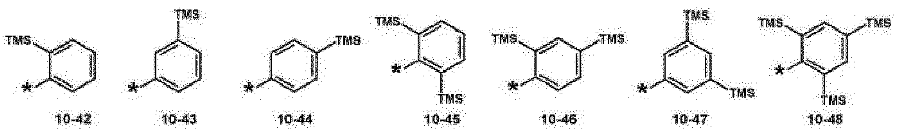

10-42 10-43 10-44 10-45 10-46 10-47 10-48

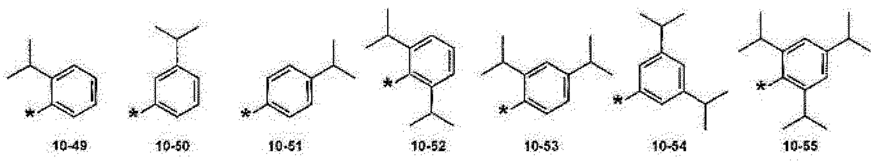

10-49 10-50 10-51 10-52 10-53 10-54 10-55

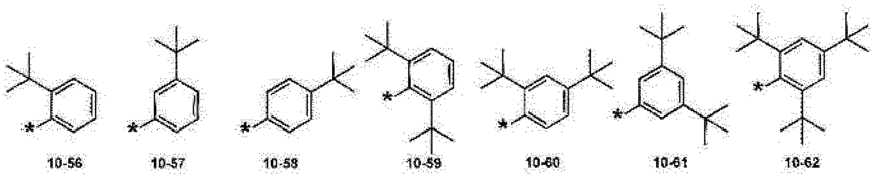

10-56 10-57 10-58 10-59 10-60 10-61 10-62

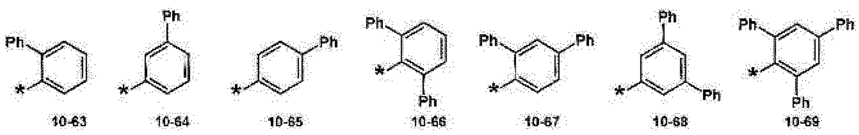

10-63 10-64 10-65 10-66 10-67 10-68 10-69

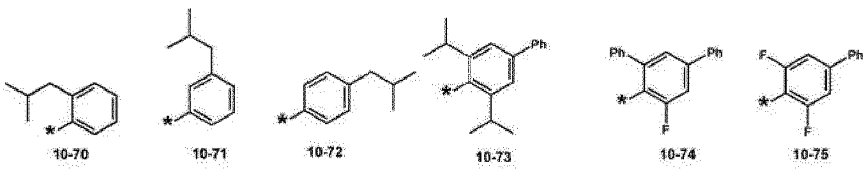

10-70 10-71 10-72 10-73 10-74 10-75

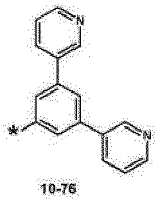
10-76
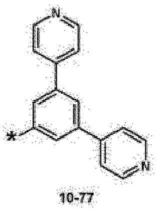
10-77
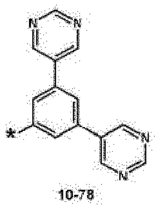
10-78
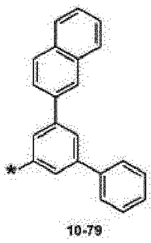
10-79
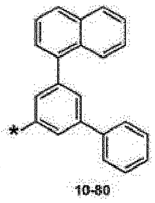
10-80

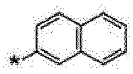
10-81
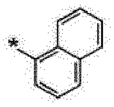
10-82
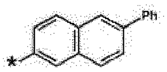
10-83
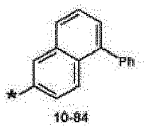
10-84
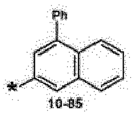
10-85

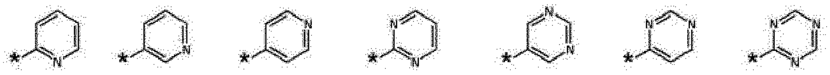
10-86
10-87
10-88
10-89
10-90
10-91
10-92

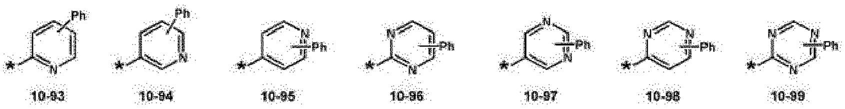
10-93
10-94
10-95
10-96
10-97
10-98
10-99

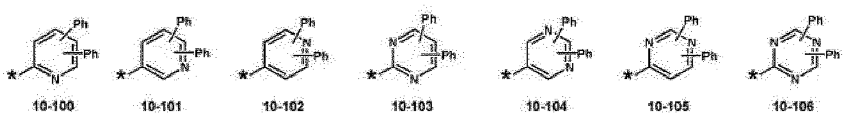
10-100
10-101
10-102
10-103
10-104
10-105
10-106

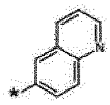
10-107
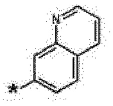
10-108
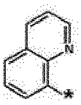
10-109
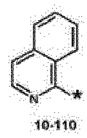
10-110
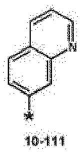
10-111
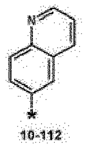
10-112

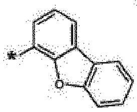
10-113
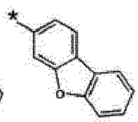
10-114
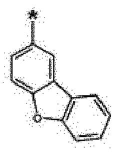
10-115
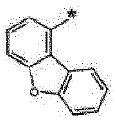
10-116
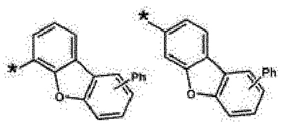
10-117
10-118

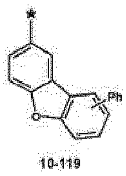
10-119
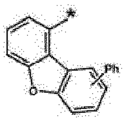
10-120
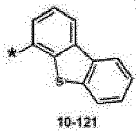
10-121
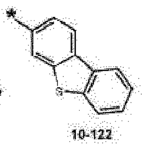
10-122
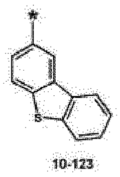
10-123
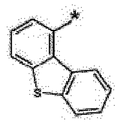
10-124

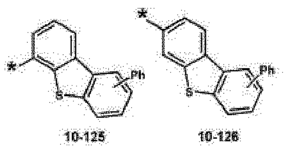
10-125
10-126
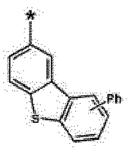
10-127
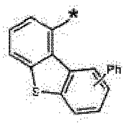
10-128
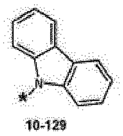
10-129
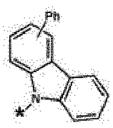
10-130

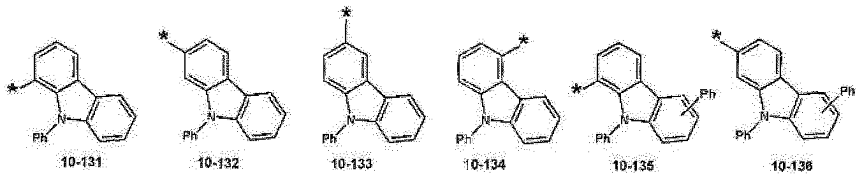

10-131 10-132 10-133 10-134 10-135 10-136

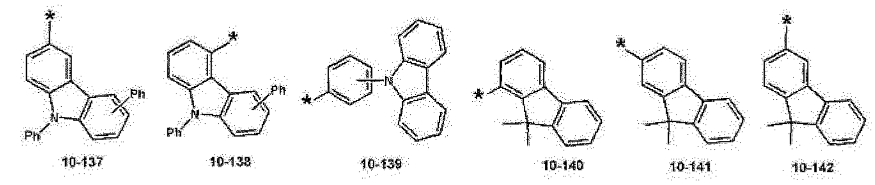

10-137 10-138 10-139 10-140 10-141 10-142

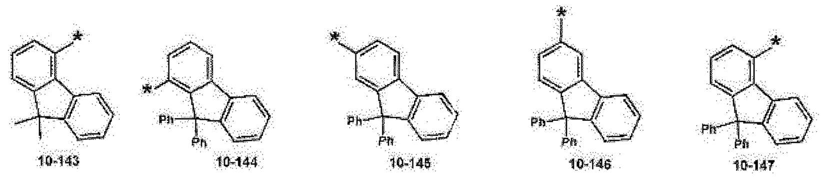

10-143 10-144 10-145 10-146 10-147

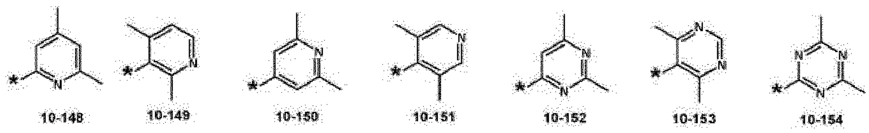

10-148 10-149 10-150 10-151 10-152 10-153 10-154

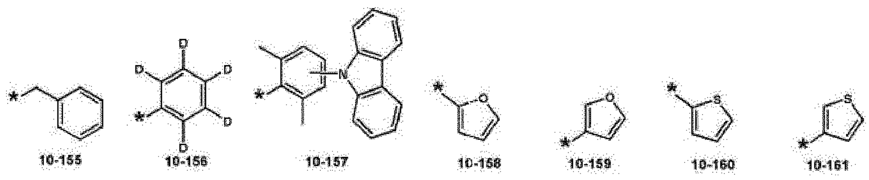

10-155 10-156 10-157 10-158 10-159 10-160 10-161

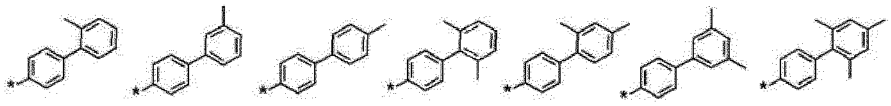

10-162 10-163 10-164 10-165 10-166 10-167 10-168

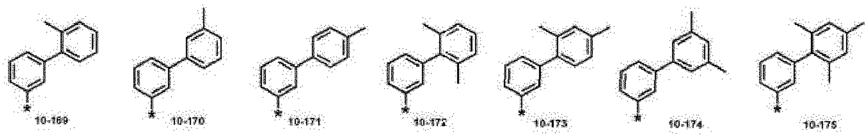

10-169 10-170 10-171 10-172 10-173 10-174 10-175

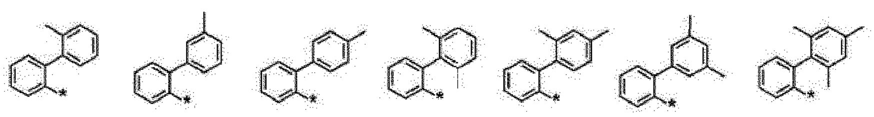

10-176 10-177 10-178 10-179 10-180 10-181 10-182

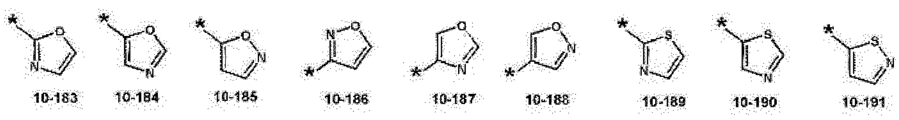

10-183 10-184 10-185 10-186 10-187 10-188 10-189 10-190 10-191

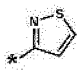 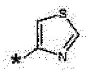 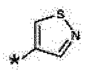

10-192 10-193 10-194

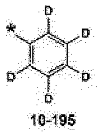 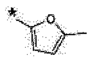 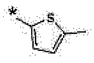 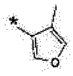 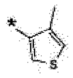 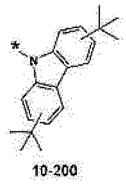 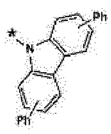 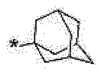

10-195 10-196 10-197 10-198 10-199 10-200 10-201 10-202

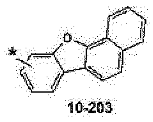 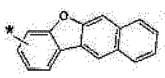 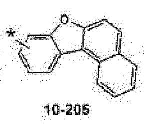 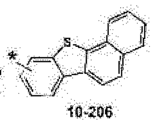 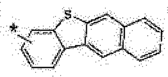 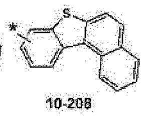

10-203 10-204 10-205 10-206 10-207 10-208

wherein, in Formulae 9-1 to 9-19 and 10-1 to 10-208, * indicates a binding site to a neighboring atom, Ph is a phenyl group, and TMS is a trimethylsilyl group.

**4.** The heterocyclic compound of any of claims 1-3, wherein $R_{10}$, $R_{20}$, $R_{31}$ to $R_{34}$, and $R_{41}$ to $R_{44}$ are each independently:

hydrogen, deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_1$-$C_{20}$ alkoxy group, or any combination thereof;
a $C_1$-$C_{20}$ alkyl group. a $C_1$-$C_{20}$ alkoxy group, or any combination thereof, each substituted with at least one deuterium, -F, -Cl, -Br, -I, a hydroxyl group, a cyano group, a nitro group, an amino group, an amidino group, a hydrazine group, a hydrazone group, a carboxylic acid or a salt thereof, a sulfonic acid or a salt thereof, a phosphoric acid or a salt thereof, a phenyl group, a naphthyl group, a pyridinyl group, a pyrimidinyl group, a pyrazinyl group, a pyridazinyl group, a triazinyl group, or any combination thereof;
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyrimidinyl group, an imidazopyridinyl group, or any combination thereof; or
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-bifluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyrimidinyl group, an imidazopyridinyl group, or any combination thereof, each substituted with at least one deuterium, -F, -Cl, -Br, -I, a hydroxyl group, a

cyano group, a nitro group, an amino group, an amidino group, a hydrazine group, a hydrazone group, a carboxylic acid or a salt thereof, a sulfonic acid or a salt thereof, a phosphoric acid or a salt thereof, a $C_1$-$C_{20}$ alkyl group, a $C_2$-$C_{20}$ alkenyl group, a $C_2$-$C_{20}$ alkynyl group, a $C_1$-$C_{20}$ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, or any combination thereof.

**5.** The heterocyclic compound of any of claims 1-4, wherein $R_{10}$, $R_{20}$, $R_{31}$ to $R_{34}$, and $R_{41}$ to $R_{44}$ are each independently:

hydrogen, deuterium, a cyano group, a $C_1$-$C_{20}$ alkyl group, a $C_1$-$C_{20}$ alkoxy group, or any combination thereof;
a $C_1$-$C_{20}$ alkyl group, a $C_1$-$C_{20}$ alkoxy group, or any combination thereof, each substituted with at least one deuterium, a cyano group, a phenyl group, a biphenyl group, a terphenyl group, a naphthyl group, or any combination thereof;
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group, a dibenzothiophenyl group, or any combination thereof; or
a cyclopentyl group, a cyclohexyl group, a cyclopentenyl group, a cyclohexenyl group, a cycloheptenyl group, a phenyl group, a biphenyl group, a terphenyl group, a naphthyl group, a fluorenyl group, a carbazolyl group, a dibenzofuranyl group, a dibenzothiophenyl group, or any combination thereof, each substituted with at least one deuterium, a cyano group, a $C_1$-$C_{20}$ alkyl group, a $C_1$-$C_{20}$ alkoxy group, a phenyl group, a biphenyl group, a terphenyl group, a naphthyl group, or any combination thereof.

**6.** The heterocyclic compound of any of claims 1-5, wherein the heterocyclic compound represented by Formulae 1-1 to 1-6 is a compound represented by one of Formulas 10-1 to 10-6:

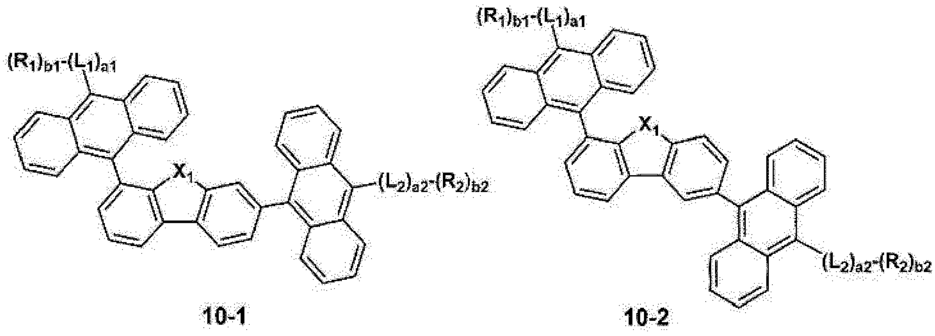

10-1 10-2

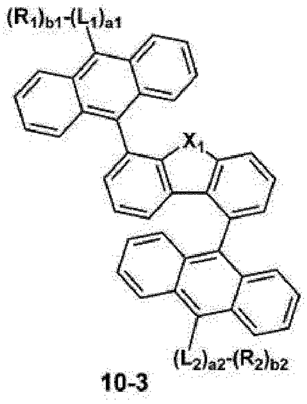

10-3

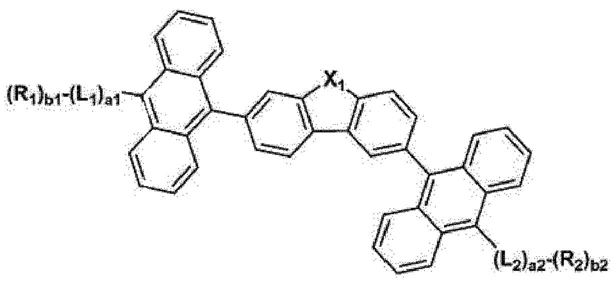

10-4

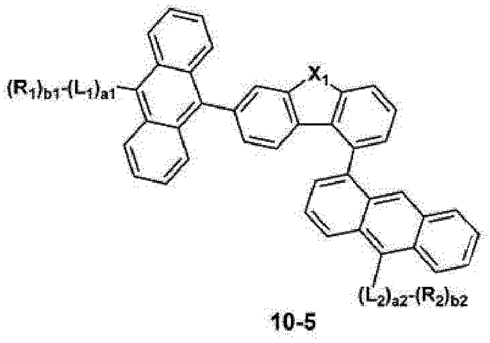

10-5

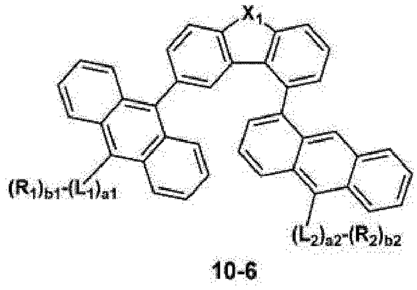

10-6

wherein, in Formulae 10-1 to 10-6,
$X_1$, $L_1$, $L_2$, a1, a2, $R_1$, $R_2$, b1, and b2 are the same as described in connection with claim 1.

7. The heterocyclic compound of any of claims 1-6, wherein the heterocyclic compound represented by one of Formula 1-1 to 1-6 is one of the following compounds:

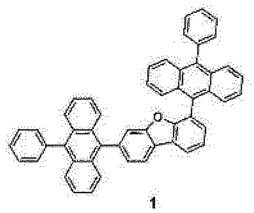

1

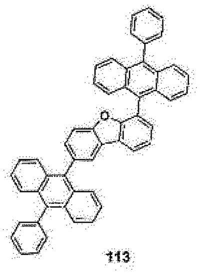

113

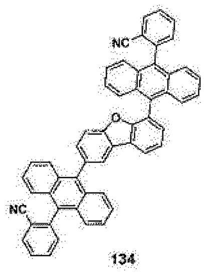

134

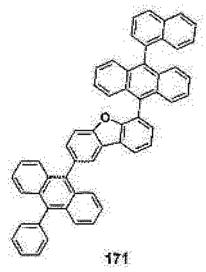

171

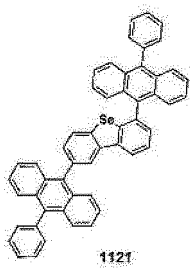

1121

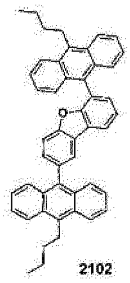

2102

.

8. An organic light-emitting device comprising:

a first electrode;
a second electrode; and
an organic layer located between the first electrode and the second electrode and comprising an emission layer, wherein the organic layer comprises at least one of the heterocyclic compounds represented by one of Formulae 1-1 to 1-6 of any of claims 1-7.

9. The organic light-emitting device of claim 8, wherein

the first electrode is an anode and the second electrode is a cathode,
the organic layer comprises a hole transport region between the first electrode and the emission layer and an electron transport region between the emission layer and the second electrode,
wherein the hole transport region comprises at least one of a hole injection layer, a hole transport layer, an electron blocking layer or any combination thereof, and
the electron transport region comprises at least one of a hole blocking layer, an electron transport layer, an electron injection layer, or any combination thereof.

10. The organic light-emitting device of claims 8 or 9, wherein the heterocyclic compound represented by one of Formulae

1-1 to 1-6 is included in the emission layer;
preferably wherein the emission layer emits light having a maximum emission wavelength of 410 nm to 490 nm.

**11.** The organic light-emitting device of claim 10, wherein the emission layer comprises a host and a dopant,

wherein the host comprises the heterocyclic compound represented by one of Formulae 1-1 to 1-6, and an amount of the host is greater than that of the dopant;
preferably wherein the dopant is a fluorescent dopant.

**12.** The organic light-emitting device of any of claims 9-11, wherein the heterocyclic compound represented by one of Formulae 1-1 to 1-6 is included in the hole transport region; and/or
wherein the heterocyclic compound represented by one of Formulae 1-1 to 1-6 is included in the electron transport region.

## Patentansprüche

**1.** Heterocyclische Verbindung, dargestellt durch eine der Formeln 1-1 bis 1-6:

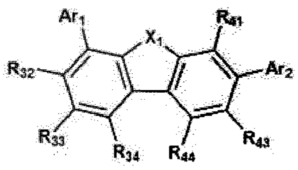
1-1

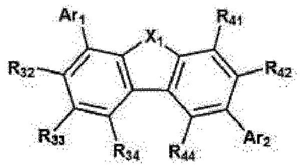
1-2

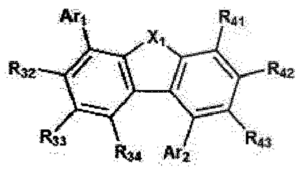
1-3

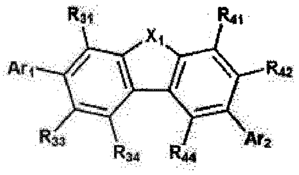
1-4

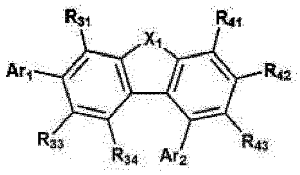
1-5

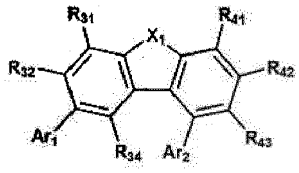
1-6

Formel 1A

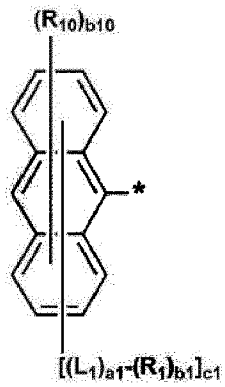

Formel 1B

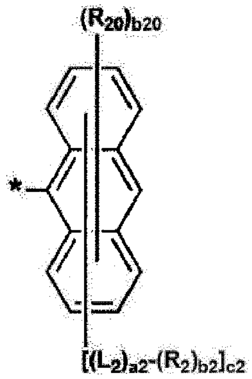

wobei, in Formel 1-1 bis 1-6, 1A und 1B,

$X_1$ O oder Se ist,
$Ar_1$ eine durch Formel 1A dargestellte Gruppe ist und $Ar_2$ eine durch Formel 1B dargestellte Gruppe ist,
$L_1$ und $L_2$ jeweils unabhängig eine substituierte oder unsubstituierte carbocyclische $C_5$-$C_{30}$-Gruppe, eine substituierte oder unsubstituierte heterocyclische $C_1$-$C_{30}$-Gruppe oder eine beliebige Kombination davon sind,
a1 und a2 jeweils unabhängig eine ganze Zahl von 0 bis 3 sind,
wobei, wenn a1 2 oder mehr ist, zwei oder mehr von $L_1$ zueinander identisch oder voneinander unterschiedlich

sind, und, wenn a2 2 oder mehr ist, zwei oder mehr von $L_2$ zueinander identisch oder voneinander unterschiedlich sind,

$R_1$, $R_2$, $R_{10}$, $R_{20}$, $R_{31}$ bis $R_{34}$ und $R_{41}$ bis $R_{44}$ jeweils unabhängig Wasserstoff, Deuterium, -F, -Cl, -Br, -I, $-SF_5$, eine Hydroxylgruppe, eine Cyanogruppe, eine Nitrogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_2$-$C_{10}$-Heterocycloalkenylgruppe, eine substituierte oder unsubstituierte $C_6$-$C_{60}$-Arylgruppe, eine substituierte oder unsubstituierte $C_6$-$C_{60}$-Aryloxygruppe, eine substituierte oder unsubstituierte $C_6$-$C_{60}$-Arylthiogruppe, eine substituierte oder unsubstituierte $C_1$-$C_{60}$-Heteroarylgruppe, eine substituierte oder unsubstituierte monovalente nicht-aromatische kondensierte polycyclische Gruppe, eine substituierte oder unsubstituierte monovalente nicht-aromatische kondensierte heteropolycyclische Gruppe, $-N(Q_1)(Q_2)$, - $Si(Q_3)(Q_4)(Q_5)$, $-B(Q_6)(Q_7)$, $-P(=O)(Q_8)(Q_9)$ oder eine beliebige Kombination davon sind,

b1 und b2 jeweils unabhängig eine ganze Zahl von 1 bis 5 sind,

wobei, wenn b1 2 oder mehr ist, zwei oder mehr von $R_1$ zueinander identisch oder voneinander unterschiedlich sind, und, wenn b2 2 oder mehr ist, zwei oder mehr von $R_2$ zueinander identisch oder voneinander unterschiedlich sind,

b10 und b20 jeweils unabhängig eine ganze Zahl von 1 bis 8 sind, wobei, wenn b10 2 oder mehr ist, zwei oder mehr von $R_{10}$ zueinander identisch oder voneinander unterschiedlich sind, und, wenn b20 2 oder mehr ist, zwei oder mehr von $R_{20}$ zueinander identisch oder voneinander unterschiedlich sind,

c1 und c2 jeweils unabhängig eine ganze Zahl von 1 bis 8 sind,

wobei, wenn c1 2 oder mehr ist, zwei oder mehr von $-(L_1)_{a1}-(R_1)_{b1}$-Gruppen zueinander identisch oder voneinander unterschiedlich sind, und, wenn c2 2 oder mehr ist, zwei oder mehr von $-(L_2)_{a2}-(R_2)_{b2}$-Gruppen zueinander identisch oder voneinander unterschiedlich sind,

die Summe von b10 und c1 9 ist und die Summe von b20 und c2 9 ist, und

mindestens ein Substituent der substituierten carbocyclischen $C_5$-$C_{30}$-Gruppe, der substituierten heterocyclischen $C_1$-$C_{30}$-Gruppe, der substituierten $C_1$-$C_{60}$-Alkylgruppe, der substituierten $C_2$-$C_{60}$-Alkenylgruppe, der substituierten $C_2$-$C_{60}$-Alkinylgruppe, der substituierten $C_1$-$C_{60}$-Alkoxygruppe, der substituierten $C_3$-$C_{10}$-Cycloalkylgruppe, der substituierten $C_1$-$C_{10}$-Heterocycloalkylgruppe, der substituierten $C_3$-$C_{10}$-Cycloalkenylgruppe, der substituierten $C_2$-$C_{10}$-Heterocycloalkenylgruppe, der substituierten $C_6$-$C_{60}$-Arylgruppe, der substituierten $C_6$-$C_{60}$-Aryloxygruppe, der substituierten $C_6$-$C_{60}$-Arylthiogruppe, der substituierten $C_1$-$C_{60}$-Heteroarylgruppe, der substituierten monovalenten nicht-aromatischen kondensierten polycyclischen Gruppe und der substituierten monovalenten nicht-aromatischen kondensierten heteropolycyclischen Gruppe Folgendes ist:

Deuterium, -F, -Cl, -Br, -I, $CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe oder eine beliebige Kombination davon;

eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe oder eine beliebige Kombination davon, jeweils substituiert mit mindestens einem Deuterium, -F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, eine monovalente nicht-aromatische kondensierte heteropolycyclische Gruppe, $-Si(Q_{11})(Q_{12})(Q_{13})$, $-N(Q_{14})(Q_{15})$, $-B(Q_{16})(Q_{17})$ oder eine beliebige Kombination davon;

eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, eine monovalente nicht-aromatische kondensierte heteropolycyclische Gruppe oder eine Kombination davon;

eine $C_3$-$C_{10}$-Cycloalkylgruppe, eine $C_1$-$C_{10}$-Heterocycloalkylgruppe, eine $C_3$-$C_{10}$-Cycloalkenylgruppe, eine

$C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, eine monovalente nicht-aromatische kondensierte heteropolycyclische Gruppe oder eine beliebige Kombination davon, jeweils substituiert mit mindestens einem Deuterium, -F, -Cl, -Br, -I, $-CD_3$, $-CD_2H$, $-CDH_2$, $-CF_3$, $-CF_2H$, $-CFH_2$, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe, eine $C_3$-$C_{10}$-Cycloalkylgruppe, eine $C_1$-$C_{10}$-Heterocycloalkylgruppe, eine $C_3$-$C_{10}$-Cycloalkenylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, eine monovalente nicht-aromatische kondensierte heteropolycyclische Gruppe, $-Si(Q_{21})(Q_{22})(Q_{23})$, $-N(Q_{24})(Q_{25})$, - $B(Q_{26})(Q_{27})$ oder eine beliebige Kombination davon; oder

$-Si(Q_{31})(Q_{32})(Q_{33})$, $-N(Q_{34})(Q_{35})$, $-B(Q_{36})(Q_{37})$ oder eine beliebige Kombination davon,

wobei $Q_1$ bis $Q_7$, $Q_{11}$ bis $Q_{17}$, $Q_{21}$ bis $Q_{27}$ und $Q_{31}$ bis $Q_{37}$ jeweils unabhängig Wasserstoff, Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine substituierte oder unsubstituierte $C_1$-$C_{60}$-Alkylgruppe, eine substituierte oder unsubstituierte $C_2$-$C_{60}$-Alkenylgruppe, eine substituierte oder unsubstituierte $C_2$-$C_{60}$-Alkinylgruppe, eine substituierte oder unsubstituierte $C_1$-$C_{60}$-Alkoxygruppe, eine substituierte oder unsubstituierte $C_3$-$C_{10}$-Cycloalkylgruppe, eine substituierte oder unsubstituierte $C_1$-$C_{10}$-Heterocycloalkylgruppe, eine substituierte oder unsubstituierte $C_3$-$C_{10}$-Cycloalkenylgruppe, eine substituierte oder unsubstituierte $C_2$-$C_{10}$-Heterocycloalkenylgruppe, eine substituierte oder unsubstituierte $C_6$-$C_{60}$-Arylgruppe, eine substituierte oder unsubstituierte $C_6$-$C_{60}$-Aryloxygruppe, eine substituierte oder unsubstituierte $C_6$-$C_{60}$-Arylthiogruppe, eine substituierte oder unsubstituierte $C_1$-$C_{60}$-Heteroarylgruppe, eine substituierte oder unsubstituierte monovalente nicht-aromatische kondensierte polycyclische Gruppe, eine substituierte oder unsubstituierte monovalente nicht-aromatische kondensierte heteropolycyclische Gruppe oder eine beliebige Kombination davon.

**2.** Heterocyclische Verbindung nach Anspruch 1, wobei $Ar_1$ dargestellt ist durch eine der Formeln 1A-1 bis 1A-5:

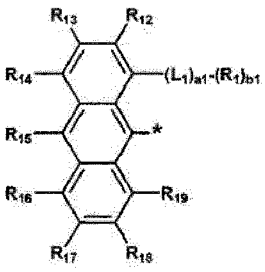

1A-1

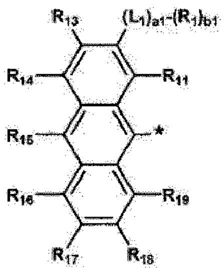

1A-2

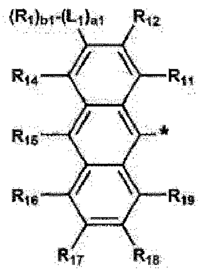

1A-3

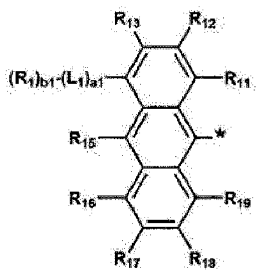

1A-4

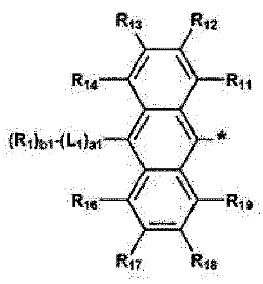

1A-5

wobei, in Formel 1A-1 bis 1A-5,

$L_1$, a1, $R_1$ und b1 dieselben wie im Zusammenhang mit Anspruch 1 beschrieben sind,
$R_{11}$ bis $R_{19}$ dieselben wie im Zusammenhang mit $R_{10}$ in Anspruch 1 beschrieben sind und

* eine Bindungsstelle zu einem benachbarten Atom angibt; und/oder

wobei $Ar_2$ dargestellt ist durch eine der Formeln 2A-1 bis 2A-5:

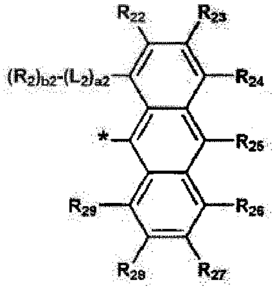

2A-1

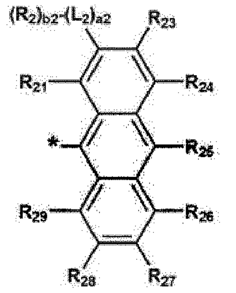

2A-2

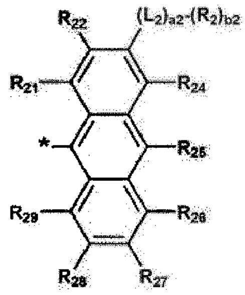

2A-3

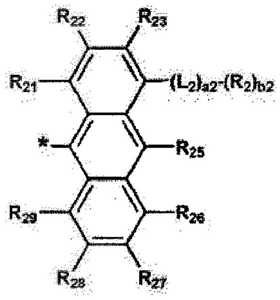

2A-4

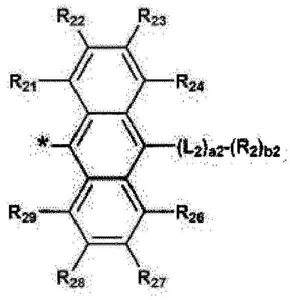

2A-5

wobei, in Formel 2A-1 bis 2A-5,

$L_2$, a2, $R_2$ und b2 dieselben wie im Zusammenhang mit Anspruch 1 beschrieben sind,
$R_{21}$ bis $R_{29}$ dieselben wie im Zusammenhang mit $R_{20}$ in Anspruch 1 beschrieben sind und
* eine Bindungsstelle zu einem benachbarten Atom angibt.

**3.** Heterocyclische Verbindung nach einem der Ansprüche 1 oder 2, wobei $L_1$ und $L_2$ jeweils unabhängig Folgendes sind:

eine Phenylengruppe, eine Pentalenylengruppe, eine Indenylengruppe, eine Naphthylengruppe, eine Azulenylengruppe, eine Heptalenylengruppe, eine Acenaphthylengruppe, eine Fluorenylengruppe, eine Phenalenylengruppe, eine Phenanthrenylengruppe, eine Anthracenylengruppe, eine Fluoranthenylengruppe, eine Triphenylenylengruppe, eine Pyrenylengruppe, eine Chrysenylenylengruppe, eine Naphthacenylengruppe, eine Picenylengruppe, eine Perylenylengruppe, eine Pentacenylengruppe oder eine beliebige Kombination davon; oder

eine Phenylengruppe, eine Pentalenylengruppe, eine Indenylengruppe, eine Naphthylengruppe, eine Azulenylengruppe, eine Heptalenylengruppe, eine Acenaphthylengruppe, eine Fluorenylengruppe, eine Phenalenylengruppe, eine Phenanthrenylengruppe, eine Anthracenylengruppe, eine Fluoranthenylengruppe, eine Triphenylenylengruppe, eine Pyrenylengruppe, eine Chrysenylenylengruppe, eine Naphthacenylengruppe, eine Picenylengruppe, eine Perylenylengruppe, eine Pentacenylengruppe oder eine beliebige Kombination davon, jeweils substituiert mit mindestens einem Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäuregruppe oder ein Salz davon, eine Sulfonsäuregruppe oder ein Salz davon, eine Phosphorsäuregruppe oder ein Salz davon, eine $C_1$-$C_{60}$-Alkylgruppe, eine $C_2$-$C_{60}$-Alkenylgruppe, eine $C_2$-$C_{60}$-Alkinylgruppe, eine $C_1$-$C_{60}$-Alkoxygruppe, eine $C_3$-$C_{10}$-Cycloalkylgruppe, eine $C_3$-$C_{10}$-Cycloalkenylgruppe, eine $C_1$-$C_{10}$-Heterocycloalkylgruppe, eine $C_2$-$C_{10}$-Heterocycloalkenylgruppe, eine $C_6$-$C_{60}$-Arylgruppe, eine $C_6$-$C_{60}$-Aryloxygruppe, eine $C_6$-$C_{60}$-Arylthiogruppe, eine $C_1$-$C_{60}$-Heteroarylgruppe, eine monovalente nicht-aromatische kondensierte polycyclische Gruppe, eine monovalente nicht-aromatische kondensierte heteropolycyclische Gruppe oder eine beliebige Kombination davon; und/oder

wobei $R_1$ und $R_2$ jeweils unabhängig Deuterium, -F, eine Cyanogruppe, eine durch Formel 9-1 bis 9-19

dargestellte Gruppe, eine durch Formel 10-1 bis 10-208 dargestellte Gruppe oder eine beliebige Kombination davon ist:

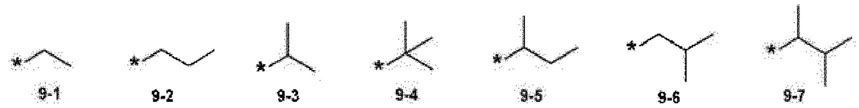

9-1 9-2 9-3 9-4 9-5 9-6 9-7

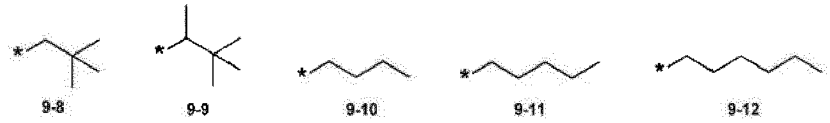

9-8 9-9 9-10 9-11 9-12

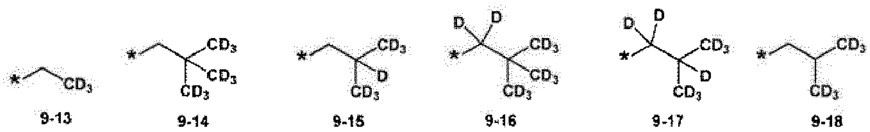

9-13 9-14 9-15 9-16 9-17 9-18

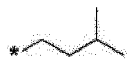

9-19

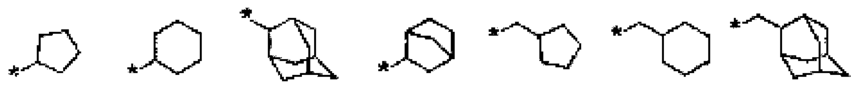

10-1 10-2 10-3 10-4 10-5 10-6 10-7

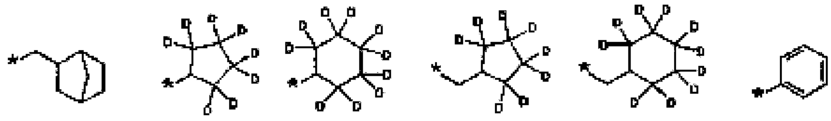

10-8 10-9 10-10 10-11 10-12 10-13

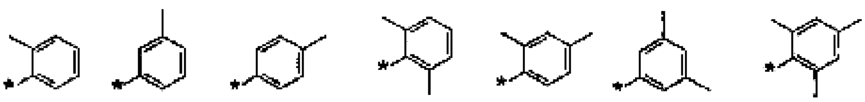

10-14 10-15 10-16 10-17 10-18 10-19 10-20

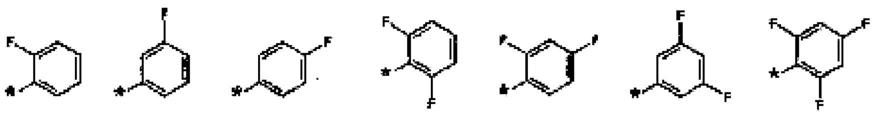

10-21 10-22 10-23 10-24 10-25 10-26 10-27

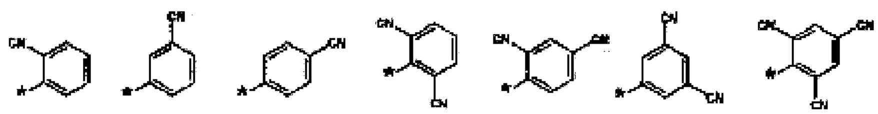

10-28 10-29 10-30 10-31 10-32 10-33 10-34

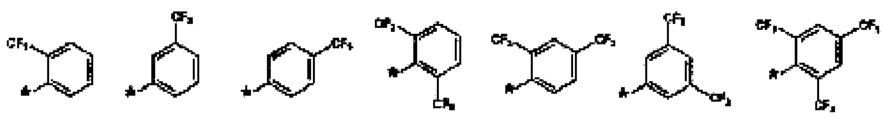

10-35 10-36 10-37 10-38 10-39 10-40 10-41

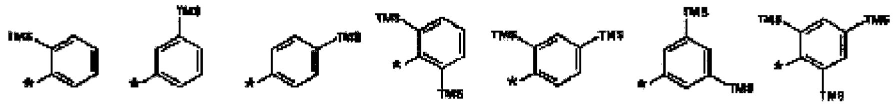

10-42 10-43 10-44 10-45 10-46 10-47 10-48

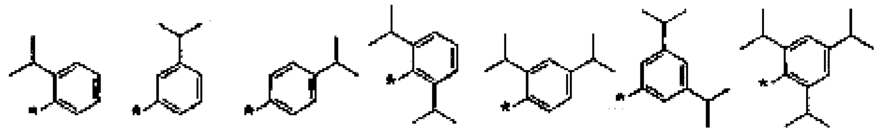

10-49 10-50 10-51 10-52 10-53 10-54 10-55

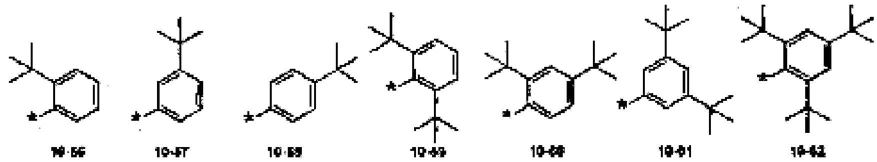

10-56 10-57 10-58 10-59 10-60 10-61 10-62

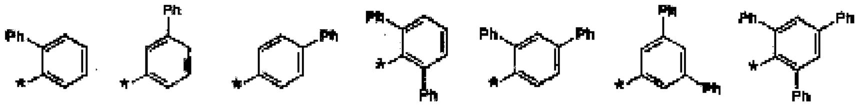

10-63 10-64 10-65 10-66 10-67 10-68 10-69

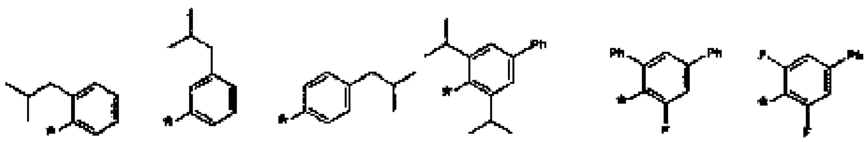

10-70 10-71 10-72 10-73 10-74 10-75

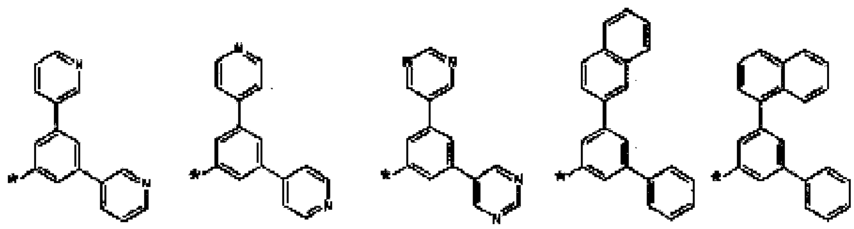

10-76 10-77 10-78 10-79 10-80

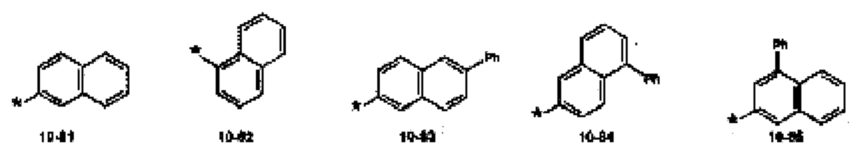

10-81 10-82 10-83 10-84 10-85

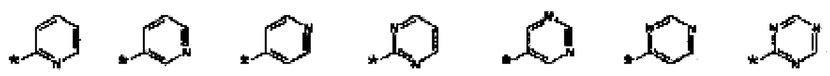

10-86 10-87 10-88 10-89 10-90 10-91 10-92

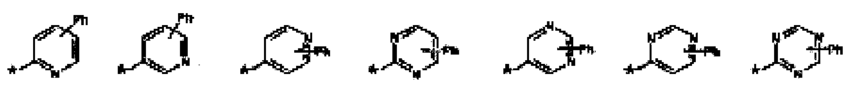

10-93 10-94 10-95 10-96 10-97 10-98 10-99

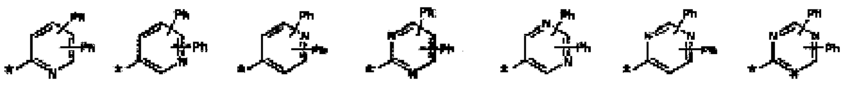
10-100 10-101 10-102 10-103 10-104 10-105 10-106

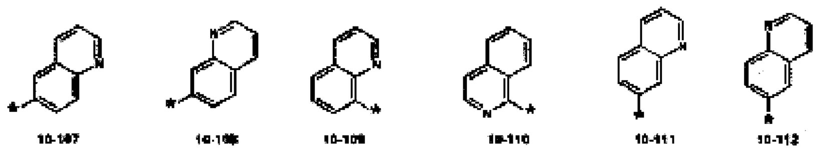
10-107 10-108 10-109 10-110 10-111 10-112

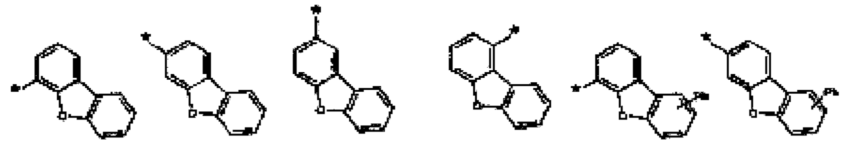
10-113 10-114 10-115 10-116 10-117 10-118

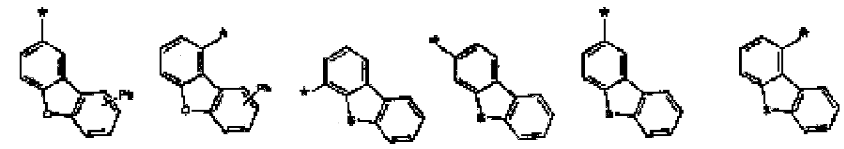
10-119 10-120 10-121 10-122 10-123 10-124

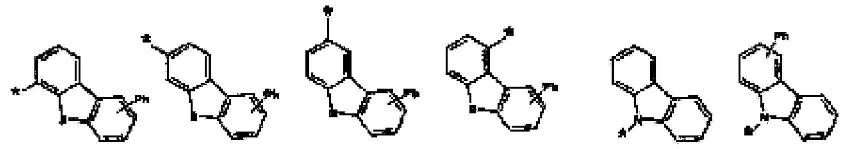
10-125 10-126 10-127 10-128 10-129 10-130

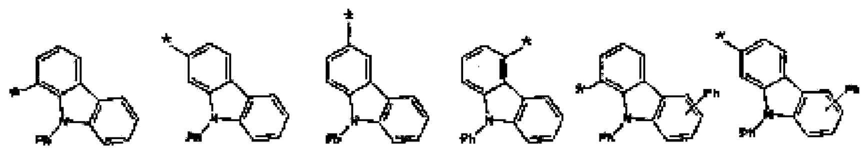
10-131 10-132 10-133 10-134 10-135 10-136

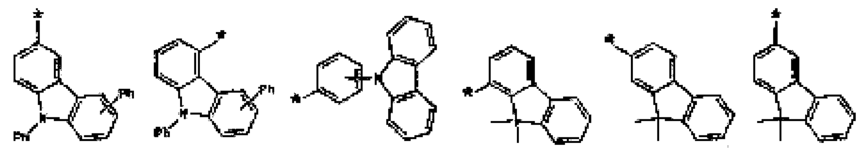
10-137 10-138 10-139 10-140 10-141 10-142

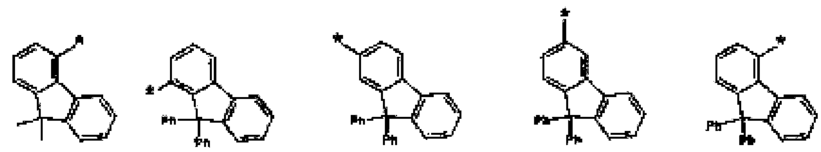
10-143 10-144 10-145 10-146 10-147

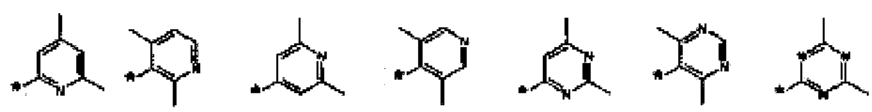
10-148 10-149 10-150 10-151 10-152 10-153 10-154

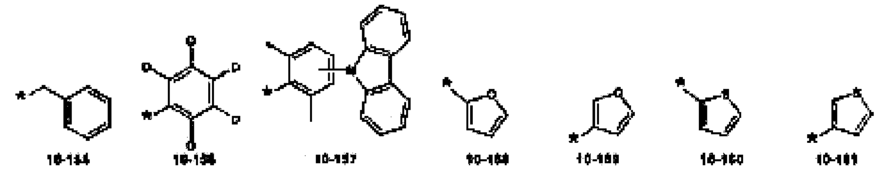

10-155 10-156 10-157 10-158 10-159 10-160 10-161

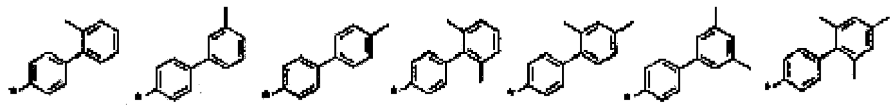

10-162 10-163 10-164 10-165 10-166 10-167 10-168

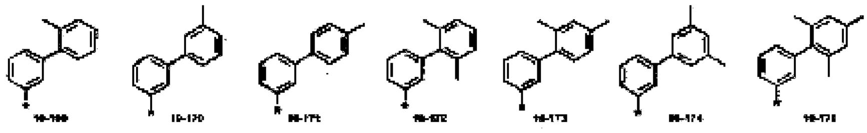

10-169 10-170 10-171 10-172 10-173 10-174 10-175

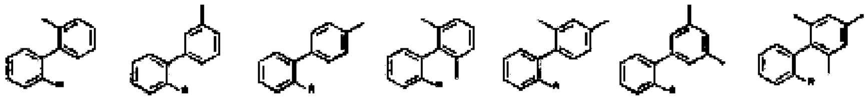

10-176 10-177 10-178 10-179 10-180 10-181 10-182

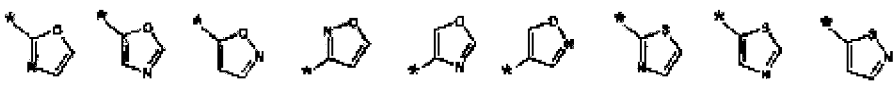

10-183 10-184 10-185 10-186 10-187 10-188 10-189 10-190 10-191

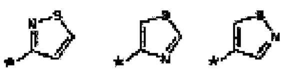

10-192 10-193 10-194

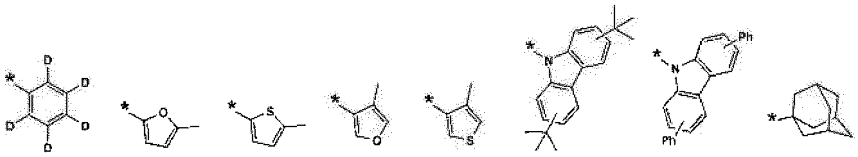

10-195 10-196 10-197 10-198 10-199 10-200 10-201 10-202

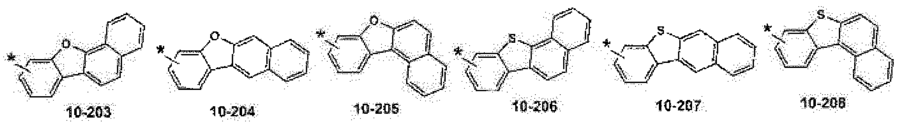

10-203 10-204 10-205 10-206 10-207 10-208

wobei, in Formel 9-1 bis 9-19 und 10-1 bis 10-208, * eine Bindungsstelle zu einem benachbarten Atom angibt, Ph eine Phenylgruppe ist und TMS eine Trimethylsilylgruppe ist.

**4.** Heterocyclische Verbindung nach einem der Ansprüche 1-3, wobei $R_{10}$, $R_{20}$, $R_{31}$ bis $R_{34}$ und $R_{41}$ bis $R_{44}$ jeweils unabhängig Folgendes sind:

Wasserstoff, Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine $C_1$-$C_{20}$-Alkylgruppe, eine $C_1$-$C_{20}$-Alkoxygruppe oder eine beliebige Kombination davon;
eine $C_1$-$C_{20}$-Alkylgruppe, eine $C_1$-$C_{20}$-Alkoxygruppe oder eine beliebige Kombination davon, jeweils substituiert mit mindestens einem Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein

Salz davon, eine Sulfonsäure oder ein Salz davon, eine Phosphorsäure oder ein Salz davon, eine Phenylgruppe, eine Naphthylgruppe, eine Pyridinylgruppe, eine Pyrimidinylgruppe, eine Pyrazinylgruppe, eine Pyridazinylgruppe, eine Triazinylgruppe oder eine beliebige Kombination davon;

eine Cyclopentylgruppe, eine Cyclohexylgruppe, eine Cyclopentenylgruppe, eine Cyclohexenylgruppe, eine Cycloheptenylgruppe, eine Phenylgruppe, eine Pentalenylgruppe, eine Indenylgruppe, eine Naphthylgruppe, eine Azulenylgruppe, eine Heptalenylgruppe, eine Indacenylgruppe, eine Acenaphthylgruppe, eine Fluorenylgruppe, eine Spiro-Bifluorenylgruppe, eine Phenalenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Naphthacenylgruppe, eine Picenylgruppe, eine Perylenylgruppe, eine Pentaphenylgruppe, eine Hexacenylgruppe, eine Pyrrolylgruppe, eine Imidazolylgruppe, eine Pyrazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Phthalazinylgruppe, eine Naphthyridinylgruppe, eine Chinoxalinylgruppe, eine Chinazolinylgruppe, eine Cinnolinylgruppe, eine Phenanthridinylgruppe, eine Acridinylgruppe, eine Phenanthrolinylgruppe, eine Phenazinylgruppe, eine Benzoxazolylgruppe, eine Benzimidazolylgruppe, eine Furanylgruppe, eine Benzofuranylgruppe, eine Thiophenylgruppe, eine Benzothiophenylgruppe, eine Thiazolylgruppe, eine Isothiazolylgruppe, eine Benzothiazolylgruppe, eine Isoxazolylgruppe, eine Oxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Imidazopyrimidinylgruppe, eine Imidazopyridinylgruppe oder eine beliebige Kombination davon; oder

eine Cyclopentylgruppe, eine Cyclohexylgruppe, eine Cyclopentenylgruppe, eine Cyclohexenylgruppe, eine Cycloheptenylgruppe, eine Phenylgruppe, eine Pentalenylgruppe, eine Indenylgruppe, eine Naphthylgruppe, eine Azulenylgruppe, eine Heptalenylgruppe, eine Indacenylgruppe, eine Acenaphthylgruppe, eine Fluorenylgruppe, eine Spiro-Bifluorenylgruppe, eine Phenalenylgruppe, eine Phenanthrenylgruppe, eine Anthracenylgruppe, eine Fluoranthenylgruppe, eine Triphenylenylgruppe, eine Pyrenylgruppe, eine Chrysenylgruppe, eine Naphthacenylgruppe, eine Picenylgruppe, eine Perylenylgruppe, eine Pentaphenylgruppe, eine Hexacenylgruppe, eine Pyrrolylgruppe, eine Imidazolylgruppe, eine Pyrazolylgruppe, eine Pyridinylgruppe, eine Pyrazinylgruppe, eine Pyrimidinylgruppe, eine Pyridazinylgruppe, eine Isoindolylgruppe, eine Indolylgruppe, eine Indazolylgruppe, eine Purinylgruppe, eine Chinolinylgruppe, eine Isochinolinylgruppe, eine Benzochinolinylgruppe, eine Phthalazinylgruppe, eine Naphthyridinylgruppe, eine Chinoxalinylgruppe, eine Chinazolinylgruppe, eine Cinnolinylgruppe, eine Phenanthridinylgruppe, eine Acridinylgruppe, eine Phenanthrolinylgruppe, eine Phenazinylgruppe, eine Benzoxazolylgruppe, eine Benzimidazolylgruppe, eine Furanylgruppe, eine Benzofuranylgruppe, eine Thiophenylgruppe, eine Benzothiophenylgruppe, eine Thiazolylgruppe, eine Isothiazolylgruppe, eine Benzothiazolylgruppe, eine Isoxazolylgruppe, eine Oxazolylgruppe, eine Triazolylgruppe, eine Tetrazolylgruppe, eine Oxadiazolylgruppe, eine Triazinylgruppe, eine Dibenzofuranylgruppe, eine Dibenzothiophenylgruppe, eine Imidazopyrimidinylgruppe, eine Imidazopyridinylgruppe oder eine beliebige Kombination davon, jeweils substituiert mit mindestens einem Deuterium, -F, -Cl, -Br, -I, eine Hydroxylgruppe, eine Cyanogruppe, eine Nitrogruppe, eine Aminogruppe, eine Amidinogruppe, eine Hydrazingruppe, eine Hydrazongruppe, eine Carboxylsäure oder ein Salz davon, eine Sulfonsäure oder ein Salz davon, eine Phosphorsäure oder ein Salz davon, eine $C_1$-$C_{20}$-Alkylgruppe, eine $C_2$-$C_{20}$-Alkenylgruppe, eine $C_2$-$C_{20}$-Alkinylgruppe, eine $C_1$-$C_{20}$-Alkoxygruppe, eine Phenylgruppe, eine Naphthylgruppe, eine Anthracenylgruppe, eine Pyrenylgruppe, eine Phenanthrenylgruppe, eine Fluorenylgruppe, eine Pyridinylgruppe, eine Pyrimidinylgruppe, eine Pyrazinylgruppe, eine Pyridazinylgruppe, eine Triazinylgruppe, eine Chinolinylgruppe, eine Isochinolinylgruppe, eine Phthalazinylgruppe, eine Chinoxalinylgruppe, eine Cinnolinylgruppe, eine Chinazolinylgruppe oder eine beliebige Kombination davon.

**5.** Heterocyclische Verbindung nach einem der Ansprüche 1 bis 4, wobei $R_{10}$, $R_{20}$, $R_{31}$ bis $R_{34}$ und $R_{41}$ bis $R_{44}$ jeweils unabhängig Folgendes sind:

Wasserstoff, Deuterium, eine Cyanogruppe, eine $C_1$-$C_{20}$-Alkylgruppe, eine $C_1$-$C_{20}$-Alkoxygruppe oder eine beliebige Kombination davon;

eine $C_1$-$C_{20}$-Alkylgruppe, eine $C_1$-$C_{20}$-Alkoxygruppe oder eine beliebige Kombination davon, jeweils substituiert mit mindestens einem Deuterium, eine Cyanogruppe, eine Phenylgruppe, eine Biphenylgruppe, eine Terphenylgruppe, eine Naphthylgruppe oder eine beliebige Kombination davon;

eine Cyclopentylgruppe, eine Cyclohexylgruppe, eine Cyclopentenylgruppe, eine Cyclohexenylgruppe, eine Cycloheptenylgruppe, eine Phenylgruppe, eine Biphenylgruppe, eine Terphenylgruppe, eine Naphthylgruppe, eine Fluorenylgruppe, eine Carbazolylgruppe, eine Dibenzofuranylgruppe, eine Dibenzothiophenylgruppe oder eine beliebige Kombination davon; oder

eine Cyclopentylgruppe, eine Cyclohexylgruppe, eine Cyclopentenylgruppe, eine Cyclohexenylgruppe, eine Cycloheptenylgruppe, eine Phenylgruppe, eine Biphenylgruppe, eine Terphenylgruppe, eine Naphthylgruppe, eine Fluorenylgruppe, eine Carbazolylgruppe, eine Dibenzofuranylgruppe, eine Dibenzothiophenylgruppe oder eine beliebige Kombination davon, jeweils substituiert mit mindestens einem Deuterium, eine Cyanogruppe, eine $C_1$-$C_{20}$-Alkylgruppe, eine $C_1$-$C_{20}$-Alkoxygruppe, eine Phenylgruppe, eine Biphenylgruppe, eine Terphenylgruppe, eine Naphthylgruppe oder eine beliebige Kombination davon.

**6.** Heterocyclische Verbindung nach einem der Ansprüche 1-5, wobei die heterocyclische Verbindung, die dargestellt ist durch Formel 1-1 bis 1-6, eine Verbindung ist, die dargestellt ist durch einen der Formeln 10-1 bis 10-6:

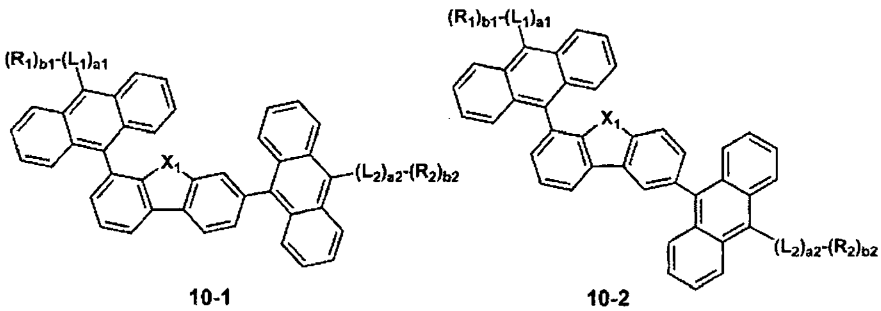

10-1 10-2

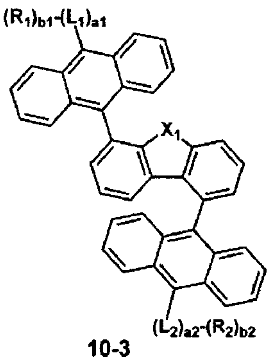

10-3

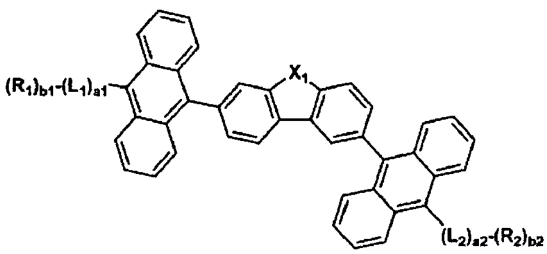

10-4

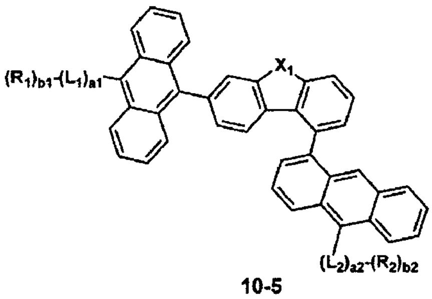

10-5

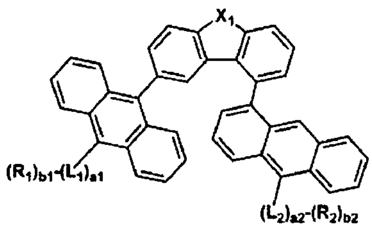

10-6

wobei, in Formel 10-1 bis 10-6,
$X_1$, $L_1$, $L_2$, a1, a2, $R_1$, $R_2$, b1 und b2 dieselben wie im Zusammenhang mit Anspruch 1 beschrieben sind.

**7.** Heterocyclische Verbindung nach einem der Ansprüche 1 bis 6, wobei die heterocyclische Verbindung, die dargestellt ist durch eine der Formeln 1-1 bis 1-6, eine der folgenden Verbindungen ist:

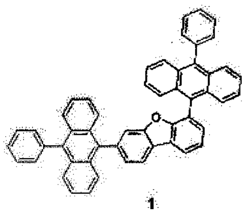
1
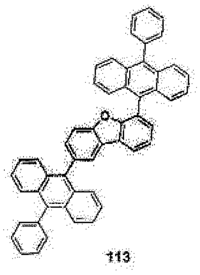
113
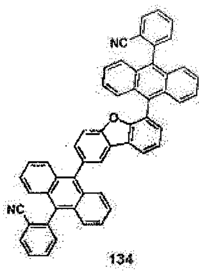
134

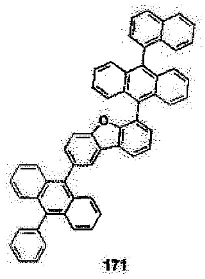
171
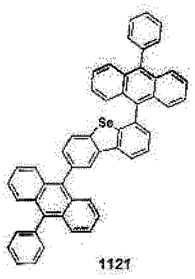
1121
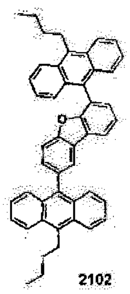
2102
.

**8.** Organische lichtemittierende Vorrichtung, umfassend:

eine erste Elektrode;
eine zweite Elektrode; und
eine organische Schicht, die zwischen der ersten Elektrode und der zweiten Elektrode befindlich ist und eine Emissionsschicht umfasst,
wobei die organische Schicht mindestens eine der heterocyclischen Verbindungen umfasst, die dargestellt sind durch eine der Formeln 1-1 bis 1-6 nach einem der Ansprüche 1-7.

**9.** Organische lichtemittierende Vorrichtung nach Anspruch 8, wobei

die erste Elektrode eine Anode ist und die zweite Elektrode eine Kathode ist,
die organische Schicht eine Lochtransportregion zwischen der ersten Elektrode und der Emissionsschicht und eine Elektronentransportregion zwischen der Emissionsschicht und der zweiten Elektrode umfasst,
wobei die Lochtransportregion mindestens eine von einer Locheinspritzschicht, einer Lochtransportschicht, einer Elektronenblockierschicht oder eine beliebige Kombination davon umfasst, und
die Elektronentransportregion mindestens eine von einer Lochblockierschicht, einer Elektronentransportschicht, einer Elektroneneinspritzschicht oder eine beliebige Kombination davon umfasst.

**10.** Organische lichtemittierende Vorrichtung nach Anspruch 8 oder 9, wobei die heterocyclische Verbindung, die dargestellt ist durch eine der Formeln 1-1 bis 1-6, in der Emissionsschicht beinhaltet ist;
vorzugsweise, wobei die Emissionsschicht Licht mit einer maximalen Emissionswellenlänge von 410 nm bis 490 nm emittiert.

**11.** Organische lichtemittierende Vorrichtung nach Anspruch 10, wobei die Emissionsschicht einen Wirt und ein Dotierungsmittel umfasst,

wobei der Wirt die heterocyclische Verbindung umfasst, die dargestellt ist durch eine der Formeln 1-1 bis 1-6, und eine Menge des Wirts größer als diejenige des Dotierungsmittels ist;
vorzugsweise, wobei das Dotierungsmittel ein fluoreszierendes Dotierungsmittel ist.

**12.** Organische lichtemittierende Vorrichtung nach einem der Ansprüche 9-11, wobei die heterocyclische Verbindung, die dargestellt ist durch eine der Formeln 1-1 bis 1-6, in der Lochtransportregion beinhaltet ist; und/oder
wobei die heterocyclische Verbindung, die dargestellt ist durch eine der Formeln 1-1 bis 1-6, in der Elektronentransportregion beinhaltet ist.

## Revendications

**1.** Composé hétérocyclique représenté par l'une des Formules 1-1 à 1-6 :

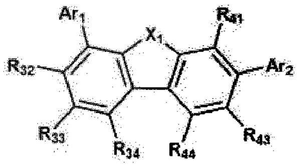
1-1

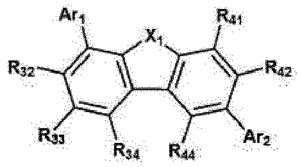
1-2

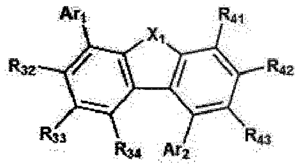
1-3

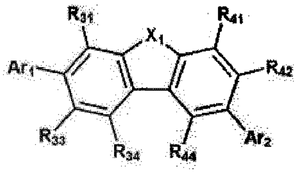
1-4

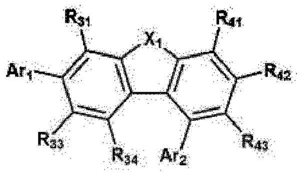
1-5

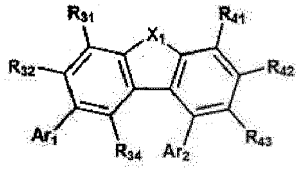
1-6

Formule 1A

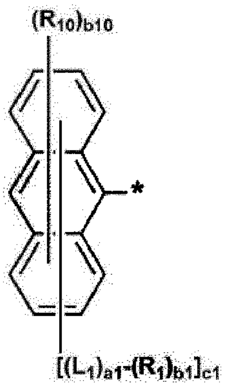

Formule 1B

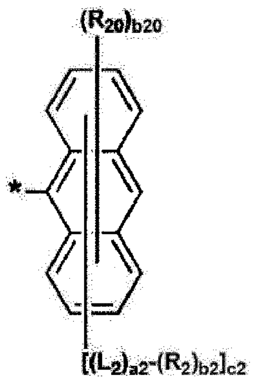

dans lequel, dans les Formules 1-1 à 1-6, 1A et 1B,

$X_1$ est O ou Se,
$Ar_1$ est un groupe représenté par la Formule 1A, et $Ar_2$ est un groupe représenté par la Formule 1B,
$L_1$ et $L_2$ sont chacun indépendamment un groupe carbocyclique en $C_5$-$C_{30}$ substitué ou non substitué, un groupe hétérocyclique en $C_1$-$C_{30}$ substitué ou non substitué, ou toute combinaison de ceux-ci,
a1 et a2 sont chacun indépendamment un nombre entier de 0 à 3,
dans lequel lorsque a1 est égal ou supérieur à 2, au moins deux de $L_1$(s) sont identiques ou différents les uns des autres, et lorsque a2 est égal ou supérieur à 2, au moins deux de $L_2$(s) sont identiques ou différents les uns des autres,
$R_1$, $R_2$, $R_{10}$, $R_{20}$, $R_{31}$ à $R_{34}$ et $R_{41}$ à $R_{44}$ sont chacun indépendamment hydrogène, deutérium, -F, -Cl, -Br, -I, $-SF_5$, un groupe hydroxyle, un groupe cyano, un groupe nitro, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$-$C_{60}$ substitué ou non substitué, un groupe alcényle en $C_2$-$C_{60}$ substitué ou non substitué, un groupe alcynyle en $C_2$-$C_{60}$ substitué ou non substitué, un groupe alcoxy en $C_1$-$C_{60}$ substitué ou non substitué, un groupe cycloalkyle en $C_3$-$C_{10}$ substitué ou non substitué, un groupe hétérocycloalkyle en $C_1$-$C_{10}$ substitué ou non substitué, un groupe cycloalcényle en $C_3$-$C_{10}$ substitué ou non substitué, un groupe hétérocycloalcényle en $C_2$-$C_{10}$ substitué ou non substitué, un groupe aryle en $C_6$-$C_{60}$ substitué ou non substitué, un groupe aryloxy en $C_6$-$C_{60}$ substitué ou non substitué, un groupe arylthio en $C_6$-$C_{60}$ substitué ou non substitué, un groupe hétéroaryle en $C_1$-$C_{60}$ substitué ou non substitué, un groupe polycyclique condensé non aromatique monovalent substitué ou non substitué, un groupe hétéropolycyclique condensé non aromatique monovalent substitué ou non substitué, $-N(Q_1)(Q_2)$, $-Si(Q_3)(Q_4)(Q_5)$, $-B(Q_6)(Q_7)$, $-P(=O)(Q_8)(Q_9)$, ou toute combinaison de ceux-ci,

b1 et b2 sont chacun indépendamment un nombre entier de 1 à 5,
dans lequel lorsque b1 est égal ou supérieur à 2, au moins deux de $R_1$(s) sont identiques ou différents les uns des autres, et lorsque b2 est égal ou supérieur à 2, au moins deux de $R_2$ sont identiques ou différents les uns des autres,
b10 et b20 sont chacun indépendamment un nombre entier de 1 à 8,
dans lequel, lorsque b10 est égal ou supérieur à 2, au moins deux de $R_{10}$(s) sont identiques ou différents les uns des autres, et lorsque b20 est égal ou supérieur à 2, au moins deux de $R_{20}$(s) sont identiques ou différents les uns des autres,
c1 et c2 sont chacun indépendamment un nombre entier de 1 à 8,
dans lequel, lorsque c1 est égal ou supérieur à 2, au moins deux des groupes $-(L_1)_{a1}-(R_1)_{b1}$ sont identiques ou différents les uns des autres, et lorsque c2 est égal ou supérieur à 2, au moins deux des groupes $-(L_2)_{a2}-(R_2)_{b2}$ sont identiques ou différents les uns des autres,
la somme de b10 et c1 vaut 9 et la somme de b20 et c2 vaut 9, et
au moins un substituant du groupe carbocyclique en $C_5$-$C_{30}$ substitué, du groupe hétérocyclique en $C_1$-$C_{30}$ substitué, du groupe alkyle en $C_1$-$C_{60}$ substitué, du groupe alcényle en $C_2$-$C_{60}$ substitué, du groupe alcynyle en $C_2$-$C_{60}$ substitué, du groupe alcoxy en $C_1$-$C_{60}$ substitué, du groupe cycloalkyle en $C_3$-$C_{10}$ substitué, du groupe hétérocycloalkyle en $C_1$-$C_{10}$ substitué, du groupe cycloalcényle en $C_3$-$C_{10}$ substitué, du groupe hétérocycloalcényle en $C_2$-$C_{10}$ substitué, du groupe aryle en $C_6$-$C_{60}$ substitué, du groupe aryloxy en $C_6$-$C_{60}$ substitué, du groupe arylthio en $C_6$-$C_{60}$ substitué, du groupe hétéroaryle en $C_1$-$C_{60}$ substitué, du groupe polycyclique condensé non aromatique monovalent substitué et du groupe hétéropolycyclique condensé non aromatique monovalent substitué est :

deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{60}$, un groupe alcényle en $C_2$-$C_{60}$, un groupe alcynyle en $C_2$-$C_{60}$, un groupe alcoxy en $C_1$-$C_{60}$, ou toute combinaison de ceux-ci ;
un groupe alkyle en $C_1$-$C_{60}$, un groupe alcényle en $C_2$-$C_{60}$, un groupe alcynyle en $C_2$-$C_{60}$, un groupe alcoxy en $C_1$-$C_{60}$, ou toute combinaison de ceux-ci, chacun étant substitué par au moins un deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe cycloalkyle en $C_3$-$C_{10}$, un groupe hétérocycloalkyle en $C_1$-$C_{10}$, un groupe cycloalcényle en $C_3$-$C_{10}$, un groupe hétérocycloalcényle en $C_2$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, un groupe hétéropolycyclique condensé non aromatique monovalent, $-Si(Q_{11})(Q_{12})(Q_{13})$, $-N(Q_{14})(Q_{15})$, $-B(Q_{16})(Q_{17})$, ou toute combinaison de ceux-ci ;
un groupe cycloalkyle en $C_3$-$C_{10}$, un groupe hétérocycloalkyle en $C_1$-$C_{10}$, un groupe cycloalcényle en $C_3$-$C_{10}$, un groupe hétérocycloalcényle en $C_2$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, un groupe hétéropolycyclique condensé non aromatique monovalent, ou toute combinaison de ceux-ci ;
un groupe cycloalkyle en $C_3$-$C_{10}$, un groupe hétérocycloalkyle en $C_{1-10}$, un groupe cycloalcényle en $C_3$-$C_{10}$, un groupe hétérocycloalcényle en $C_2$-$C_{10}$, un groupe aryle en $C_6$-$C_{60}$, un groupe aryloxy en $C_{6-}C_{60}$, un groupe arylthio en $C_{6-}C_{60}$, un groupe hétéroaryle en $C_1$-$C_{60}$, un groupe polycyclique condensé non aromatique monovalent, un groupe hétéropolycyclique condensé non aromatique monovalent, ou toute combinaison de ceux-ci, chacun étant substitué par au moins un deutérium, -F, -Cl, -Br, -I, $-CD_3$, $-CD_2H$, $-CDH_2$, $-CF_3$, $-CF_2H$, $-CFH_2$, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{60}$, un groupe alcényle en $C_2$-$C_{60}$, un groupe alcynyle en $C_2$-$C_{60}$, un groupe alcoxy en $C_1$-$C_{60}$, un groupe cycloalkyle en $C_3$-$C_{10}$, un groupe hétérocycloalkyle en $C_{1-}C_{10}$, un groupe cycloalcényle en $C_3$-$C_{10}$, un groupe hétérocycloalcényle en $C_2$-$C_{10}$, un groupe aryle en $C_{6-}C_{60}$, un groupe aryloxy en $C_{6-}C_{60}$, un groupe arylthio en $C_{6-}C_{60}$, un groupe hétéroaryle en $C_1$-$C_{60}$, un groupe polycyclique condensé non aromatique monovalent, un groupe hétéropolycyclique condensé non aromatique monovalent, $-Si(Q_{21})(Q_{22})(Q_{23})$, $-N(Q_{24})(Q_{25})$, $-B(Q_{26})(Q_{27})$, ou toute combinaison de ceux-ci ; ou
$-Si(Q_{31})(Q_{32})(Q_{33})$, $-N(Q_{34})(Q_{35})$, $-B(Q_{36})(Q_{37})$, ou toute combinaison de ceux-ci,
dans lequel $Q_1$ à $Q_7$, $Q_{11}$ à $Q_{17}$, $Q_{21}$ à $Q_{27}$ et $Q_{31}$ à $Q_{37}$ sont chacun indépendamment hydrogène, deutérium,

-F, -Cl, -Br, -I, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{60}$ substitué ou non substitué, un groupe alcényle en $C_2$-$C_{60}$ substitué ou non substitué, un groupe alcynyle en $C_2$-$C_{60}$ substitué ou non substitué, un groupe alcoxy en $C_1$-$C_{60}$ substitué ou non substitué, un groupe cycloalkyle en $C_3$-$C_{10}$ substitué ou non substitué, un groupe hétérocycloalkyle en $C_1$-$C_{10}$ substitué ou non substitué, un groupe cycloalcényle en $C_3$-$C_{10}$ substitué ou non substitué, un groupe hétérocycloalcényle en $C_2$-$C_{10}$ substitué ou non substitué, un groupe aryle en $C_6$-$C_{60}$ substitué ou non substitué, un groupe aryloxy en $C_6$-$C_{60}$ substitué ou non substitué, un groupe arylthio en $C_6$-$C_{60}$ substitué ou non substitué, un groupe hétéroaryle en $C_1$-$C_{60}$ substitué ou non substitué, un groupe polycyclique condensé non aromatique monovalent substitué ou non substitué, un groupe hétéropolycyclique condensé non aromatique monovalent substitué ou non substitué, ou toute combinaison de ceux-ci.

**2.** Composé hétérocyclique de la revendication 1, dans lequel $Ar_1$ est représenté par l'une des Formules 1A-1 à 1A-5 :

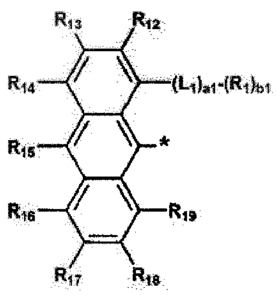
1A-1

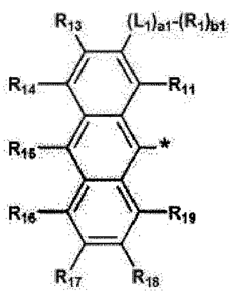
1A-2

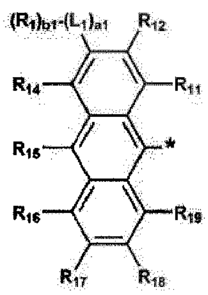
1A-3

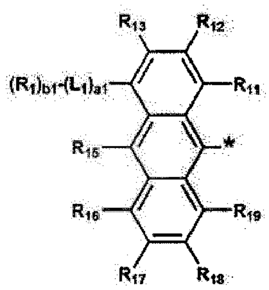
1A-4

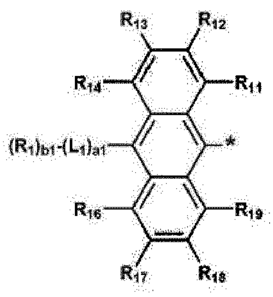
1A-5

dans lequel, dans les Formules 1A-1 à 1A-5,

$L_1$, al, $R_1$ et b1 sont les mêmes que décrits en relation avec la revendication 1,
$R_{11}$ à $R_{19}$ sont les mêmes que décrits en relation avec $R_{10}$ dans la revendication 1, et
* indique un site de liaison à un atome voisin ; et/ou
dans lequel $Ar_2$ est représenté par l'une des Formules 2A-1 à 2A-5 :

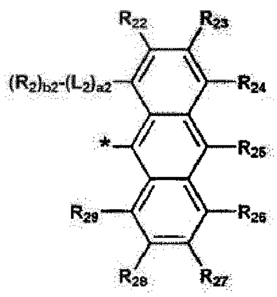
2A-1

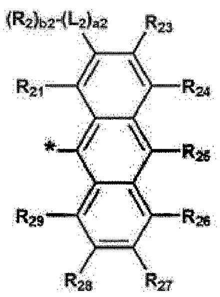
2A-2

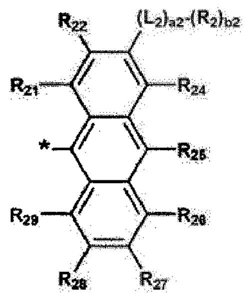
2A-3

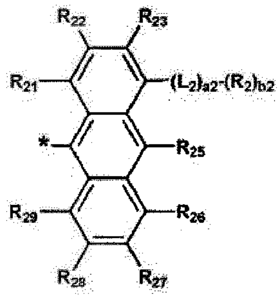

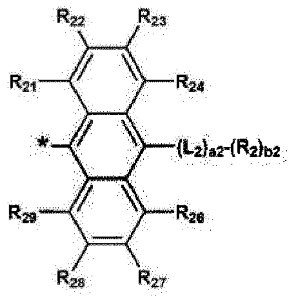

2A-4 2A-5

dans lequel, dans les formules 2A-1 à 2A-5,

$L_2$, a2, $R_2$ et b2 sont les mêmes que décrits en relation avec la revendication 1,
$R_{21}$ à $R_{29}$ sont les mêmes que décrits en relation avec $R_{20}$ dans la revendication 1, et
* indique un site de liaison à un atome voisin.

**3.** Composé hétérocyclique de l'une quelconque de la revendication 1 ou 2, dans lequel $L_1$ et $L_2$ sont chacun indépendamment :

un groupe phénylène, un groupe pentalénylène, un groupe indénylène, un groupe naphtylène, un groupe azulénylène, un groupe heptalénylène, un groupe acénaphtylène, un groupe fluorénylène, un groupe phénalénylène, un groupe phénanthrénylène, un groupe anthracénylène, un groupe fluoranthénylène, un groupe triphénylénylène groupe, un groupe pyrénylène, un groupe chrysénylénylène groupe, un groupe naphtacénylène groupe, un groupe picénylène, un groupe pérylénylène groupe, un groupe pentacénylène, ou toute combinaison de ceux-ci ; ou
un groupe phénylène, un groupe pentalénylène, un groupe indénylène, un groupe naphtylène, un groupe azulénylène, un groupe heptalénylène, un groupe acénaphtylène, un groupe fluorénylène, un groupe phénalénylène, un groupe phénanthrénylène, un groupe anthracénylène, un groupe fluoranthénylène, un groupe triphénylénylène, un groupe pyrénylène, un groupe chrysénylénylène, un groupe naphtacénylène, un groupe picénylène, un groupe pérylénylène, un groupe pentacénylène, ou toute combinaison de ceux-ci, chacun étant substitué par au moins un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en $C_1$-$C_{60}$, un groupe alcényle en $C_2$-$C_{60}$, un groupe alcynyle en $C_2$-$C_{60}$, un groupe alcoxy en $C_1$-$C_{60}$, un groupe cycloalkyle en $C_3$-$C_{10}$, un groupe cycloalcényle en $C_3$-$C_{10}$, un groupe hétérocycloalkyle en $C_1$-$C_{10}$, un groupe hétérocycloalcényle en $C_2$-$C_{10}$, un groupe aryle en $C_6$-$C_{60}$, un groupe aryloxy en $C_6$-$C_{60}$, un groupe arylthio en $C_6$-$C_{60}$, un groupe hétéroaryle en $C_1$-$C_{60}$, un groupe polycyclique condensé non aromatique monovalent, un groupe hétéropolycyclique condensé non aromatique monovalent, ou toute combinaison de ceux-ci ; et/ou
dans lequel $R_1$ et $R_2$ sont chacun indépendamment deutérium, -F, un groupe cyano, un groupe représenté par les Formules 9-1 à 9-19, un groupe représenté par les Formules 10-1 à 10-208, ou toute combinaison de ceux-ci :

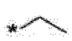
9-1
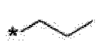
9-2
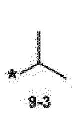
9-3
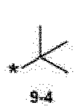
9-4
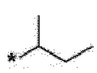
9-5
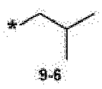
9-6
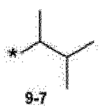
9-7

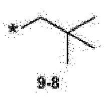
9-8
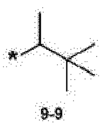
9-9
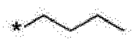
9-10
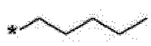
9-11
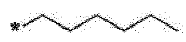
9-12

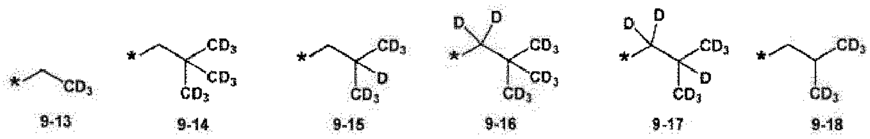

9-13 9-14 9-15 9-16 9-17 9-18

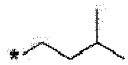

9-19

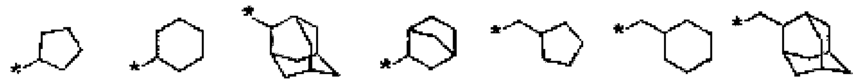

10-1 10-2 10-3 10-4 10-5 10-6 10-7

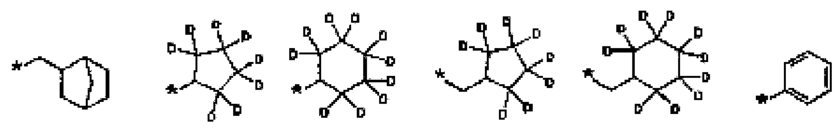

10-8 10-9 10-10 10-11 10-12 10-13

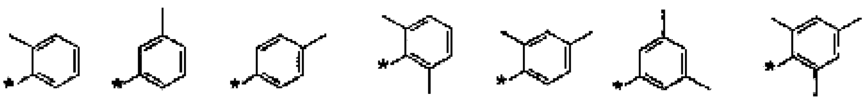

10-14 10-15 10-16 10-17 10-18 10-19 10-20

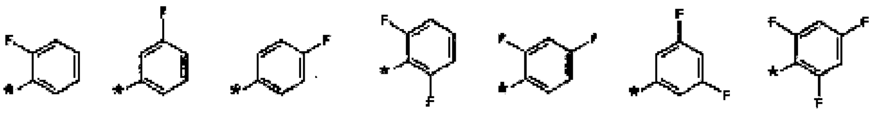

10-21 10-22 10-23 10-24 10-25 10-26 10-27

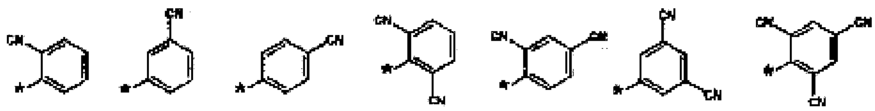

10-28 10-29 10-30 10-31 10-32 10-33 10-34

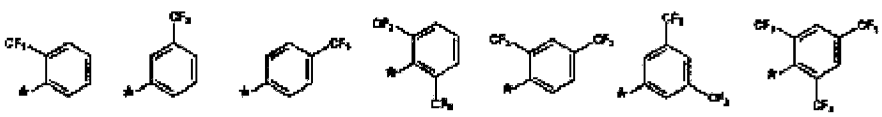

10-35 10-36 10-37 10-38 10-39 10-40 10-41

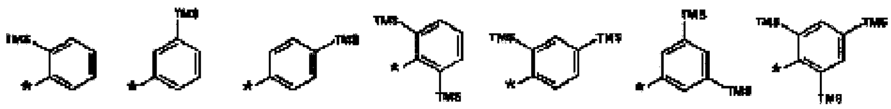

10-42 10-43 10-44 10-45 10-46 10-47 10-48

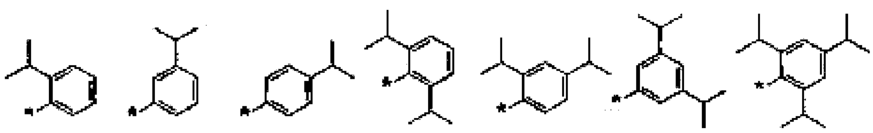

10-49 10-50 10-51 10-52 10-53 10-54 10-55

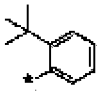
10-56
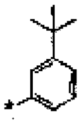
10-57
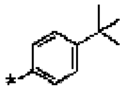
10-58
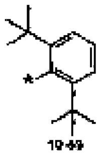
10-59
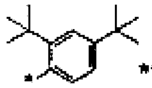
10-60
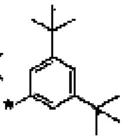
10-61
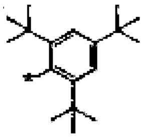
10-62

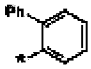
10-63
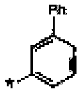
10-64
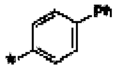
10-65
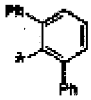
10-66
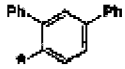
10-67
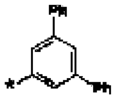
10-68
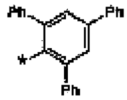
10-69

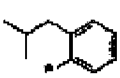
10-70
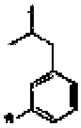
10-71
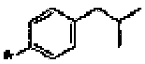
10-72
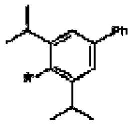
10-73
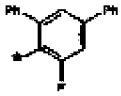
10-74
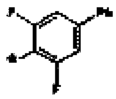
10-75

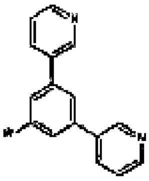
10-76
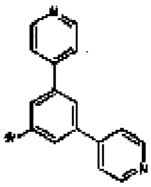
10-77
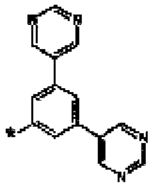
10-78
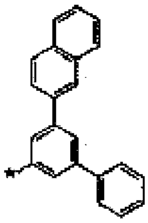
10-79
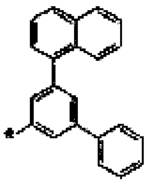
10-80

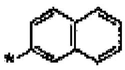
10-81
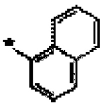
10-82
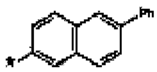
10-83
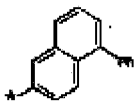
10-84
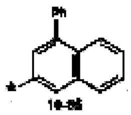
10-85

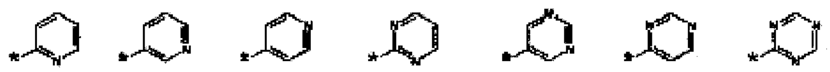
10-86 10-87 10-88 10-89 10-90 10-91 10-92

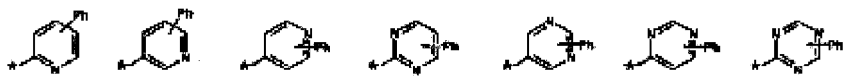
10-93 10-94 10-95 10-96 10-97 10-98 10-99

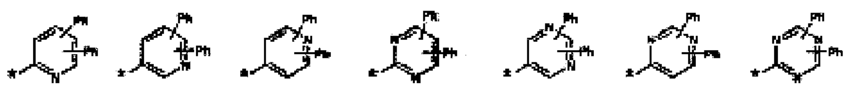
10-100 10-101 10-102 10-103 10-104 10-105 10-106

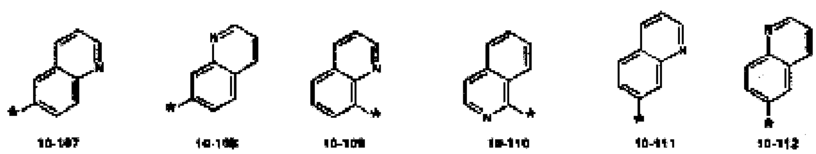
10-107 10-108 10-109 10-110 10-111 10-112

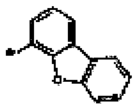
10-113
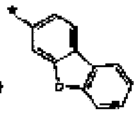
10-114
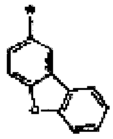
10-115
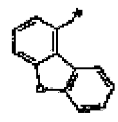
10-116
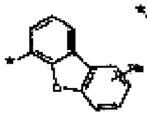
10-117
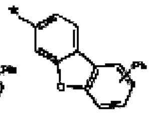
10-118

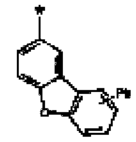
10-119
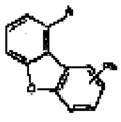
10-120
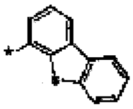
10-121
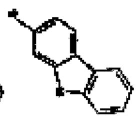
10-122
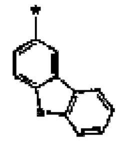
10-123
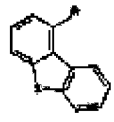
10-124

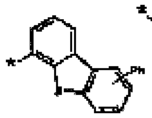
10-125
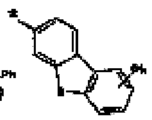
10-126
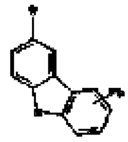
10-127
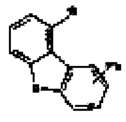
10-128
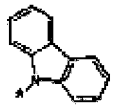
10-129
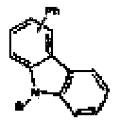
10-130

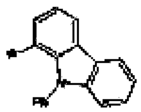
10-131
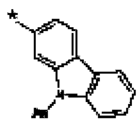
10-132
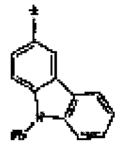
10-133
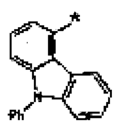
10-134
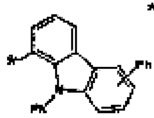
10-135
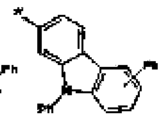
10-136

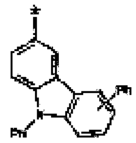
10-137
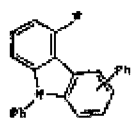
10-138
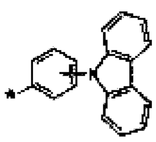
10-139
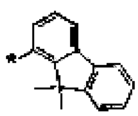
10-140
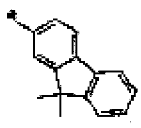
10-141
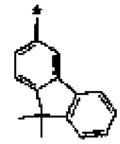
10-142

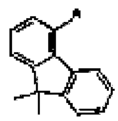
10-143
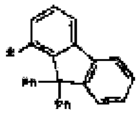
10-144
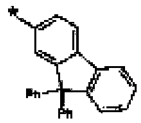
10-145
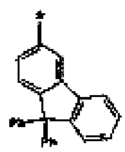
10-146
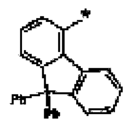
10-147

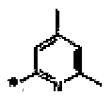
10-148
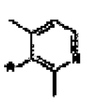
10-149
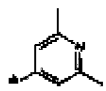
10-150
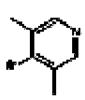
10-151
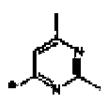
10-152
10-153
10-154

10-155
10-156
10-157
10-158
10-159
10-160
10-161

10-162
10-163
10-164
10-165
10-166
10-167
10-168

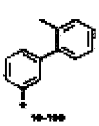
10-169
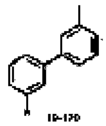
10-170
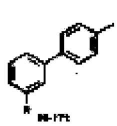
10-171
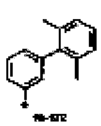
10-172
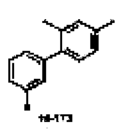
10-173
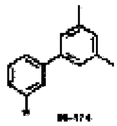
10-174
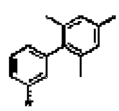
10-175

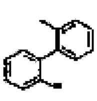
10-176
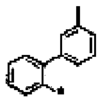
10-177
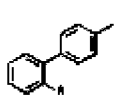
10-178
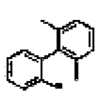
10-179
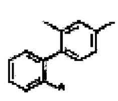
10-180
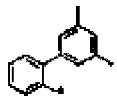
10-181
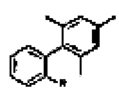
10-182

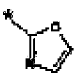
10-183
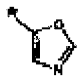
10-184
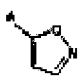
10-185
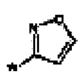
10-186
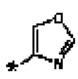
10-187
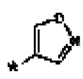
10-188
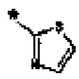
10-189
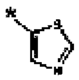
10-190
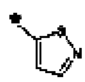
10-191

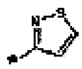
10-192
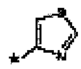
10-193
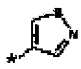
10-194

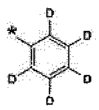
10-195
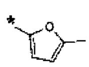
10-196
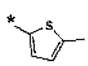
10-197
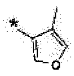
10-198
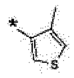
10-199
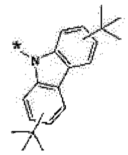
10-200
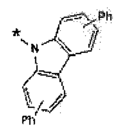
10-201
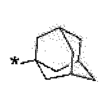
10-202

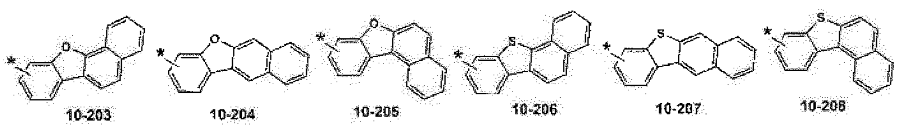
10-203 10-204 10-205 10-206 10-207 10-208

dans lequel, dans les Formules 9-1 à 9-19 et 10-1 à 10-208, * indique un site de liaison à un atome voisin, Ph est un groupe phényle et TMS est un groupe triméthylsilyle.

**4.** Composé hétérocyclique de l'une quelconque des revendications 1 à 3, dans lequel $R_{10}$, $R_{20}$, $R_{31}$ à $R_{34}$ et $R_{41}$ à $R_{44}$ sont chacun indépendamment :

hydrogène, deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{20}$, un groupe alcoxy en $C_1$-$C_{20}$, ou toute combinaison de ceux-ci ;
un groupe alkyle en $C_1$-$C_{20}$, un groupe alcoxy en $C_1$-$C_{20}$, ou toute combinaison de ceux-ci, chacun étant substitué par au moins un deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe phényle, un groupe naphtyle, un groupe pyridinyle, un groupe pyrimidinyle, un groupe pyrazinyle, un groupe pyridazinyle, un groupe triazinyle, ou toute combinaison de ceux-ci ;
un groupe cyclopentyle, un groupe cyclohexyle, un groupe cyclopentényle, un groupe cyclohexényle, un groupe cycloheptényle, un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spiro-bifluorényle, un groupe phénalényle, un groupe phénanthrényle, un groupe anthracényle, un groupe fluoranthényle, un groupe triphénylényle, un groupe pyrényle, un groupe chrysényle, un groupe naphtacényle, un groupe picényle, un groupe pérylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe

phtalazinyle, un groupe naphtyridinyle, un groupe quinoxalinyle, un groupe quinazolinyle, un groupe cinnolinyle, un groupe phénanthridinyle, un groupe acridinyle, un groupe phénanthrolinyle, un groupe phénazinyle, un groupe benzoxazolyle, un groupe benzimidazolyle, un groupe furanyle, un groupe benzofuranyle, un groupe thiophényle, un groupe benzothiophényle, un groupe thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe imidazopyrimidinyle, un groupe imidazopyridinyle, ou toute combinaison de ceux-ci ; ou

un groupe cyclopentyle, un groupe cyclohexyle, un groupe cyclopentényle, un groupe cyclohexényle, un groupe cycloheptényle, un groupe phényle, un groupe pentalenyle, un groupe indényle, un groupe naphthyle, un groupe azulényle, un groupe heptalényle, un groupe indacényle, un groupe acénaphtyle, un groupe fluorényle, un groupe spiro-bifluorényle, un groupe phénalényle, un groupe phénanthrényle, un groupe anthracényle, un groupe fluoranthényle, un groupe triphénylényle, un groupe pyrényle, un groupe chrysényle, un groupe naphthacényle, un groupe picényle, un groupe pérylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phthalazinyle, un groupe naphthyridinyle, un groupe quinoxalinyle, un groupe quinazolinyle, un groupe cinnolinyle, un groupe phénanthridinyle, un groupe acridinyle, un groupe phénanthrolinyle, un groupe phénazinyle, un groupe benzoxazolyle, un groupe benzimidazolyle, un groupe furanyle, un groupe benzofuranyle, un groupe thiophényle, un groupe benzothiophényle, un groupe thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe imidazopyrimidinyle, un groupe imidazopyridinyle, ou toute combinaison de ceux-ci, chacun étant substitué par au moins un deutérium, -F, -Cl, -Br, -I, un groupe hydroxyle, un groupe cyano, un groupe nitro, un groupe amino, un groupe amidino, un groupe hydrazine, un groupe hydrazone, un acide carboxylique ou un sel de celui-ci, un acide sulfonique ou un sel de celui-ci, un acide phosphorique ou un sel de celui-ci, un groupe alkyle en $C_1$-$C_{20}$, un groupe alcényle en $C_2$-$C_{20}$, un groupe alcynyle en $C_2$-$C_{20}$, un groupe alcoxy en $C_1$-$C_{20}$, un groupe phényle, un groupe naphthyle, un groupe anthracényle, un groupe pyrényle, un groupe phénanthrényle, un groupe fluorényle, un groupe pyridinyle, un groupe pyrimidinyle, un groupe pyrazinyle, un groupe pyridazinyle, un groupe triazinyle, un groupe quinolinyle, un groupe isoquinolinyle, un groupe phthalazinyle, un groupe quinoxalinyle, un groupe cinnolinyle, un groupe quinazolinyle, ou toute combinaison de ceux-ci.

**5.** Composé hétérocyclique de l'une quelconque des revendications 1 à 4, dans lequel $R_{10}$, $R_{20}$, $R_{31}$ à $R_{34}$ et $R_{41}$ à $R_{44}$ sont chacun indépendamment :

hydrogène, deutérium, un groupe cyano, un groupe alkyle en $C_1$-$C_{20}$, un groupe alcoxy en $C_1$-$C_{20}$, ou toute combinaison de ceux-ci ;

un groupe alkyle en $C_1$-$C_{20}$, un groupe alcoxy en $C_1$-$C_{20}$, ou toute combinaison de ceux-ci, chacun étant substitué par au moins un deutérium, un groupe cyano, un groupe phényle, un groupe biphényle, un groupe terphényle, un groupe naphtyle, ou toute combinaison de ceux-ci ;

un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle, un groupe dibenzothiophényle, ou toute combinaison de ceux-ci ; ou

un groupe cyclopentyle, un groupe cyclohexyle, un groupe cyclopentényle, un groupe cyclohexényle, un groupe cycloheptényle, un groupe phényle, un groupe biphényle, un groupe terphényle, un groupe naphtyle, un groupe fluorényle, un groupe carbazolyle, un groupe dibenzofuranyle, un groupe dibenzothiophényle, ou toute combinaison de ceux-ci, chacun étant substitué par au moins un deutérium, un groupe cyano, un groupe alkyle en $C_1$-$C_{20}$, un groupe alcoxy en $C_1$-$C_{20}$, un groupe phényle, un groupe biphényle, un groupe terphényle, un groupe naphtyle, ou toute combinaison de ceux-ci.

**6.** Composé hétérocyclique de l'une quelconque des revendications 1 à 5, ledit composé hétérocyclique représenté par les Formules 1-1 à 1-6 étant un composé représenté par l'une des Formules 10-1 à 10-6 :

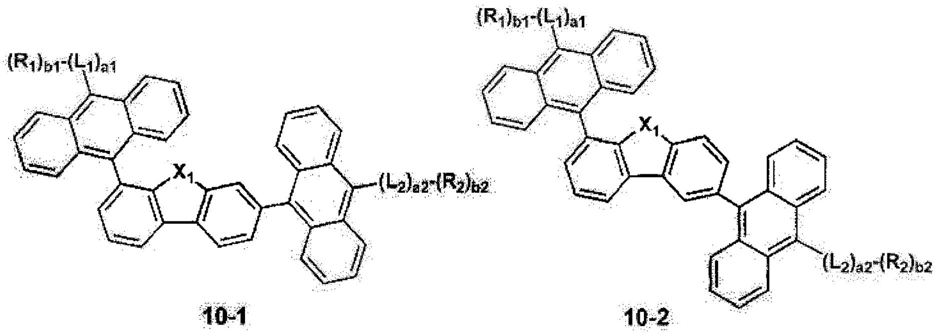

10-1 10-2

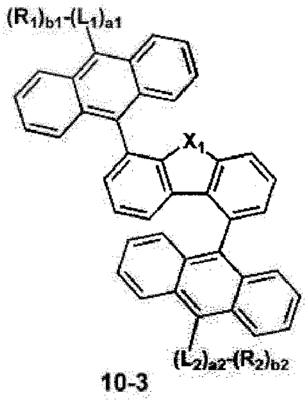

10-3

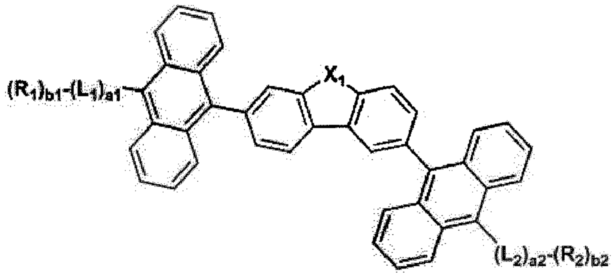

10-4

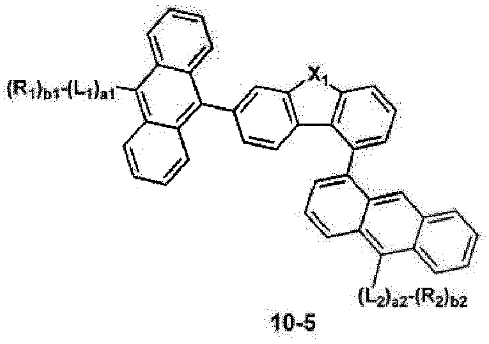

10-5

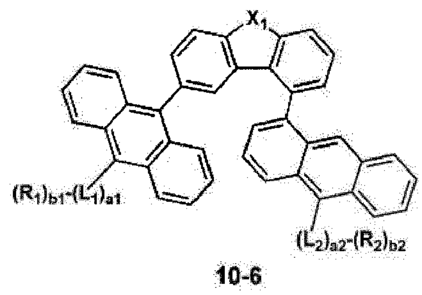

10-6

dans lequel, dans les Formules 10-1 à 10-6,
$X_1$, $L_1$, $L_2$, al, a2, $R_1$, $R_2$, bl et b2 sont les mêmes que décrits en relation avec la revendication 1.

**7.** Composé hétérocyclique de l'une quelconque des revendications 1 à 6, ledit composé hétérocyclique représenté par l'une des Formules 1-1 à 1-6 étant l'un des composés suivants :

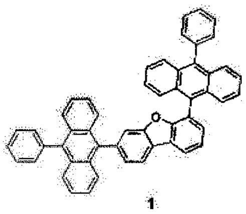

1

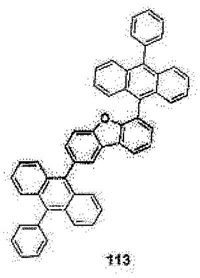

113

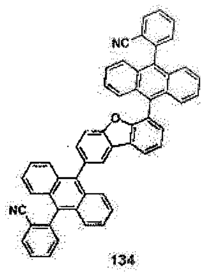

134

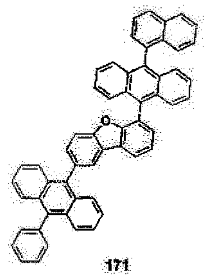

171

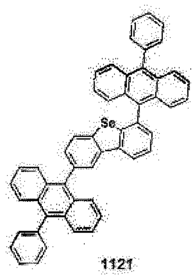

1121

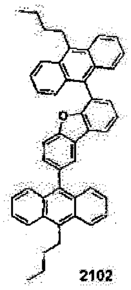

2102 .

**8.** Dispositif électroluminescent organique comprenant :

une première électrode ;
une seconde électrode ; et
une couche organique située entre la première électrode et la seconde électrode et comprenant une couche d'émission,
ladite couche organique comprenant au moins l'un des composés hétérocycliques représentés par l'une des Formules 1-1 à 1-6 de l'une quelconque des revendications 1 à 7.

**9.** Dispositif électroluminescent organique de la revendication 8, dans lequel

la première électrode est une anode et la seconde électrode est une cathode,
la couche organique comprend une région de transport de trous disposée entre la première électrode et la couche d'émission et une région de transport d'électrons disposée entre la couche d'émission et la seconde électrode,
ladite région de transport de trous comprenant au moins une couche d'injection de trous, une couche de transport de trous, une couche de blocage d'électrons, ou toute combinaison de celles-ci, et
ladite région de transport d'électrons comprenant au moins une couche de blocage de trous, une couche de transport d'électrons, une couche d'injection d'électrons ou toute combinaison de celles-ci.

**10.** Dispositif électroluminescent organique de la revendication 8 ou 9, dans lequel le composé hétérocyclique représenté par l'une des Formules 1-1 à 1-6 est compris dans la couche d'émission ;
de préférence ladite couche d'émission émettant une lumière possédant une longueur d'onde d'émission maximale de 410 nm à 490 nm.

**11.** Dispositif électroluminescent organique de la revendication 10, dans lequel la couche d'émission comprend un hôte et un dopant,

ledit hôte comprenant le composé hétérocyclique représenté par l'une des Formules 1-1 à 1-6, et une quantité de l'hôte étant supérieure à celle du dopant ;
de préférence ledit dopant étant un dopant fluorescent.

**12.** Dispositif électroluminescent organique de l'une quelconque des revendications 9 à 11, dans lequel le composé hétérocyclique représenté par l'une des Formules 1-1 à 1-6 est compris dans la région de transport de trous ; et/ou ledit composé hétérocyclique représenté par l'une des Formules 1-1 à 1-6 étant compris dans la région de transport d'électrons.

FIGURE

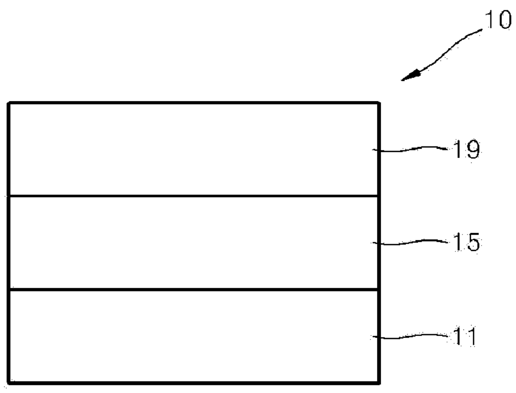

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 109867646
- KR 20180131963
- KR 20170096860


### Non-patent literature cited in the description


- **Y.S. KIM et al.** *Blue fluorescent materials based on bis(10-phenylanthracen-9-yl) derivatives containing heterocyclic moiety*